# EUROPEAN PATENT APPLICATION

(11) **EP 1 533 370 A1**
(43) Date of publication of application: **25.05.2005**
(21) Application number: 03078772.5
(22) Date of filing: 01.12.2003
(51) Int. Cl.: C12N 7/00, C12N 7/02, C12N 5/10, C12N 15/63, C07K 14/165, C07K 16/10, A61K 39/215, G01N 33/569

(54) **Novel atypical pneumonia-causing virus**

(30) Priority: 18.11.2003 EP 03078613
(71) Applicant: ViroNovative B.V., 3062 PA Rotterdam (NL)
(72) Inventor: de Jong, Jan Cornelis, 2806 EH Gouda (NL); Bestebroer, Theodorus Marinus, 2924 VE Krimpen aan den Ijssel (NL); Simon, James Henry Matthew, 1016 RK Amsterdam (NL); Fouchier, Ronaldus Adrianus Maria, 3021 AR Rotterdam (NL); Osterhaus, Albertus Dominicus Marcellinus Erasmus, 2000 Antwerpen-Zuid (BE)
(74) Representative: Prins, Adrianus Willem, Mr. Ir.

(57) **Abstract**

The invention relates to the field of virology. The invention provides a new isolated essentially mammalian positive-sense single stranded RNA virus (EMCR-CoV) within the group of coronaviuses and components thereof.

SEQUENCE LISTING

<110> ViroNovative B.V.

<120> Novel atypical pneumonia-causing virus

<130> P67316EP00

<140> EP 03078772.5
<141> 2003-12-01

<160> 9

<170> PatentIn version 3.1

<210> 1
<211> 27532
<212> DNA
<213> EMCR Coronavirus

<220>
<221> 5'UTR
<222> (1)..(264)
<223>

<220>
<221> CDS
<222> (265)..(12432)
<223> Product = Replicase 1a

<220>
<221> misc_feature
<222> (14931)..(14931)
<223> "n" on pos. 14931 stands for "k"

<220>

<221>  CDS

<222>  (20435)..(24502)

<223>  Product = Spike protein


<220>

<221>  CDS

<222>  (25163)..(25396)

<223>  Product = E protein


<220>

<221>  CDS

<222>  (25405)..(26085)

<223>  Product = M protein


<220>

<221>  CDS

<222>  (26096)..(27229)

<223>  Product = N protein


<220>

<221>  3'UTR

<222>  (27230)..(27532)

<223>


<400>  1
   agatagagaa ttttcttatt tagactttgt gtctactcct ctcaactaaa cgaaattttt      60
   ctagtgctgt catttgttat ggcagtccta gtgtaattga aatttcgtca agtttgtaaa     120
   ctggttaggc aagtgttgta ttttctgtgt ttaagcactg gtggttctgt ccactagtgc     180
   acacattgat acttaagtgg tgttctgtca ctgcttattg tggaagcaac gttctgtcgt     240
   tgtggaaacc aataactgct aacc atg ttt tac aat caa gtg aca ctt gct        291
                              Met Phe Tyr Asn Gln Val Thr Leu Ala
                              1               5
   gtt gca agt gat tcg gaa att tca ggt ttt ggt ttt gcc att cct tct       339
   Val Ala Ser Asp Ser Glu Ile Ser Gly Phe Gly Phe Ala Ile Pro Ser
   10                  15                  20                  25
   gta gcc gtt cgc gct tat agc gaa gcc gct gca caa ggt ttt cag gca 387
   Val Ala Val Arg Ala Tyr Ser Glu Ala Ala Ala Gln Gly Phe Gln Ala
   30 35 40
   tgc cgc ttt gtt gct ttt ggc tta cag gat tgt gta acc ggt att aat 435
   Cys Arg Phe Val Ala Phe Gly Leu Gln Asp Cys Val Thr Gly Ile Asn
   45 50 55
   gat gac gat tat gtc att gca ttg act ggt act aat cag ctt tgt gcc 483
   Asp Asp Asp Tyr Val Ile Ala Leu Thr Gly Thr Asn Gln Leu Cys Ala
   60 65 70
   aaa att tta ctt ttt tct gat aga cct ctt aat ttg cga ggt tgg ctc 531
   Lys Ile Leu Leu Phe Ser Asp Arg Pro Leu Asn Leu Arg Gly Trp Leu
   75 80 85
   att ttt tct aac agc aat tat gtt ctt cag gac ttt gat gtt gtt ttt 579
   Ile Phe Ser Asn Ser Asn Tyr Val Leu Gln Asp Phe Asp Val Val Phe
   90 95 100 105
   ggc cat ggt gca gga agt gtg gtt ttt gtg gat aag tat atg tgt ggt 627
   Gly His Gly Ala Gly Ser Val Val Phe Val Asp Lys Tyr Met Cys Gly
   110 115 120
   ttt gat ggt aaa cct gtg tta cct aaa aac atg tgg gaa ttt aga gat 675
   Phe Asp Gly Lys Pro Val Leu Pro Lys Asn Met Trp Glu Phe Arg Asp
   125 130 135
   tac ttt aat gat aat act gat agt att gtt att ggt ggt gtc act tat 723
   Tyr Phe Asn Asp Asn Thr Asp Ser Ile Val Ile Gly Gly Val Thr Tyr
   140 145 150
   caa tta gca tgg gat gtt ata cgt aaa gac ctt tct tat gaa cag caa 771
   Gln Leu Ala Trp Asp Val Ile Arg Lys Asp Leu Ser Tyr Glu Gln Gln
   155 160 165
   aat gtt tta gct att gag agc att cat tat ctt ggc act aca ggt cat 819
   Asn Val Leu Ala Ile Glu Ser Ile His Tyr Leu Gly Thr Thr Gly His
   170 175 180 185
   act ttg aag tct ggt tgc aaa ctc att aat gcc aag ccg cct aaa tat 867
   Thr Leu Lys Ser Gly Cys Lys Leu Ile Asn Ala Lys Pro Pro Lys Tyr
   190 195 200
   tct tct aag gtt gtt ttg agt ggt gaa tgg aat gct gtg tat aag gcg 915
   Ser Ser Lys Val Val Leu Ser Gly Glu Trp Asn Ala Val Tyr Lys Ala
   205 210 215
   ttt ggt tca cca ttt att aca aat ggt ata tca ttg cta gat ata att 963
   Phe Gly Ser Pro Phe Ile Thr Asn Gly Ile Ser Leu Leu Asp Ile Ile
   220 225 230
   gtt aaa cca gtt ttc ttt aat gct ttt gtt aaa tgc aat tgt ggt tct 1011
   Val Lys Pro Val Phe Phe Asn Ala Phe Val Lys Cys Asn Cys Gly Ser
   235 240 245
   gag aat tgg agt gtt ggt gca tgg gat ggt tat cta tct tct tgt tgt 1059
   Glu Asn Trp Ser Val Gly Ala Trp Asp Gly Tyr Leu Ser Ser Cys Cys
   250 255 260 265
   ggc aca cct gct aag aaa ctt tgt gtt gtt cct ggt aat gtt gtt cct 1107
   Gly Thr Pro Ala Lys Lys Leu Cys Val Val Pro Gly Asn Val Val Pro
   270 275 280
   ggt gat gtg atc atc acc tca act gat gct ggt tgt ggt gtt aaa tac 1155
   Gly Asp Val Ile Ile Thr Ser Thr Asp Ala Gly Cys Gly Val Lys Tyr
   285 290 295
   tat gct ggc tta gtt gtt aaa cat att act aac att act ggt gtg tct 1203
   Tyr Ala Gly Leu Val Val Lys His Ile Thr Asn Ile Thr Gly Val Ser
   300 305 310
   tta tgg cgt gtt aca gct gtt cat tct gat gga atg ttt gtg gca aca 1251
   Leu Trp Arg Val Thr Ala Val His Ser Asp Gly Met Phe Val Ala Thr
   315 320 325
   tct tct tat gat gca ctt ttg cat aga aat tca tta gac cct ttt tgc 1299
   Ser Ser Tyr Asp Ala Leu Leu His Arg Asn Ser Leu Asp Pro Phe Cys
   330 335 340 345
   ttt gat gtt aac act tta ctt tct aat caa tta cgt cta gct ttt ctt 1347
   Phe Asp Val Asn Thr Leu Leu Ser Asn Gln Leu Arg Leu Ala Phe Leu
   350 355 360
   ggt gct tct gtt aca gaa gat gtt aaa ttt gct gct agc act ggt gtt 1395
   Gly Ala Ser Val Thr Glu Asp Val Lys Phe Ala Ala Ser Thr Gly Val
   365 370 375
   att gac att agt gct ggt atg ttt ggt ctt tac gat gac ata ttg aca 1443
   Ile Asp Ile Ser Ala Gly Met Phe Gly Leu Tyr Asp Asp Ile Leu Thr
   380 385 390
   aac aat aaa cct tgg ttt gta cgc aaa gct tct ggg ctt ttt gat gca 1491
   Asn Asn Lys Pro Trp Phe Val Arg Lys Ala Ser Gly Leu Phe Asp Ala
   395 400 405
   atc tgg gat gct ttt gtt gcc gct att aag ctt gtg cca act act act 1539
   Ile Trp Asp Ala Phe Val Ala Ala Ile Lys Leu Val Pro Thr Thr Thr
   410 415 420 425
   ggt ggt ttg gtt agg ttt gtt aag tct atc gct tca act gtt tta act 1587
   Gly Gly Leu Val Arg Phe Val Lys Ser Ile Ala Ser Thr Val Leu Thr
   430 435 440
   gtt tct aat ggt gtt att att atg tgt gca gat gtt cca gat gct ttt 1635
   Val Ser Asn Gly Val Ile Ile Met Cys Ala Asp Val Pro Asp Ala Phe
   445 450 455
   caa cca gtt tac cgc aca ttt aca caa gct att tgt gct gca ttt gat 1683
   Gln Pro Val Tyr Arg Thr Phe Thr Gln Ala Ile Cys Ala Ala Phe Asp
   460 465 470
   ttt tct tta gat gta ttt aaa att ggt gat gtt aaa ttt aaa cga ctt 1731
   Phe Ser Leu Asp Val Phe Lys Ile Gly Asp Val Lys Phe Lys Arg Leu
   475 480 485
   ggt gat tat gtt ctt act gaa aat gct ctt gtt cgt ttg act act gaa 1779
   Gly Asp Tyr Val Leu Thr Glu Asn Ala Leu Val Arg Leu Thr Thr Glu
   490 495 500 505
   gtt gtt cgt ggt gtt cgt gat gct cgc ata aag aaa gcc atg ttt act 1827
   Val Val Arg Gly Val Arg Asp Ala Arg Ile Lys Lys Ala Met Phe Thr
   510 515 520
   aaa gta gtt gta ggt cct aca act gaa gtt aag ttt tct gtt att gaa 1875
   Lys Val Val Val Gly Pro Thr Thr Glu Val Lys Phe Ser Val Ile Glu
   525 530 535
   ctt gcc act gtt aat ttg cgt ctt gtt gat tgt gca cct gta gtt tgc 1923
   Leu Ala Thr Val Asn Leu Arg Leu Val Asp Cys Ala Pro Val Val Cys
   540 545 550
   cct aaa ggt aaa att gtt gtt att gct gga caa gct ttt ttc tat agt 1971
   Pro Lys Gly Lys Ile Val Val Ile Ala Gly Gln Ala Phe Phe Tyr Ser
   555 560 565
   ggt ggt ttt tat cgt ttt atg gtt gat tct aca act gta tta aat gac 2019
   Gly Gly Phe Tyr Arg Phe Met Val Asp Ser Thr Thr Val Leu Asn Asp
   570 575 580 585
   cct gtt ttt act ggt gag tta ttt tat act att aag ttt agt ggt ttt 2067
   Pro Val Phe Thr Gly Glu Leu Phe Tyr Thr Ile Lys Phe Ser Gly Phe
   590 595 600
   aag ctt gat ggt ttt aac cat cag ttt gtt aat gct agt tct gct aca 2115
   Lys Leu Asp Gly Phe Asn His Gln Phe Val Asn Ala Ser Ser Ala Thr
   605 610 615
   gat gcc att att gct gtt gag ctg ttg tta tcg gat ttt aaa act gca 2163
   Asp Ala Ile Ile Ala Val Glu Leu Leu Leu Ser Asp Phe Lys Thr Ala
   620 625 630
   gtt ttt gtg tac aca tgt gtg gtt gat ggt tgt agt gtc att gtt aga 2211
   Val Phe Val Tyr Thr Cys Val Val Asp Gly Cys Ser Val Ile Val Arg
   635 640 645
   cgt gat gct aca ttc gcc aca cat gtg tgt ttt aag gac tgt tat agt 2259
   Arg Asp Ala Thr Phe Ala Thr His Val Cys Phe Lys Asp Cys Tyr Ser
   650 655 660 665
   att tgg gag caa ttc tgc att gat aat tgt ggt gag cca tgg ttt ttg 2307
   Ile Trp Glu Gln Phe Cys Ile Asp Asn Cys Gly Glu Pro Trp Phe Leu
   670 675 680
   act gat tat aat gct atc ttg cag agt aat aac cct caa tgt gct att 2355
   Thr Asp Tyr Asn Ala Ile Leu Gln Ser Asn Asn Pro Gln Cys Ala Ile
   685 690 695
   gtt caa gca tcg gag tct aaa gtt ttg ctt gag agg ttt tta cct aag 2403
   Val Gln Ala Ser Glu Ser Lys Val Leu Leu Glu Arg Phe Leu Pro Lys
   700 705 710
   tgt cct gaa ata ctg ttg agt att gat gat ggc cat tta tgg aat ctt 2451
   Cys Pro Glu Ile Leu Leu Ser Ile Asp Asp Gly His Leu Trp Asn Leu
   715 720 725
   ttt gtt gaa aag ttt aat ttt gtt aca gat tgg tta aaa act ctt aag 2499
   Phe Val Glu Lys Phe Asn Phe Val Thr Asp Trp Leu Lys Thr Leu Lys
   730 735 740 745
   ctt aca ctt act tct aat ggt ctt tta ggt aat tgt gcc aaa cgt ttt 2547
   Leu Thr Leu Thr Ser Asn Gly Leu Leu Gly Asn Cys Ala Lys Arg Phe
   750 755 760
   aga cgt gtt ttg gta aaa ttg ctt gat gtc tat aat ggt ttt ctt gaa 2595
   Arg Arg Val Leu Val Lys Leu Leu Asp Val Tyr Asn Gly Phe Leu Glu
   765 770 775
   act gtc tgt agt gtc gta cac act gct ggt gtt tgc att aaa tat tat 2643
   Thr Val Cys Ser Val Val His Thr Ala Gly Val Cys Ile Lys Tyr Tyr
   780 785 790
   gct gtt aat gtt cca tat gta gtt att agt ggt ttt gta agt cgt gta 2691
   Ala Val Asn Val Pro Tyr Val Val Ile Ser Gly Phe Val Ser Arg Val
   795 800 805
   att cgt aga gaa agg tgt gac gtg act ttt cct tgt gtt agt tgt gtc 2739
   Ile Arg Arg Glu Arg Cys Asp Val Thr Phe Pro Cys Val Ser Cys Val
   810 815 820 825
   act ttt ttc tat gaa ttt tta gac acg tgt ttt ggt gtt agt aaa cct 2787
   Thr Phe Phe Tyr Glu Phe Leu Asp Thr Cys Phe Gly Val Ser Lys Pro
   830 835 840
   aat gcc att gat gtt gaa cat tta gag ctt aaa gaa act gtt ttt gtt 2835
   Asn Ala Ile Asp Val Glu His Leu Glu Leu Lys Glu Thr Val Phe Val
   845 850 855
   gaa cct aag gat ggt ggt caa ttt ttt gtt tct gat gat tat ctt tgg 2883
   Glu Pro Lys Asp Gly Gly Gln Phe Phe Val Ser Asp Asp Tyr Leu Trp
   860 865 870
   tat gtt gta gat gac att tat tat cca gct tca tgt aat ggt gta ttg 2931
   Tyr Val Val Asp Asp Ile Tyr Tyr Pro Ala Ser Cys Asn Gly Val Leu
   875 880 885
   cca gtt gct ttt aca aaa ttg gca ggt ggt aaa ata tct ttt tct gat 2979
   Pro Val Ala Phe Thr Lys Leu Ala Gly Gly Lys Ile Ser Phe Ser Asp
   890 895 900 905
   gat gtt ata gtt cat gat gtt gaa cct acc cat aaa gtc aag ctc ata 3027
   Asp Val Ile Val His Asp Val Glu Pro Thr His Lys Val Lys Leu Ile
   910 915 920
   ttt gag ttt gaa gat gat gtt gtt acc agt ctt tgt aag aag agt ttt 3075
   Phe Glu Phe Glu Asp Asp Val Val Thr Ser Leu Cys Lys Lys Ser Phe
   925 930 935
   ggt aag tct att att tat aca ggt gat tgg gaa ggt tta cat gaa gtt 3123
   Gly Lys Ser Ile Ile Tyr Thr Gly Asp Trp Glu Gly Leu His Glu Val
   940 945 950
   ctt aca tct gca atg aat gtc att ggg caa cat att aag ttg cca caa 3171
   Leu Thr Ser Ala Met Asn Val Ile Gly Gln His Ile Lys Leu Pro Gln
   955 960 965
   ttt tat att tat gat gaa gag ggt ggt tat gat gtt tct aaa cca gtt 3219
   Phe Tyr Ile Tyr Asp Glu Glu Gly Gly Tyr Asp Val Ser Lys Pro Val
   970 975 980 985
   atg att tca caa tgg cct att agt gat gat agt gat ggt tgt gtt gtt 3267
   Met Ile Ser Gln Trp Pro Ile Ser Asp Asp Ser Asp Gly Cys Val Val
   990 995 1000
   gaa gcg agc act gat ttt cat caa tta gaa tct gtt aga gaa gag 3312
   Glu Ala Ser Thr Asp Phe His Gln Leu Glu Ser Val Arg Glu Glu
   1005 1010 1015
   gtt gat ata att gaa caa cct ttt ggg gaa gtt gaa cat gcg ctc 3357
   Val Asp Ile Ile Glu Gln Pro Phe Gly Glu Val Glu His Ala Leu
   1020 1025 1030
   tca att aga caa cct ttt tct ttt tct ttt aga gat gaa ttg ggt 3402
   Ser Ile Arg Gln Pro Phe Ser Phe Ser Phe Arg Asp Glu Leu Gly
   1035 1040 1045
   gtt cgt gtt tta gat caa tct gat aat aat tgt tgg att agt acc 3447
   Val Arg Val Leu Asp Gln Ser Asp Asn Asn Cys Trp Ile Ser Thr
   1050 1055 1060
   aca ctt ata cag ttg caa ctt aca aag ctt ttg gat gat tct att 3492
   Thr Leu Ile Gln Leu Gln Leu Thr Lys Leu Leu Asp Asp Ser Ile
   1065 1070 1075
   gag atg caa ttg ttt aaa gtt ggt aaa gtt gat tca att gtt caa 3537
   Glu Met Gln Leu Phe Lys Val Gly Lys Val Asp Ser Ile Val Gln
   1080 1085 1090
   aag tgt tat gag ttg tct cat tta att agt ggt tca ctt ggt gat 3582
   Lys Cys Tyr Glu Leu Ser His Leu Ile Ser Gly Ser Leu Gly Asp
   1095 1100 1105
   agt ggt aaa ctt ctt agt gaa ctt ctt aaa gat aaa tat aca tgt 3627
   Ser Gly Lys Leu Leu Ser Glu Leu Leu Lys Asp Lys Tyr Thr Cys
   1110 1115 1120
   tct ata act ttt gag atg tct tgt gat tgt ggt aaa aag ttt gat 3672
   Ser Ile Thr Phe Glu Met Ser Cys Asp Cys Gly Lys Lys Phe Asp
   1125 1130 1135
   gag caa gtt ggt tgt ttg ttt tgg att atg cct tac aca aaa ctt 3717
   Glu Gln Val Gly Cys Leu Phe Trp Ile Met Pro Tyr Thr Lys Leu
   1140 1145 1150
   ttt caa aaa ggt gag tgt tgt att tgt cat aaa atg cag act tat 3762
   Phe Gln Lys Gly Glu Cys Cys Ile Cys His Lys Met Gln Thr Tyr
   1155 1160 1165
   aag ctt gtt agt atg aaa ggt act ggt gtg ttt gta cag gat cca 3807
   Lys Leu Val Ser Met Lys Gly Thr Gly Val Phe Val Gln Asp Pro
   1170 1175 1180
   gca cct att gac att gat gct ttc cct gtt aga cct ata tgt tca 3852
   Ala Pro Ile Asp Ile Asp Ala Phe Pro Val Arg Pro Ile Cys Ser
   1185 1190 1195
   tct gta tat tta ggt gtt aag ggt tct ggt cat tat caa aca aat 3897
   Ser Val Tyr Leu Gly Val Lys Gly Ser Gly His Tyr Gln Thr Asn
   1200 1205 1210
   tta tac agt ttt gac aaa gct att gat ggt ttt ggt gtc ttt gac 3942
   Leu Tyr Ser Phe Asp Lys Ala Ile Asp Gly Phe Gly Val Phe Asp
   1215 1220 1225
   att aaa aat agt agt gtt aat act gtt tgt ttt gtt gat gtt gat 3987
   Ile Lys Asn Ser Ser Val Asn Thr Val Cys Phe Val Asp Val Asp
   1230 1235 1240
   ttt cat agt gta gaa ata gaa gct ggt gaa gtt aaa cct ttt gct 4032
   Phe His Ser Val Glu Ile Glu Ala Gly Glu Val Lys Pro Phe Ala
   1245 1250 1255
   gta tat aaa aat gtt aaa ttt tat tta ggt gat att tca cac ctt 4077
   Val Tyr Lys Asn Val Lys Phe Tyr Leu Gly Asp Ile Ser His Leu
   1260 1265 1270
   gta aac tgt gtt tct ttt gac ttt gtt gtc aat gct gct aat gaa 4122
   Val Asn Cys Val Ser Phe Asp Phe Val Val Asn Ala Ala Asn Glu
   1275 1280 1285
   aat ctc atg cat gga ggc ggt gtc gca cgt gct att gat att ttg 4167
   Asn Leu Met His Gly Gly Gly Val Ala Arg Ala Ile Asp Ile Leu
   1290 1295 1300
   act gaa ggt caa ctt cag tca tta tct aaa gat tac att agt agt 4212
   Thr Glu Gly Gln Leu Gln Ser Leu Ser Lys Asp Tyr Ile Ser Ser
   1305 1310 1315
   aat ggt cca ctt aag gtt gga gca ggt gtt atg ttg gag tgt gaa 4257
   Asn Gly Pro Leu Lys Val Gly Ala Gly Val Met Leu Glu Cys Glu
   1320 1325 1330
   aaa ttc aat gta ttt aat gtt gtt ggt ccg cga act ggt aaa cat 4302
   Lys Phe Asn Val Phe Asn Val Val Gly Pro Arg Thr Gly Lys His
   1335 1340 1345
   gag cat tca tta ctt gtt gaa gct tat aat tct att tta ttt gaa 4347
   Glu His Ser Leu Leu Val Glu Ala Tyr Asn Ser Ile Leu Phe Glu
   1350 1355 1360
   aat ggt att cca ctt atg cct ctt ctt agt tgt ggt att ttt ggt 4392
   Asn Gly Ile Pro Leu Met Pro Leu Leu Ser Cys Gly Ile Phe Gly
   1365 1370 1375
   gta agg att gaa aat tct ctt aaa gct ttg ttt agt tgt gac att 4437
   Val Arg Ile Glu Asn Ser Leu Lys Ala Leu Phe Ser Cys Asp Ile
   1380 1385 1390
   aat aaa cca ttg caa gtt ttt gtt tat tct tca aat gaa gaa caa 4482
   Asn Lys Pro Leu Gln Val Phe Val Tyr Ser Ser Asn Glu Glu Gln
   1395 1400 1405
   gct gtt ctt aag ttt tta gat ggt tta gat tta aca cca gtc att 4527
   Ala Val Leu Lys Phe Leu Asp Gly Leu Asp Leu Thr Pro Val Ile
   1410 1415 1420
   gac gat gtt gat gtt gtt aaa cct ttt aga gtt gaa ggt aat ttt 4572
   Asp Asp Val Asp Val Val Lys Pro Phe Arg Val Glu Gly Asn Phe
   1425 1430 1435
   tca ttc ttt gat tgt ggt gtc aat gcc ttg gat ggt gat att tac 4617
   Ser Phe Phe Asp Cys Gly Val Asn Ala Leu Asp Gly Asp Ile Tyr
   1440 1445 1450
   tta tta ttt act aac tct att tta atg ttg gat aaa caa gga caa 4662
   Leu Leu Phe Thr Asn Ser Ile Leu Met Leu Asp Lys Gln Gly Gln
   1455 1460 1465
   tta ttg gac aca aaa ctt aat ggt att ttg caa cag gca gtt ctt 4707
   Leu Leu Asp Thr Lys Leu Asn Gly Ile Leu Gln Gln Ala Val Leu
   1470 1475 1480
   gat tat ctt gct aca gtt aaa act gta cca gct ggt aat ttg gtt 4752
   Asp Tyr Leu Ala Thr Val Lys Thr Val Pro Ala Gly Asn Leu Val
   1485 1490 1495
   aaa ctt gtt gtt gag agt tgt acc att tat atg tgt gtt gta cca 4797
   Lys Leu Val Val Glu Ser Cys Thr Ile Tyr Met Cys Val Val Pro
   1500 1505 1510
   tcg ata aat gat ctt tct ttt gat aaa aat ctt ggt cgt tgt gtg 4842
   Ser Ile Asn Asp Leu Ser Phe Asp Lys Asn Leu Gly Arg Cys Val
   1515 1520 1525
   cgt aaa ctt aat aga ttg aaa act tgt gtt att gcc aat gtt cct 4887
   Arg Lys Leu Asn Arg Leu Lys Thr Cys Val Ile Ala Asn Val Pro
   1530 1535 1540
   gct att gat gtt ttg aaa aag ctt ctt tca agt ttg act tta act 4932
   Ala Ile Asp Val Leu Lys Lys Leu Leu Ser Ser Leu Thr Leu Thr
   1545 1550 1555
   gtt aaa ttt gtt gta gag agt aat gtt atg gat gtt aac gac tgt 4977
   Val Lys Phe Val Val Glu Ser Asn Val Met Asp Val Asn Asp Cys
   1560 1565 1570
   ttt aag aat gat aat gta gtt ttg aaa att act gaa gat ggt att 5022
   Phe Lys Asn Asp Asn Val Val Leu Lys Ile Thr Glu Asp Gly Ile
   1575 1580 1585
   aat gtt aaa gat gtt gtt gtt gag tct tct aag tca ctt ggt aaa 5067
   Asn Val Lys Asp Val Val Val Glu Ser Ser Lys Ser Leu Gly Lys
   1590 1595 1600
   caa ttg ggt gtt gtg agt gat ggt gtt gac tct ttt gaa ggt gtt 5112
   Gln Leu Gly Val Val Ser Asp Gly Val Asp Ser Phe Glu Gly Val
   1605 1610 1615
   tta cct att aat act gat act gtc tta tct gta gct cca gaa gtt 5157
   Leu Pro Ile Asn Thr Asp Thr Val Leu Ser Val Ala Pro Glu Val
   1620 1625 1630
   gac tgg gtt gct ttt tac ggt ttt gaa aag gca gca ctt ttt gct 5202
   Asp Trp Val Ala Phe Tyr Gly Phe Glu Lys Ala Ala Leu Phe Ala
   1635 1640 1645
   tct ttg gat gta aag cca tat ggt tac cct aat gat ttt gtt ggt 5247
   Ser Leu Asp Val Lys Pro Tyr Gly Tyr Pro Asn Asp Phe Val Gly
   1650 1655 1660
   ggt ttt aga gtt ctt ggg acc acc gac aat aat tgt tgg gtt aat 5292
   Gly Phe Arg Val Leu Gly Thr Thr Asp Asn Asn Cys Trp Val Asn
   1665 1670 1675
   gca act tgt ata att tta cag tat ctt aag cct act ttt aaa tct 5337
   Ala Thr Cys Ile Ile Leu Gln Tyr Leu Lys Pro Thr Phe Lys Ser
   1680 1685 1690
   aag ggt tta aat gtt ctt tgg aac aaa ttt gtt aca ggt gat gtt 5382
   Lys Gly Leu Asn Val Leu Trp Asn Lys Phe Val Thr Gly Asp Val
   1695 1700 1705
   gga cct ttt gtt agt ttt att tat ttt ata act atg tct tca aag 5427
   Gly Pro Phe Val Ser Phe Ile Tyr Phe Ile Thr Met Ser Ser Lys
   1710 1715 1720
   ggt caa aag ggt gat gct gaa gag gca tta tct aaa ttg tca gag 5472
   Gly Gln Lys Gly Asp Ala Glu Glu Ala Leu Ser Lys Leu Ser Glu
   1725 1730 1735
   tat ttg att agt gat tct att gtt act ctt gaa caa tat tca act 5517
   Tyr Leu Ile Ser Asp Ser Ile Val Thr Leu Glu Gln Tyr Ser Thr
   1740 1745 1750
   tgt gac att tgt aaa agt act gta gtt gaa gtt aaa agt gct gtt 5562
   Cys Asp Ile Cys Lys Ser Thr Val Val Glu Val Lys Ser Ala Val
   1755 1760 1765
   gtc tgt gct agt gtg ctt aaa gat ggt tgt gat gtt ggt ttt tgt 5607
   Val Cys Ala Ser Val Leu Lys Asp Gly Cys Asp Val Gly Phe Cys
   1770 1775 1780
   cca cac aga cat aaa ttg cgt tca cgt gtt aag ttt gtt aat gga 5652
   Pro His Arg His Lys Leu Arg Ser Arg Val Lys Phe Val Asn Gly
   1785 1790 1795
   cgt gtt gtt att acc aat gtt ggt gaa cct ata att tca caa cct 5697
   Arg Val Val Ile Thr Asn Val Gly Glu Pro Ile Ile Ser Gln Pro
   1800 1805 1810
   tct aag ttg ctt aat ggt att gct tat aca aca ttt tca ggt tct 5742
   Ser Lys Leu Leu Asn Gly Ile Ala Tyr Thr Thr Phe Ser Gly Ser
   1815 1820 1825
   ttt gat aac ggt cac tat gta gtt tat gat gct gct aat aat gct 5787
   Phe Asp Asn Gly His Tyr Val Val Tyr Asp Ala Ala Asn Asn Ala
   1830 1835 1840
   gtc tat gat ggt gct cgt tta ttt gct tca gat ttg tct act tta 5832
   Val Tyr Asp Gly Ala Arg Leu Phe Ala Ser Asp Leu Ser Thr Leu
   1845 1850 1855
   gct gtt aca gct att gtt gta gta ggt ggt tgt gta aca tct aat 5877
   Ala Val Thr Ala Ile Val Val Val Gly Gly Cys Val Thr Ser Asn
   1860 1865 1870
   gtt cca cca att gtt agt gag aaa att tct gtt atg gat aaa ctt 5922
   Val Pro Pro Ile Val Ser Glu Lys Ile Ser Val Met Asp Lys Leu
   1875 1880 1885
   gat act ggt gca caa aaa ttt ttc caa ttt ggt gat ttt gtt atg 5967
   Asp Thr Gly Ala Gln Lys Phe Phe Gln Phe Gly Asp Phe Val Met
   1890 1895 1900
   aat aac att gtt ctg ttt tta act tgg ttg ctt agt atg ttt agt 6012
   Asn Asn Ile Val Leu Phe Leu Thr Trp Leu Leu Ser Met Phe Ser
   1905 1910 1915
   ctt tta cgt act tct att atg aag cat gat att aaa gtt att gcc 6057
   Leu Leu Arg Thr Ser Ile Met Lys His Asp Ile Lys Val Ile Ala
   1920 1925 1930
   aag gct cct aaa cgt aca ggt gtt att ttg aca cgt agt ttt aag 6102
   Lys Ala Pro Lys Arg Thr Gly Val Ile Leu Thr Arg Ser Phe Lys
   1935 1940 1945
   tat aac att aga tct gct ttg ttt gtt gta aag cag aag tgg tgt 6147
   Tyr Asn Ile Arg Ser Ala Leu Phe Val Val Lys Gln Lys Trp Cys
   1950 1955 1960
   gtt att gtt act ttg ttt aag ttc tta ttg tta tta tat gct att 6192
   Val Ile Val Thr Leu Phe Lys Phe Leu Leu Leu Leu Tyr Ala Ile
   1965 1970 1975
   tat gca ctt gtt ttt atg att gtg caa ttt agt cct ttt aat agt 6237
   Tyr Ala Leu Val Phe Met Ile Val Gln Phe Ser Pro Phe Asn Ser
   1980 1985 1990
   ctt tta tgt ggt gac att gta agt ggt tat gaa aaa tcc act ttt 6282
   Leu Leu Cys Gly Asp Ile Val Ser Gly Tyr Glu Lys Ser Thr Phe
   1995 2000 2005
   aat aag gat att tat tgt ggt aat tct atg gtt tgt aag atg tgt 6327
   Asn Lys Asp Ile Tyr Cys Gly Asn Ser Met Val Cys Lys Met Cys
   2010 2015 2020
   ttg ttt agt tat caa gag ttt aat gat ttg gat cat act agt ctt 6372
   Leu Phe Ser Tyr Gln Glu Phe Asn Asp Leu Asp His Thr Ser Leu
   2025 2030 2035
   gtt tgg aag cac att cgt gat cct ata tta atc agt tta caa cca 6417
   Val Trp Lys His Ile Arg Asp Pro Ile Leu Ile Ser Leu Gln Pro
   2040 2045 2050
   ttt gtt ata ctt gtt att ttg tta att ttt ggt aat atg tat ttg 6462
   Phe Val Ile Leu Val Ile Leu Leu Ile Phe Gly Asn Met Tyr Leu
   2055 2060 2065
   cgt ttt gga ctt tta tat ttt gtt gca caa ttt att agt act ttt 6507
   Arg Phe Gly Leu Leu Tyr Phe Val Ala Gln Phe Ile Ser Thr Phe
   2070 2075 2080
   ggt tct ttc tta ggc ttt cat cag aaa cag tgg ttt tta cat ttt 6552
   Gly Ser Phe Leu Gly Phe His Gln Lys Gln Trp Phe Leu His Phe
   2085 2090 2095
   gtg ccg ttt gat gtt tta tgt aat gag ttt tta gct aca ttt att 6597
   Val Pro Phe Asp Val Leu Cys Asn Glu Phe Leu Ala Thr Phe Ile
   2100 2105 2110
   gtc tgc aaa att gtt tta ttt gtt aga cat att att gtt ggc tgt 6642
   Val Cys Lys Ile Val Leu Phe Val Arg His Ile Ile Val Gly Cys
   2115 2120 2125
   aat aat gct gac tgt gta gct tgt tct aaa agt gct aga ctt aaa 6687
   Asn Asn Ala Asp Cys Val Ala Cys Ser Lys Ser Ala Arg Leu Lys
   2130 2135 2140
   cgt gta cca ctt caa act att att aat ggt atg cat aaa tca ttc 6732
   Arg Val Pro Leu Gln Thr Ile Ile Asn Gly Met His Lys Ser Phe
   2145 2150 2155
   tat gtt aat gct aat ggt ggt act tgt ttc tgt aat aaa cat aac 6777
   Tyr Val Asn Ala Asn Gly Gly Thr Cys Phe Cys Asn Lys His Asn
   2160 2165 2170
   ttc ttt tgt gtt aat tgt gat tct ttt ggg cct ggt aat act ttt 6822
   Phe Phe Cys Val Asn Cys Asp Ser Phe Gly Pro Gly Asn Thr Phe
   2175 2180 2185
   att aat ggt gat att gca aga gag ctt ggt aat gtt gtt aaa aca 6867
   Ile Asn Gly Asp Ile Ala Arg Glu Leu Gly Asn Val Val Lys Thr
   2190 2195 2200
   gct gtt caa ccc aca gct cct gca tat gtt att att gat aag gta 6912
   Ala Val Gln Pro Thr Ala Pro Ala Tyr Val Ile Ile Asp Lys Val
   2205 2210 2215
   gat ttt gtt aat gga ttt tat cgt ctt tat agt ggt gac act ttt 6957
   Asp Phe Val Asn Gly Phe Tyr Arg Leu Tyr Ser Gly Asp Thr Phe
   2220 2225 2230
   tgg cgg tat gac ttt gac att act gaa tct aag tat agt tgt aaa 7002
   Trp Arg Tyr Asp Phe Asp Ile Thr Glu Ser Lys Tyr Ser Cys Lys
   2235 2240 2245
   gag gtt ctg aag aat tgt aat gtt tta gaa aat ttt att gtt tac 7047
   Glu Val Leu Lys Asn Cys Asn Val Leu Glu Asn Phe Ile Val Tyr
   2250 2255 2260
   aat aat agt ggt agt aac att aca cag att aaa aat gct tgt gtt 7092
   Asn Asn Ser Gly Ser Asn Ile Thr Gln Ile Lys Asn Ala Cys Val
   2265 2270 2275
   tat ttt tct caa ttg ttg tgt gaa cct ata aag ttg gta aat tca 7137
   Tyr Phe Ser Gln Leu Leu Cys Glu Pro Ile Lys Leu Val Asn Ser
   2280 2285 2290
   gag ttg ttg tca act tta tca gtt gat ttt aat ggt gtt ttg cat 7182
   Glu Leu Leu Ser Thr Leu Ser Val Asp Phe Asn Gly Val Leu His
   2295 2300 2305
   aag gca tat gtt gat gtt ttg tgt aat agt ttt ttt aag gag cta 7227
   Lys Ala Tyr Val Asp Val Leu Cys Asn Ser Phe Phe Lys Glu Leu
   2310 2315 2320
   act gct aac atg tcc atg gct gaa tgt aaa gct aca ctt ggt ttg 7272
   Thr Ala Asn Met Ser Met Ala Glu Cys Lys Ala Thr Leu Gly Leu
   2325 2330 2335
   act gtt tct gat gat gat ttt gtt tca gct gtt gcc aat gca cat 7317
   Thr Val Ser Asp Asp Asp Phe Val Ser Ala Val Ala Asn Ala His
   2340 2345 2350
   agg tat gac gtt ttg ctt tca gat ttg tca ttt aat aat ttt ttt 7362
   Arg Tyr Asp Val Leu Leu Ser Asp Leu Ser Phe Asn Asn Phe Phe
   2355 2360 2365
   att tct tat gct aaa cct gaa gat aag ttg tcc gtt tat gac att 7407
   Ile Ser Tyr Ala Lys Pro Glu Asp Lys Leu Ser Val Tyr Asp Ile
   2370 2375 2380
   gct tgt tgt atg cgt gcc ggt tct aag gtt gtt aac cat aat gtt 7452
   Ala Cys Cys Met Arg Ala Gly Ser Lys Val Val Asn His Asn Val
   2385 2390 2395
   tta atc aaa gag tca ata cct att gtt tgg ggt gtc aag gac ttt 7497
   Leu Ile Lys Glu Ser Ile Pro Ile Val Trp Gly Val Lys Asp Phe
   2400 2405 2410
   aat act ctt tct caa gaa ggt aag aag tac ctt gtt aaa aca act 7542
   Asn Thr Leu Ser Gln Glu Gly Lys Lys Tyr Leu Val Lys Thr Thr
   2415 2420 2425
   aaa gca aag ggt ttg act ttt tta tta act ttt aat gat aac caa 7587
   Lys Ala Lys Gly Leu Thr Phe Leu Leu Thr Phe Asn Asp Asn Gln
   2430 2435 2440
   gca att aca caa gtt cct gct act agt ata gtt gca aaa cag ggt 7632
   Ala Ile Thr Gln Val Pro Ala Thr Ser Ile Val Ala Lys Gln Gly
   2445 2450 2455
   gct ggt ttt aaa cgt act tat aat ttt ctg tgg tat gta tgt tta 7677
   Ala Gly Phe Lys Arg Thr Tyr Asn Phe Leu Trp Tyr Val Cys Leu
   2460 2465 2470
   ttt gtt gtt gca ttg ttt att ggt gtc tca ttt att gat tat aca 7722
   Phe Val Val Ala Leu Phe Ile Gly Val Ser Phe Ile Asp Tyr Thr
   2475 2480 2485
   acc act gta act agc ttt cat ggt tat gat ttt aag tac att gag 7767
   Thr Thr Val Thr Ser Phe His Gly Tyr Asp Phe Lys Tyr Ile Glu
   2490 2495 2500
   aat ggt cag ttg aag gtg ttt gaa gca cct tta cac tgt gtt cgt 7812
   Asn Gly Gln Leu Lys Val Phe Glu Ala Pro Leu His Cys Val Arg
   2505 2510 2515
   aat gtt ttt gat aat ttt aat caa tgg cat gag gct aag ttt ggt 7857
   Asn Val Phe Asp Asn Phe Asn Gln Trp His Glu Ala Lys Phe Gly
   2520 2525 2530
   gtt gtt act act aat agt gat aaa tgt cct ata gtt gtt ggt gtt 7902
   Val Val Thr Thr Asn Ser Asp Lys Cys Pro Ile Val Val Gly Val
   2535 2540 2545
   tca gag cgt att aat gtt gtt cct ggt gtt cca aca aat gta tat 7947
   Ser Glu Arg Ile Asn Val Val Pro Gly Val Pro Thr Asn Val Tyr
   2550 2555 2560
   ttg gta gga aag act ctt gtt ttt aca tta cag gct gct ttt gga 7992
   Leu Val Gly Lys Thr Leu Val Phe Thr Leu Gln Ala Ala Phe Gly
   2565 2570 2575
   aac aca ggt gtt tgt tat gac ttt gat ggt gtt acc act agt gat 8037
   Asn Thr Gly Val Cys Tyr Asp Phe Asp Gly Val Thr Thr Ser Asp
   2580 2585 2590
   aag tgt att ttt aat tct gct tgt act agg ttg gaa ggt ttg ggt 8082
   Lys Cys Ile Phe Asn Ser Ala Cys Thr Arg Leu Glu Gly Leu Gly
   2595 2600 2605
   ggt gac aat gtt tat tgt tac aac act gat ctt att gaa ggt tct 8127
   Gly Asp Asn Val Tyr Cys Tyr Asn Thr Asp Leu Ile Glu Gly Ser
   2610 2615 2620
   aaa cct tat agt att tta cag ccc aat gct tat tat aag tat gat 8172
   Lys Pro Tyr Ser Ile Leu Gln Pro Asn Ala Tyr Tyr Lys Tyr Asp
   2625 2630 2635
   gtt aaa aat tat gta cgt ttt cca gaa att tta gct aga ggt ttt 8217
   Val Lys Asn Tyr Val Arg Phe Pro Glu Ile Leu Ala Arg Gly Phe
   2640 2645 2650
   ggc tta cgt act att aga act ttg gct aca cgt tat tgt aga gtt 8262
   Gly Leu Arg Thr Ile Arg Thr Leu Ala Thr Arg Tyr Cys Arg Val
   2655 2660 2665
   ggt gaa tgc cgt gac tca cat aaa ggt gtt tgt ttt ggt ttt gat 8307
   Gly Glu Cys Arg Asp Ser His Lys Gly Val Cys Phe Gly Phe Asp
   2670 2675 2680
   aaa tgg tat gtt aat gat gga cgt gtt gat gac ggt tac att tgt 8352
   Lys Trp Tyr Val Asn Asp Gly Arg Val Asp Asp Gly Tyr Ile Cys
   2685 2690 2695
   ggt gat ggt ctt ata gac ctt ctt gtt aat gta ctc tca atc ttt 8397
   Gly Asp Gly Leu Ile Asp Leu Leu Val Asn Val Leu Ser Ile Phe
   2700 2705 2710
   agt tca tct ttt agc gtt gtg gct atg tct gga cat atg ttg ttt 8442
   Ser Ser Ser Phe Ser Val Val Ala Met Ser Gly His Met Leu Phe
   2715 2720 2725
   aat ttt ctt ttt gca gca ttt att aca ttt ttg tgc ttt tta gtt 8487
   Asn Phe Leu Phe Ala Ala Phe Ile Thr Phe Leu Cys Phe Leu Val
   2730 2735 2740
   act aaa ttt aaa cgt gtt ttt ggt gat ctt tct tat ggt gtt ttt 8532
   Thr Lys Phe Lys Arg Val Phe Gly Asp Leu Ser Tyr Gly Val Phe
   2745 2750 2755
   act gtt gtt tgt gca act ttg att aat aac att tct tat gtt gtt 8577
   Thr Val Val Cys Ala Thr Leu Ile Asn Asn Ile Ser Tyr Val Val
   2760 2765 2770
   act caa aat tta ttt ttt atg ttg ctt tat gct att ttg tat ttt 8622
   Thr Gln Asn Leu Phe Phe Met Leu Leu Tyr Ala Ile Leu Tyr Phe
   2775 2780 2785
   gtt ttt act agg aca gtg cgt tat gct tgg att tgg cat att gca 8667
   Val Phe Thr Arg Thr Val Arg Tyr Ala Trp Ile Trp His Ile Ala
   2790 2795 2800
   tac att gtt gca tac ttc ttg tta ata cca tgg tgg ctt ctc aca 8712
   Tyr Ile Val Ala Tyr Phe Leu Leu Ile Pro Trp Trp Leu Leu Thr
   2805 2810 2815
   tgg ttt agt ttt gct gca ttt tta gag ctt tta cct aat gtt ttt 8757
   Trp Phe Ser Phe Ala Ala Phe Leu Glu Leu Leu Pro Asn Val Phe
   2820 2825 2830
   aag tta aaa atc tct act caa ttg ttt gaa ggt gat aag ttt ata 8802
   Lys Leu Lys Ile Ser Thr Gln Leu Phe Glu Gly Asp Lys Phe Ile
   2835 2840 2845
   ggt act ttt gag agt gct gct gca ggt aca ttt gtt ctt gac atg 8847
   Gly Thr Phe Glu Ser Ala Ala Ala Gly Thr Phe Val Leu Asp Met
   2850 2855 2860
   cgt tct tat gaa agg ctg ata aat act att tca cct gag aaa ctt 8892
   Arg Ser Tyr Glu Arg Leu Ile Asn Thr Ile Ser Pro Glu Lys Leu
   2865 2870 2875
   aag aat tat gct gca agt tat aat aaa tat aaa tat tat agt ggt 8937
   Lys Asn Tyr Ala Ala Ser Tyr Asn Lys Tyr Lys Tyr Tyr Ser Gly
   2880 2885 2890
   agt gct agt gag gct gat tat cgt tgt gct tgt tat gct cat tta 8982
   Ser Ala Ser Glu Ala Asp Tyr Arg Cys Ala Cys Tyr Ala His Leu
   2895 2900 2905
   gcc aag gct atg tta gat tac gca aaa gat cat aat gac atg tta 9027
   Ala Lys Ala Met Leu Asp Tyr Ala Lys Asp His Asn Asp Met Leu
   2910 2915 2920
   tat tct cca cct acc att agc tac aat tcc acc tta caa tct ggt 9072
   Tyr Ser Pro Pro Thr Ile Ser Tyr Asn Ser Thr Leu Gln Ser Gly
   2925 2930 2935
   ctt aag aag atg gca caa cca tct ggt tgt gtt gag aga tgt gtg 9117
   Leu Lys Lys Met Ala Gln Pro Ser Gly Cys Val Glu Arg Cys Val
   2940 2945 2950
   gtt cgc gtc tgt tat ggt agt act gtg ctt aat gga gtt tgg tta 9162
   Val Arg Val Cys Tyr Gly Ser Thr Val Leu Asn Gly Val Trp Leu
   2955 2960 2965
   ggt gac act gtt act tgt cct aga cat gtc ata gca cca tca acc 9207
   Gly Asp Thr Val Thr Cys Pro Arg His Val Ile Ala Pro Ser Thr
   2970 2975 2980
   act gtt ctt att gat tat gat cat gca tat agt act atg cgt ttg 9252
   Thr Val Leu Ile Asp Tyr Asp His Ala Tyr Ser Thr Met Arg Leu
   2985 2990 2995
   cat aat ttt tca gtg tct cat aat ggt gtc ttc ttg gga gtt gtt 9297
   His Asn Phe Ser Val Ser His Asn Gly Val Phe Leu Gly Val Val
   3000 3005 3010
   ggt gtt aca atg cat ggt tct gtg ttg cgt att aag gtt tca caa 9342
   Gly Val Thr Met His Gly Ser Val Leu Arg Ile Lys Val Ser Gln
   3015 3020 3025
   tct aat gta cat aca cct aaa cat gtt ttt aaa acg ttg aaa cct 9387
   Ser Asn Val His Thr Pro Lys His Val Phe Lys Thr Leu Lys Pro
   3030 3035 3040
   ggt gct tct ttt aat att tta gca tgt tat gaa ggt att gca tct 9432
   Gly Ala Ser Phe Asn Ile Leu Ala Cys Tyr Glu Gly Ile Ala Ser
   3045 3050 3055
   ggt gtt ttt ggt gtt aat tta cgt aca aac ttt act att aaa ggt 9477
   Gly Val Phe Gly Val Asn Leu Arg Thr Asn Phe Thr Ile Lys Gly
   3060 3065 3070
   tct ttt ata aat gga gct tgt ggt tct cct ggt tat aat gtt aga 9522
   Ser Phe Ile Asn Gly Ala Cys Gly Ser Pro Gly Tyr Asn Val Arg
   3075 3080 3085
   aat gat ggt act gtt gag ttt tgt tat tta cac caa att gag tta 9567
   Asn Asp Gly Thr Val Glu Phe Cys Tyr Leu His Gln Ile Glu Leu
   3090 3095 3100
   ggt agt ggt gct cat gtt ggt tct gat ttt act ggt agt gtt tat 9612
   Gly Ser Gly Ala His Val Gly Ser Asp Phe Thr Gly Ser Val Tyr
   3105 3110 3115
   ggt aat ttt gat gac caa cct agt ttg caa gtt gag agt gcc aac 9657
   Gly Asn Phe Asp Asp Gln Pro Ser Leu Gln Val Glu Ser Ala Asn
   3120 3125 3130
   ctt atg cta tca gat aat gtt gtt gcc ttt ttg tat gct gct ttg 9702
   Leu Met Leu Ser Asp Asn Val Val Ala Phe Leu Tyr Ala Ala Leu
   3135 3140 3145
   ttg aat ggt tgt agg tgg tgg ttg cgt tca act aga gtt aat gtt 9747
   Leu Asn Gly Cys Arg Trp Trp Leu Arg Ser Thr Arg Val Asn Val
   3150 3155 3160
   gat ggt ttt aat gaa tgg gct atg gct aat ggt tat aca att gtt 9792
   Asp Gly Phe Asn Glu Trp Ala Met Ala Asn Gly Tyr Thr Ile Val
   3165 3170 3175
   tct agt gtt gag tgc tat tct att ttg gca gca aaa act ggt gtt 9837
   Ser Ser Val Glu Cys Tyr Ser Ile Leu Ala Ala Lys Thr Gly Val
   3180 3185 3190
   agt gtt gaa caa ttg tta gct tcc att caa cat ctt cat gaa ggt 9882
   Ser Val Glu Gln Leu Leu Ala Ser Ile Gln His Leu His Glu Gly
   3195 3200 3205
   ttt ggt ggt aaa aac ata ctt ggt tat tct agt tta tgt gat gag 9927
   Phe Gly Gly Lys Asn Ile Leu Gly Tyr Ser Ser Leu Cys Asp Glu
   3210 3215 3220
   ttc aca cta gct gaa gtt gtg aag cag atg tat ggt gtt aac ttg 9972
   Phe Thr Leu Ala Glu Val Val Lys Gln Met Tyr Gly Val Asn Leu
   3225 3230 3235
   caa agt ggt aag gtt att ttt ggt tta aaa aca atg ttt tta ttt 10017
   Gln Ser Gly Lys Val Ile Phe Gly Leu Lys Thr Met Phe Leu Phe
   3240 3245 3250
   agc gtt ttc ttc aca atg ttt tgg gca gaa ctc ttt att tat aca 10062
   Ser Val Phe Phe Thr Met Phe Trp Ala Glu Leu Phe Ile Tyr Thr
   3255 3260 3265
   aac act ata tgg ata aac cct gtt ata ctt aca cct ata ttt tgt 10107
   Asn Thr Ile Trp Ile Asn Pro Val Ile Leu Thr Pro Ile Phe Cys
   3270 3275 3280
   tta ctt ttg ttt ttg tca tta gtt tta act atg ttt ctt aaa cat 10152
   Leu Leu Leu Phe Leu Ser Leu Val Leu Thr Met Phe Leu Lys His
   3285 3290 3295
   aag ttt ttg ttt ttg caa gta ttt tta tta cct act gtt att gca 10197
   Lys Phe Leu Phe Leu Gln Val Phe Leu Leu Pro Thr Val Ile Ala
   3300 3305 3310
   act gct tta tat aat tgt gtt ttg gat tat tac ata gta aaa ttt 10242
   Thr Ala Leu Tyr Asn Cys Val Leu Asp Tyr Tyr Ile Val Lys Phe
   3315 3320 3325
   ttg gct gac cat ttt aac tat aat gtt tca gta tta caa atg gat 10287
   Leu Ala Asp His Phe Asn Tyr Asn Val Ser Val Leu Gln Met Asp
   3330 3335 3340
   gtt cag ggt tta gtt aat gtt ttg gtc tgt tta ttt gtt gta ttt 10332
   Val Gln Gly Leu Val Asn Val Leu Val Cys Leu Phe Val Val Phe
   3345 3350 3355
   tta cac aca tgg cgt ttt tct aaa gaa cgt ttc aca cat tgg ttt 10377
   Leu His Thr Trp Arg Phe Ser Lys Glu Arg Phe Thr His Trp Phe
   3360 3365 3370
   aca tat gtg tgt tct ctt ata gca gtt gct tac act tat ttt tat 10422
   Thr Tyr Val Cys Ser Leu Ile Ala Val Ala Tyr Thr Tyr Phe Tyr
   3375 3380 3385
   agt ggt gac ttt ttg agt ttg ctt gtt atg ttt tta tgt gct ata 10467
   Ser Gly Asp Phe Leu Ser Leu Leu Val Met Phe Leu Cys Ala Ile
   3390 3395 3400
   tct agt gat tgg tac att ggt gcc att gtt ttt agg ttg tca cgt 10512
   Ser Ser Asp Trp Tyr Ile Gly Ala Ile Val Phe Arg Leu Ser Arg
   3405 3410 3415
   ttg att ata ttt ttt tca cct gaa agt gta ttt agt gtt ttt ggt 10557
   Leu Ile Ile Phe Phe Ser Pro Glu Ser Val Phe Ser Val Phe Gly
   3420 3425 3430
   gat gtg aaa ctc act tta gtt gtt tat tta att tgt ggt tat tta 10602
   Asp Val Lys Leu Thr Leu Val Val Tyr Leu Ile Cys Gly Tyr Leu
   3435 3440 3445
   gtt tgt act tat tgg ggc att ttg tat tgg ttc aat agg ttt ttt 10647
   Val Cys Thr Tyr Trp Gly Ile Leu Tyr Trp Phe Asn Arg Phe Phe
   3450 3455 3460
   aaa tgt act atg ggt gtt tat gat ttt aag gtg agt gct gct gaa 10692
   Lys Cys Thr Met Gly Val Tyr Asp Phe Lys Val Ser Ala Ala Glu
   3465 3470 3475
   ttt aaa tac atg gtt gct aat gga ctt cat gca cca tat gga cct 10737
   Phe Lys Tyr Met Val Ala Asn Gly Leu His Ala Pro Tyr Gly Pro
   3480 3485 3490
   ttt gat gca ctt tgg tta tca ttc aaa tta ctt ggt att ggt ggt 10782
   Phe Asp Ala Leu Trp Leu Ser Phe Lys Leu Leu Gly Ile Gly Gly
   3495 3500 3505
   gac cgt tgt ata aaa att tca act gtc caa tcc aaa ctg act gat 10827
   Asp Arg Cys Ile Lys Ile Ser Thr Val Gln Ser Lys Leu Thr Asp
   3510 3515 3520
   ttg aag tgt act aat gtt gtg tta ttg ggt tgt ttg tct agt atg 10872
   Leu Lys Cys Thr Asn Val Val Leu Leu Gly Cys Leu Ser Ser Met
   3525 3530 3535
   aac att gca gct aat tct agt gaa tgg gct tat tgt gtt gat tta 10917
   Asn Ile Ala Ala Asn Ser Ser Glu Trp Ala Tyr Cys Val Asp Leu
   3540 3545 3550
   cac aat aag att aat ctt tgt gat gac cca gaa aaa gct caa ggt 10962
   His Asn Lys Ile Asn Leu Cys Asp Asp Pro Glu Lys Ala Gln Gly
   3555 3560 3565
   atg ttg tta gca ctc ctt gcg ttc ttt cta agt aaa cat agt gat 11007
   Met Leu Leu Ala Leu Leu Ala Phe Phe Leu Ser Lys His Ser Asp
   3570 3575 3580
   ttt ggt ctt gat ggc ctt att gat tct tat ttt gat aat agt agc 11052
   Phe Gly Leu Asp Gly Leu Ile Asp Ser Tyr Phe Asp Asn Ser Ser
   3585 3590 3595
   acc ctg cag agt gtt gct tca tca ttt gtt agt atg cca tca tat 11097
   Thr Leu Gln Ser Val Ala Ser Ser Phe Val Ser Met Pro Ser Tyr
   3600 3605 3610
   att gct tat gaa aat gct aga caa gct tat gag gat gct att gct 11142
   Ile Ala Tyr Glu Asn Ala Arg Gln Ala Tyr Glu Asp Ala Ile Ala
   3615 3620 3625
   aat gga tct tct tct caa ctt att aaa caa ttg aag cgt gcc atg 11187
   Asn Gly Ser Ser Ser Gln Leu Ile Lys Gln Leu Lys Arg Ala Met
   3630 3635 3640
   aat atc gca aag tct gaa ttt gat cat gag ata tct gtt cag aag 11232
   Asn Ile Ala Lys Ser Glu Phe Asp His Glu Ile Ser Val Gln Lys
   3645 3650 3655
   aaa att aat aga  atg gct gaa caa gct  gct act cag atg tat  aaa      11277
   Lys Ile Asn Arg  Met Ala Glu Gln Ala  Ala Thr Gln Met Tyr  Lys
               3660                 3665                 3670
   gaa gca cgc tct  gtt aat aga aaa tct  aaa gtt att agt gct  atg      11322
   Glu Ala Arg Ser  Val Asn Arg Lys Ser  Lys Val Ile Ser Ala  Met
               3675                 3680                 3685
   cac tct tta ctt  ttt gga atg tta aga  cgt ttg gat atg tct  agt      11367
   His Ser Leu Leu  Phe Gly Met Leu Arg  Arg Leu Asp Met Ser  Ser
               3690                 3695                 3700
   gtt gaa act gtt  ttg aat tta gca cgt  gat ggt gtt gtg cca  ttg      11412
   Val Glu Thr Val  Leu Asn Leu Ala Arg  Asp Gly Val Val Pro  Leu
               3705                 3710                 3715
   tca gtt ata cct  gca act tca gct tcc  aaa cta act att gtt  agt      11457
   Ser Val Ile Pro  Ala Thr Ser Ala Ser  Lys Leu Thr Ile Val  Ser
               3720                 3725                 3730
   cca gat ctt gaa  tct tat tct aag att  gtt tgt gat ggt tct  gtt      11502
   Pro Asp Leu Glu  Ser Tyr Ser Lys Ile  Val Cys Asp Gly Ser  Val
               3735                 3740                 3745
   cat tat gct gga  gtt gtt tgg aca ctt  aat gat gtt aaa gac  aat      11547
   His Tyr Ala Gly  Val Val Trp Thr Leu  Asn Asp Val Lys Asp  Asn
               3750                 3755                 3760
   gat ggt aga cct  gtt cat gtt aaa gag  att aca agg gag aat  gtt      11592
   Asp Gly Arg Pro  Val His Val Lys Glu  Ile Thr Arg Glu Asn  Val
               3765                 3770                 3775
   gaa act ttg aca  tgg cct ctt atc ctt  aat tgt gaa cgt gtt  gtt      11637
   Glu Thr Leu Thr  Trp Pro Leu Ile Leu  Asn Cys Glu Arg Val  Val
               3780                 3785                 3790
   aaa ctt caa aat  aat gaa att atg cct  ggt aaa ctt aag caa  aaa      11682
   Lys Leu Gln Asn  Asn Glu Ile Met Pro  Gly Lys Leu Lys Gln  Lys
               3795                 3800                 3805
   cct atg aaa gct  gag ggt gat ggt ggt  gtt tta ggt gat ggt  aat      11727
   Pro Met Lys Ala  Glu Gly Asp Gly Gly  Val Leu Gly Asp Gly  Asn
               3810                 3815                 3820
   gct ttg tat aat  act gag ggt ggt aaa  act ttt atg tat gct  tat      11772
   Ala Leu Tyr Asn  Thr Glu Gly Gly Lys  Thr Phe Met Tyr Ala  Tyr
               3825                 3830                 3835
   att tct aat aaa  gct gac ctt aaa ttt  gtt aag tgg gag tat  gag      11817
   Ile Ser Asn Lys  Ala Asp Leu Lys Phe  Val Lys Trp Glu Tyr  Glu
               3840                 3845                 3850
   ggt ggt tgc aac  aca atc gag tta gac  tct cct tgt cga ttt  atg      11862
   Gly Gly Cys Asn  Thr Ile Glu Leu Asp  Ser Pro Cys Arg Phe  Met
               3855                 3860                 3865
   gtc gaa aca cct  aat ggt cct caa gtg  aag tat ttg tat ttt  gtt      11907
   Val Glu Thr Pro  Asn Gly Pro Gln Val  Lys Tyr Leu Tyr Phe  Val
               3870                 3875                 3880
   aaa aat tta aat  acc tta cgt aga ggt  gcc gtt ctt ggt ttt  ata      11952
   Lys Asn Leu Asn  Thr Leu Arg Arg Gly  Ala Val Leu Gly Phe  Ile
               3885                 3890                 3895
   ggt gcc aca att  cgt cta caa gct ggt  aaa caa act gaa ttg  gct      11997
   Gly Ala Thr Ile  Arg Leu Gln Ala Gly  Lys Gln Thr Glu Leu  Ala
               3900                 3905                 3910
   gtt aat tct gga  ctt tta act gct tgt  gct ttt tct gtt gat  cca      12042
   Val Asn Ser Gly  Leu Leu Thr Ala Cys  Ala Phe Ser Val Asp  Pro
               3915                 3920                 3925
   gca acc act tac  ttg gaa gct gtt aaa  cat ggt gca aaa cct  gta      12087
   Ala Thr Thr Tyr  Leu Glu Ala Val Lys  His Gly Ala Lys Pro  Val
               3930                 3935                 3940
   agt aat tgt att  aag atg tta tct aat  ggt gct ggt aat ggt  caa      12132
   Ser Asn Cys Ile  Lys Met Leu Ser Asn  Gly Ala Gly Asn Gly  Gln
               3945                 3950                 3955
   gct ata aca act  agt gta gat gct aac  acc aat caa gat tct  tat      12177
   Ala Ile Thr Thr  Ser Val Asp Ala Asn  Thr Asn Gln Asp Ser  Tyr
               3960                 3965                 3970
   ggt gga gcg tct  att tgt ttg tat tgt  cgg gcc cac gtt cct  cac      12222
   Gly Gly Ala Ser  Ile Cys Leu Tyr Cys  Arg Ala His Val Pro  His
               3975                 3980                 3985
   cct agt atg gat  ggt tac tgt aag ttt  aag ggt aaa tgt gtt  cag      12267
   Pro Ser Met Asp  Gly Tyr Cys Lys Phe  Lys Gly Lys Cys Val  Gln
               3990                 3995                 4000
   gtt cct att ggt  tgt ttg gat cct att  agg ttt tgt tta gaa  aat      12312
   Val Pro Ile Gly  Cys Leu Asp Pro Ile  Arg Phe Cys Leu Glu  Asn
               4005                 4010                 4015
   aat gtg tgt aat  gtt tgt ggt tgt tgg  ttg gga cac ggg tgt  gct      12357
   Asn Val Cys Asn  Val Cys Gly Cys Trp  Leu Gly His Gly Cys  Ala
               4020                 4025                 4030
   tgt gat cgt aca  acc att caa agt gtt  gac att tct tat tta  aac      12402
   Cys Asp Arg Thr  Thr Ile Gln Ser Val  Asp Ile Ser Tyr Leu  Asn
               4035                 4040                 4045
   gag caa ggg gtt  cta gtg cag ctc gac  tag aaccctgtaa tggcacggac     12452
   Glu Gln Gly Val  Leu Val Gln Leu Asp
               4050                 4055
   atcgataagt gtgttcgtgc ttttgacatt tataataaaa atgtttcatt cttgggtaag   12512
   tgtttgaaga tgaactgtgt tcgttttaaa aatgctgatc ttaaggatgg ttattttgtt   12572
   ataaagaggt gtactaagtc ggttatggaa cacgagcaat ccatgtataa cctacttaac   12632
   ttttctggtg ctttggctga gcatgatttc tttacttgga aagatggcag agtcatttat   12692
   ggtaatgtta gtagacataa tcttactaaa tatactatga tggacttggt ttatgctatg   12752
   cgtaactttg atgaacaaaa ttgtgatgtt ctaaaagaag tattagtttt aactggttgt   12812
   tgtgacaatt cttattttga tagtaagggt tggtatgacc cagttgaaaa tgaagatata   12872
   catagagttt atgcatctct tggcaaaatt gtagctagag ctatgcttaa atgcgttgct   12932
   ctatgtgatg cgatggttgc taaaggtgtt gttggtgttt taacattaga taaccaagat   12992
   cttaatggta acttttatga ttttggtgat tttgttgtta gcttacctaa tatgggtgtt   13052
   ccctgttgta catcatatta ttcttatatg atgcctatta tgggtttaac taattgttta   13112
   gctagtgagt gttttgtcaa gagtgatatt tttggtagtg attttaaaac ttttgatttg   13172
   cttaagtatg atttcactga acataaagaa aatttattca ataagtactt taagcattgg   13232
   agttttgatt atcatcctaa ttgtagtgac tgttatgatg atatgtgtgt tatacattgt   13292
   gctaatttta atacactatt tgccacaact ataccaggta ctgcttttgg tccactatgt 13352
   cgtaaagttt ttatagatgg tgttccactt gttacaactg ctggttatca ttttaagcaa 13412
   ttaggtttgg tttggaataa agatgttaac acacactcag ttaggttgac aatcactgaa 13472
   cttttgcaat ttgttactga cccttccttg ataatagctt cttctccagc actcgttgat 13532
   caacgcacta tttgtttttc tgttgcagca ttgagtactg gtttgacaaa tcaagttgtt 13592
   aagccaggtc attttaatga agagttttat aactttcttc gtttaagagg tttctttgat 13652
   gaaggttctg aacttacatt aaaacatttc ttcttcgcac agaatggtga tgctgctgtt 13712
   aaagattttg acttttaccg ttataataag cctaccattt tagatatttg tcaagctaga 13772
   gttacatata agatagtctc tcgttatttt gacatttatg aaggtggctg tattaaggca 13832
   tgtgaagttg ttgtaacaaa tcttaataag agtgctggtt ggccattaaa taagtttggt 13892
   aaagctagtt tgtattacga atctatatct tatgaagaac aggatgcttt gtttgctttg 13952
   acaaagcgta atgtcctccc tactatgaca cagctgaatc ttaagtatgc tattagtggt 14012
   aaagaacgtg ctagaactgt tggtggtgtt tctctgttgt ccacaatgac cacaagacaa 14072
   taccatcaaa aacatcttaa atccattgtt aatacacgca atgccactgt tgttattggt 14132
   actaccaaat tttatggtgg ttggaataat atgttgcgta ctttaattga tggtgttgaa 14192
   aaccctatgc tcatgggttg ggattatccc aaatgtgata gagctttgcc taacatgata 14252
   cgtatgattt cagccatggt gttgggttct aagcatgtta attgttgtac tgtaacagat 14312
   aggttttata ggcttggtaa cgagttggca caagttttaa cagaagttgt ttattctaat 14372
   ggtggttttt attttaagcc aggtggtacg acttctggtg acgctagtac agcttatgct 14432
   aattctattt ttaacatttt tcaagccgtg agttctaaca ttaacaggtt gcttagtgtc 14492
   ccatcagatt catgtaataa tgttaatgtt agggatctac aacgacgtct gtatgataat 14552
   tgctataggt taactagtgt tgaagagtca ttcattgatg attattatgg ttatcttagg 14612
   aaacattttt caatgatgat tctctctgat gacggtgttg tctgttataa caaggattat 14672
   gctgagttag gttatatagc agacattagt gcttttaaag ccactttgta ttaccagaat 14732
   aatgtcttta tgagtacttc taaatgttgg gttgaagaag atttaactaa gggaccacat 14792
   gagttttgtt cccagcatac tatgcaaata gttgataaag atggtaccta ttatttgcct 14852
   tacccagatc ctagtaggat cttgtcagct ggtgtttttg ttgatgatgt tgttaagaca 14912
   gatgctgttg ttttgttana acgttatgtg tctttagcta ttgatgcata ccctctttca 14972
   aaacacccta attctgaata tcgtaaggtt ttttacgtat tacttgattg ggttaagcat 15032
   cttaacaaaa atttgaatga gggtgttctt gaatcttttt ctgttacact tcttgataat 15092
   caagaagata agttttggtg tgaagatttt tatgctagta tgtatgaaaa ttctacaata 15152
   ttgcaagctg ctggcttatg tgttgtttgt ggttcacaaa ctgttcttcg ttgtggtgat 15212
   tgtctgcgta agcctatgtt gtgcactaaa tgtgcatatg atcatgtatt tggtaccgac 15272
   cacaagttta ttttggctat aacaccgtat gtatgtaatg catcaggttg tggtgttagt 15332
   gatgttaaaa aattgtatct tggtggtttg aattactatt gtacaaatca taaaccacag 15392
   ttgtcttttc cattatgttc tgctggtaat atatttggtt tatataaaaa ttcagcaact 15452
   ggttccttag atgttgaagt ttttaatagg cttgcaacgt ctgattggac tgatgttagg 15512
   gactataaac ttgctaatga tgttaaagat acacttagac tctttgcggc tgaaactatt 15572
   aaagctaaag aagagagtgt taagtcttct tatgcttttg caactcttaa agaggttgtt 15632
   ggacctaaag aattgcttct tagttgggaa agtggtaaag ttaaaccacc tttgaatcgt 15692
   aattctgttt tcacctgttt tcaaataagt aaggactcaa aattccaaat aggtgagttc 15752
   atctttgaaa aggttgaata tggttctgat actgttacgt ataagtctac tgtaaccact 15812
   aagttagttc ctggtatgat ttttgtctta acatctcaca atgttcaacc tttacgtgca 15872
   ccaactattg caaaccaaga gaagtattct agcatttata aattgcaccc tgcttttaat 15932
   gtcagtgatg catatgctaa tttggttcca tattaccaac ttattggtaa acaaaagata 15992
   actacaatac agggtcctcc tggtagtggt aagtcacatt gttccattgg acttggattg 16052
   tactatccag gtgcgcgtat tgtttttgtt gcttgtgccc atgctgctgt tgattcctta 16112
   tgtgcaaaag ctatgactgt ttatagcatt gataagtgta ctaggattat acctgcaaga 16172
   gctcgggttg agtgttatag tggctttaaa ccaaataaca ctagtgcaca atacatattt 16232
   agcactgtta acgcattacc tgagtgtaat gctgatattg ttgttgtaga tgaagtttca 16292
   atgtgtacaa attatgacct ttctgttatt aatcagcgtt tatcatataa acatattgtt 16352
   tatgttggtg atccacaaca acttcctgca cctagagtaa tgattactaa aggtgttatg 16412
   gagcctgttg attataacgt tgttactcaa cgtatgtgtg ctataggccc tgatgttttt 16472
   cttcataaat gttatagatg tcctgctgaa atagttaata cagtttctga acttgtttat 16532
   gagaacaagt ttgtccctgt taaacctgct agtaaacagt gttttaaaat cttttttaag 16592
   ggtaatgtac aggttgacaa tggctctagt attaacagaa agcagcttga aatagttaag 16652
   ctgtttttag ttaaaaatcc aagttggagt aaggctgtgt ttatttctcc ttataatagt 16712
   cagaattatg ttgctagtag atttttagga cttcaaattc aaactgttga ttcttctcaa 16772
   ggtagtgagt atgattatgt aatctatgca caaacttctg acactgcaca tgcttgcaat 16832
   gtaaaccgtt ttaatgttgc tataacacgt gctaagaagg gtatattttg tgtaatgtgt 16892
   gataaaactt tgtttgattc acttaagttt tttgagatta aacatgcaga tttacactct 16952
   agccaggttt gtggcttgtt taaaaattgt acacgcactc ctcttaattt accaccaact 17012
   catgcacaca ctttcttgtc gttgtcagat cagtttaaga ctacaggtga tttagctgtt 17072
   caaataggtt caaataatgt ttgtacttat gaacatgtta tatcatttat gggttttagg 17132
   tttgatatta gtattcctgg tagtcatagt ttgttttgta cacgtgactt tgctattcgt 17192
   aatgtgcgtg gttggttggg tatggatgtt gaaagtgctc atgtttgtgg cgataacata 17252
   ggtactaatg ttcctttaca ggttggtttt tcaaatggtg ttaattttgt tgtgcaaact 17312
   gaaggttgtg tgtctaccaa ttttggtgat gttattaaac ctgtttgtgc aaaatctcca 17372
   ccaggtgaac aatttagaca ccttgttcct tttttacgta aaggacaacc ttggttaatt 17432
   gttcgtagac gcattgtgca aatgatatct gattatttgt ccaatttgtc tgacattctt 17492
   gtctttgttt tgtgggcagg tagtttggaa ttaactacaa tgcgttactt tgtaaaaata 17552
   gggccaatta aatattgtta ttgtggtaat tctgccactt gttataattc agttagtaat 17612
   gaatattgtt gttttaaaca tgcattgggt tgtgattatg tttacaatcc gtatgctttt 17672
   gatatacaac agtggggtta tgttggttcc ttgagccaga accaccacac gttctgtaac 17732
   attcatagaa acgagcatga tgcttctggt gatgctgtta tgacacgttg tttggcagta 17792
   catgattgtt ttgtcaaaaa tgttgattgg actgtaacgt acccctttat tgcaaatgag 17852
   aaatttatca atggctgtgg gcgtaatgtc cagggacatg ttgttcgcgc agccttgaaa 17912
   ttgtataaac ctagtgttat tcatgatatt ggtaatccta aaggtgtacg ttgtgctgtt 17972
   actgatgcca aatggtactg ttatgacaag caacctgtta atagtaatgt caagttgttg 18032
   gattatgatt atgcaaccca tggtcaactt gatggtcttt gtttattctg gaattgtaat 18092
   gttgatatgt atccagaatt ttcaattgtg tgtcgctttg acacacgtac tcgttctgtt 18152
   tttaatttag aaggtgttaa tggtggttct ctttatgtta acaaacatgc gtttcataca 18212
   ccagcatatg ataaacgtgc ttttgttaaa ttaaaaccta tgcccttttt ttactttgat 18272
   gacagtgatt gtgatgttgt gcaagaacaa gttaattatg taccccttcg cgctagtagt 18332
   tgtgttaccc gttgtaatat aggtggtgct gtttgttcaa aacatgcaaa tttgtatcaa 18392
   aaatatgttg aggcatataa tacatttaca caggctggtt ttaacatttg ggtaccacat 18452
   agttttgatg tttataattt gtggcaaatt tttattgaaa ctaatttaca aagtcttgaa 18512
   aatatagcat ttaatgttgt aaaaaaaggg tgttttactg gtgttgatgg tgagttacct 18572
   gttgcagttg ttaacgacaa agtttttgtt cgctatggcg atgttgacaa cttggttttt 18632
   acaaataaaa caacattgcc tactaatgtt gcttttgaat tgtttgcaaa acgaaaaatg 18692
   ggtttaacac caccattgtc tattctcaaa aatcttggtg ttgttgctac atataaattt 18752
   gttttatggg attatgaagc tgaaagacct tttacctcat atactaagag tgtatgtaaa 18812
   tacactgatt ttaatgagga tgtttgtgtt tgttttgaca atagtattca gggttcgtat 18872
   gagcgtttta cgcttactac gaacgctgtt ttattttcta ctgttgtcat taaaaattta 18932
   acacctataa agttgaattt tggtatgttg aatggtatgc cagtttcttc tattaagagt 18992
   gataaaggtg ttgaaaaatt agttaattgg tacacatatg ttcgtaaaaa tggtcaattt 19052
   caagatcatt atgatggttt ttacactcaa ggtaggaatt tatcagactt tacaccaaga 19112
   agtgatatgg agtatgattt tcttaacatg gatatgggtg tttttattaa taaatatggt 19172
   cttgaggatt ttaattttga acatgttgta tatggtgatg tttcaaaaac tacattagga 19232
   ggtcttcatt tgttgatatc acagtttagg cttagtaaaa tgggtgtttt gaaagctgat 19292
   gattttgtca ctgcttctga cacaactttg aggtgctgta ctgttactta tcttaatgaa 19352
   cttagttcaa aagttgtttg tacttatatg gatttgttgt tggacgactt tgttactata 19412
   ctaaagagtt tagatcttgg tgtaatatct aaagttcatg aagttattat agataataaa 19472
   ccttataggt ggatgttgtg gtgtaaagat aaccacttgt ccacttttta tccacagttg 19532
   cagtctgctg aatggaagtg tggttatgct atgccacaaa tttataagct tcaacgtatg 19592
   tgtttggaac cttgtaattt atataattat ggtgctggta ttaagttgcc tagtggtata 19652
   atgttaaatg ttgttaaata cactcagctt tgtcaatacc taaatagcac tacaatgtgc 19712
   gtacctcata atatgcgtgt tttgcactat ggtgctggtt ctgacaaagg tgtggcacct 19772
   ggtacaactg ttttaaaacg ttggctacca cctgatgcaa taatcattga taatgatatc 19832
   aatgattatg ttagtgatgc agattttagc attacaggtg attgtgctac tgtttacctt 19892
   gaagataagt ttgacttact tatttctgat atgtatgatg gtagaattaa attttgtgat 19952
   ggtgaaaacg tctctaaaga tggttttttt acttatctta atggtgttat tagagaaaaa 20012
   ttagctattg gtggtagtgt tgccattaag attacagaat atagttggaa taagtatctt 20072
   tatgaattaa tacaaagatt tgctttttgg actttgttct gcacgtctgt taatacatcc 20132
   tcttcagaag cttttcttat tggtattaat tatttaggtg actttattca aggtcctttt 20192
   atagctggta acactgttca tgctaattat atattttggc gtaattctac tattatgtct 20252
   ttgtcataca attcagtttt agatttaagt aagtttgaat gtaaacataa ggccactgtt 20312
   gttgttacac ttaaagatag tgatgtaaat gatatggttt tgagtttgat taagagtggt 20372
   aggttgttgt tacgtaatag tggccgtttt ggtggtttta gtaatcattt agtctcaact 20432
   aa atg aaa ctt ttc ttg att ttg ctt att ttg ccc ctg gtt tct tgc 20479
      Met Lys Leu Phe Leu Ile Leu Leu Ile Leu Pro Leu Val Ser Cys
                      4060                4065                4070
   ttt tct aca tgt aac agt aat gct agt att tct atg tta caa tta 20524
   Phe Ser Thr Cys Asn Ser Asn Ala Ser Ile Ser Met Leu Gln Leu
                   4075                4080                4085
   ggt gtt cct gat aac tct tca act att gtc aca ggt ttg ttg cca 20569
   Gly Val Pro Asp Asn Ser Ser Thr Ile Val Thr Gly Leu Leu Pro
                   4090                4095                4100
   gtc cat tgg att tgt gct aat cag agt aca tct agt tac cca gcc 20614
   Val His Trp Ile Cys Ala Asn Gln Ser Thr Ser Ser Tyr Pro Ala
                   4105                4110                4115
   aac ggc ttt ttc tat att gat gtt ggt aaa cac cgt agt gcc ttt 20659
   Asn Gly Phe Phe Tyr Ile Asp Val Gly Lys His Arg Ser Ala Phe
                   4120                4125                4130
   gca ctc cat agt ggt tat tat gat gct aac cag tat tat att tat 20704
   Ala Leu His Ser Gly Tyr Tyr Asp Ala Asn Gln Tyr Tyr Ile Tyr
                   4135                4140                4145
   ctc act aat aaa ata cat tta aat gct cct gtc act ctg aag att 20749
   Leu Thr Asn Lys Ile His Leu Asn Ala Pro Val Thr Leu Lys Ile
                   4150                4155                4160
   tgt aag ttt gga aac act tct ttt gat ttt tta agt aat gtt tct 20794
   Cys Lys Phe Gly Asn Thr Ser Phe Asp Phe Leu Ser Asn Val Ser
                   4165                4170                4175
   act tct cat gat tgt ata gtt aat ttg tca ttc aca gaa cag tta 20839
   Thr Ser His Asp Cys Ile Val Asn Leu Ser Phe Thr Glu Gln Leu
   4180 4185 4190
   ggt gtg cct ttg ggc ata act ata tcg ggt gaa act gta cgt ttg 20884
   Gly Val Pro Leu Gly Ile Thr Ile Ser Gly Glu Thr Val Arg Leu
   4195 4200 4205
   cat tta tat aat gca act cgt act ttt tat gtg ccg gcc gct tat 20929
   His Leu Tyr Asn Ala Thr Arg Thr Phe Tyr Val Pro Ala Ala Tyr
   4210 4215 4220
   aaa ctt act aaa ctt agt gtt aaa tgt tac ttt agt gaa tcc tgt 20974
   Lys Leu Thr Lys Leu Ser Val Lys Cys Tyr Phe Ser Glu Ser Cys
   4225 4230 4235
   gtt ttt agt gtt gtc aat gcc acc att act gtt aat gtc acc aca 21019
   Val Phe Ser Val Val Asn Ala Thr Ile Thr Val Asn Val Thr Thr
   4240 4245 4250
   ctt aat ggc cgt ata gtt aac tac act gtt tgt gat gat tgt aat 21064
   Leu Asn Gly Arg Ile Val Asn Tyr Thr Val Cys Asp Asp Cys Asn
   4255 4260 4265
   ggt tat act gat aac ata ttt tct gtt caa cag gat ggc cgc att 21109
   Gly Tyr Thr Asp Asn Ile Phe Ser Val Gln Gln Asp Gly Arg Ile
   4270 4275 4280
   cct aat ggt ttc cct ttt aat aat tgg ttt ttg tta act aat ggt 21154
   Pro Asn Gly Phe Pro Phe Asn Asn Trp Phe Leu Leu Thr Asn Gly
   4285 4290 4295
   tcc aca tta gtg gac ggg gtc tct aga ctt tat caa cca ctc cgt 21199
   Ser Thr Leu Val Asp Gly Val Ser Arg Leu Tyr Gln Pro Leu Arg
   4300 4305 4310
   tta act tgt tta tgg cct gta cct ggt ctt aaa tct tca act ggt 21244
   Leu Thr Cys Leu Trp Pro Val Pro Gly Leu Lys Ser Ser Thr Gly
   4315 4320 4325
   ttt gtt tat ttt aat gcc act ggt tct gat gtt aat tgt aac ggc 21289
   Phe Val Tyr Phe Asn Ala Thr Gly Ser Asp Val Asn Cys Asn Gly
   4330 4335 4340
   tat caa cat aat tct gtt gct gat gtt atg cgt tac aat ctt aac 21334
   Tyr Gln His Asn Ser Val Ala Asp Val Met Arg Tyr Asn Leu Asn
   4345 4350 4355
   ctc agt gct aat tct gtg gac aat ctt aag agt ggt gtt ata gtt 21379
   Leu Ser Ala Asn Ser Val Asp Asn Leu Lys Ser Gly Val Ile Val
   4360 4365 4370
   ttt aaa act tta cag tac gat gtt ttg ttt tat tgt agt aat tct 21424
   Phe Lys Thr Leu Gln Tyr Asp Val Leu Phe Tyr Cys Ser Asn Ser
   4375 4380 4385
   tct tca ggt gtt ctt gac acc aca ata cct ttt ggc cct tcc tct 21469
   Ser Ser Gly Val Leu Asp Thr Thr Ile Pro Phe Gly Pro Ser Ser
   4390 4395 4400
   caa cct tat tac tgt ttt ata aac agt act atc aac act act cat 21514
   Gln Pro Tyr Tyr Cys Phe Ile Asn Ser Thr Ile Asn Thr Thr His
   4405 4410 4415
   gtt agc act ttt gtg ggt att tta cca ccc act gtg cgt gaa att 21559
   Val Ser Thr Phe Val Gly Ile Leu Pro Pro Thr Val Arg Glu Ile
   4420 4425 4430
   gtt gtt gct aga act ggt cag ttt tat att aat ggt ttt aag tat 21604
   Val Val Ala Arg Thr Gly Gln Phe Tyr Ile Asn Gly Phe Lys Tyr
   4435 4440 4445
   ttc gat ttg ggt ttc ata gaa gct gtc aat ttt aat gtc acg act 21649
   Phe Asp Leu Gly Phe Ile Glu Ala Val Asn Phe Asn Val Thr Thr
   4450 4455 4460
   gct agt gcc aca gat ttt tgg acg gtt gca ttt gct act ttt gtt 21694
   Ala Ser Ala Thr Asp Phe Trp Thr Val Ala Phe Ala Thr Phe Val
   4465 4470 4475
   gat gtt ttg gtt aat gtt agt gca act aac att caa aac tta ctt 21739
   Asp Val Leu Val Asn Val Ser Ala Thr Asn Ile Gln Asn Leu Leu
   4480 4485 4490
   tat tgc gat tct cca ttt gaa aag ttg cag tgt gag cac ttg cag 21784
   Tyr Cys Asp Ser Pro Phe Glu Lys Leu Gln Cys Glu His Leu Gln
   4495 4500 4505
   ttt gga ttg caa gat ggt ttt tat tct gca aat ttt ctt gat gat 21829
   Phe Gly Leu Gln Asp Gly Phe Tyr Ser Ala Asn Phe Leu Asp Asp
   4510 4515 4520
   aat gtt ttg cct gag act tat gtt gca ctc ccc att tat tat caa 21874
   Asn Val Leu Pro Glu Thr Tyr Val Ala Leu Pro Ile Tyr Tyr Gln
   4525 4530 4535
   cat acg gac ata aat ttt act gca act gca tct ttt ggt ggt tct 21919
   His Thr Asp Ile Asn Phe Thr Ala Thr Ala Ser Phe Gly Gly Ser
   4540 4545 4550
   tgt tat gtt tgt aaa cca cgc cag gtt aat ata tct ctt aat ggt 21964
   Cys Tyr Val Cys Lys Pro Arg Gln Val Asn Ile Ser Leu Asn Gly
   4555 4560 4565
   aac act tca gtg tgt gtt aga aca tct cat ttt tca att agg tat 22009
   Asn Thr Ser Val Cys Val Arg Thr Ser His Phe Ser Ile Arg Tyr
   4570 4575 4580
   att tat aac cgc gtt aag agt ggt tca cca ggt gac tct tca tgg 22054
   Ile Tyr Asn Arg Val Lys Ser Gly Ser Pro Gly Asp Ser Ser Trp
   4585 4590 4595
   cat att tat tta aag agt ggc act tgt cca ttt tct ttt tct aag 22099
   His Ile Tyr Leu Lys Ser Gly Thr Cys Pro Phe Ser Phe Ser Lys
   4600 4605 4610
   tta aat aat ttt caa aag ttt aag act att tgt ttc tca acc gtc 22144
   Leu Asn Asn Phe Gln Lys Phe Lys Thr Ile Cys Phe Ser Thr Val
   4615 4620 4625
   gaa gtg cct ggt agt tgt aat ttt cca ctt gaa gcc acc tgg cat 22189
   Glu Val Pro Gly Ser Cys Asn Phe Pro Leu Glu Ala Thr Trp His
   4630 4635 4640
   tac act tct tat act att gtt ggt gct ttg tat gtt act tgg tct 22234
   Tyr Thr Ser Tyr Thr Ile Val Gly Ala Leu Tyr Val Thr Trp Ser
   4645 4650 4655
   gaa ggt aat tcc att act ggt gta cct tat cct gtc tct ggt att 22279
   Glu Gly Asn Ser Ile Thr Gly Val Pro Tyr Pro Val Ser Gly Ile
   4660 4665 4670
   cgt gag ttt agt aat tta gtt tta aat aat tgt acc aaa tat aat 22324
   Arg Glu Phe Ser Asn Leu Val Leu Asn Asn Cys Thr Lys Tyr Asn
   4675 4680 4685
   att tat gat tat gtt ggt act gga att ata cgt tct tca aac cag 22369
   Ile Tyr Asp Tyr Val Gly Thr Gly Ile Ile Arg Ser Ser Asn Gln
   4690 4695 4700
   tca ctt gct ggt ggt att aca tat gtt tct aac tct ggt aat tta 22414
   Ser Leu Ala Gly Gly Ile Thr Tyr Val Ser Asn Ser Gly Asn Leu
   4705 4710 4715
   ctt ggt ttt aaa aat gtt tcc act ggt aac att ttt att gtg aca 22459
   Leu Gly Phe Lys Asn Val Ser Thr Gly Asn Ile Phe Ile Val Thr
   4720 4725 4730
   cca tgt aac caa cca gat caa gta gct gtt tat caa caa agc att 22504
   Pro Cys Asn Gln Pro Asp Gln Val Ala Val Tyr Gln Gln Ser Ile
   4735 4740 4745
   att ggt gcc atg acc gct gtt aat gag tct aga tat ggc ttg caa 22549
   Ile Gly Ala Met Thr Ala Val Asn Glu Ser Arg Tyr Gly Leu Gln
   4750 4755 4760
   aac tta cta cag tta cct aac ttt tat tat gtt agt aat ggt ggt 22594
   Asn Leu Leu Gln Leu Pro Asn Phe Tyr Tyr Val Ser Asn Gly Gly
   4765 4770 4775
   aac aat tgc act acg gct gtt atg att tat tct aat ttt ggt att 22639
   Asn Asn Cys Thr Thr Ala Val Met Ile Tyr Ser Asn Phe Gly Ile
   4780 4785 4790
   tgt gct gat ggt tct tta att cct gtt cgt ccg cgt aat tct agt 22684
   Cys Ala Asp Gly Ser Leu Ile Pro Val Arg Pro Arg Asn Ser Ser
   4795 4800 4805
   gat aat ggt att tca gcc ata atc act gct aat tta tcc att ccc 22729
   Asp Asn Gly Ile Ser Ala Ile Ile Thr Ala Asn Leu Ser Ile Pro
   4810 4815 4820
   tct aac tgg act act tca gtt caa gtt gag tac ctc caa att act 22774
   Ser Asn Trp Thr Thr Ser Val Gln Val Glu Tyr Leu Gln Ile Thr
   4825 4830 4835
   agt act cca ata gtt gtt gat tgt gct act tat gtg tgt aat ggt 22819
   Ser Thr Pro Ile Val Val Asp Cys Ala Thr Tyr Val Cys Asn Gly
   4840 4845 4850
   aac cct cgt tgt aag aat cta ctt aag cag tat act tct gct tgt 22864
   Asn Pro Arg Cys Lys Asn Leu Leu Lys Gln Tyr Thr Ser Ala Cys
   4855 4860 4865
   aaa act att gaa gat gcc tta cga ctt agt gct cat ttg gaa act 22909
   Lys Thr Ile Glu Asp Ala Leu Arg Leu Ser Ala His Leu Glu Thr
   4870 4875 4880
   aat gat gtt agt agt atg cta act ttc gat agc aat gct ttt agt 22954
   Asn Asp Val Ser Ser Met Leu Thr Phe Asp Ser Asn Ala Phe Ser
   4885 4890 4895
   ttg gct aat gtt act agt ttt gga gat tat aac ctt tct agt gtt 22999
   Leu Ala Asn Val Thr Ser Phe Gly Asp Tyr Asn Leu Ser Ser Val
   4900 4905 4910
   tta cct cag aga aac att cat tca agc cgt ata gca gga cgt agt 23044
   Leu Pro Gln Arg Asn Ile His Ser Ser Arg Ile Ala Gly Arg Ser
   4915 4920 4925
   gct ttg gaa gat ttg ttg ttt agc aaa gtt gtt aca tct ggt ttg 23089
   Ala Leu Glu Asp Leu Leu Phe Ser Lys Val Val Thr Ser Gly Leu
   4930 4935 4940
   ggt act gtt gat gtt gac tat aag tct tgt act aaa ggt ctt tct 23134
   Gly Thr Val Asp Val Asp Tyr Lys Ser Cys Thr Lys Gly Leu Ser
   4945 4950 4955
   att gct gac ctt gct tgt gct cag tac tac aat ggc ata atg gtt 23179
   Ile Ala Asp Leu Ala Cys Ala Gln Tyr Tyr Asn Gly Ile Met Val
   4960 4965 4970
   ttg cca ggt gtt gct gat gct gaa cgt atg gcc atg tac aca ggt 23224
   Leu Pro Gly Val Ala Asp Ala Glu Arg Met Ala Met Tyr Thr Gly
   4975 4980 4985
   tct ctt ata ggt ggc atg gtg ctc gga ggt ctt aca tca gca gcc 23269
   Ser Leu Ile Gly Gly Met Val Leu Gly Gly Leu Thr Ser Ala Ala
   4990 4995 5000
   gcc ata cct ttt tct ttg gca ctg caa gca cga ctt aac tat gtt 23314
   Ala Ile Pro Phe Ser Leu Ala Leu Gln Ala Arg Leu Asn Tyr Val
   5005 5010 5015
   gct tta caa act gat gtg ctt caa gaa aat cag aaa att ttg gct 23359
   Ala Leu Gln Thr Asp Val Leu Gln Glu Asn Gln Lys Ile Leu Ala
   5020 5025 5030
   gca tca ttt aat aag gct att aat aat att gtt gct tct ttt agt 23404
   Ala Ser Phe Asn Lys Ala Ile Asn Asn Ile Val Ala Ser Phe Ser
   5035 5040 5045
   agc gtt aat gat gct att aca cat act gca gag gct ata cat act 23449
   Ser Val Asn Asp Ala Ile Thr His Thr Ala Glu Ala Ile His Thr
   5050 5055 5060
   gtt act att gca ctt aat aag att cag gat gtt gtt aat caa cag 23494
   Val Thr Ile Ala Leu Asn Lys Ile Gln Asp Val Val Asn Gln Gln
   5065 5070 5075
   ggt agt gct ctt aac cat ctc act tca caa ttg aga cat aat ttt 23539
   Gly Ser Ala Leu Asn His Leu Thr Ser Gln Leu Arg His Asn Phe
   5080 5085 5090
   cag gcc att tct aat tca att cat gct att tat gac cgg ctt gat 23584
   Gln Ala Ile Ser Asn Ser Ile His Ala Ile Tyr Asp Arg Leu Asp
   5095 5100 5105
   tca att caa gcc gat caa caa gtt gac aga tta att act gga cgg 23629
   Ser Ile Gln Ala Asp Gln Gln Val Asp Arg Leu Ile Thr Gly Arg
   5110 5115 5120
   ctt gca gct ttg aat gca ttt gtt tcc caa gtt ttg aat aaa tat 23674
   Leu Ala Ala Leu Asn Ala Phe Val Ser Gln Val Leu Asn Lys Tyr
   5125 5130 5135
   act gaa gtt cgt ggt tcc aga cgc tta gca cag cag aag att aat 23719
   Thr Glu Val Arg Gly Ser Arg Arg Leu Ala Gln Gln Lys Ile Asn
   5140 5145 5150
   gaa tgt gtc aag tca caa tct aat aga tat ggt ttt tgt ggc aat 23764
   Glu Cys Val Lys Ser Gln Ser Asn Arg Tyr Gly Phe Cys Gly Asn
   5155 5160 5165
   ggc act cac atc ttt tca atc gtc aac tca gct cca gat ggt ttg 23809
   Gly Thr His Ile Phe Ser Ile Val Asn Ser Ala Pro Asp Gly Leu
   5170 5175 5180
   ctt ttt ctt cat act gtt ttg ctg cca act gat tac aag aat gta 23854
   Leu Phe Leu His Thr Val Leu Leu Pro Thr Asp Tyr Lys Asn Val
   5185 5190 5195
   aag gcg tgg tct ggt atc tgt gtt gat ggc att tat ggc tat gtt 23899
   Lys Ala Trp Ser Gly Ile Cys Val Asp Gly Ile Tyr Gly Tyr Val
   5200 5205 5210
   ctg cgt caa cct aac ttg gtt ctt tat tct gat aat ggt gtc ttt 23944
   Leu Arg Gln Pro Asn Leu Val Leu Tyr Ser Asp Asn Gly Val Phe
   5215 5220 5225
   cgt gta act tcc agg gtc atg ttt caa cct cgt tta cct gtt ttg 23989
   Arg Val Thr Ser Arg Val Met Phe Gln Pro Arg Leu Pro Val Leu
   5230 5235 5240
   tct gat ttt gtg caa ata tat aat tgt aat gtt act ttt gtt aac 24034
   Ser Asp Phe Val Gln Ile Tyr Asn Cys Asn Val Thr Phe Val Asn
   5245 5250 5255
   ata tct cgt gtc gag tta cat act gtc ata cct gac tac gtt gat 24079
   Ile Ser Arg Val Glu Leu His Thr Val Ile Pro Asp Tyr Val Asp
   5260 5265 5270
   gtt aat aaa aca tta caa gag ttt gca caa aac tta cca aag tat 24124
   Val Asn Lys Thr Leu Gln Glu Phe Ala Gln Asn Leu Pro Lys Tyr
   5275 5280 5285
   gtt aag cct aat ttt gac ttg act cct ttt aat tta aca tat ctt 24169
   Val Lys Pro Asn Phe Asp Leu Thr Pro Phe Asn Leu Thr Tyr Leu
   5290 5295 5300
   aat ttg agt tct gag ttg aag caa ctc gaa gct aaa act gct agt 24214
   Asn Leu Ser Ser Glu Leu Lys Gln Leu Glu Ala Lys Thr Ala Ser
   5305 5310 5315
   ctt ttc caa act act gtt gaa tta caa ggt ctt att gat cag att 24259
   Leu Phe Gln Thr Thr Val Glu Leu Gln Gly Leu Ile Asp Gln Ile
   5320 5325 5330
   aac agt aca tat gtt gat ttg aag ttg ctt aat agg ttt gaa aat 24304
   Asn Ser Thr Tyr Val Asp Leu Lys Leu Leu Asn Arg Phe Glu Asn
   5335 5340 5345
   tat atc aaa tgg cct tgg tgg gtt tgg ctc att att tct gtt gtt 24349
   Tyr Ile Lys Trp Pro Trp Trp Val Trp Leu Ile Ile Ser Val Val
   5350 5355 5360
   ttt gtt gta ttg ttg agt ctt ctt gtg ttt tgt tgt ctt tct aca 24394
   Phe Val Val Leu Leu Ser Leu Leu Val Phe Cys Cys Leu Ser Thr
   5365 5370 5375
   ggt tgt tgt ggt tgt tgc aat tgt tta act tca tca atg cga ggc 24439
   Gly Cys Cys Gly Cys Cys Asn Cys Leu Thr Ser Ser Met Arg Gly
   5380 5385 5390
   tgt tgt gat tgt ggt tca act aaa ctt cct tat tat gaa ttt gaa 24484
   Cys Cys Asp Cys Gly Ser Thr Lys Leu Pro Tyr Tyr Glu Phe Glu
   5395 5400 5405
   aag gtc cac gtt caa taa tgcctttcgg tggcctattt caacttactc 24532
   Lys Val His Val Gln
   5410
   ttgaaagtac tattaataag agtgtggcta atctcaaatt accacctcat gatgttactg 24592
   tcttgcgtga caatcttaaa cctgttacta cacttagtac tatcactgct tatttgttag 24652
   ttagtttgtt tgtcacttat tttgctttat tcaaacctct tactgctaga ggtcgtgttg 24712
   cttgttttgt tttaaaacta ttgacactat ctgtctatgt gcctttattg gttctttttg 24772
   gtatgtatct tgacagtttt ataatttttt ttctacgctg ttgtttcgat tcatacatgt 24832
   tggctattat gcctatctct aataaaaatt tttcatttgt tttgttcaat gttactaaac 24892
   tatgcttcgt ttcaggcaag tgttggtatc ttgaacaatc attttatgaa aatcgttttg 24952
   ctgctattta tggtggtgac cactatgtcg ttttaggtgg tgaaactatt acttttgttt 25012
   cttttgatga cctttatgtt gctattagag gttcttgtga aaagaaccta caacttatgc 25072
   gtaaggttga cttgtataat ggtgctgtca tttacatttt tgccgaagag cctgttgttg 25132
   gtatagttta ctcctctcaa ctatacgaag atg ttc ctt cga tta att gat gac 25186
   Met Phe Leu Arg Leu Ile Asp Asp
   5415
   aat ggc att gtc ctc aat tct att tta tgg ctc ctt gtt atg ata 25231
   Asn Gly Ile Val Leu Asn Ser Ile Leu Trp Leu Leu Val Met Ile
   5420 5425 5430
   ttt ttc ttt gtg ttg gca atg acc ttt att aaa ctg att caa ttg 25276
   Phe Phe Phe Val Leu Ala Met Thr Phe Ile Lys Leu Ile Gln Leu
   5435 5440 5445
   tgt ttt act tgt cat tat ttt ttt agt agg aca tta tat caa cca 25321
   Cys Phe Thr Cys His Tyr Phe Phe Ser Arg Thr Leu Tyr Gln Pro
   5450 5455 5460
   gtt tat aaa att ttt ctt gct tac caa gat tat atg caa ata gca 25366
   Val Tyr Lys Ile Phe Leu Ala Tyr Gln Asp Tyr Met Gln Ile Ala
   5465 5470 5475
   cct gtt cca gct gaa gta cta aat gtc taa actaaacg atg tct aat 25413
   Pro Val Pro Ala Glu Val Leu Asn Val Met Ser Asn
   5480 5485 5490
   agt agt gtg cct ctt tca gag gtt tat gtc cat tta cgt aac tgg 25458
   Ser Ser Val Pro Leu Ser Glu Val Tyr Val His Leu Arg Asn Trp
   5495 5500 5505
   aac ttt agt tgg aat tta att cta aca gtt ttt ata gtt gtg ttg 25503
   Asn Phe Ser Trp Asn Leu Ile Leu Thr Val Phe Ile Val Val Leu
   5510 5515 5520
   cag tat ggg cat tat aag tat agc aga ctt ctt tat ggt tta aag 25548
   Gln Tyr Gly His Tyr Lys Tyr Ser Arg Leu Leu Tyr Gly Leu Lys
   5525 5530 5535
   atg tct gtt tta tgg tgt tta tgg cca ctt gtt cta gct ttg tct 25593
   Met Ser Val Leu Trp Cys Leu Trp Pro Leu Val Leu Ala Leu Ser
   5540 5545 5550
   att ttt gac tgt ttt gtc aat ttt aat gtg gac tgg gtc ttt ttt 25638
   Ile Phe Asp Cys Phe Val Asn Phe Asn Val Asp Trp Val Phe Phe
   5555 5560 5565
   ggt ttt agt att ctt atg tct att att aca ctt tgt tta tgg gtt 25683
   Gly Phe Ser Ile Leu Met Ser Ile Ile Thr Leu Cys Leu Trp Val
   5570 5575 5580
   atg tat ttt gtt aat agt ttc aga ctt tgg cgc cgt gtt aaa act 25728
   Met Tyr Phe Val Asn Ser Phe Arg Leu Trp Arg Arg Val Lys Thr
   5585 5590 5595
   ttt tgg gct ttt aat cct gaa act aat gca atc atc tct ctc cag 25773
   Phe Trp Ala Phe Asn Pro Glu Thr Asn Ala Ile Ile Ser Leu Gln
   5600 5605 5610
   gtt tat gga cat aat tat tac tta ccg gtg atg gct gca cct aca 25818
   Val Tyr Gly His Asn Tyr Tyr Leu Pro Val Met Ala Ala Pro Thr
   5615 5620 5625
   ggt gtt aca tta aca ctt ctt agt ggt gta ctt ctt gtt gat ggc 25863
   Gly Val Thr Leu Thr Leu Leu Ser Gly Val Leu Leu Val Asp Gly
   5630 5635 5640
   cat aag att gct act cgt gtt caa gtg ggt cag ttg cct aaa tat 25908
   His Lys Ile Ala Thr Arg Val Gln Val Gly Gln Leu Pro Lys Tyr
   5645 5650 5655
   gta ata gtt gct aca cct agt acc aca att gtt tgt gac cgt gtt 25953
   Val Ile Val Ala Thr Pro Ser Thr Thr Ile Val Cys Asp Arg Val
   5660 5665 5670
   ggt cgc tct gtt aat gaa aca agc cag act ggt tgg gca ttc tac 25998
   Gly Arg Ser Val Asn Glu Thr Ser Gln Thr Gly Trp Ala Phe Tyr
   5675 5680 5685
   gtc cgt gct aaa cat ggt gat ttt tct ggt gtt gcc tct cag gag 26043
   Val Arg Ala Lys His Gly Asp Phe Ser Gly Val Ala Ser Gln Glu
   5690 5695 5700
   ggt gtt ttg tca gaa aga gag aag ttg ctt cat tta atc taa 26085
   Gly Val Leu Ser Glu Arg Glu Lys Leu Leu His Leu Ile
   5705 5710
   actaaacaaa atg gct agt gta aat tgg gcc gat gac aga gct gct agg 26134
   Met Ala Ser Val Asn Trp Ala Asp Asp Arg Ala Ala Arg
   5715 5720 5725
   aag aaa ttt cct cct cct tca ttt tac atg cct ctt ttg gtt agt 26179
   Lys Lys Phe Pro Pro Pro Ser Phe Tyr Met Pro Leu Leu Val Ser
   5730 5735 5740
   tct gat aag gca cca tat agg gtc att ccc agg aat ctt gtc cct 26224
   Ser Asp Lys Ala Pro Tyr Arg Val Ile Pro Arg Asn Leu Val Pro
   5745 5750 5755
   att ggt aag ggt aat aaa gat gag cag att ggt tat tgg aat gtt 26269
   Ile Gly Lys Gly Asn Lys Asp Glu Gln Ile Gly Tyr Trp Asn Val
   5760 5765 5770
   caa gag cgt tgg cgt atg cgc agg ggg caa cgt gtt gat ttg cct 26314
   Gln Glu Arg Trp Arg Met Arg Arg Gly Gln Arg Val Asp Leu Pro
   5775 5780 5785
   cct aaa gtt cat ttt tat tac cta ggt act gga cct cat aag gac 26359
   Pro Lys Val His Phe Tyr Tyr Leu Gly Thr Gly Pro His Lys Asp
   5790 5795 5800
   ctt aaa ttc aga caa cgt tct gat ggt gtt gtt tgg gtt gct aag 26404
   Leu Lys Phe Arg Gln Arg Ser Asp Gly Val Val Trp Val Ala Lys
   5805 5810 5815
   gaa ggt gct aaa act gtt aat acc agt ctt ggt aat cgc aaa cgt 26449
   Glu Gly Ala Lys Thr Val Asn Thr Ser Leu Gly Asn Arg Lys Arg
   5820 5825 5830
   aat cag aaa cct ttg gaa cca aag ttc tct att gct ttg cct cca 26494
   Asn Gln Lys Pro Leu Glu Pro Lys Phe Ser Ile Ala Leu Pro Pro
   5835 5840 5845
   gag ctc tct gtt gtt gag ttt gag gat cgc tct aat aac tca tct 26539
   Glu Leu Ser Val Val Glu Phe Glu Asp Arg Ser Asn Asn Ser Ser
   5850 5855 5860
   cgt gct agc agt cgt tct tca act cgt aac aac tca cga gac tct 26584
   Arg Ala Ser Ser Arg Ser Ser Thr Arg Asn Asn Ser Arg Asp Ser
   5865 5870 5875
   tct cgt agt act tca aga caa cag tct cgc act cgt tct gat tct 26629
   Ser Arg Ser Thr Ser Arg Gln Gln Ser Arg Thr Arg Ser Asp Ser
   5880 5885 5890
   aac cag tct tct tca gat ctt gtt gct gct gtt act ttg gct tta 26674
   Asn Gln Ser Ser Ser Asp Leu Val Ala Ala Val Thr Leu Ala Leu
   5895 5900 5905
   aag aac tta ggt ttt gat aac cag tcg aag tca cct agt tct tct 26719
   Lys Asn Leu Gly Phe Asp Asn Gln Ser Lys Ser Pro Ser Ser Ser
   5910 5915 5920
   ggt act tcc act cct aag aaa cct aat aag cct ctt tct caa ccc 26764
   Gly Thr Ser Thr Pro Lys Lys Pro Asn Lys Pro Leu Ser Gln Pro
   5925 5930 5935
   agg gct gat aag cct tct cag ttg aag aaa cct cgt tgg aag cgt 26809
   Arg Ala Asp Lys Pro Ser Gln Leu Lys Lys Pro Arg Trp Lys Arg
   5940 5945 5950
   gtt cct acc aga gag gaa aat gtt att cag tgc ttt ggt cct cgt 26854
   Val Pro Thr Arg Glu Glu Asn Val Ile Gln Cys Phe Gly Pro Arg
   5955 5960 5965
   gat ttt aat cac aat atg ggg gat tca gat ctt gtt cag aat ggt 26899
   Asp Phe Asn His Asn Met Gly Asp Ser Asp Leu Val Gln Asn Gly
   5970 5975 5980
   gtt gat gcc aag ggt ttt cca cag ctt gct gaa ttg att cct aat 26944
   Val Asp Ala Lys Gly Phe Pro Gln Leu Ala Glu Leu Ile Pro Asn
   5985 5990 5995
   cag gct gcg tta ttc ttt gat agt gag gtt agc act gat gaa gtg 26989
   Gln Ala Ala Leu Phe Phe Asp Ser Glu Val Ser Thr Asp Glu Val
   6000 6005 6010
   ggt gat aat gtt cag att acc tac acc tac aaa atg ctt gta gct 27034
   Gly Asp Asn Val Gln Ile Thr Tyr Thr Tyr Lys Met Leu Val Ala
   6015 6020 6025
   aag gat aat aag aac ctt cct aag ttc att gag cag att agt gct 27079
   Lys Asp Asn Lys Asn Leu Pro Lys Phe Ile Glu Gln Ile Ser Ala
   6030 6035 6040
   ttt act aaa ccc agt tct atc aaa gaa atg cag tca caa tca tct 27124
   Phe Thr Lys Pro Ser Ser Ile Lys Glu Met Gln Ser Gln Ser Ser
   6045 6050 6055
   cat gtt gct cag aac aca gta ctt aat gct tct att cca gaa tct 27169
   His Val Ala Gln Asn Thr Val Leu Asn Ala Ser Ile Pro Glu Ser
   6060 6065 6070
   aaa cca ttg gct gat gat gat tca gcc att ata gaa att gtc aac 27214
   Lys Pro Leu Ala Asp Asp Asp Ser Ala Ile Ile Glu Ile Val Asn
   6075 6080 6085
   gag gtt ttg cat taa attgttttgt aattccagtt gaatgtttat tattattagt 27269
   Glu Val Leu His
   6090
   tgcaacccca tgcgtttagc gcatgataag ggtttagtct tacacacaat ggtaggccag 27329
   tgatagtaaa gtgtaagtaa tttgctatca tattaacatg tctagaggaa agtcagaact 27389
   ttttctgttt gtgttgttgg agtacttaaa gatcgcatag gcgcgccaac aatggaagag 27449
   ccaacaacat atctaaaaat gttttgtctg gtacttgtta atgatattgt ttttgatatg 27509
   gatacacaaa aaaaaaaaaa aaa 27532

<210> 2
<211> 4055
<212> PRT
<213> EMCR Coronavirus

<400> 2

Met Phe Tyr Asn Gln Val Thr Leu Ala Val Ala Ser Asp Ser Glu Ile
1               5                   10                  15

Ser Gly Phe Gly Phe Ala Ile Pro Ser Val Ala Val Arg Ala Tyr Ser
            20                  25                  30

Glu Ala Ala Ala Gln Gly Phe Gln Ala Cys Arg Phe Val Ala Phe Gly
        35                  40                  45

Leu Gln Asp Cys Val Thr Gly Ile Asn Asp Asp Asp Tyr Val Ile Ala
    50                  55                  60

Leu Thr Gly Thr Asn Gln Leu Cys Ala Lys Ile Leu Leu Phe Ser Asp
65                  70                  75                  80

Arg Pro Leu Asn Leu Arg Gly Trp Leu Ile Phe Ser Asn Ser Asn Tyr
                85                  90                  95

Val Leu Gln Asp Phe Asp Val Val Phe Gly His Gly Ala Gly Ser Val
            100                 105                 110

Val Phe Val Asp Lys Tyr Met Cys Gly Phe Asp Gly Lys Pro Val Leu
        115                 120                 125

Pro Lys Asn Met Trp Glu Phe Arg Asp Tyr Phe Asn Asp Asn Thr Asp
    130                 135                 140

Ser Ile Val Ile Gly Gly Val Thr Tyr Gln Leu Ala Trp Asp Val Ile
145                 150                 155                 160

Arg Lys Asp Leu Ser Tyr Glu Gln Gln Asn Val Leu Ala Ile Glu Ser
                165                 170                 175

Ile His Tyr Leu Gly Thr Thr Gly His Thr Leu Lys Ser Gly Cys Lys
            180                 185                 190

Leu Ile Asn Ala Lys Pro Pro Lys Tyr Ser Ser Lys Val Val Leu Ser

P67316EP00.ST25
        195                     200                     205

Gly Glu Trp Asn Ala Val Tyr Lys Ala Phe Gly Ser Pro Phe Ile Thr
    210                     215                     220

Asn Gly Ile Ser Leu Leu Asp Ile Ile Val Lys Pro Val Phe Phe Asn
225                     230                     235                     240

Ala Phe Val Lys Cys Asn Cys Gly Ser Glu Asn Trp Ser Val Gly Ala
                245                     250                     255

Trp Asp Gly Tyr Leu Ser Ser Cys Cys Gly Thr Pro Ala Lys Lys Leu
            260                     265                     270

Cys Val Val Pro Gly Asn Val Val Pro Gly Asp Val Ile Ile Thr Ser
        275                     280                     285

Thr Asp Ala Gly Cys Gly Val Lys Tyr Tyr Ala Gly Leu Val Val Lys
    290                     295                     300

His Ile Thr Asn Ile Thr Gly Val Ser Leu Trp Arg Val Thr Ala Val
305                     310                     315                     320

His Ser Asp Gly Met Phe Val Ala Thr Ser Ser Tyr Asp Ala Leu Leu
                325                     330                     335

His Arg Asn Ser Leu Asp Pro Phe Cys Phe Asp Val Asn Thr Leu Leu
            340                     345                     350

Ser Asn Gln Leu Arg Leu Ala Phe Leu Gly Ala Ser Val Thr Glu Asp
        355                     360                     365

Val Lys Phe Ala Ala Ser Thr Gly Val Ile Asp Ile Ser Ala Gly Met
    370                     375                     380

Phe Gly Leu Tyr Asp Asp Ile Leu Thr Asn Asn Lys Pro Trp Phe Val
385                     390                     395                     400

Arg Lys Ala Ser Gly Leu Phe Asp Ala Ile Trp Asp Ala Phe Val Ala
                405                     410                     415

Ala Ile Lys Leu Val Pro Thr Thr Thr Gly Gly Leu Val Arg Phe Val
            420                     425                     430

Lys Ser Ile Ala Ser Thr Val Leu Thr Val Ser Asn Gly Val Ile Ile
        435                     440                     445

Met Cys Ala Asp Val Pro Asp Ala Phe Gln Pro Val Tyr Arg Thr Phe
    450                     455                     460

Thr Gln Ala Ile Cys Ala Ala Phe Asp Phe Ser Leu Asp Val Phe Lys

465                 470                 475                 480

Ile Gly Asp Val Lys Phe Lys Arg Leu Gly Asp Tyr Val Leu Thr Glu
                485                 490                 495

Asn Ala Leu Val Arg Leu Thr Thr Glu Val Val Arg Gly Val Arg Asp
            500                 505                 510

Ala Arg Ile Lys Lys Ala Met Phe Thr Lys Val Val Val Gly Pro Thr
        515                 520                 525

Thr Glu Val Lys Phe Ser Val Ile Glu Leu Ala Thr Val Asn Leu Arg
    530                 535                 540

Leu Val Asp Cys Ala Pro Val Val Cys Pro Lys Gly Lys Ile Val Val
545                 550                 555                 560

Ile Ala Gly Gln Ala Phe Phe Tyr Ser Gly Gly Phe Tyr Arg Phe Met
                565                 570                 575

Val Asp Ser Thr Thr Val Leu Asn Asp Pro Val Phe Thr Gly Glu Leu
            580                 585                 590

Phe Tyr Thr Ile Lys Phe Ser Gly Phe Lys Leu Asp Gly Phe Asn His
        595                 600                 605

Gln Phe Val Asn Ala Ser Ser Ala Thr Asp Ala Ile Ile Ala Val Glu
    610                 615                 620

Leu Leu Leu Ser Asp Phe Lys Thr Ala Val Phe Val Tyr Thr Cys Val
625                 630                 635                 640

Val Asp Gly Cys Ser Val Ile Val Arg Arg Asp Ala Thr Phe Ala Thr
                645                 650                 655

His Val Cys Phe Lys Asp Cys Tyr Ser Ile Trp Glu Gln Phe Cys Ile
            660                 665                 670

Asp Asn Cys Gly Glu Pro Trp Phe Leu Thr Asp Tyr Asn Ala Ile Leu
        675                 680                 685

Gln Ser Asn Asn Pro Gln Cys Ala Ile Val Gln Ala Ser Glu Ser Lys
    690                 695                 700

Val Leu Leu Glu Arg Phe Leu Pro Lys Cys Pro Glu Ile Leu Leu Ser
705                 710                 715                 720

Ile Asp Asp Gly His Leu Trp Asn Leu Phe Val Glu Lys Phe Asn Phe
                725                 730                 735

Val Thr Asp Trp Leu Lys Thr Leu Lys Leu Thr Leu Thr Ser Asn Gly

740 745 750

Leu Leu Gly Asn Cys Ala Lys Arg Phe Arg Arg Val Leu Val Lys Leu
755 760 765

Leu Asp Val Tyr Asn Gly Phe Leu Glu Thr Val Cys Ser Val Val His
770 775 780

Thr Ala Gly Val Cys Ile Lys Tyr Tyr Ala Val Asn Val Pro Tyr Val
785 790 795 800

Val Ile Ser Gly Phe Val Ser Arg Val Ile Arg Arg Glu Arg Cys Asp
805 810 815

Val Thr Phe Pro Cys Val Ser Cys Val Thr Phe Phe Tyr Glu Phe Leu
820 825 830

Asp Thr Cys Phe Gly Val Ser Lys Pro Asn Ala Ile Asp Val Glu His
835 840 845

Leu Glu Leu Lys Glu Thr Val Phe Val Glu Pro Lys Asp Gly Gly Gln
850 855 860

Phe Phe Val Ser Asp Asp Tyr Leu Trp Tyr Val Val Asp Asp Ile Tyr
865 870 875 880

Tyr Pro Ala Ser Cys Asn Gly Val Leu Pro Val Ala Phe Thr Lys Leu
885 890 895

Ala Gly Gly Lys Ile Ser Phe Ser Asp Asp Val Ile Val His Asp Val
900 905 910

Glu Pro Thr His Lys Val Lys Leu Ile Phe Glu Phe Glu Asp Asp Val
915 920 925

Val Thr Ser Leu Cys Lys Lys Ser Phe Gly Lys Ser Ile Ile Tyr Thr
930 935 940

Gly Asp Trp Glu Gly Leu His Glu Val Leu Thr Ser Ala Met Asn Val
945 950 955 960

Ile Gly Gln His Ile Lys Leu Pro Gln Phe Tyr Ile Tyr Asp Glu Glu
965 970 975

Gly Gly Tyr Asp Val Ser Lys Pro Val Met Ile Ser Gln Trp Pro Ile
980 985 990

Ser Asp Asp Ser Asp Gly Cys Val Val Glu Ala Ser Thr Asp Phe His
995 1000 1005

Gln Leu Glu Ser Val Arg Glu Glu Val Asp Ile Ile Glu Gln Pro

1010                    1015                    1020

Phe Gly Glu Val Glu His Ala Leu Ser Ile Arg Gln Pro Phe Ser
    1025                    1030                    1035

Phe Ser Phe Arg Asp Glu Leu Gly Val Arg Val Leu Asp Gln Ser
    1040                    1045                    1050

Asp Asn Asn Cys Trp Ile Ser Thr Thr Leu Ile Gln Leu Gln Leu
    1055                    1060                    1065

Thr Lys Leu Leu Asp Asp Ser Ile Glu Met Gln Leu Phe Lys Val
    1070                    1075                    1080

Gly Lys Val Asp Ser Ile Val Gln Lys Cys Tyr Glu Leu Ser His
    1085                    1090                    1095

Leu Ile Ser Gly Ser Leu Gly Asp Ser Gly Lys Leu Leu Ser Glu
    1100                    1105                    1110

Leu Leu Lys Asp Lys Tyr Thr Cys Ser Ile Thr Phe Glu Met Ser
    1115                    1120                    1125

Cys Asp Cys Gly Lys Lys Phe Asp Glu Gln Val Gly Cys Leu Phe
    1130                    1135                    1140

Trp Ile Met Pro Tyr Thr Lys Leu Phe Gln Lys Gly Glu Cys Cys
    1145                    1150                    1155

Ile Cys His Lys Met Gln Thr Tyr Lys Leu Val Ser Met Lys Gly
    1160                    1165                    1170

Thr Gly Val Phe Val Gln Asp Pro Ala Pro Ile Asp Ile Asp Ala
    1175                    1180                    1185

Phe Pro Val Arg Pro Ile Cys Ser Ser Val Tyr Leu Gly Val Lys
    1190                    1195                    1200

Gly Ser Gly His Tyr Gln Thr Asn Leu Tyr Ser Phe Asp Lys Ala
    1205                    1210                    1215

Ile Asp Gly Phe Gly Val Phe Asp Ile Lys Asn Ser Ser Val Asn
    1220                    1225                    1230

Thr Val Cys Phe Val Asp Val Asp Phe His Ser Val Glu Ile Glu
    1235                    1240                    1245

Ala Gly Glu Val Lys Pro Phe Ala Val Tyr Lys Asn Val Lys Phe
    1250                    1255                    1260

Tyr Leu Gly Asp Ile Ser His Leu Val Asn Cys Val Ser Phe Asp

1265                 1270                1275

Phe Val Val Asn Ala Ala Asn Glu Asn Leu Met His Gly Gly Gly
    1280                1285                1290

Val Ala Arg Ala Ile Asp Ile Leu Thr Glu Gly Gln Leu Gln Ser
    1295                1300                1305

Leu Ser Lys Asp Tyr Ile Ser Ser Asn Gly Pro Leu Lys Val Gly
    1310                1315                1320

Ala Gly Val Met Leu Glu Cys Glu Lys Phe Asn Val Phe Asn Val
    1325                1330                1335

Val Gly Pro Arg Thr Gly Lys His Glu His Ser Leu Leu Val Glu
    1340                1345                1350

Ala Tyr Asn Ser Ile Leu Phe Glu Asn Gly Ile Pro Leu Met Pro
    1355                1360                1365

Leu Leu Ser Cys Gly Ile Phe Gly Val Arg Ile Glu Asn Ser Leu
    1370                1375                1380

Lys Ala Leu Phe Ser Cys Asp Ile Asn Lys Pro Leu Gln Val Phe
    1385                1390                1395

Val Tyr Ser Ser Asn Glu Glu Gln Ala Val Leu Lys Phe Leu Asp
    1400                1405                1410

Gly Leu Asp Leu Thr Pro Val Ile Asp Asp Val Asp Val Val Lys
    1415                1420                1425

Pro Phe Arg Val Glu Gly Asn Phe Ser Phe Phe Asp Cys Gly Val
    1430                1435                1440

Asn Ala Leu Asp Gly Asp Ile Tyr Leu Leu Phe Thr Asn Ser Ile
    1445                1450                1455

Leu Met Leu Asp Lys Gln Gly Gln Leu Leu Asp Thr Lys Leu Asn
    1460                1465                1470

Gly Ile Leu Gln Gln Ala Val Leu Asp Tyr Leu Ala Thr Val Lys
    1475                1480                1485

Thr Val Pro Ala Gly Asn Leu Val Lys Leu Val Val Glu Ser Cys
    1490                1495                1500

Thr Ile Tyr Met Cys Val Val Pro Ser Ile Asn Asp Leu Ser Phe
    1505                1510                1515

Asp Lys Asn Leu Gly Arg Cys Val Arg Lys Leu Asn Arg Leu Lys

1520                    1525                    1530

Thr Cys  Val Ile Ala Asn Val  Pro Ala Ile Asp Val  Leu Lys Lys
    1535                    1540                    1545

Leu Leu  Ser Ser Leu Thr Leu  Thr Val Lys Phe Val  Val Glu Ser
    1550                    1555                    1560

Asn Val  Met Asp Val Asn Asp  Cys Phe Lys Asn Asp  Asn Val Val
    1565                    1570                    1575

Leu Lys  Ile Thr Glu Asp Gly  Ile Asn Val Lys Asp  Val Val Val
    1580                    1585                    1590

Glu Ser  Ser Lys Ser Leu Gly  Lys Gln Leu Gly Val  Val Ser Asp
    1595                    1600                    1605

Gly Val  Asp Ser Phe Glu Gly  Val Leu Pro Ile Asn  Thr Asp Thr
    1610                    1615                    1620

Val Leu  Ser Val Ala Pro Glu  Val Asp Trp Val Ala  Phe Tyr Gly
    1625                    1630                    1635

Phe Glu  Lys Ala Ala Leu Phe  Ala Ser Leu Asp Val  Lys Pro Tyr
    1640                    1645                    1650

Gly Tyr  Pro Asn Asp Phe Val  Gly Gly Phe Arg Val  Leu Gly Thr
    1655                    1660                    1665

Thr Asp  Asn Asn Cys Trp Val  Asn Ala Thr Cys Ile  Ile Leu Gln
    1670                    1675                    1680

Tyr Leu  Lys Pro Thr Phe Lys  Ser Lys Gly Leu Asn  Val Leu Trp
    1685                    1690                    1695

Asn Lys  Phe Val Thr Gly Asp  Val Gly Pro Phe Val  Ser Phe Ile
    1700                    1705                    1710

Tyr Phe  Ile Thr Met Ser Ser  Lys Gly Gln Lys Gly  Asp Ala Glu
    1715                    1720                    1725

Glu Ala  Leu Ser Lys Leu Ser  Glu Tyr Leu Ile Ser  Asp Ser Ile
    1730                    1735                    1740

Val Thr  Leu Glu Gln Tyr Ser  Thr Cys Asp Ile Cys  Lys Ser Thr
    1745                    1750                    1755

Val Val  Glu Val Lys Ser Ala  Val Val Cys Ala Ser  Val Leu Lys
    1760                    1765                    1770

Asp Gly  Cys Asp Val Gly Phe  Cys Pro His Arg His  Lys Leu Arg

1775                 1780                 1785

Ser Arg  Val Lys Phe Val Asn  Gly Arg Val Val Ile  Thr Asn Val
    1790                 1795                 1800

Gly Glu  Pro Ile Ile Ser Gln  Pro Ser Lys Leu Leu  Asn Gly Ile
    1805                 1810                 1815

Ala Tyr  Thr Thr Phe Ser Gly  Ser Phe Asp Asn Gly  His Tyr Val
    1820                 1825                 1830

Val Tyr  Asp Ala Ala Asn Asn  Ala Val Tyr Asp Gly  Ala Arg Leu
    1835                 1840                 1845

Phe Ala  Ser Asp Leu Ser Thr  Leu Ala Val Thr Ala  Ile Val Val
    1850                 1855                 1860

Val Gly  Gly Cys Val Thr Ser  Asn Val Pro Pro Ile  Val Ser Glu
    1865                 1870                 1875

Lys Ile  Ser Val Met Asp Lys  Leu Asp Thr Gly Ala  Gln Lys Phe
    1880                 1885                 1890

Phe Gln  Phe Gly Asp Phe Val  Met Asn Asn Ile Val  Leu Phe Leu
    1895                 1900                 1905

Thr Trp  Leu Leu Ser Met Phe  Ser Leu Leu Arg Thr  Ser Ile Met
    1910                 1915                 1920

Lys His  Asp Ile Lys Val Ile  Ala Lys Ala Pro Lys  Arg Thr Gly
    1925                 1930                 1935

Val Ile  Leu Thr Arg Ser Phe  Lys Tyr Asn Ile Arg  Ser Ala Leu
    1940                 1945                 1950

Phe Val  Val Lys Gln Lys Trp  Cys Val Ile Val Thr  Leu Phe Lys
    1955                 1960                 1965

Phe Leu  Leu Leu Leu Tyr Ala  Ile Tyr Ala Leu Val  Phe Met Ile
    1970                 1975                 1980

Val Gln  Phe Ser Pro Phe Asn  Ser Leu Leu Cys Gly  Asp Ile Val
    1985                 1990                 1995

Ser Gly  Tyr Glu Lys Ser Thr  Phe Asn Lys Asp Ile  Tyr Cys Gly
    2000                 2005                 2010

Asn Ser  Met Val Cys Lys Met  Cys Leu Phe Ser Tyr  Gln Glu Phe
    2015                 2020                 2025

Asn Asp  Leu Asp His Thr Ser  Leu Val Trp Lys His  Ile Arg Asp

2030                    2035                    2040

Pro Ile Leu Ile Ser Leu Gln Pro Phe Val Ile Leu Val Ile Leu
    2045                    2050                    2055

Leu Ile Phe Gly Asn Met Tyr Leu Arg Phe Gly Leu Leu Tyr Phe
    2060                    2065                    2070

Val Ala Gln Phe Ile Ser Thr Phe Gly Ser Phe Leu Gly Phe His
    2075                    2080                    2085

Gln Lys Gln Trp Phe Leu His Phe Val Pro Phe Asp Val Leu Cys
    2090                    2095                    2100

Asn Glu Phe Leu Ala Thr Phe Ile Val Cys Lys Ile Val Leu Phe
    2105                    2110                    2115

Val Arg His Ile Ile Val Gly Cys Asn Asn Ala Asp Cys Val Ala
    2120                    2125                    2130

Cys Ser Lys Ser Ala Arg Leu Lys Arg Val Pro Leu Gln Thr Ile
    2135                    2140                    2145

Ile Asn Gly Met His Lys Ser Phe Tyr Val Asn Ala Asn Gly Gly
    2150                    2155                    2160

Thr Cys Phe Cys Asn Lys His Asn Phe Phe Cys Val Asn Cys Asp
    2165                    2170                    2175

Ser Phe Gly Pro Gly Asn Thr Phe Ile Asn Gly Asp Ile Ala Arg
    2180                    2185                    2190

Glu Leu Gly Asn Val Val Lys Thr Ala Val Gln Pro Thr Ala Pro
    2195                    2200                    2205

Ala Tyr Val Ile Ile Asp Lys Val Asp Phe Val Asn Gly Phe Tyr
    2210                    2215                    2220

Arg Leu Tyr Ser Gly Asp Thr Phe Trp Arg Tyr Asp Phe Asp Ile
    2225                    2230                    2235

Thr Glu Ser Lys Tyr Ser Cys Lys Glu Val Leu Lys Asn Cys Asn
    2240                    2245                    2250

Val Leu Glu Asn Phe Ile Val Tyr Asn Asn Ser Gly Ser Asn Ile
    2255                    2260                    2265

Thr Gln Ile Lys Asn Ala Cys Val Tyr Phe Ser Gln Leu Leu Cys
    2270                    2275                    2280

Glu Pro Ile Lys Leu Val Asn Ser Glu Leu Leu Ser Thr Leu Ser

2285                 2290                 2295

Val Asp Phe Asn Gly Val Leu His Lys Ala Tyr Val Asp Val Leu
    2300                2305                2310

Cys Asn Ser Phe Phe Lys Glu Leu Thr Ala Asn Met Ser Met Ala
    2315                2320                2325

Glu Cys Lys Ala Thr Leu Gly Leu Thr Val Ser Asp Asp Asp Phe
    2330                2335                2340

Val Ser Ala Val Ala Asn Ala His Arg Tyr Asp Val Leu Leu Ser
    2345                2350                2355

Asp Leu Ser Phe Asn Asn Phe Phe Ile Ser Tyr Ala Lys Pro Glu
    2360                2365                2370

Asp Lys Leu Ser Val Tyr Asp Ile Ala Cys Cys Met Arg Ala Gly
    2375                2380                2385

Ser Lys Val Val Asn His Asn Val Leu Ile Lys Glu Ser Ile Pro
    2390                2395                2400

Ile Val Trp Gly Val Lys Asp Phe Asn Thr Leu Ser Gln Glu Gly
    2405                2410                2415

Lys Lys Tyr Leu Val Lys Thr Thr Lys Ala Lys Gly Leu Thr Phe
    2420                2425                2430

Leu Leu Thr Phe Asn Asp Asn Gln Ala Ile Thr Gln Val Pro Ala
    2435                2440                2445

Thr Ser Ile Val Ala Lys Gln Gly Ala Gly Phe Lys Arg Thr Tyr
    2450                2455                2460

Asn Phe Leu Trp Tyr Val Cys Leu Phe Val Val Ala Leu Phe Ile
    2465                2470                2475

Gly Val Ser Phe Ile Asp Tyr Thr Thr Thr Val Thr Ser Phe His
    2480                2485                2490

Gly Tyr Asp Phe Lys Tyr Ile Glu Asn Gly Gln Leu Lys Val Phe
    2495                2500                2505

Glu Ala Pro Leu His Cys Val Arg Asn Val Phe Asp Asn Phe Asn
    2510                2515                2520

Gln Trp His Glu Ala Lys Phe Gly Val Val Thr Thr Asn Ser Asp
    2525                2530                2535

Lys Cys Pro Ile Val Val Gly Val Ser Glu Arg Ile Asn Val Val

2540                    2545                    2550

Pro Gly Val Pro Thr Asn Val Tyr Leu Val Gly Lys Thr Leu Val
    2555                    2560                    2565

Phe Thr Leu Gln Ala Ala Phe Gly Asn Thr Gly Val Cys Tyr Asp
    2570                    2575                    2580

Phe Asp Gly Val Thr Thr Ser Asp Lys Cys Ile Phe Asn Ser Ala
    2585                    2590                    2595

Cys Thr Arg Leu Glu Gly Leu Gly Gly Asp Asn Val Tyr Cys Tyr
    2600                    2605                    2610

Asn Thr Asp Leu Ile Glu Gly Ser Lys Pro Tyr Ser Ile Leu Gln
    2615                    2620                    2625

Pro Asn Ala Tyr Tyr Lys Tyr Asp Val Lys Asn Tyr Val Arg Phe
    2630                    2635                    2640

Pro Glu Ile Leu Ala Arg Gly Phe Gly Leu Arg Thr Ile Arg Thr
    2645                    2650                    2655

Leu Ala Thr Arg Tyr Cys Arg Val Gly Glu Cys Arg Asp Ser His
    2660                    2665                    2670

Lys Gly Val Cys Phe Gly Phe Asp Lys Trp Tyr Val Asn Asp Gly
    2675                    2680                    2685

Arg Val Asp Asp Gly Tyr Ile Cys Gly Asp Gly Leu Ile Asp Leu
    2690                    2695                    2700

Leu Val Asn Val Leu Ser Ile Phe Ser Ser Ser Phe Ser Val Val
    2705                    2710                    2715

Ala Met Ser Gly His Met Leu Phe Asn Phe Leu Phe Ala Ala Phe
    2720                    2725                    2730

Ile Thr Phe Leu Cys Phe Leu Val Thr Lys Phe Lys Arg Val Phe
    2735                    2740                    2745

Gly Asp Leu Ser Tyr Gly Val Phe Thr Val Val Cys Ala Thr Leu
    2750                    2755                    2760

Ile Asn Asn Ile Ser Tyr Val Val Thr Gln Asn Leu Phe Phe Met
    2765                    2770                    2775

Leu Leu Tyr Ala Ile Leu Tyr Phe Val Phe Thr Arg Thr Val Arg
    2780                    2785                    2790

Tyr Ala Trp Ile Trp His Ile Ala Tyr Ile Val Ala Tyr Phe Leu

2795                    2800                    2805

Leu Ile  Pro Trp Trp Leu Leu  Thr Trp Phe Ser Phe  Ala Ala Phe
    2810                    2815                    2820

Leu Glu  Leu Leu Pro Asn Val  Phe Lys Leu Lys Ile  Ser Thr Gln
    2825                    2830                    2835

Leu Phe  Glu Gly Asp Lys Phe  Ile Gly Thr Phe Glu  Ser Ala Ala
    2840                    2845                    2850

Ala Gly  Thr Phe Val Leu Asp  Met Arg Ser Tyr Glu  Arg Leu Ile
    2855                    2860                    2865

Asn Thr  Ile Ser Pro Glu Lys  Leu Lys Asn Tyr Ala  Ala Ser Tyr
    2870                    2875                    2880

Asn Lys  Tyr Lys Tyr Tyr Ser  Gly Ser Ala Ser Glu  Ala Asp Tyr
    2885                    2890                    2895

Arg Cys  Ala Cys Tyr Ala His  Leu Ala Lys Ala Met  Leu Asp Tyr
    2900                    2905                    2910

Ala Lys  Asp His Asn Asp Met  Leu Tyr Ser Pro Pro  Thr Ile Ser
    2915                    2920                    2925

Tyr Asn  Ser Thr Leu Gln Ser  Gly Leu Lys Lys Met  Ala Gln Pro
    2930                    2935                    2940

Ser Gly  Cys Val Glu Arg Cys  Val Val Arg Val Cys  Tyr Gly Ser
    2945                    2950                    2955

Thr Val  Leu Asn Gly Val Trp  Leu Gly Asp Thr Val  Thr Cys Pro
    2960                    2965                    2970

Arg His  Val Ile Ala Pro Ser  Thr Thr Val Leu Ile  Asp Tyr Asp
    2975                    2980                    2985

His Ala  Tyr Ser Thr Met Arg  Leu His Asn Phe Ser  Val Ser His
    2990                    2995                    3000

Asn Gly  Val Phe Leu Gly Val  Val Gly Val Thr Met  His Gly Ser
    3005                    3010                    3015

Val Leu  Arg Ile Lys Val Ser  Gln Ser Asn Val His  Thr Pro Lys
    3020                    3025                    3030

His Val  Phe Lys Thr Leu Lys  Pro Gly Ala Ser Phe  Asn Ile Leu
    3035                    3040                    3045

Ala Cys  Tyr Glu Gly Ile Ala  Ser Gly Val Phe Gly  Val Asn Leu

3050                    3055                    3060

Arg Thr  Asn Phe Thr Ile Lys  Gly Ser Phe Ile Asn  Gly Ala Cys
    3065                    3070                    3075

Gly Ser  Pro Gly Tyr Asn Val  Arg Asn Asp Gly Thr  Val Glu Phe
    3080                    3085                    3090

Cys Tyr  Leu His Gln Ile Glu  Leu Gly Ser Gly Ala  His Val Gly
    3095                    3100                    3105

Ser Asp  Phe Thr Gly Ser Val  Tyr Gly Asn Phe Asp  Asp Gln Pro
    3110                    3115                    3120

Ser Leu  Gln Val Glu Ser Ala  Asn Leu Met Leu Ser  Asp Asn Val
    3125                    3130                    3135

Val Ala  Phe Leu Tyr Ala Ala  Leu Leu Asn Gly Cys  Arg Trp Trp
    3140                    3145                    3150

Leu Arg  Ser Thr Arg Val Asn  Val Asp Gly Phe Asn  Glu Trp Ala
    3155                    3160                    3165

Met Ala  Asn Gly Tyr Thr Ile  Val Ser Ser Val Glu  Cys Tyr Ser
    3170                    3175                    3180

Ile Leu  Ala Ala Lys Thr Gly  Val Ser Val Glu Gln  Leu Leu Ala
    3185                    3190                    3195

Ser Ile  Gln His Leu His Glu  Gly Phe Gly Gly Lys  Asn Ile Leu
    3200                    3205                    3210

Gly Tyr  Ser Ser Leu Cys Asp  Glu Phe Thr Leu Ala  Glu Val Val
    3215                    3220                    3225

Lys Gln  Met Tyr Gly Val Asn  Leu Gln Ser Gly Lys  Val Ile Phe
    3230                    3235                    3240

Gly Leu  Lys Thr Met Phe Leu  Phe Ser Val Phe Phe  Thr Met Phe
    3245                    3250                    3255

Trp Ala  Glu Leu Phe Ile Tyr  Thr Asn Thr Ile Trp  Ile Asn Pro
    3260                    3265                    3270

Val Ile  Leu Thr Pro Ile Phe  Cys Leu Leu Leu Phe  Leu Ser Leu
    3275                    3280                    3285

Val Leu  Thr Met Phe Leu Lys  His Lys Phe Leu Phe  Leu Gln Val
    3290                    3295                    3300

Phe Leu  Leu Pro Thr Val Ile  Ala Thr Ala Leu Tyr  Asn Cys Val

3305                    3310                    3315

Leu Asp Tyr Tyr Ile Val Lys Phe Leu Ala Asp His Phe Asn Tyr
    3320                    3325                    3330

Asn Val Ser Val Leu Gln Met Asp Val Gln Gly Leu Val Asn Val
    3335                    3340                    3345

Leu Val Cys Leu Phe Val Val Phe Leu His Thr Trp Arg Phe Ser
    3350                    3355                    3360

Lys Glu Arg Phe Thr His Trp Phe Thr Tyr Val Cys Ser Leu Ile
    3365                    3370                    3375

Ala Val Ala Tyr Thr Tyr Phe Tyr Ser Gly Asp Phe Leu Ser Leu
    3380                    3385                    3390

Leu Val Met Phe Leu Cys Ala Ile Ser Ser Asp Trp Tyr Ile Gly
    3395                    3400                    3405

Ala Ile Val Phe Arg Leu Ser Arg Leu Ile Ile Phe Phe Ser Pro
    3410                    3415                    3420

Glu Ser Val Phe Ser Val Phe Gly Asp Val Lys Leu Thr Leu Val
    3425                    3430                    3435

Val Tyr Leu Ile Cys Gly Tyr Leu Val Cys Thr Tyr Trp Gly Ile
    3440                    3445                    3450

Leu Tyr Trp Phe Asn Arg Phe Phe Lys Cys Thr Met Gly Val Tyr
    3455                    3460                    3465

Asp Phe Lys Val Ser Ala Ala Glu Phe Lys Tyr Met Val Ala Asn
    3470                    3475                    3480

Gly Leu His Ala Pro Tyr Gly Pro Phe Asp Ala Leu Trp Leu Ser
    3485                    3490                    3495

Phe Lys Leu Leu Gly Ile Gly Gly Asp Arg Cys Ile Lys Ile Ser
    3500                    3505                    3510

Thr Val Gln Ser Lys Leu Thr Asp Leu Lys Cys Thr Asn Val Val
    3515                    3520                    3525

Leu Leu Gly Cys Leu Ser Ser Met Asn Ile Ala Ala Asn Ser Ser
    3530                    3535                    3540

Glu Trp Ala Tyr Cys Val Asp Leu His Asn Lys Ile Asn Leu Cys
    3545                    3550                    3555

Asp Asp Pro Glu Lys Ala Gln Gly Met Leu Leu Ala Leu Leu Ala

3560                    3565                    3570

Phe Phe  Leu Ser Lys His Ser  Asp Phe Gly Leu Asp  Gly Leu Ile
    3575                    3580                    3585

Asp Ser  Tyr Phe Asp Asn Ser  Ser Thr Leu Gln Ser  Val Ala Ser
    3590                    3595                    3600

Ser Phe  Val Ser Met Pro Ser  Tyr Ile Ala Tyr Glu  Asn Ala Arg
    3605                    3610                    3615

Gln Ala  Tyr Glu Asp Ala Ile  Ala Asn Gly Ser Ser  Ser Gln Leu
    3620                    3625                    3630

Ile Lys  Gln Leu Lys Arg Ala  Met Asn Ile Ala Lys  Ser Glu Phe
    3635                    3640                    3645

Asp His  Glu Ile Ser Val Gln  Lys Lys Ile Asn Arg  Met Ala Glu
    3650                    3655                    3660

Gln Ala  Ala Thr Gln Met Tyr  Lys Glu Ala Arg Ser  Val Asn Arg
    3665                    3670                    3675

Lys Ser  Lys Val Ile Ser Ala  Met His Ser Leu Leu  Phe Gly Met
    3680                    3685                    3690

Leu Arg  Arg Leu Asp Met Ser  Ser Val Glu Thr Val  Leu Asn Leu
    3695                    3700                    3705

Ala Arg  Asp Gly Val Val Pro  Leu Ser Val Ile Pro  Ala Thr Ser
    3710                    3715                    3720

Ala Ser  Lys Leu Thr Ile Val  Ser Pro Asp Leu Glu  Ser Tyr Ser
    3725                    3730                    3735

Lys Ile  Val Cys Asp Gly Ser  Val His Tyr Ala Gly  Val Val Trp
    3740                    3745                    3750

Thr Leu  Asn Asp Val Lys Asp  Asn Asp Gly Arg Pro  Val His Val
    3755                    3760                    3765

Lys Glu  Ile Thr Arg Glu Asn  Val Glu Thr Leu Thr  Trp Pro Leu
    3770                    3775                    3780

Ile Leu  Asn Cys Glu Arg Val  Val Lys Leu Gln Asn  Asn Glu Ile
    3785                    3790                    3795

Met Pro  Gly Lys Leu Lys Gln  Lys Pro Met Lys Ala  Glu Gly Asp
    3800                    3805                    3810

Gly Gly  Val Leu Gly Asp Gly  Asn Ala Leu Tyr Asn  Thr Glu Gly

3815                    3820                    3825

Gly Lys Thr Phe Met Tyr Ala Tyr Ile Ser Asn Lys Ala Asp Leu
    3830                    3835                    3840

Lys Phe Val Lys Trp Glu Tyr Glu Gly Gly Cys Asn Thr Ile Glu
    3845                    3850                    3855

Leu Asp Ser Pro Cys Arg Phe Met Val Glu Thr Pro Asn Gly Pro
    3860                    3865                    3870

Gln Val Lys Tyr Leu Tyr Phe Val Lys Asn Leu Asn Thr Leu Arg
    3875                    3880                    3885

Arg Gly Ala Val Leu Gly Phe Ile Gly Ala Thr Ile Arg Leu Gln
    3890                    3895                    3900

Ala Gly Lys Gln Thr Glu Leu Ala Val Asn Ser Gly Leu Leu Thr
    3905                    3910                    3915

Ala Cys Ala Phe Ser Val Asp Pro Ala Thr Thr Tyr Leu Glu Ala
    3920                    3925                    3930

Val Lys His Gly Ala Lys Pro Val Ser Asn Cys Ile Lys Met Leu
    3935                    3940                    3945

Ser Asn Gly Ala Gly Asn Gly Gln Ala Ile Thr Thr Ser Val Asp
    3950                    3955                    3960

Ala Asn Thr Asn Gln Asp Ser Tyr Gly Gly Ala Ser Ile Cys Leu
    3965                    3970                    3975

Tyr Cys Arg Ala His Val Pro His Pro Ser Met Asp Gly Tyr Cys
    3980                    3985                    3990

Lys Phe Lys Gly Lys Cys Val Gln Val Pro Ile Gly Cys Leu Asp
    3995                    4000                    4005

Pro Ile Arg Phe Cys Leu Glu Asn Asn Val Cys Asn Val Cys Gly
    4010                    4015                    4020

Cys Trp Leu Gly His Gly Cys Ala Cys Asp Arg Thr Thr Ile Gln
    4025                    4030                    4035

Ser Val Asp Ile Ser Tyr Leu Asn Glu Gln Gly Val Leu Val Gln
    4040                    4045                    4050

Leu Asp
    4055

<210>  3

<211>  1355

<212>  PRT

<213>  EMCR Coronavirus

<400>  3

Met Lys Leu Phe Leu Ile Leu Leu Ile Leu Pro Leu Val Ser Cys Phe
1               5                   10                  15

Ser Thr Cys Asn Ser Asn Ala Ser Ile Ser Met Leu Gln Leu Gly Val
            20                  25                  30

Pro Asp Asn Ser Ser Thr Ile Val Thr Gly Leu Leu Pro Val His Trp
        35                  40                  45

Ile Cys Ala Asn Gln Ser Thr Ser Ser Tyr Pro Ala Asn Gly Phe Phe
    50                  55                  60

Tyr Ile Asp Val Gly Lys His Arg Ser Ala Phe Ala Leu His Ser Gly
65                  70                  75                  80

Tyr Tyr Asp Ala Asn Gln Tyr Tyr Ile Tyr Leu Thr Asn Lys Ile His
                85                  90                  95

Leu Asn Ala Pro Val Thr Leu Lys Ile Cys Lys Phe Gly Asn Thr Ser
            100                 105                 110

Phe Asp Phe Leu Ser Asn Val Ser Thr Ser His Asp Cys Ile Val Asn
        115                 120                 125

Leu Ser Phe Thr Glu Gln Leu Gly Val Pro Leu Gly Ile Thr Ile Ser
    130                 135                 140

Gly Glu Thr Val Arg Leu His Leu Tyr Asn Ala Thr Arg Thr Phe Tyr
145                 150                 155                 160

Val Pro Ala Ala Tyr Lys Leu Thr Lys Leu Ser Val Lys Cys Tyr Phe
                165                 170                 175

Ser Glu Ser Cys Val Phe Ser Val Val Asn Ala Thr Ile Thr Val Asn
            180                 185                 190

Val Thr Thr Leu Asn Gly Arg Ile Val Asn Tyr Thr Val Cys Asp Asp
        195                 200                 205

Cys Asn Gly Tyr Thr Asp Asn Ile Phe Ser Val Gln Gln Asp Gly Arg
    210                 215                 220

Ile Pro Asn Gly Phe Pro Phe Asn Asn Trp Phe Leu Leu Thr Asn Gly

225                 230                 235                 240

Ser Thr Leu Val Asp Gly Val Ser Arg Leu Tyr Gln Pro Leu Arg Leu
                245                 250                 255

Thr Cys Leu Trp Pro Val Pro Gly Leu Lys Ser Ser Thr Gly Phe Val
            260                 265                 270

Tyr Phe Asn Ala Thr Gly Ser Asp Val Asn Cys Asn Gly Tyr Gln His
        275                 280                 285

Asn Ser Val Ala Asp Val Met Arg Tyr Asn Leu Asn Leu Ser Ala Asn
    290                 295                 300

Ser Val Asp Asn Leu Lys Ser Gly Val Ile Val Phe Lys Thr Leu Gln
305                 310                 315                 320

Tyr Asp Val Leu Phe Tyr Cys Ser Asn Ser Ser Ser Gly Val Leu Asp
                325                 330                 335

Thr Thr Ile Pro Phe Gly Pro Ser Ser Gln Pro Tyr Tyr Cys Phe Ile
            340                 345                 350

Asn Ser Thr Ile Asn Thr Thr His Val Ser Thr Phe Val Gly Ile Leu
        355                 360                 365

Pro Pro Thr Val Arg Glu Ile Val Val Ala Arg Thr Gly Gln Phe Tyr
    370                 375                 380

Ile Asn Gly Phe Lys Tyr Phe Asp Leu Gly Phe Ile Glu Ala Val Asn
385                 390                 395                 400

Phe Asn Val Thr Thr Ala Ser Ala Thr Asp Phe Trp Thr Val Ala Phe
                405                 410                 415

Ala Thr Phe Val Asp Val Leu Val Asn Val Ser Ala Thr Asn Ile Gln
            420                 425                 430

Asn Leu Leu Tyr Cys Asp Ser Pro Phe Glu Lys Leu Gln Cys Glu His
        435                 440                 445

Leu Gln Phe Gly Leu Gln Asp Gly Phe Tyr Ser Ala Asn Phe Leu Asp
    450                 455                 460

Asp Asn Val Leu Pro Glu Thr Tyr Val Ala Leu Pro Ile Tyr Tyr Gln
465                 470                 475                 480

His Thr Asp Ile Asn Phe Thr Ala Thr Ala Ser Phe Gly Gly Ser Cys
                485                 490                 495

Tyr Val Cys Lys Pro Arg Gln Val Asn Ile Ser Leu Asn Gly Asn Thr

            500                 505                 510

Ser Val Cys Val Arg Thr Ser His Phe Ser Ile Arg Tyr Ile Tyr Asn
        515                 520                 525

Arg Val Lys Ser Gly Ser Pro Gly Asp Ser Ser Trp His Ile Tyr Leu
    530                 535                 540

Lys Ser Gly Thr Cys Pro Phe Ser Phe Ser Lys Leu Asn Asn Phe Gln
545                 550                 555                 560

Lys Phe Lys Thr Ile Cys Phe Ser Thr Val Glu Val Pro Gly Ser Cys
                565                 570                 575

Asn Phe Pro Leu Glu Ala Thr Trp His Tyr Thr Ser Tyr Thr Ile Val
            580                 585                 590

Gly Ala Leu Tyr Val Thr Trp Ser Glu Gly Asn Ser Ile Thr Gly Val
        595                 600                 605

Pro Tyr Pro Val Ser Gly Ile Arg Glu Phe Ser Asn Leu Val Leu Asn
    610                 615                 620

Asn Cys Thr Lys Tyr Asn Ile Tyr Asp Tyr Val Gly Thr Gly Ile Ile
625                 630                 635                 640

Arg Ser Ser Asn Gln Ser Leu Ala Gly Gly Ile Thr Tyr Val Ser Asn
                645                 650                 655

Ser Gly Asn Leu Leu Gly Phe Lys Asn Val Ser Thr Gly Asn Ile Phe
            660                 665                 670

Ile Val Thr Pro Cys Asn Gln Pro Asp Gln Val Ala Val Tyr Gln Gln
        675                 680                 685

Ser Ile Ile Gly Ala Met Thr Ala Val Asn Glu Ser Arg Tyr Gly Leu
    690                 695                 700

Gln Asn Leu Leu Gln Leu Pro Asn Phe Tyr Tyr Val Ser Asn Gly Gly
705                 710                 715                 720

Asn Asn Cys Thr Thr Ala Val Met Ile Tyr Ser Asn Phe Gly Ile Cys
                725                 730                 735

Ala Asp Gly Ser Leu Ile Pro Val Arg Pro Arg Asn Ser Ser Asp Asn
            740                 745                 750

Gly Ile Ser Ala Ile Ile Thr Ala Asn Leu Ser Ile Pro Ser Asn Trp
        755                 760                 765

Thr Thr Ser Val Gln Val Glu Tyr Leu Gln Ile Thr Ser Thr Pro Ile

770                 775                 780

Val Val Asp Cys Ala Thr Tyr Val Cys Asn Gly Asn Pro Arg Cys Lys
785                 790                 795                 800

Asn Leu Leu Lys Gln Tyr Thr Ser Ala Cys Lys Thr Ile Glu Asp Ala
                805                 810                 815

Leu Arg Leu Ser Ala His Leu Glu Thr Asn Asp Val Ser Ser Met Leu
            820                 825                 830

Thr Phe Asp Ser Asn Ala Phe Ser Leu Ala Asn Val Thr Ser Phe Gly
        835                 840                 845

Asp Tyr Asn Leu Ser Ser Val Leu Pro Gln Arg Asn Ile His Ser Ser
    850                 855                 860

Arg Ile Ala Gly Arg Ser Ala Leu Glu Asp Leu Leu Phe Ser Lys Val
865                 870                 875                 880

Val Thr Ser Gly Leu Gly Thr Val Asp Val Asp Tyr Lys Ser Cys Thr
                885                 890                 895

Lys Gly Leu Ser Ile Ala Asp Leu Ala Cys Ala Gln Tyr Tyr Asn Gly
            900                 905                 910

Ile Met Val Leu Pro Gly Val Ala Asp Ala Glu Arg Met Ala Met Tyr
        915                 920                 925

Thr Gly Ser Leu Ile Gly Gly Met Val Leu Gly Gly Leu Thr Ser Ala
    930                 935                 940

Ala Ala Ile Pro Phe Ser Leu Ala Leu Gln Ala Arg Leu Asn Tyr Val
945                 950                 955                 960

Ala Leu Gln Thr Asp Val Leu Gln Glu Asn Gln Lys Ile Leu Ala Ala
                965                 970                 975

Ser Phe Asn Lys Ala Ile Asn Asn Ile Val Ala Ser Phe Ser Ser Val
            980                 985                 990

Asn Asp Ala Ile Thr His Thr Ala  Glu Ala Ile His Thr  Val Thr Ile
        995                 1000                 1005

Ala Leu  Asn Lys Ile Gln Asp  Val Val Asn Gln Gln  Gly Ser Ala
    1010                 1015                 1020

Leu Asn  His Leu Thr Ser Gln  Leu Arg His Asn Phe  Gln Ala Ile
    1025                 1030                 1035

Ser Asn  Ser Ile His Ala Ile  Tyr Asp Arg Leu Asp  Ser Ile Gln

1040                    1045                    1050

Ala Asp  Gln Gln Val Asp Arg  Leu Ile Thr Gly Arg  Leu Ala Ala
    1055                    1060                    1065

Leu Asn  Ala Phe Val Ser Gln  Val Leu Asn Lys Tyr  Thr Glu Val
    1070                    1075                    1080

Arg Gly  Ser Arg Arg Leu Ala  Gln Gln Lys Ile Asn  Glu Cys Val
    1085                    1090                    1095

Lys Ser  Gln Ser Asn Arg Tyr  Gly Phe Cys Gly Asn  Gly Thr His
    1100                    1105                    1110

Ile Phe  Ser Ile Val Asn Ser  Ala Pro Asp Gly Leu  Leu Phe Leu
    1115                    1120                    1125

His Thr  Val Leu Leu Pro Thr  Asp Tyr Lys Asn Val  Lys Ala Trp
    1130                    1135                    1140

Ser Gly  Ile Cys Val Asp Gly  Ile Tyr Gly Tyr Val  Leu Arg Gln
    1145                    1150                    1155

Pro Asn  Leu Val Leu Tyr Ser  Asp Asn Gly Val Phe  Arg Val Thr
    1160                    1165                    1170

Ser Arg  Val Met Phe Gln Pro  Arg Leu Pro Val Leu  Ser Asp Phe
    1175                    1180                    1185

Val Gln  Ile Tyr Asn Cys Asn  Val Thr Phe Val Asn  Ile Ser Arg
    1190                    1195                    1200

Val Glu  Leu His Thr Val Ile  Pro Asp Tyr Val Asp  Val Asn Lys
    1205                    1210                    1215

Thr Leu  Gln Glu Phe Ala Gln  Asn Leu Pro Lys Tyr  Val Lys Pro
    1220                    1225                    1230

Asn Phe  Asp Leu Thr Pro Phe  Asn Leu Thr Tyr Leu  Asn Leu Ser
    1235                    1240                    1245

Ser Glu  Leu Lys Gln Leu Glu  Ala Lys Thr Ala Ser  Leu Phe Gln
    1250                    1255                    1260

Thr Thr  Val Glu Leu Gln Gly  Leu Ile Asp Gln Ile  Asn Ser Thr
    1265                    1270                    1275

Tyr Val  Asp Leu Lys Leu Leu  Asn Arg Phe Glu Asn  Tyr Ile Lys
    1280                    1285                    1290

Trp Pro  Trp Trp Val Trp Leu  Ile Ile Ser Val Val  Phe Val Val

1295                    1300                    1305

Leu Leu  Ser Leu Leu Val Phe  Cys Cys Leu Ser Thr  Gly Cys Cys
    1310                    1315                    1320

Gly Cys  Cys Asn Cys Leu Thr  Ser Ser Met Arg Gly  Cys Cys Asp
    1325                    1330                    1335

Cys Gly  Ser Thr Lys Leu Pro  Tyr Tyr Glu Phe Glu  Lys Val His
    1340                    1345                    1350

Val Gln
    1355

<210>  4

<211>  77

<212>  PRT

<213>  EMCR Coronavirus

<400>  4

Met Phe Leu Arg Leu Ile Asp Asp Asn Gly Ile Val Leu Asn Ser Ile
1               5                   10                  15

Leu Trp Leu Leu Val Met Ile Phe Phe Phe Val Leu Ala Met Thr Phe
            20                  25                  30

Ile Lys Leu Ile Gln Leu Cys Phe Thr Cys His Tyr Phe Phe Ser Arg
        35                  40                  45

Thr Leu Tyr Gln Pro Val Tyr Lys Ile Phe Leu Ala Tyr Gln Asp Tyr
    50                  55                  60

Met Gln Ile Ala Pro Val Pro Ala Glu Val Leu Asn Val
65                  70                  75

<210>  5

<211>  226

<212>  PRT

<213>  EMCR Coronavirus

<400>  5

Met Ser Asn Ser Ser Val Pro Leu Ser Glu Val Tyr Val His Leu Arg
1               5                   10                  15

Asn Trp Asn Phe Ser Trp Asn Leu Ile Leu Thr Val Phe Ile Val Val
            20                  25                  30

Leu Gln Tyr Gly His Tyr Lys Tyr Ser Arg Leu Leu Tyr Gly Leu Lys
        35                  40                  45

Met Ser Val Leu Trp Cys Leu Trp Pro Leu Val Leu Ala Leu Ser Ile
    50                  55                  60

Phe Asp Cys Phe Val Asn Phe Asn Val Asp Trp Val Phe Phe Gly Phe
65                  70                  75                  80

Ser Ile Leu Met Ser Ile Ile Thr Leu Cys Leu Trp Val Met Tyr Phe
                85                  90                  95

Val Asn Ser Phe Arg Leu Trp Arg Arg Val Lys Thr Phe Trp Ala Phe
            100                 105                 110

Asn Pro Glu Thr Asn Ala Ile Ile Ser Leu Gln Val Tyr Gly His Asn
        115                 120                 125

Tyr Tyr Leu Pro Val Met Ala Ala Pro Thr Gly Val Thr Leu Thr Leu
    130                 135                 140

Leu Ser Gly Val Leu Leu Val Asp Gly His Lys Ile Ala Thr Arg Val
145                 150                 155                 160

Gln Val Gly Gln Leu Pro Lys Tyr Val Ile Val Ala Thr Pro Ser Thr
                165                 170                 175

Thr Ile Val Cys Asp Arg Val Gly Arg Ser Val Asn Glu Thr Ser Gln
            180                 185                 190

Thr Gly Trp Ala Phe Tyr Val Arg Ala Lys His Gly Asp Phe Ser Gly
        195                 200                 205

Val Ala Ser Gln Glu Gly Val Leu Ser Glu Arg Glu Lys Leu Leu His
    210                 215                 220

Leu Ile
225

<210>  6

<211>  377

<212>  PRT

<213>  EMCR Coronavirus

<400>  6

Met Ala Ser Val Asn Trp Ala Asp Asp Arg Ala Ala Arg Lys Lys Phe
1               5                   10                  15

Pro Pro Pro Ser Phe Tyr Met Pro Leu Leu Val Ser Ser Asp Lys Ala
            20                  25                  30

Pro Tyr Arg Val Ile Pro Arg Asn Leu Val Pro Ile Gly Lys Gly Asn
        35                  40                  45

Lys Asp Glu Gln Ile Gly Tyr Trp Asn Val Gln Glu Arg Trp Arg Met
    50                  55                  60

Arg Arg Gly Gln Arg Val Asp Leu Pro Pro Lys Val His Phe Tyr Tyr
65                  70                  75                  80

Leu Gly Thr Gly Pro His Lys Asp Leu Lys Phe Arg Gln Arg Ser Asp
                85                  90                  95

Gly Val Val Trp Val Ala Lys Glu Gly Ala Lys Thr Val Asn Thr Ser
            100                 105                 110

Leu Gly Asn Arg Lys Arg Asn Gln Lys Pro Leu Glu Pro Lys Phe Ser
        115                 120                 125

Ile Ala Leu Pro Pro Glu Leu Ser Val Val Glu Phe Glu Asp Arg Ser
    130                 135                 140

Asn Asn Ser Ser Arg Ala Ser Ser Arg Ser Ser Thr Arg Asn Asn Ser
145                 150                 155                 160

Arg Asp Ser Ser Arg Ser Thr Ser Arg Gln Gln Ser Arg Thr Arg Ser
                165                 170                 175

Asp Ser Asn Gln Ser Ser Ser Asp Leu Val Ala Ala Val Thr Leu Ala
            180                 185                 190

Leu Lys Asn Leu Gly Phe Asp Asn Gln Ser Lys Ser Pro Ser Ser Ser
        195                 200                 205

Gly Thr Ser Thr Pro Lys Lys Pro Asn Lys Pro Leu Ser Gln Pro Arg
    210                 215                 220

Ala Asp Lys Pro Ser Gln Leu Lys Lys Pro Arg Trp Lys Arg Val Pro
225                 230                 235                 240

Thr Arg Glu Glu Asn Val Ile Gln Cys Phe Gly Pro Arg Asp Phe Asn
                245                 250                 255

His Asn Met Gly Asp Ser Asp Leu Val Gln Asn Gly Val Asp Ala Lys
            260                 265                 270

Gly Phe Pro Gln Leu Ala Glu Leu Ile Pro Asn Gln Ala Ala Leu Phe
        275                 280                 285

Phe Asp Ser Glu Val Ser Thr Asp Glu Val Gly Asp Asn Val Gln Ile
    290                 295                 300

Thr Tyr Thr Tyr Lys Met Leu Val Ala Lys Asp Asn Lys Asn Leu Pro
305                 310                 315                 320

Lys Phe Ile Glu Gln Ile Ser Ala Phe Thr Lys Pro Ser Ser Ile Lys
                325                 330                 335

Glu Met Gln Ser Gln Ser Ser His Val Ala Gln Asn Thr Val Leu Asn
            340                 345                 350

Ala Ser Ile Pro Glu Ser Lys Pro Leu Ala Asp Asp Asp Ser Ala Ile
        355                 360                 365

Ile Glu Ile Val Asn Glu Val Leu His
    370                 375

<210>  7

<211>  27532

<212>  DNA

<213>  EMCR Coronavirus


<220>

<221>  CDS

<222>  (12402)..(20438)

<223>  Product = Replicase 1b


<220>

<221>  CDS

<222>  (24502)..(25182)

<223>  Product = ORF 4ab


<220>

<221>  misc_feature

<222>  (14931)..(14931)

<223>  "n" stands for "k"


<400>  7
agatagagaa ttttcttatt tagactttgt gtctactcct ctcaactaaa cgaaattttt      60

ctagtgctgt catttgttat ggcagtccta gtgtaattga aatttcgtca agtttgtaaa 120
ctggttaggc aagtgttgta ttttctgtgt ttaagcactg gtggttctgt ccactagtgc 180
acacattgat acttaagtgg tgttctgtca ctgcttattg tggaagcaac gttctgtcgt 240
tgtggaaacc aataactgct aaccatgttt tacaatcaag tgacacttgc tgttgcaagt 300
gattcggaaa tttcaggttt tggttttgcc attccttctg tagccgttcg cgcttatagc 360
gaagccgctg cacaaggttt tcaggcatgc cgctttgttg cttttggctt acaggattgt 420
gtaaccggta ttaatgatga cgattatgtc attgcattga ctggtactaa tcagctttgt 480
gccaaaattt tacttttttc tgatagacct cttaatttgc gaggttggct cattttttct 540
aacagcaatt atgttcttca ggactttgat gttgtttttg gccatggtgc aggaagtgtg 600
gtttttgtgg ataagtatat gtgtggtttt gatggtaaac ctgtgttacc taaaaacatg 660
tgggaattta gagattactt taatgataat actgatagta ttgttattgg tggtgtcact 720
tatcaattag catgggatgt tatacgtaaa gacctttctt atgaacagca aaatgtttta 780
gctattgaga gcattcatta tcttggcact acaggtcata ctttgaagtc tggttgcaaa 840
ctcattaatg ccaagccgcc taaatattct tctaaggttg ttttgagtgg tgaatggaat 900
gctgtgtata aggcgtttgg ttcaccattt attacaaatg gtatatcatt gctagatata 960
attgttaaac cagttttctt taatgctttt gttaaatgca attgtggttc tgagaattgg 1020
agtgttggtg catgggatgg ttatctatct tcttgttgtg gcacacctgc taagaaactt 1080
tgtgttgttc ctggtaatgt tgttcctggt gatgtgatca tcacctcaac tgatgctggt 1140
tgtggtgtta aatactatgc tggcttagtt gttaaacata ttactaacat tactggtgtg 1200
tctttatggc gtgttacagc tgttcattct gatggaatgt ttgtggcaac atcttcttat 1260
gatgcacttt tgcatagaaa ttcattagac cctttttgct ttgatgttaa cactttactt 1320
tctaatcaat tacgtctagc ttttcttggt gcttctgtta cagaagatgt taaatttgct 1380
gctagcactg gtgttattga cattagtgct ggtatgtttg gtctttacga tgacatattg 1440
acaaacaata aaccttggtt tgtacgcaaa gcttctgggc tttttgatgc aatctgggat 1500
gcttttgttg ccgctattaa gcttgtgcca actactactg gtggtttggt taggtttgtt 1560
aagtctatcg cttcaactgt tttaactgtt tctaatggtg ttattattat gtgtgcagat 1620
gttccagatg cttttcaacc agtttaccgc acatttacac aagctatttg tgctgcattt 1680
gatttttctt tagatgtatt taaaattggt gatgttaaat ttaaacgact tggtgattat 1740
gttcttactg aaaatgctct tgttcgtttg actactgaag ttgttcgtgg tgttcgtgat 1800
gctcgcataa agaaagccat gtttactaaa gtagttgtag gtcctacaac tgaagttaag 1860
ttttctgtta ttgaacttgc cactgttaat ttgcgtcttg ttgattgtgc acctgtagtt 1920
tgccctaaag gtaaaattgt tgttattgct ggacaagctt ttttctatag tggtggtttt 1980
tatcgtttta tggttgattc tacaactgta ttaaatgacc ctgtttttac tggtgagtta 2040
ttttatacta ttaagtttag tggttttaag cttgatggtt ttaaccatca gtttgttaat 2100

gctagttctg ctacagatgc cattattgct gttgagctgt tgttatcgga ttttaaaact 2160
gcagtttttg tgtacacatg tgtggttgat ggttgtagtg tcattgttag acgtgatgct 2220
acattcgcca cacatgtgtg ttttaaggac tgttatagta tttgggagca attctgcatt 2280
gataattgtg gtgagccatg gtttttgact gattataatg ctatcttgca gagtaataac 2340
cctcaatgtg ctattgttca agcatcggag tctaaagttt tgcttgagag gtttttacct 2400
aagtgtcctg aaatactgtt gagtattgat gatggccatt tatggaatct ttttgttgaa 2460
aagtttaatt ttgttacaga ttggttaaaa actcttaagc ttacacttac ttctaatggt 2520
cttttaggta attgtgccaa acgttttaga cgtgttttgg taaaattgct tgatgtctat 2580
aatggttttc ttgaaactgt ctgtagtgtc gtacacactg ctggtgtttg cattaaatat 2640
tatgctgtta atgttccata tgtagttatt agtggttttg taagtcgtgt aattcgtaga 2700
gaaaggtgtg acgtgacttt tccttgtgtt agttgtgtca cttttttcta tgaattttta 2760
gacacgtgtt ttggtgttag taaacctaat gccattgatg ttgaacattt agagcttaaa 2820
gaaactgttt ttgttgaacc taaggatggt ggtcaatttt ttgtttctga tgattatctt 2880
tggtatgttg tagatgacat ttattatcca gcttcatgta atggtgtatt gccagttgct 2940
tttacaaaat tggcaggtgg taaaatatct ttttctgatg atgttatagt tcatgatgtt 3000
gaacctaccc ataaagtcaa gctcatattt gagtttgaag atgatgttgt taccagtctt 3060
tgtaagaaga gttttggtaa gtctattatt tatacaggtg attgggaagg tttacatgaa 3120
gttcttacat ctgcaatgaa tgtcattggg caacatatta agttgccaca attttatatt 3180
tatgatgaag agggtggtta tgatgtttct aaaccagtta tgatttcaca atggcctatt 3240
agtgatgata gtgatggttg tgttgttgaa gcgagcactg attttcatca attagaatct 3300
gttagagaag aggttgatat aattgaacaa ccttttgggg aagttgaaca tgcgctctca 3360
attagacaac ctttttcttt ttcttttaga gatgaattgg gtgttcgtgt tttagatcaa 3420
tctgataata attgttggat tagtaccaca cttatacagt tgcaacttac aaagcttttg 3480
gatgattcta ttgagatgca attgtttaaa gttggtaaag ttgattcaat tgttcaaaag 3540
tgttatgagt tgtctcattt aattagtggt tcacttggtg atagtggtaa acttcttagt 3600
gaacttctta aagataaata tacatgttct ataacttttg agatgtcttg tgattgtggt 3660
aaaaagtttg atgagcaagt tggttgtttg ttttggatta tgccttacac aaaacttttt 3720
caaaaaggtg agtgttgtat ttgtcataaa atgcagactt ataagcttgt tagtatgaaa 3780
ggtactggtg tgtttgtaca ggatccagca cctattgaca ttgatgcttt ccctgttaga 3840
cctatatgtt catctgtata tttaggtgtt aagggttctg gtcattatca aacaaattta 3900
tacagttttg acaaagctat tgatggtttt ggtgtctttg acattaaaaa tagtagtgtt 3960
aatactgttt gttttgttga tgttgatttt catagtgtag aaatagaagc tggtgaagtt 4020
aaaccttttg ctgtatataa aaatgttaaa ttttatttag gtgatatttc acaccttgta 4080
aactgtgttt cttttgactt tgttgtcaat gctgctaatg aaaatctcat gcatggaggc 4140

ggtgtcgcac gtgctattga tattttgact gaaggtcaac ttcagtcatt atctaaagat 4200
tacattagta gtaatggtcc acttaaggtt ggagcaggtg ttatgttgga gtgtgaaaaa 4260
ttcaatgtat ttaatgttgt tggtccgcga actggtaaac atgagcattc attacttgtt 4320
gaagcttata attctatttt atttgaaaat ggtattccac ttatgcctct tcttagttgt 4380
ggtatttttg gtgtaaggat tgaaaattct cttaaagctt tgtttagttg tgacattaat 4440
aaaccattgc aagtttttgt ttattcttca aatgaagaac aagctgttct taagttttta 4500
gatggtttag atttaacacc agtcattgac gatgttgatg ttgttaaacc ttttagagtt 4560
gaaggtaatt tttcattctt tgattgtggt gtcaatgcct tggatggtga tatttactta 4620
ttatttacta actctatttt aatgttggat aaacaaggac aattattgga cacaaaactt 4680
aatggtattt tgcaacaggc agttcttgat tatcttgcta cagttaaaac tgtaccagct 4740
ggtaatttgg ttaaacttgt tgttgagagt tgtaccattt atatgtgtgt tgtaccatcg 4800
ataaatgatc tttcttttga taaaaatctt ggtcgttgtg tgcgtaaact taatagattg 4860
aaaacttgtg ttattgccaa tgttcctgct attgatgttt tgaaaaagct tctttcaagt 4920
ttgactttaa ctgttaaatt tgttgtagag agtaatgtta tggatgttaa cgactgtttt 4980
aagaatgata atgtagtttt gaaaattact gaagatggta ttaatgttaa agatgttgtt 5040
gttgagtctt ctaagtcact tggtaaacaa ttgggtgttg tgagtgatgg tgttgactct 5100
tttgaaggtg ttttacctat taatactgat actgtcttat ctgtagctcc agaagttgac 5160
tgggttgctt tttacggttt tgaaaaggca gcactttttg cttctttgga tgtaaagcca 5220
tatggttacc ctaatgattt tgttggtggt tttagagttc ttgggaccac cgacaataat 5280
tgttgggtta atgcaacttg tataatttta cagtatctta agcctacttt taaatctaag 5340
ggtttaaatg ttctttggaa caaatttgtt acaggtgatg ttggaccttt tgttagtttt 5400
atttatttta taactatgtc ttcaaagggt caaaagggtg atgctgaaga ggcattatct 5460
aaattgtcag agtatttgat tagtgattct attgttactc ttgaacaata ttcaacttgt 5520
gacatttgta aaagtactgt agttgaagtt aaaagtgctg ttgtctgtgc tagtgtgctt 5580
aaagatggtt gtgatgttgg tttttgtcca cacagacata aattgcgttc acgtgttaag 5640
tttgttaatg gacgtgttgt tattaccaat gttggtgaac ctataatttc acaaccttct 5700
aagttgctta atggtattgc ttatacaaca ttttcaggtt cttttgataa cggtcactat 5760
gtagtttatg atgctgctaa taatgctgtc tatgatggtg ctcgtttatt tgcttcagat 5820
ttgtctactt tagctgttac agctattgtt gtagtaggtg gttgtgtaac atctaatgtt 5880
ccaccaattg ttagtgagaa aatttctgtt atggataaac ttgatactgg tgcacaaaaa 5940
tttttccaat ttggtgattt tgttatgaat aacattgttc tgtttttaac ttggttgctt 6000
agtatgttta gtcttttacg tacttctatt atgaagcatg atattaaagt tattgccaag 6060
gctcctaaac gtacaggtgt tattttgaca cgtagtttta agtataacat tagatctgct 6120
ttgtttgttg taaagcagaa gtggtgtgtt attgttactt tgtttaagtt cttattgtta 6180

ttatatgcta tttatgcact tgtttttatg attgtgcaat ttagtccttt taatagtctt 6240
ttatgtggtg acattgtaag tggttatgaa aaatccactt ttaataagga tatttattgt 6300
ggtaattcta tggtttgtaa gatgtgtttg tttagttatc aagagtttaa tgatttggat 6360
catactagtc ttgtttggaa gcacattcgt gatcctatat taatcagttt acaaccattt 6420
gttatacttg ttattttgtt aatttttggt aatatgtatt tgcgttttgg acttttatat 6480
tttgttgcac aatttattag tacttttggt tctttcttag gctttcatca gaaacagtgg 6540
tttttacatt ttgtgccgtt tgatgtttta tgtaatgagt ttttagctac atttattgtc 6600
tgcaaaattg ttttatttgt tagacatatt attgttggct gtaataatgc tgactgtgta 6660
gcttgttcta aaagtgctag acttaaacgt gtaccacttc aaactattat taatggtatg 6720
cataaatcat tctatgttaa tgctaatggt ggtacttgtt tctgtaataa acataacttc 6780
ttttgtgtta attgtgattc ttttgggcct ggtaatactt ttattaatgg tgatattgca 6840
agagagcttg gtaatgttgt taaaacagct gttcaaccca cagctcctgc atatgttatt 6900
attgataagg tagattttgt taatggattt tatcgtcttt atagtggtga cactttttgg 6960
cggtatgact ttgacattac tgaatctaag tatagttgta aagaggttct gaagaattgt 7020
aatgttttag aaaattttat tgtttacaat aatagtggta gtaacattac acagattaaa 7080
aatgcttgtg tttatttttc tcaattgttg tgtgaaccta taaagttggt aaattcagag 7140
ttgttgtcaa ctttatcagt tgattttaat ggtgttttgc ataaggcata tgttgatgtt 7200
ttgtgtaata gtttttttaa ggagctaact gctaacatgt ccatggctga atgtaaagct 7260
acacttggtt tgactgtttc tgatgatgat tttgtttcag ctgttgccaa tgcacatagg 7320
tatgacgttt tgctttcaga tttgtcattt aataattttt ttatttctta tgctaaacct 7380
gaagataagt tgtccgttta tgacattgct tgttgtatgc gtgccggttc taaggttgtt 7440
aaccataatg ttttaatcaa agagtcaata cctattgttt ggggtgtcaa ggactttaat 7500
actctttctc aagaaggtaa gaagtacctt gttaaaacaa ctaaagcaaa gggtttgact 7560
tttttattaa cttttaatga taaccaagca attacacaag ttcctgctac tagtatagtt 7620
gcaaaacagg gtgctggttt taaacgtact tataattttc tgtggtatgt atgtttattt 7680
gttgttgcat tgtttattgg tgtctcattt attgattata caaccactgt aactagcttt 7740
catggttatg attttaagta cattgagaat ggtcagttga aggtgtttga agcaccttta 7800
cactgtgttc gtaatgtttt tgataatttt aatcaatggc atgaggctaa gtttggtgtt 7860
gttactacta atagtgataa atgtcctata gttgttggtg tttcagagcg tattaatgtt 7920
gttcctggtg ttccaacaaa tgtatatttg gtaggaaaga ctcttgtttt tacattacag 7980
gctgcttttg gaaacacagg tgtttgttat gactttgatg gtgttaccac tagtgataag 8040
tgtattttta attctgcttg tactaggttg gaaggtttgg gtggtgacaa tgtttattgt 8100
tacaacactg atcttattga aggttctaaa ccttatagta ttttacagcc caatgcttat 8160
tataagtatg atgttaaaaa ttatgtacgt tttccagaaa ttttagctag aggttttggc 8220

ttacgtacta ttagaacttt ggctacacgt tattgtagag ttggtgaatg ccgtgactca 8280
cataaaggtg tttgttttgg ttttgataaa tggtatgtta atgatggacg tgttgatgac 8340
ggttacattt gtggtgatgg tcttatagac cttcttgtta atgtactctc aatctttagt 8400
tcatctttta gcgttgtggc tatgtctgga catatgttgt ttaattttct ttttgcagca 8460
tttattacat ttttgtgctt tttagttact aaatttaaac gtgtttttgg tgatctttct 8520
tatggtgttt ttactgttgt ttgtgcaact ttgattaata acatttctta tgttgttact 8580
caaaatttat tttttatgtt gctttatgct attttgtatt ttgtttttac taggacagtg 8640
cgttatgctt ggatttggca tattgcatac attgttgcat acttcttgtt aataccatgg 8700
tggcttctca catggtttag ttttgctgca tttttagagc ttttacctaa tgtttttaag 8760
ttaaaaatct ctactcaatt gtttgaaggt gataagttta taggtacttt tgagagtgct 8820
gctgcaggta catttgttct tgacatgcgt tcttatgaaa ggctgataaa tactatttca 8880
cctgagaaac ttaagaatta tgctgcaagt tataataaat ataaatatta tagtggtagt 8940
gctagtgagg ctgattatcg ttgtgcttgt tatgctcatt tagccaaggc tatgttagat 9000
tacgcaaaag atcataatga catgttatat tctccaccta ccattagcta caattccacc 9060
ttacaatctg gtcttaagaa gatggcacaa ccatctggtt gtgttgagag atgtgtggtt 9120
cgcgtctgtt atggtagtac tgtgcttaat ggagtttggt taggtgacac tgttacttgt 9180
cctagacatg tcatagcacc atcaaccact gttcttattg attatgatca tgcatatagt 9240
actatgcgtt tgcataattt ttcagtgtct cataatggtg tcttcttggg agttgttggt 9300
gttacaatgc atggttctgt gttgcgtatt aaggtttcac aatctaatgt acatacacct 9360
aaacatgttt ttaaaacgtt gaaacctggt gcttctttta atattttagc atgttatgaa 9420
ggtattgcat ctggtgtttt tggtgttaat ttacgtacaa actttactat taaaggttct 9480
tttataaatg gagcttgtgg ttctcctggt tataatgtta gaaatgatgg tactgttgag 9540
ttttgttatt tacaccaaat tgagttaggt agtggtgctc atgttggttc tgattttact 9600
ggtagtgttt atggtaattt tgatgaccaa cctagtttgc aagttgagag tgccaacctt 9660
atgctatcag ataatgttgt tgcctttttg tatgctgctt tgttgaatgg ttgtaggtgg 9720
tggttgcgtt caactagagt taatgttgat ggttttaatg aatgggctat ggctaatggt 9780
tatacaattg tttctagtgt tgagtgctat tctattttgg cagcaaaaac tggtgttagt 9840
gttgaacaat tgttagcttc cattcaacat cttcatgaag gttttggtgg taaaaacata 9900
cttggttatt ctagtttatg tgatgagttc acactagctg aagttgtgaa gcagatgtat 9960
ggtgttaact tgcaaagtgg taaggttatt tttggtttaa aaacaatgtt tttatttagc 10020
gttttcttca caatgttttg ggcagaactc tttatttata caaacactat atggataaac 10080
cctgttatac ttacacctat attttgttta cttttgtttt tgtcattagt tttaactatg 10140
tttcttaaac ataagttttt gtttttgcaa gtatttttat tacctactgt tattgcaact 10200
gctttatata attgtgtttt ggattattac atagtaaaat ttttggctga ccattttaac 10260

tataatgttt cagtattaca aatggatgtt cagggtttag ttaatgtttt ggtctgttta 10320
tttgttgtat ttttacacac atggcgtttt tctaaagaac gtttcacaca ttggtttaca 10380
tatgtgtgtt ctcttatagc agttgcttac acttattttt atagtggtga ctttttgagt 10440
ttgcttgtta tgtttttatg tgctatatct agtgattggt acattggtgc cattgttttt 10500
aggttgtcac gtttgattat atttttttca cctgaaagtg tatttagtgt ttttggtgat 10560
gtgaaactca ctttagttgt ttatttaatt tgtggttatt tagtttgtac ttattggggc 10620
attttgtatt ggttcaatag gttttttaaa tgtactatgg gtgtttatga ttttaaggtg 10680
agtgctgctg aatttaaata catggttgct aatggacttc atgcaccata tggacctttt 10740
gatgcacttt ggttatcatt caaattactt ggtattggtg gtgaccgttg tataaaaatt 10800
tcaactgtcc aatccaaact gactgatttg aagtgtacta atgttgtgtt attgggttgt 10860
ttgtctagta tgaacattgc agctaattct agtgaatggg cttattgtgt tgatttacac 10920
aataagatta atctttgtga tgacccagaa aaagctcaag gtatgttgtt agcactcctt 10980
gcgttctttc taagtaaaca tagtgatttt ggtcttgatg gccttattga ttcttatttt 11040
gataatagta gcaccctgca gagtgttgct tcatcatttg ttagtatgcc atcatatatt 11100
gcttatgaaa atgctagaca agcttatgag gatgctattg ctaatggatc ttcttctcaa 11160
cttattaaac aattgaagcg tgccatgaat atcgcaaagt ctgaatttga tcatgagata 11220
tctgttcaga agaaaattaa tagaatggct gaacaagctg ctactcagat gtataaagaa 11280
gcacgctctg ttaatagaaa atctaaagtt attagtgcta tgcactcttt actttttgga 11340
atgttaagac gtttggatat gtctagtgtt gaaactgttt tgaatttagc acgtgatggt 11400
gttgtgccat tgtcagttat acctgcaact tcagcttcca aactaactat tgttagtcca 11460
gatcttgaat cttattctaa gattgtttgt gatggttctg ttcattatgc tggagttgtt 11520
tggacactta atgatgttaa agacaatgat ggtagacctg ttcatgttaa agagattaca 11580
agggagaatg ttgaaacttt gacatggcct cttatcctta attgtgaacg tgttgttaaa 11640
cttcaaaata atgaaattat gcctggtaaa cttaagcaaa aacctatgaa agctgagggt 11700
gatggtggtg ttttaggtga tggtaatgct ttgtataata ctgagggtgg taaaactttt 11760
atgtatgctt atatttctaa taaagctgac cttaaatttg ttaagtggga gtatgagggt 11820
ggttgcaaca caatcgagtt agactctcct tgtcgattta tggtcgaaac acctaatggt 11880
cctcaagtga agtatttgta ttttgttaaa aatttaaata ccttacgtag aggtgccgtt 11940
cttggtttta taggtgccac aattcgtcta caagctggta aacaaactga attggctgtt 12000
aattctggac ttttaactgc ttgtgctttt tctgttgatc cagcaaccac ttacttggaa 12060
gctgttaaac atggtgcaaa acctgtaagt aattgtatta agatgttatc taatggtgct 12120
ggtaatggtc aagctataac aactagtgta gatgctaaca ccaatcaaga ttcttatggt 12180
ggagcgtcta tttgtttgta ttgtcgggcc cacgttcctc accctagtat ggatggttac 12240
tgtaagttta agggtaaatg tgttcaggtt cctattggtt gtttggatcc tattaggttt 12300

tgtttagaaa ataatgtgtg taatgtttgt ggttgttggt tgggacacgg gtgtgcttgt 12360

gatcgtacaa ccattcaaag tgttgacatt tcttatttaa a cga gca agg ggt tct 12416
Arg Ala Arg Gly Ser
1               5

agt gca gct cga cta gaa ccc tgt aat ggc acg gac atc gat aag tgt 12464
Ser Ala Ala Arg Leu Glu Pro Cys Asn Gly Thr Asp Ile Asp Lys Cys
                10                  15                  20

gtt cgt gct ttt gac att tat aat aaa aat gtt tca ttc ttg ggt aag 12512
Val Arg Ala Phe Asp Ile Tyr Asn Lys Asn Val Ser Phe Leu Gly Lys
            25                  30                  35

tgt ttg aag atg aac tgt gtt cgt ttt aaa aat gct gat ctt aag gat 12560
Cys Leu Lys Met Asn Cys Val Arg Phe Lys Asn Ala Asp Leu Lys Asp
        40                  45                  50

ggt tat ttt gtt ata aag agg tgt act aag tcg gtt atg gaa cac gag 12608
Gly Tyr Phe Val Ile Lys Arg Cys Thr Lys Ser Val Met Glu His Glu
    55                  60                  65

caa tcc atg tat aac cta ctt aac ttt tct ggt gct ttg gct gag cat 12656
Gln Ser Met Tyr Asn Leu Leu Asn Phe Ser Gly Ala Leu Ala Glu His
70                  75                  80                  85

gat ttc ttt act tgg aaa gat ggc aga gtc att tat ggt aat gtt agt 12704
Asp Phe Phe Thr Trp Lys Asp Gly Arg Val Ile Tyr Gly Asn Val Ser
                90                  95                  100

aga cat aat ctt act aaa tat act atg atg gac ttg gtt tat gct atg 12752
Arg His Asn Leu Thr Lys Tyr Thr Met Met Asp Leu Val Tyr Ala Met
            105                 110                 115

cgt aac ttt gat gaa caa aat tgt gat gtt cta aaa gaa gta tta gtt 12800
Arg Asn Phe Asp Glu Gln Asn Cys Asp Val Leu Lys Glu Val Leu Val
        120                 125                 130

tta act ggt tgt tgt gac aat tct tat ttt gat agt aag ggt tgg tat 12848
Leu Thr Gly Cys Cys Asp Asn Ser Tyr Phe Asp Ser Lys Gly Trp Tyr
    135                 140                 145

gac cca gtt gaa aat gaa gat ata cat aga gtt tat gca tct ctt ggc 12896
Asp Pro Val Glu Asn Glu Asp Ile His Arg Val Tyr Ala Ser Leu Gly
150                 155                 160                 165

aaa att gta gct aga gct atg ctt aaa tgc gtt gct cta tgt gat gcg 12944
Lys Ile Val Ala Arg Ala Met Leu Lys Cys Val Ala Leu Cys Asp Ala
                170                 175                 180

atg gtt gct aaa ggt gtt gtt ggt gtt tta aca tta gat aac caa gat 12992
Met Val Ala Lys Gly Val Val Gly Val Leu Thr Leu Asp Asn Gln Asp
            185                 190                 195

ctt aat ggt aac ttt tat gat ttt ggt gat ttt gtt gtt agc tta cct 13040
Leu Asn Gly Asn Phe Tyr Asp Phe Gly Asp Phe Val Val Ser Leu Pro
        200                 205                 210

aat atg ggt gtt ccc tgt tgt aca tca tat tat tct tat atg atg cct 13088
Asn Met Gly Val Pro Cys Cys Thr Ser Tyr Tyr Ser Tyr Met Met Pro
    215                 220                 225

att atg ggt tta act aat tgt tta gct agt gag tgt ttt gtc aag agt 13136
Ile Met Gly Leu Thr Asn Cys Leu Ala Ser Glu Cys Phe Val Lys Ser
230                 235                 240                 245

gat att ttt ggt agt gat ttt aaa act ttt gat ttg ctt aag tat gat 13184

Asp Ile Phe Gly Ser Asp Phe Lys Thr Phe Asp Leu Leu Lys Tyr Asp
                250                 255                 260

ttc act gaa cat aaa gaa aat tta ttc aat aag tac ttt aag cat tgg 13232
Phe Thr Glu His Lys Glu Asn Leu Phe Asn Lys Tyr Phe Lys His Trp
            265                 270                 275

agt ttt gat tat cat cct aat tgt agt gac tgt tat gat gat atg tgt 13280
Ser Phe Asp Tyr His Pro Asn Cys Ser Asp Cys Tyr Asp Asp Met Cys
        280                 285                 290

gtt ata cat tgt gct aat ttt aat aca cta ttt gcc aca act ata cca 13328
Val Ile His Cys Ala Asn Phe Asn Thr Leu Phe Ala Thr Thr Ile Pro
    295                 300                 305

ggt act gct ttt ggt cca cta tgt cgt aaa gtt ttt ata gat ggt gtt 13376
Gly Thr Ala Phe Gly Pro Leu Cys Arg Lys Val Phe Ile Asp Gly Val
310                 315                 320                 325

cca ctt gtt aca act gct ggt tat cat ttt aag caa tta ggt ttg gtt 13424
Pro Leu Val Thr Thr Ala Gly Tyr His Phe Lys Gln Leu Gly Leu Val
                330                 335                 340

tgg aat aaa gat gtt aac aca cac tca gtt agg ttg aca atc act gaa 13472
Trp Asn Lys Asp Val Asn Thr His Ser Val Arg Leu Thr Ile Thr Glu
            345                 350                 355

ctt ttg caa ttt gtt act gac cct tcc ttg ata ata gct tct tct cca 13520
Leu Leu Gln Phe Val Thr Asp Pro Ser Leu Ile Ile Ala Ser Ser Pro
        360                 365                 370

gca ctc gtt gat caa cgc act att tgt ttt tct gtt gca gca ttg agt 13568
Ala Leu Val Asp Gln Arg Thr Ile Cys Phe Ser Val Ala Ala Leu Ser
    375                 380                 385

act ggt ttg aca aat caa gtt gtt aag cca ggt cat ttt aat gaa gag 13616
Thr Gly Leu Thr Asn Gln Val Val Lys Pro Gly His Phe Asn Glu Glu
390                 395                 400                 405

ttt tat aac ttt ctt cgt tta aga ggt ttc ttt gat gaa ggt tct gaa 13664
Phe Tyr Asn Phe Leu Arg Leu Arg Gly Phe Phe Asp Glu Gly Ser Glu
                410                 415                 420

ctt aca tta aaa cat ttc ttc ttc gca cag aat ggt gat gct gct gtt 13712
Leu Thr Leu Lys His Phe Phe Phe Ala Gln Asn Gly Asp Ala Ala Val
            425                 430                 435

aaa gat ttt gac ttt tac cgt tat aat aag cct acc att tta gat att 13760
Lys Asp Phe Asp Phe Tyr Arg Tyr Asn Lys Pro Thr Ile Leu Asp Ile
        440                 445                 450

tgt caa gct aga gtt aca tat aag ata gtc tct cgt tat ttt gac att 13808
Cys Gln Ala Arg Val Thr Tyr Lys Ile Val Ser Arg Tyr Phe Asp Ile
    455                 460                 465

tat gaa ggt ggc tgt att aag gca tgt gaa gtt gtt gta aca aat ctt 13856
Tyr Glu Gly Gly Cys Ile Lys Ala Cys Glu Val Val Val Thr Asn Leu
470                 475                 480                 485

aat aag agt gct ggt tgg cca tta aat aag ttt ggt aaa gct agt ttg 13904
Asn Lys Ser Ala Gly Trp Pro Leu Asn Lys Phe Gly Lys Ala Ser Leu
                490                 495                 500

tat tac gaa tct ata tct tat gaa gaa cag gat gct ttg ttt gct ttg 13952
Tyr Tyr Glu Ser Ile Ser Tyr Glu Glu Gln Asp Ala Leu Phe Ala Leu
            505                 510                 515

aca aag cgt aat gtc ctc cct act atg aca cag ctg aat ctt aag tat 14000

Thr Lys Arg Asn Val Leu Pro Thr Met Thr Gln Leu Asn Leu Lys Tyr
        520                 525                 530

gct att agt ggt aaa gaa cgt gct aga act gtt ggt ggt gtt tct ctg     14048
Ala Ile Ser Gly Lys Glu Arg Ala Arg Thr Val Gly Gly Val Ser Leu
    535                 540                 545

ttg tcc aca atg acc aca aga caa tac cat caa aaa cat ctt aaa tcc     14096
Leu Ser Thr Met Thr Thr Arg Gln Tyr His Gln Lys His Leu Lys Ser
550                 555                 560                 565

att gtt aat aca cgc aat gcc act gtt gtt att ggt act acc aaa ttt     14144
Ile Val Asn Thr Arg Asn Ala Thr Val Val Ile Gly Thr Thr Lys Phe
                570                 575                 580

tat ggt ggt tgg aat aat atg ttg cgt act tta att gat ggt gtt gaa     14192
Tyr Gly Gly Trp Asn Asn Met Leu Arg Thr Leu Ile Asp Gly Val Glu
            585                 590                 595

aac cct atg ctc atg ggt tgg gat tat ccc aaa tgt gat aga gct ttg     14240
Asn Pro Met Leu Met Gly Trp Asp Tyr Pro Lys Cys Asp Arg Ala Leu
        600                 605                 610

cct aac atg ata cgt atg att tca gcc atg gtg ttg ggt tct aag cat     14288
Pro Asn Met Ile Arg Met Ile Ser Ala Met Val Leu Gly Ser Lys His
    615                 620                 625

gtt aat tgt tgt act gta aca gat agg ttt tat agg ctt ggt aac gag     14336
Val Asn Cys Cys Thr Val Thr Asp Arg Phe Tyr Arg Leu Gly Asn Glu
630                 635                 640                 645

ttg gca caa gtt tta aca gaa gtt gtt tat tct aat ggt ggt ttt tat     14384
Leu Ala Gln Val Leu Thr Glu Val Val Tyr Ser Asn Gly Gly Phe Tyr
                650                 655                 660

ttt aag cca ggt ggt acg act tct ggt gac gct agt aca gct tat gct     14432
Phe Lys Pro Gly Gly Thr Thr Ser Gly Asp Ala Ser Thr Ala Tyr Ala
            665                 670                 675

aat tct att ttt aac att ttt caa gcc gtg agt tct aac att aac agg     14480
Asn Ser Ile Phe Asn Ile Phe Gln Ala Val Ser Ser Asn Ile Asn Arg
        680                 685                 690

ttg ctt agt gtc cca tca gat tca tgt aat aat gtt aat gtt agg gat     14528
Leu Leu Ser Val Pro Ser Asp Ser Cys Asn Asn Val Asn Val Arg Asp
    695                 700                 705

cta caa cga cgt ctg tat gat aat tgc tat agg tta act agt gtt gaa     14576
Leu Gln Arg Arg Leu Tyr Asp Asn Cys Tyr Arg Leu Thr Ser Val Glu
710                 715                 720                 725

gag tca ttc att gat gat tat tat ggt tat ctt agg aaa cat ttt tca     14624
Glu Ser Phe Ile Asp Asp Tyr Tyr Gly Tyr Leu Arg Lys His Phe Ser
                730                 735                 740

atg atg att ctc tct gat gac ggt gtt gtc tgt tat aac aag gat tat     14672
Met Met Ile Leu Ser Asp Asp Gly Val Val Cys Tyr Asn Lys Asp Tyr
            745                 750                 755

gct gag tta ggt tat ata gca gac att agt gct ttt aaa gcc act ttg     14720
Ala Glu Leu Gly Tyr Ile Ala Asp Ile Ser Ala Phe Lys Ala Thr Leu
        760                 765                 770

tat tac cag aat aat gtc ttt atg agt act tct aaa tgt tgg gtt gaa     14768
Tyr Tyr Gln Asn Asn Val Phe Met Ser Thr Ser Lys Cys Trp Val Glu
    775                 780                 785

gaa gat tta act aag gga cca cat gag ttt tgt tcc cag cat act atg     14816

Glu Asp Leu Thr Lys Gly Pro His Glu Phe Cys Ser Gln His Thr Met
790                 795                 800                 805

caa ata gtt gat aaa gat ggt acc tat tat ttg cct tac cca gat cct     14864
Gln Ile Val Asp Lys Asp Gly Thr Tyr Tyr Leu Pro Tyr Pro Asp Pro
            810                 815                 820

agt agg atc ttg tca gct ggt gtt ttt gtt gat gat gtt gtt aag aca     14912
Ser Arg Ile Leu Ser Ala Gly Val Phe Val Asp Asp Val Val Lys Thr
        825                 830                 835

gat gct gtt gtt ttg tta naa cgt tat gtg tct tta gct att gat gca     14960
Asp Ala Val Val Leu Leu Xaa Arg Tyr Val Ser Leu Ala Ile Asp Ala
    840                 845                 850

tac cct ctt tca aaa cac cct aat tct gaa tat cgt aag gtt ttt tac     15008
Tyr Pro Leu Ser Lys His Pro Asn Ser Glu Tyr Arg Lys Val Phe Tyr
    855                 860                 865

gta tta ctt gat tgg gtt aag cat ctt aac aaa aat ttg aat gag ggt     15056
Val Leu Leu Asp Trp Val Lys His Leu Asn Lys Asn Leu Asn Glu Gly
870                 875                 880                 885

gtt ctt gaa tct ttt tct gtt aca ctt ctt gat aat caa gaa gat aag     15104
Val Leu Glu Ser Phe Ser Val Thr Leu Leu Asp Asn Gln Glu Asp Lys
            890                 895                 900

ttt tgg tgt gaa gat ttt tat gct agt atg tat gaa aat tct aca ata     15152
Phe Trp Cys Glu Asp Phe Tyr Ala Ser Met Tyr Glu Asn Ser Thr Ile
            905                 910                 915

ttg caa gct gct ggc tta tgt gtt gtt tgt ggt tca caa act gtt ctt     15200
Leu Gln Ala Ala Gly Leu Cys Val Val Cys Gly Ser Gln Thr Val Leu
        920                 925                 930

cgt tgt ggt gat tgt ctg cgt aag cct atg ttg tgc act aaa tgt gca     15248
Arg Cys Gly Asp Cys Leu Arg Lys Pro Met Leu Cys Thr Lys Cys Ala
    935                 940                 945

tat gat cat gta ttt ggt acc gac cac aag ttt att ttg gct ata aca     15296
Tyr Asp His Val Phe Gly Thr Asp His Lys Phe Ile Leu Ala Ile Thr
950                 955                 960                 965

ccg tat gta tgt aat gca tca ggt tgt ggt gtt agt gat gtt aaa aaa     15344
Pro Tyr Val Cys Asn Ala Ser Gly Cys Gly Val Ser Asp Val Lys Lys
            970                 975                 980

ttg tat ctt ggt ggt ttg aat tac tat tgt aca aat cat aaa cca cag     15392
Leu Tyr Leu Gly Gly Leu Asn Tyr Tyr Cys Thr Asn His Lys Pro Gln
        985                 990                 995

ttg tct ttt  cca tta tgt tct gct  ggt aat ata ttt ggt  tta tat     15437
Leu Ser Phe  Pro Leu Cys Ser Ala  Gly Asn Ile Phe Gly  Leu Tyr
        1000                 1005                 1010

aaa aat tca  gca act ggt tcc tta  gat gtt gaa gtt ttt  aat agg     15482
Lys Asn Ser  Ala Thr Gly Ser Leu  Asp Val Glu Val Phe  Asn Arg
        1015                 1020                 1025

ctt gca acg  tct gat tgg act gat  gtt agg gac tat aaa  ctt gct     15527
Leu Ala Thr  Ser Asp Trp Thr Asp  Val Arg Asp Tyr Lys  Leu Ala
        1030                 1035                 1040

aat gat gtt  aaa gat aca ctt aga  ctc ttt gcg gct gaa  act att     15572
Asn Asp Val  Lys Asp Thr Leu Arg  Leu Phe Ala Ala Glu  Thr Ile
        1045                 1050                 1055

aaa gct aaa  gaa gag agt gtt aag  tct tct tat gct ttt  gca act     15617

Lys Ala Lys Glu Glu Ser Val Lys Ser Ser Tyr Ala Phe Ala Thr
        1060                1065                1070

ctt aaa gag gtt gtt gga cct aaa gaa ttg ctt ctt agt tgg gaa 15662
Leu Lys Glu Val Val Gly Pro Lys Glu Leu Leu Leu Ser Trp Glu
        1075                1080                1085

agt ggt aaa gtt aaa cca cct ttg aat cgt aat tct gtt ttc acc 15707
Ser Gly Lys Val Lys Pro Pro Leu Asn Arg Asn Ser Val Phe Thr
        1090                1095                1100

tgt ttt caa ata agt aag gac tca aaa ttc caa ata ggt gag ttc 15752
Cys Phe Gln Ile Ser Lys Asp Ser Lys Phe Gln Ile Gly Glu Phe
        1105                1110                1115

atc ttt gaa aag gtt gaa tat ggt tct gat act gtt acg tat aag 15797
Ile Phe Glu Lys Val Glu Tyr Gly Ser Asp Thr Val Thr Tyr Lys
        1120                1125                1130

tct act gta acc act aag tta gtt cct ggt atg att ttt gtc tta 15842
Ser Thr Val Thr Thr Lys Leu Val Pro Gly Met Ile Phe Val Leu
        1135                1140                1145

aca tct cac aat gtt caa cct tta cgt gca cca act att gca aac 15887
Thr Ser His Asn Val Gln Pro Leu Arg Ala Pro Thr Ile Ala Asn
        1150                1155                1160

caa gag aag tat tct agc att tat aaa ttg cac cct gct ttt aat 15932
Gln Glu Lys Tyr Ser Ser Ile Tyr Lys Leu His Pro Ala Phe Asn
        1165                1170                1175

gtc agt gat gca tat gct aat ttg gtt cca tat tac caa ctt att 15977
Val Ser Asp Ala Tyr Ala Asn Leu Val Pro Tyr Tyr Gln Leu Ile
        1180                1185                1190

ggt aaa caa aag ata act aca ata cag ggt cct cct ggt agt ggt 16022
Gly Lys Gln Lys Ile Thr Thr Ile Gln Gly Pro Pro Gly Ser Gly
        1195                1200                1205

aag tca cat tgt tcc att gga ctt gga ttg tac tat cca ggt gcg 16067
Lys Ser His Cys Ser Ile Gly Leu Gly Leu Tyr Tyr Pro Gly Ala
        1210                1215                1220

cgt att gtt ttt gtt gct tgt gcc cat gct gct gtt gat tcc tta 16112
Arg Ile Val Phe Val Ala Cys Ala His Ala Ala Val Asp Ser Leu
        1225                1230                1235

tgt gca aaa gct atg act gtt tat agc att gat aag tgt act agg 16157
Cys Ala Lys Ala Met Thr Val Tyr Ser Ile Asp Lys Cys Thr Arg
        1240                1245                1250

att ata cct gca aga gct cgg gtt gag tgt tat agt ggc ttt aaa 16202
Ile Ile Pro Ala Arg Ala Arg Val Glu Cys Tyr Ser Gly Phe Lys
        1255                1260                1265

cca aat aac act agt gca caa tac ata ttt agc act gtt aac gca 16247
Pro Asn Asn Thr Ser Ala Gln Tyr Ile Phe Ser Thr Val Asn Ala
        1270                1275                1280

tta cct gag tgt aat gct gat att gtt gtt gta gat gaa gtt tca 16292
Leu Pro Glu Cys Asn Ala Asp Ile Val Val Val Asp Glu Val Ser
        1285                1290                1295

atg tgt aca aat tat gac ctt tct gtt att aat cag cgt tta tca 16337
Met Cys Thr Asn Tyr Asp Leu Ser Val Ile Asn Gln Arg Leu Ser
        1300                1305                1310

tat aaa cat att gtt tat gtt ggt gat cca caa caa ctt cct gca 16382

Tyr Lys His Ile Val Tyr Val Gly Asp Pro Gln Gln Leu Pro Ala
        1315                1320                1325

cct aga gta atg att act aaa ggt gtt atg gag cct gtt gat tat      16427
Pro Arg Val Met Ile Thr Lys Gly Val Met Glu Pro Val Asp Tyr
        1330                1335                1340

aac gtt gtt act caa cgt atg tgt gct ata ggc cct gat gtt ttt      16472
Asn Val Val Thr Gln Arg Met Cys Ala Ile Gly Pro Asp Val Phe
        1345                1350                1355

ctt cat aaa tgt tat aga tgt cct gct gaa ata gtt aat aca gtt      16517
Leu His Lys Cys Tyr Arg Cys Pro Ala Glu Ile Val Asn Thr Val
        1360                1365                1370

tct gaa ctt gtt tat gag aac aag ttt gtc cct gtt aaa cct gct      16562
Ser Glu Leu Val Tyr Glu Asn Lys Phe Val Pro Val Lys Pro Ala
        1375                1380                1385

agt aaa cag tgt ttt aaa atc ttt ttt aag ggt aat gta cag gtt      16607
Ser Lys Gln Cys Phe Lys Ile Phe Phe Lys Gly Asn Val Gln Val
        1390                1395                1400

gac aat ggc tct agt att aac aga aag cag ctt gaa ata gtt aag      16652
Asp Asn Gly Ser Ser Ile Asn Arg Lys Gln Leu Glu Ile Val Lys
        1405                1410                1415

ctg ttt tta gtt aaa aat cca agt tgg agt aag gct gtg ttt att      16697
Leu Phe Leu Val Lys Asn Pro Ser Trp Ser Lys Ala Val Phe Ile
        1420                1425                1430

tct cct tat aat agt cag aat tat gtt gct agt aga ttt tta gga      16742
Ser Pro Tyr Asn Ser Gln Asn Tyr Val Ala Ser Arg Phe Leu Gly
        1435                1440                1445

ctt caa att caa act gtt gat tct tct caa ggt agt gag tat gat      16787
Leu Gln Ile Gln Thr Val Asp Ser Ser Gln Gly Ser Glu Tyr Asp
        1450                1455                1460

tat gta atc tat gca caa act tct gac act gca cat gct tgc aat      16832
Tyr Val Ile Tyr Ala Gln Thr Ser Asp Thr Ala His Ala Cys Asn
        1465                1470                1475

gta aac cgt ttt aat gtt gct ata aca cgt gct aag aag ggt ata      16877
Val Asn Arg Phe Asn Val Ala Ile Thr Arg Ala Lys Lys Gly Ile
        1480                1485                1490

ttt tgt gta atg tgt gat aaa act ttg ttt gat tca ctt aag ttt      16922
Phe Cys Val Met Cys Asp Lys Thr Leu Phe Asp Ser Leu Lys Phe
        1495                1500                1505

ttt gag att aaa cat gca gat tta cac tct agc cag gtt tgt ggc      16967
Phe Glu Ile Lys His Ala Asp Leu His Ser Ser Gln Val Cys Gly
        1510                1515                1520

ttg ttt aaa aat tgt aca cgc act cct ctt aat tta cca cca act      17012
Leu Phe Lys Asn Cys Thr Arg Thr Pro Leu Asn Leu Pro Pro Thr
        1525                1530                1535

cat gca cac act ttc ttg tcg ttg tca gat cag ttt aag act aca      17057
His Ala His Thr Phe Leu Ser Leu Ser Asp Gln Phe Lys Thr Thr
        1540                1545                1550

ggt gat tta gct gtt caa ata ggt tca aat aat gtt tgt act tat      17102
Gly Asp Leu Ala Val Gln Ile Gly Ser Asn Asn Val Cys Thr Tyr
        1555                1560                1565

gaa cat gtt ata tca ttt atg ggt ttt agg ttt gat att agt att      17147

Glu His Val Ile Ser Phe Met Gly Phe Arg Phe Asp Ile Ser Ile
        1570                1575                1580

cct ggt agt cat agt ttg ttt tgt aca cgt gac ttt gct att cgt      17192
Pro Gly Ser His Ser Leu Phe Cys Thr Arg Asp Phe Ala Ile Arg
        1585                1590                1595

aat gtg cgt ggt tgg ttg ggt atg gat gtt gaa agt gct cat gtt      17237
Asn Val Arg Gly Trp Leu Gly Met Asp Val Glu Ser Ala His Val
        1600                1605                1610

tgt ggc gat aac ata ggt act aat gtt cct tta cag gtt ggt ttt      17282
Cys Gly Asp Asn Ile Gly Thr Asn Val Pro Leu Gln Val Gly Phe
        1615                1620                1625

tca aat ggt gtt aat ttt gtt gtg caa act gaa ggt tgt gtg tct      17327
Ser Asn Gly Val Asn Phe Val Val Gln Thr Glu Gly Cys Val Ser
        1630                1635                1640

acc aat ttt ggt gat gtt att aaa cct gtt tgt gca aaa tct cca      17372
Thr Asn Phe Gly Asp Val Ile Lys Pro Val Cys Ala Lys Ser Pro
        1645                1650                1655

cca ggt gaa caa ttt aga cac ctt gtt cct ttt tta cgt aaa gga      17417
Pro Gly Glu Gln Phe Arg His Leu Val Pro Phe Leu Arg Lys Gly
        1660                1665                1670

caa cct tgg tta att gtt cgt aga cgc att gtg caa atg ata tct      17462
Gln Pro Trp Leu Ile Val Arg Arg Arg Ile Val Gln Met Ile Ser
        1675                1680                1685

gat tat ttg tcc aat ttg tct gac att ctt gtc ttt gtt ttg tgg      17507
Asp Tyr Leu Ser Asn Leu Ser Asp Ile Leu Val Phe Val Leu Trp
        1690                1695                1700

gca ggt agt ttg gaa tta act aca atg cgt tac ttt gta aaa ata      17552
Ala Gly Ser Leu Glu Leu Thr Thr Met Arg Tyr Phe Val Lys Ile
        1705                1710                1715

ggg cca att aaa tat tgt tat tgt ggt aat tct gcc act tgt tat      17597
Gly Pro Ile Lys Tyr Cys Tyr Cys Gly Asn Ser Ala Thr Cys Tyr
        1720                1725                1730

aat tca gtt agt aat gaa tat tgt tgt ttt aaa cat gca ttg ggt      17642
Asn Ser Val Ser Asn Glu Tyr Cys Cys Phe Lys His Ala Leu Gly
        1735                1740                1745

tgt gat tat gtt tac aat ccg tat gct ttt gat ata caa cag tgg      17687
Cys Asp Tyr Val Tyr Asn Pro Tyr Ala Phe Asp Ile Gln Gln Trp
        1750                1755                1760

ggt tat gtt ggt tcc ttg agc cag aac cac cac acg ttc tgt aac      17732
Gly Tyr Val Gly Ser Leu Ser Gln Asn His His Thr Phe Cys Asn
        1765                1770                1775

att cat aga aac gag cat gat gct tct ggt gat gct gtt atg aca      17777
Ile His Arg Asn Glu His Asp Ala Ser Gly Asp Ala Val Met Thr
        1780                1785                1790

cgt tgt ttg gca gta cat gat tgt ttt gtc aaa aat gtt gat tgg      17822
Arg Cys Leu Ala Val His Asp Cys Phe Val Lys Asn Val Asp Trp
        1795                1800                1805

act gta acg tac ccc ttt att gca aat gag aaa ttt atc aat ggc      17867
Thr Val Thr Tyr Pro Phe Ile Ala Asn Glu Lys Phe Ile Asn Gly
        1810                1815                1820

tgt ggg cgt aat gtc cag gga cat gtt gtt cgc gca gcc ttg aaa      17912

Cys Gly Arg Asn Val Gln Gly His Val Val Arg Ala Ala Leu Lys
        1825                1830                1835

ttg tat aaa cct agt gtt att cat gat att ggt aat cct aaa ggt 17957
Leu Tyr Lys Pro Ser Val Ile His Asp Ile Gly Asn Pro Lys Gly
        1840                1845                1850

gta cgt tgt gct gtt act gat gcc aaa tgg tac tgt tat gac aag 18002
Val Arg Cys Ala Val Thr Asp Ala Lys Trp Tyr Cys Tyr Asp Lys
        1855                1860                1865

caa cct gtt aat agt aat gtc aag ttg ttg gat tat gat tat gca 18047
Gln Pro Val Asn Ser Asn Val Lys Leu Leu Asp Tyr Asp Tyr Ala
        1870                1875                1880

acc cat ggt caa ctt gat ggt ctt tgt tta ttc tgg aat tgt aat 18092
Thr His Gly Gln Leu Asp Gly Leu Cys Leu Phe Trp Asn Cys Asn
        1885                1890                1895

gtt gat atg tat cca gaa ttt tca att gtg tgt cgc ttt gac aca 18137
Val Asp Met Tyr Pro Glu Phe Ser Ile Val Cys Arg Phe Asp Thr
        1900                1905                1910

cgt act cgt tct gtt ttt aat tta gaa ggt gtt aat ggt ggt tct 18182
Arg Thr Arg Ser Val Phe Asn Leu Glu Gly Val Asn Gly Gly Ser
        1915                1920                1925

ctt tat gtt aac aaa cat gcg ttt cat aca cca gca tat gat aaa 18227
Leu Tyr Val Asn Lys His Ala Phe His Thr Pro Ala Tyr Asp Lys
        1930                1935                1940

cgt gct ttt gtt aaa tta aaa cct atg ccc ttt ttt tac ttt gat 18272
Arg Ala Phe Val Lys Leu Lys Pro Met Pro Phe Phe Tyr Phe Asp
        1945                1950                1955

gac agt gat tgt gat gtt gtg caa gaa caa gtt aat tat gta ccc 18317
Asp Ser Asp Cys Asp Val Val Gln Glu Gln Val Asn Tyr Val Pro
        1960                1965                1970

ctt cgc gct agt agt tgt gtt acc cgt tgt aat ata ggt ggt gct 18362
Leu Arg Ala Ser Ser Cys Val Thr Arg Cys Asn Ile Gly Gly Ala
        1975                1980                1985

gtt tgt tca aaa cat gca aat ttg tat caa aaa tat gtt gag gca 18407
Val Cys Ser Lys His Ala Asn Leu Tyr Gln Lys Tyr Val Glu Ala
        1990                1995                2000

tat aat aca ttt aca cag gct ggt ttt aac att tgg gta cca cat 18452
Tyr Asn Thr Phe Thr Gln Ala Gly Phe Asn Ile Trp Val Pro His
        2005                2010                2015

agt ttt gat gtt tat aat ttg tgg caa att ttt att gaa act aat 18497
Ser Phe Asp Val Tyr Asn Leu Trp Gln Ile Phe Ile Glu Thr Asn
        2020                2025                2030

tta caa agt ctt gaa aat ata gca ttt aat gtt gta aaa aaa ggg 18542
Leu Gln Ser Leu Glu Asn Ile Ala Phe Asn Val Val Lys Lys Gly
        2035                2040                2045

tgt ttt act ggt gtt gat ggt gag tta cct gtt gca gtt gtt aac 18587
Cys Phe Thr Gly Val Asp Gly Glu Leu Pro Val Ala Val Val Asn
        2050                2055                2060

gac aaa gtt ttt gtt cgc tat ggc gat gtt gac aac ttg gtt ttt 18632
Asp Lys Val Phe Val Arg Tyr Gly Asp Val Asp Asn Leu Val Phe
        2065                2070                2075

aca aat aaa aca aca ttg cct act aat gtt gct ttt gaa ttg ttt 18677

Thr Asn Lys Thr Thr Leu Pro Thr Asn Val Ala Phe Glu Leu Phe
        2080                2085                2090

gca aaa cga aaa atg ggt tta aca cca cca ttg tct att ctc aaa 18722
Ala Lys Arg Lys Met Gly Leu Thr Pro Pro Leu Ser Ile Leu Lys
        2095                2100                2105

aat ctt ggt gtt gtt gct aca tat aaa ttt gtt tta tgg gat tat 18767
Asn Leu Gly Val Val Ala Thr Tyr Lys Phe Val Leu Trp Asp Tyr
        2110                2115                2120

gaa gct gaa aga cct ttt acc tca tat act aag agt gta tgt aaa 18812
Glu Ala Glu Arg Pro Phe Thr Ser Tyr Thr Lys Ser Val Cys Lys
        2125                2130                2135

tac act gat ttt aat gag gat gtt tgt gtt tgt ttt gac aat agt 18857
Tyr Thr Asp Phe Asn Glu Asp Val Cys Val Cys Phe Asp Asn Ser
        2140                2145                2150

att cag ggt tcg tat gag cgt ttt acg ctt act acg aac gct gtt 18902
Ile Gln Gly Ser Tyr Glu Arg Phe Thr Leu Thr Thr Asn Ala Val
        2155                2160                2165

tta ttt tct act gtt gtc att aaa aat tta aca cct ata aag ttg 18947
Leu Phe Ser Thr Val Val Ile Lys Asn Leu Thr Pro Ile Lys Leu
        2170                2175                2180

aat ttt ggt atg ttg aat ggt atg cca gtt tct tct att aag agt 18992
Asn Phe Gly Met Leu Asn Gly Met Pro Val Ser Ser Ile Lys Ser
        2185                2190                2195

gat aaa ggt gtt gaa aaa tta gtt aat tgg tac aca tat gtt cgt 19037
Asp Lys Gly Val Glu Lys Leu Val Asn Trp Tyr Thr Tyr Val Arg
        2200                2205                2210

aaa aat ggt caa ttt caa gat cat tat gat ggt ttt tac act caa 19082
Lys Asn Gly Gln Phe Gln Asp His Tyr Asp Gly Phe Tyr Thr Gln
        2215                2220                2225

ggt agg aat tta tca gac ttt aca cca aga agt gat atg gag tat 19127
Gly Arg Asn Leu Ser Asp Phe Thr Pro Arg Ser Asp Met Glu Tyr
        2230                2235                2240

gat ttt ctt aac atg gat atg ggt gtt ttt att aat aaa tat ggt 19172
Asp Phe Leu Asn Met Asp Met Gly Val Phe Ile Asn Lys Tyr Gly
        2245                2250                2255

ctt gag gat ttt aat ttt gaa cat gtt gta tat ggt gat gtt tca 19217
Leu Glu Asp Phe Asn Phe Glu His Val Val Tyr Gly Asp Val Ser
        2260                2265                2270

aaa act aca tta gga ggt ctt cat ttg ttg ata tca cag ttt agg 19262
Lys Thr Thr Leu Gly Gly Leu His Leu Leu Ile Ser Gln Phe Arg
        2275                2280                2285

ctt agt aaa atg ggt gtt ttg aaa gct gat gat ttt gtc act gct 19307
Leu Ser Lys Met Gly Val Leu Lys Ala Asp Asp Phe Val Thr Ala
        2290                2295                2300

tct gac aca act ttg agg tgc tgt act gtt act tat ctt aat gaa 19352
Ser Asp Thr Thr Leu Arg Cys Cys Thr Val Thr Tyr Leu Asn Glu
        2305                2310                2315

ctt agt tca aaa gtt gtt tgt act tat atg gat ttg ttg ttg gac 19397
Leu Ser Ser Lys Val Val Cys Thr Tyr Met Asp Leu Leu Leu Asp
        2320                2325                2330

gac ttt gtt act ata cta aag agt tta gat ctt ggt gta ata tct 19442

Asp Phe Val Thr Ile Leu Lys Ser Leu Asp Leu Gly Val Ile Ser
        2335                2340                2345

aaa gtt cat gaa gtt att ata gat aat aaa cct tat agg tgg atg 19487
Lys Val His Glu Val Ile Ile Asp Asn Lys Pro Tyr Arg Trp Met
        2350                2355                2360

ttg tgg tgt aaa gat aac cac ttg tcc act ttt tat cca cag ttg 19532
Leu Trp Cys Lys Asp Asn His Leu Ser Thr Phe Tyr Pro Gln Leu
        2365                2370                2375

cag tct gct gaa tgg aag tgt ggt tat gct atg cca caa att tat 19577
Gln Ser Ala Glu Trp Lys Cys Gly Tyr Ala Met Pro Gln Ile Tyr
        2380                2385                2390

aag ctt caa cgt atg tgt ttg gaa cct tgt aat tta tat aat tat 19622
Lys Leu Gln Arg Met Cys Leu Glu Pro Cys Asn Leu Tyr Asn Tyr
        2395                2400                2405

ggt gct ggt att aag ttg cct agt ggt ata atg tta aat gtt gtt 19667
Gly Ala Gly Ile Lys Leu Pro Ser Gly Ile Met Leu Asn Val Val
        2410                2415                2420

aaa tac act cag ctt tgt caa tac cta aat agc act aca atg tgc 19712
Lys Tyr Thr Gln Leu Cys Gln Tyr Leu Asn Ser Thr Thr Met Cys
        2425                2430                2435

gta cct cat aat atg cgt gtt ttg cac tat ggt gct ggt tct gac 19757
Val Pro His Asn Met Arg Val Leu His Tyr Gly Ala Gly Ser Asp
        2440                2445                2450

aaa ggt gtg gca cct ggt aca act gtt tta aaa cgt tgg cta cca 19802
Lys Gly Val Ala Pro Gly Thr Thr Val Leu Lys Arg Trp Leu Pro
        2455                2460                2465

cct gat gca ata atc att gat aat gat atc aat gat tat gtt agt 19847
Pro Asp Ala Ile Ile Ile Asp Asn Asp Ile Asn Asp Tyr Val Ser
        2470                2475                2480

gat gca gat ttt agc att aca ggt gat tgt gct act gtt tac ctt 19892
Asp Ala Asp Phe Ser Ile Thr Gly Asp Cys Ala Thr Val Tyr Leu
        2485                2490                2495

gaa gat aag ttt gac tta ctt att tct gat atg tat gat ggt aga 19937
Glu Asp Lys Phe Asp Leu Leu Ile Ser Asp Met Tyr Asp Gly Arg
        2500                2505                2510

att aaa ttt tgt gat ggt gaa aac gtc tct aaa gat ggt ttt ttt 19982
Ile Lys Phe Cys Asp Gly Glu Asn Val Ser Lys Asp Gly Phe Phe
        2515                2520                2525

act tat ctt aat ggt gtt att aga gaa aaa tta gct att ggt ggt 20027
Thr Tyr Leu Asn Gly Val Ile Arg Glu Lys Leu Ala Ile Gly Gly
        2530                2535                2540

agt gtt gcc att aag att aca gaa tat agt tgg aat aag tat ctt 20072
Ser Val Ala Ile Lys Ile Thr Glu Tyr Ser Trp Asn Lys Tyr Leu
        2545                2550                2555

tat gaa tta ata caa aga ttt gct ttt tgg act ttg ttc tgc acg 20117
Tyr Glu Leu Ile Gln Arg Phe Ala Phe Trp Thr Leu Phe Cys Thr
        2560                2565                2570

tct gtt aat aca tcc tct tca gaa gct ttt ctt att ggt att aat 20162
Ser Val Asn Thr Ser Ser Ser Glu Ala Phe Leu Ile Gly Ile Asn
        2575                2580                2585

tat tta ggt gac ttt att caa ggt cct ttt ata gct ggt aac act 20207

Tyr Leu Gly Asp Phe Ile Gln Gly Pro Phe Ile Ala Gly Asn Thr
        2590                2595                2600

gtt cat gct aat tat ata ttt tgg cgt aat tct act att atg tct 20252
Val His Ala Asn Tyr Ile Phe Trp Arg Asn Ser Thr Ile Met Ser
        2605                2610                2615

ttg tca tac aat tca gtt tta gat tta agt aag ttt gaa tgt aaa 20297
Leu Ser Tyr Asn Ser Val Leu Asp Leu Ser Lys Phe Glu Cys Lys
        2620                2625                2630

cat aag gcc act gtt gtt gtt aca ctt aaa gat agt gat gta aat 20342
His Lys Ala Thr Val Val Val Thr Leu Lys Asp Ser Asp Val Asn
        2635                2640                2645

gat atg gtt ttg agt ttg att aag agt ggt agg ttg ttg tta cgt 20387
Asp Met Val Leu Ser Leu Ile Lys Ser Gly Arg Leu Leu Leu Arg
        2650                2655                2660

aat agt ggc cgt ttt ggt ggt ttt agt aat cat tta gtc tca act 20432
Asn Ser Gly Arg Phe Gly Gly Phe Ser Asn His Leu Val Ser Thr
        2665                2670                2675

aaa tga aacttttctt gattttgctt attttgcccc tggtttcttg cttttctaca 20488
Lys

tgtaacagta atgctagtat ttctatgtta caattaggtg ttcctgataa ctcttcaact 20548

attgtcacag gtttgttgcc agtccattgg atttgtgcta atcagagtac atctagttac 20608

ccagccaacg gctttttcta tattgatgtt ggtaaacacc gtagtgcctt tgcactccat 20668

agtggttatt atgatgctaa ccagtattat atttatctca ctaataaaat acatttaaat 20728

gctcctgtca ctctgaagat ttgtaagttt ggaaacactt cttttgattt tttaagtaat 20788

gtttctactt ctcatgattg tatagttaat ttgtcattca cagaacagtt aggtgtgcct 20848

ttgggcataa ctatatcggg tgaaactgta cgtttgcatt tatataatgc aactcgtact 20908

ttttatgtgc cggccgctta taaacttact aaacttagtg ttaaatgtta ctttagtgaa 20968

tcctgtgttt ttagtgttgt caatgccacc attactgtta atgtcaccac acttaatggc 21028

cgtatagtta actacactgt ttgtgatgat tgtaatggtt atactgataa catattttct 21088

gttcaacagg atggccgcat tcctaatggt ttccctttta ataattggtt tttgttaact 21148

aatggttcca cattagtgga cggggtctct agactttatc aaccactccg tttaacttgt 21208

ttatggcctg tacctggtct taaatcttca actggttttg tttattttaa tgccactggt 21268

tctgatgtta attgtaacgg ctatcaacat aattctgttg ctgatgttat gcgttacaat 21328

cttaacctca gtgctaattc tgtggacaat cttaagagtg gtgttatagt ttttaaaact 21388

ttacagtacg atgttttgtt ttattgtagt aattcttctt caggtgttct tgacaccaca 21448

ataccttttg gcccttcctc tcaaccttat tactgtttta taaacagtac tatcaacact 21508

actcatgtta gcacttttgt gggtatttta ccacccactg tgcgtgaaat tgttgttgct 21568

agaactggtc agttttatat taatggtttt aagtatttcg atttgggttt catagaagct 21628

gtcaatttta atgtcacgac tgctagtgcc acagattttt ggacggttgc atttgctact 21688

tttgttgatg ttttggttaa tgttagtgca actaacattc aaaacttact ttattgcgat 21748

tctccatttg aaaagttgca gtgtgagcac ttgcagtttg gattgcaaga tggtttttat 21808
tctgcaaatt ttcttgatga taatgttttg cctgagactt atgttgcact ccccatttat 21868
tatcaacata cggacataaa ttttactgca actgcatctt ttggtggttc ttgttatgtt 21928
tgtaaaccac gccaggttaa tatatctctt aatggtaaca cttcagtgtg tgttagaaca 21988
tctcattttt caattaggta tatttataac cgcgttaaga gtggttcacc aggtgactct 22048
tcatggcata tttatttaaa gagtggcact tgtccatttt ctttttctaa gttaaataat 22108
tttcaaaagt ttaagactat ttgtttctca accgtcgaag tgcctggtag ttgtaatttt 22168
ccacttgaag ccacctggca ttacacttct tatactattg ttggtgcttt gtatgttact 22228
tggtctgaag gtaattccat tactggtgta ccttatcctg tctctggtat tcgtgagttt 22288
agtaatttag ttttaaataa ttgtaccaaa tataatattt atgattatgt tggtactgga 22348
attatacgtt cttcaaacca gtcacttgct ggtggtatta catatgtttc taactctggt 22408
aatttacttg gttttaaaaa tgtttccact ggtaacattt ttattgtgac accatgtaac 22468
caaccagatc aagtagctgt ttatcaacaa agcattattg gtgccatgac cgctgttaat 22528
gagtctagat atggcttgca aaacttacta cagttaccta acttttatta tgttagtaat 22588
ggtggtaaca attgcactac ggctgttatg atttattcta attttggtat ttgtgctgat 22648
ggttctttaa ttcctgttcg tccgcgtaat tctagtgata atggtatttc agccataatc 22708
actgctaatt tatccattcc ctctaactgg actacttcag ttcaagttga gtacctccaa 22768
attactagta ctccaatagt tgttgattgt gctacttatg tgtgtaatgg taaccctcgt 22828
tgtaagaatc tacttaagca gtatacttct gcttgtaaaa ctattgaaga tgccttacga 22888
cttagtgctc atttggaaac taatgatgtt agtagtatgc taactttcga tagcaatgct 22948
tttagtttgg ctaatgttac tagttttgga gattataacc tttctagtgt tttacctcag 23008
agaaacattc attcaagccg tatagcagga cgtagtgctt tggaagattt gttgtttagc 23068
aaagttgtta catctggttt gggtactgtt gatgttgact ataagtcttg tactaaaggt 23128
ctttctattg ctgaccttgc ttgtgctcag tactacaatg gcataatggt tttgccaggt 23188
gttgctgatg ctgaacgtat ggccatgtac acaggttctc ttataggtgg catggtgctc 23248
ggaggtctta catcagcagc cgccatacct ttttctttgg cactgcaagc acgacttaac 23308
tatgttgctt tacaaactga tgtgcttcaa gaaaatcaga aaattttggc tgcatcattt 23368
aataaggcta ttaataatat tgttgcttct tttagtagcg ttaatgatgc tattacacat 23428
actgcagagg ctatacatac tgttactatt gcacttaata agattcagga tgttgttaat 23488
caacagggta gtgctcttaa ccatctcact tcacaattga gacataattt tcaggccatt 23548
tctaattcaa ttcatgctat ttatgaccgg cttgattcaa ttcaagccga tcaacaagtt 23608
gacagattaa ttactggacg gcttgcagct ttgaatgcat ttgtttccca agttttgaat 23668
aaatatactg aagttcgtgg ttccagacgc ttagcacagc agaagattaa tgaatgtgtc 23728
aagtcacaat ctaatagata tggtttttgt ggcaatggca ctcacatctt ttcaatcgtc 23788

aactcagctc cagatggttt gctttttctt catactgttt tgctgccaac tgattacaag 23848

aatgtaaagg cgtggtctgg tatctgtgtt gatggcattt atggctatgt tctgcgtcaa 23908

cctaacttgg ttctttattc tgataatggt gtctttcgtg taacttccag ggtcatgttt 23968

caacctcgtt tacctgtttt gtctgatttt gtgcaaatat ataattgtaa tgttactttt 24028

gttaacatat ctcgtgtcga gttacatact gtcatacctg actacgttga tgttaataaa 24088

acattacaag agtttgcaca aaacttacca aagtatgtta agcctaattt tgacttgact 24148

ccttttaatt taacatatct taatttgagt tctgagttga agcaactcga agctaaaact 24208

gctagtcttt tccaaactac tgttgaatta caaggtctta ttgatcagat taacagtaca 24268

tatgttgatt tgaagttgct taataggttt gaaaattata tcaaatggcc ttggtgggtt 24328

tggctcatta tttctgttgt ttttgttgta ttgttgagtc ttcttgtgtt ttgttgtctt 24388

tctacaggtt gttgtggttg ttgcaattgt ttaacttcat caatgcgagg ctgttgtgat 24448

tgtggttcaa ctaaacttcc ttattatgaa tttgaaaagg tccacgttca ata atg 24504
Met

cct ttc ggt ggc cta ttt caa ctt act ctt gaa agt act att aat 24549
Pro Phe Gly Gly Leu Phe Gln Leu Thr Leu Glu Ser Thr Ile Asn
2680                2685                2690

aag agt gtg gct aat ctc aaa tta cca cct cat gat gtt act gtc 24594
Lys Ser Val Ala Asn Leu Lys Leu Pro Pro His Asp Val Thr Val
2695                2700                2705

ttg cgt gac aat ctt aaa cct gtt act aca ctt agt act atc act 24639
Leu Arg Asp Asn Leu Lys Pro Val Thr Thr Leu Ser Thr Ile Thr
2710                2715                2720

gct tat ttg tta gtt agt ttg ttt gtc act tat ttt gct tta ttc 24684
Ala Tyr Leu Leu Val Ser Leu Phe Val Thr Tyr Phe Ala Leu Phe
2725                2730                2735

aaa cct ctt act gct aga ggt cgt gtt gct tgt ttt gtt tta aaa 24729
Lys Pro Leu Thr Ala Arg Gly Arg Val Ala Cys Phe Val Leu Lys
2740                2745                2750

cta ttg aca cta tct gtc tat gtg cct tta ttg gtt ctt ttt ggt 24774
Leu Leu Thr Leu Ser Val Tyr Val Pro Leu Leu Val Leu Phe Gly
2755                2760                2765

atg tat ctt gac agt ttt ata att ttt ttt cta cgc tgt tgt ttc 24819
Met Tyr Leu Asp Ser Phe Ile Ile Phe Phe Leu Arg Cys Cys Phe
2770                2775                2780

gat tca tac atg ttg gct att atg cct atc tct aat aaa aat ttt 24864
Asp Ser Tyr Met Leu Ala Ile Met Pro Ile Ser Asn Lys Asn Phe
2785                2790                2795

tca ttt gtt ttg ttc aat gtt act aaa cta tgc ttc gtt tca ggc 24909
Ser Phe Val Leu Phe Asn Val Thr Lys Leu Cys Phe Val Ser Gly
2800                2805                2810

aag tgt tgg tat ctt gaa caa tca ttt tat gaa aat cgt ttt gct 24954
Lys Cys Trp Tyr Leu Glu Gln Ser Phe Tyr Glu Asn Arg Phe Ala
2815                2820                2825

gct att tat ggt ggt gac cac tat gtc gtt tta ggt ggt gaa act 24999

Ala Ile Tyr Gly Gly Asp His Tyr Val Val Leu Gly Gly Glu Thr
2830                2835                2840

att act ttt gtt tct ttt gat gac ctt tat gtt gct att aga ggt 25044
Ile Thr Phe Val Ser Phe Asp Asp Leu Tyr Val Ala Ile Arg Gly
2845                2850                2855

tct tgt gaa aag aac cta caa ctt atg cgt aag gtt gac ttg tat 25089
Ser Cys Glu Lys Asn Leu Gln Leu Met Arg Lys Val Asp Leu Tyr
2860                2865                2870

aat ggt gct gtc att tac att ttt gcc gaa gag cct gtt gtt ggt 25134
Asn Gly Ala Val Ile Tyr Ile Phe Ala Glu Glu Pro Val Val Gly
2875                2880                2885

ata gtt tac tcc tct caa cta tac gaa gat gtt cct tcg att aat 25179
Ile Val Tyr Ser Ser Gln Leu Tyr Glu Asp Val Pro Ser Ile Asn
2890                2895                2900

tga tgacaatggc attgtcctca attctatttt atggctcctt gttatgatat 25232

ttttctttgt gttggcaatg acctttatta aactgattca attgtgtttt acttgtcatt 25292

atttttttag taggacatta tatcaaccag tttataaaat ttttcttgct taccaagatt 25352

atatgcaaat agcacctgtt ccagctgaag tactaaatgt ctaaactaaa cgatgtctaa 25412

tagtagtgtg cctctttcag aggtttatgt ccatttacgt aactggaact ttagttggaa 25472

tttaattcta acagttttta tagttgtgtt gcagtatggg cattataagt atagcagact 25532

tctttatggt ttaaagatgt ctgttttatg gtgtttatgg ccacttgttc tagctttgtc 25592

tatttttgac tgttttgtca attttaatgt ggactgggtc ttttttggtt ttagtattct 25652

tatgtctatt attacacttt gtttatgggt tatgtatttt gttaatagtt tcagactttg 25712

gcgccgtgtt aaaacttttt gggcttttaa tcctgaaact aatgcaatca tctctctcca 25772

ggtttatgga cataattatt acttaccggt gatggctgca cctacaggtg ttacattaac 25832

acttcttagt ggtgtacttc ttgttgatgg ccataagatt gctactcgtg ttcaagtggg 25892

tcagttgcct aaatatgtaa tagttgctac acctagtacc acaattgttt gtgaccgtgt 25952

tggtcgctct gttaatgaaa caagccagac tggttgggca ttctacgtcc gtgctaaaca 26012

tggtgatttt tctggtgttg cctctcagga gggtgttttg tcagaaagag agaagttgct 26072

tcatttaatc taaactaaac aaaatggcta gtgtaaattg ggccgatgac agagctgcta 26132

ggaagaaatt tcctcctcct tcattttaca tgcctctttt ggttagttct gataaggcac 26192

catatagggt cattcccagg aatcttgtcc ctattggtaa gggtaataaa gatgagcaga 26252

ttggttattg gaatgttcaa gagcgttggc gtatgcgcag ggggcaacgt gttgatttgc 26312

ctcctaaagt tcatttttat tacctaggta ctggacctca taaggacctt aaattcagac 26372

aacgttctga tggtgttgtt tgggttgcta aggaaggtgc taaaactgtt aataccagtc 26432

ttggtaatcg caaacgtaat cagaaacctt tggaaccaaa gttctctatt gctttgcctc 26492

cagagctctc tgttgttgag tttgaggatc gctctaataa ctcatctcgt gctagcagtc 26552

gttcttcaac tcgtaacaac tcacgagact cttctcgtag tacttcaaga caacagtctc 26612

gcactcgttc tgattctaac cagtcttctt cagatcttgt tgctgctgtt actttggctt 26672

taaagaactt aggttttgat aaccagtcga agtcacctag ttcttctggt acttccactc 26732

ctaagaaacc taataagcct ctttctcaac ccagggctga taagccttct cagttgaaga 26792

aacctcgttg gaagcgtgtt cctaccagag aggaaaatgt tattcagtgc tttggtcctc 26852

gtgattttaa tcacaatatg ggggattcag atcttgttca gaatggtgtt gatgccaagg 26912

gttttccaca gcttgctgaa ttgattccta atcaggctgc gttattcttt gatagtgagg 26972

ttagcactga tgaagtgggt gataatgttc agattaccta cacctacaaa atgcttgtag 27032

ctaaggataa taagaacctt cctaagttca ttgagcagat tagtgctttt actaaaccca 27092

gttctatcaa agaaatgcag tcacaatcat ctcatgttgc tcagaacaca gtacttaatg 27152

cttctattcc agaatctaaa ccattggctg atgatgattc agccattata gaaattgtca 27212

acgaggtttt gcattaaatt gttttgtaat tccagttgaa tgtttattat tattagttgc 27272

aaccccatgc gtttagcgca tgataagggt ttagtcttac acacaatggt aggccagtga 27332

tagtaaagtg taagtaattt gctatcatat taacatgtct agaggaaagt cagaactttt 27392

tctgtttgtg ttgttggagt acttaaagat cgcataggcg cgccaacaat ggaagagcca 27452

acaacatatc taaaaatgtt ttgtctggta cttgttaatg atattgtttt tgatatggat 27512

acacaaaaaa aaaaaaaaaa 27532

<210> 8

<211> 2678

<212> PRT

<213> EMCR Coronavirus

<220>

<221> misc_feature

<222> (844)..(844)

<223> The 'Xaa' at location 844 stands for Lys, Glu, Gln, or a stop cod
on.

<220>

<221> misc_feature

<222> (14931)..(14931)

<223> "n" stands for "k"

<400> 8

Arg Ala Arg Gly Ser Ser Ala Ala Arg Leu Glu Pro Cys Asn Gly Thr
1               5                   10                  15

Asp Ile Asp Lys Cys Val Arg Ala Phe Asp Ile Tyr Asn Lys Asn Val
            20                  25                  30

Ser Phe Leu Gly Lys Cys Leu Lys Met Asn Cys Val Arg Phe Lys Asn
        35                  40                  45

Ala Asp Leu Lys Asp Gly Tyr Phe Val Ile Lys Arg Cys Thr Lys Ser
    50                  55                  60

Val Met Glu His Glu Gln Ser Met Tyr Asn Leu Leu Asn Phe Ser Gly
65                  70                  75                  80

Ala Leu Ala Glu His Asp Phe Phe Thr Trp Lys Asp Gly Arg Val Ile
                85                  90                  95

Tyr Gly Asn Val Ser Arg His Asn Leu Thr Lys Tyr Thr Met Met Asp
            100                 105                 110

Leu Val Tyr Ala Met Arg Asn Phe Asp Glu Gln Asn Cys Asp Val Leu
        115                 120                 125

Lys Glu Val Leu Val Leu Thr Gly Cys Cys Asp Asn Ser Tyr Phe Asp
    130                 135                 140

Ser Lys Gly Trp Tyr Asp Pro Val Glu Asn Glu Asp Ile His Arg Val
145                 150                 155                 160

Tyr Ala Ser Leu Gly Lys Ile Val Ala Arg Ala Met Leu Lys Cys Val
                165                 170                 175

Ala Leu Cys Asp Ala Met Val Ala Lys Gly Val Val Gly Val Leu Thr
            180                 185                 190

Leu Asp Asn Gln Asp Leu Asn Gly Asn Phe Tyr Asp Phe Gly Asp Phe
        195                 200                 205

Val Val Ser Leu Pro Asn Met Gly Val Pro Cys Cys Thr Ser Tyr Tyr
    210                 215                 220

Ser Tyr Met Met Pro Ile Met Gly Leu Thr Asn Cys Leu Ala Ser Glu
225                 230                 235                 240

Cys Phe Val Lys Ser Asp Ile Phe Gly Ser Asp Phe Lys Thr Phe Asp
                245                 250                 255

Leu Leu Lys Tyr Asp Phe Thr Glu His Lys Glu Asn Leu Phe Asn Lys
            260                 265                 270

Tyr Phe Lys His Trp Ser Phe Asp Tyr His Pro Asn Cys Ser Asp Cys
        275                 280                 285

Tyr Asp Asp Met Cys Val Ile His Cys Ala Asn Phe Asn Thr Leu Phe
    290                 295                 300

Ala Thr Thr Ile Pro Gly Thr Ala Phe Gly Pro Leu Cys Arg Lys Val
305                 310                 315                 320

Phe Ile Asp Gly Val Pro Leu Val Thr Thr Ala Gly Tyr His Phe Lys
                325                 330                 335

Gln Leu Gly Leu Val Trp Asn Lys Asp Val Asn Thr His Ser Val Arg
            340                 345                 350

Leu Thr Ile Thr Glu Leu Leu Gln Phe Val Thr Asp Pro Ser Leu Ile
        355                 360                 365

Ile Ala Ser Ser Pro Ala Leu Val Asp Gln Arg Thr Ile Cys Phe Ser
    370                 375                 380

Val Ala Ala Leu Ser Thr Gly Leu Thr Asn Gln Val Val Lys Pro Gly
385                 390                 395                 400

His Phe Asn Glu Glu Phe Tyr Asn Phe Leu Arg Leu Arg Gly Phe Phe
                405                 410                 415

Asp Glu Gly Ser Glu Leu Thr Leu Lys His Phe Phe Phe Ala Gln Asn
            420                 425                 430

Gly Asp Ala Ala Val Lys Asp Phe Asp Phe Tyr Arg Tyr Asn Lys Pro
        435                 440                 445

Thr Ile Leu Asp Ile Cys Gln Ala Arg Val Thr Tyr Lys Ile Val Ser
    450                 455                 460

Arg Tyr Phe Asp Ile Tyr Glu Gly Gly Cys Ile Lys Ala Cys Glu Val
465                 470                 475                 480

Val Val Thr Asn Leu Asn Lys Ser Ala Gly Trp Pro Leu Asn Lys Phe
                485                 490                 495

Gly Lys Ala Ser Leu Tyr Tyr Glu Ser Ile Ser Tyr Glu Glu Gln Asp
            500                 505                 510

Ala Leu Phe Ala Leu Thr Lys Arg Asn Val Leu Pro Thr Met Thr Gln
        515                 520                 525

Leu Asn Leu Lys Tyr Ala Ile Ser Gly Lys Glu Arg Ala Arg Thr Val
    530                 535                 540

Gly Gly Val Ser Leu Leu Ser Thr Met Thr Thr Arg Gln Tyr His Gln
545                 550                 555                 560

Lys His Leu Lys Ser Ile Val Asn Thr Arg Asn Ala Thr Val Val Ile
                565                 570                 575

Gly Thr Thr Lys Phe Tyr Gly Gly Trp Asn Asn Met Leu Arg Thr Leu
            580                 585                 590

Ile Asp Gly Val Glu Asn Pro Met Leu Met Gly Trp Asp Tyr Pro Lys
        595                 600                 605

Cys Asp Arg Ala Leu Pro Asn Met Ile Arg Met Ile Ser Ala Met Val
    610                 615                 620

Leu Gly Ser Lys His Val Asn Cys Cys Thr Val Thr Asp Arg Phe Tyr
625                 630                 635                 640

Arg Leu Gly Asn Glu Leu Ala Gln Val Leu Thr Glu Val Val Tyr Ser
                645                 650                 655

Asn Gly Gly Phe Tyr Phe Lys Pro Gly Gly Thr Thr Ser Gly Asp Ala
            660                 665                 670

Ser Thr Ala Tyr Ala Asn Ser Ile Phe Asn Ile Phe Gln Ala Val Ser
        675                 680                 685

Ser Asn Ile Asn Arg Leu Leu Ser Val Pro Ser Asp Ser Cys Asn Asn
    690                 695                 700

Val Asn Val Arg Asp Leu Gln Arg Arg Leu Tyr Asp Asn Cys Tyr Arg
705                 710                 715                 720

Leu Thr Ser Val Glu Glu Ser Phe Ile Asp Asp Tyr Tyr Gly Tyr Leu
                725                 730                 735

Arg Lys His Phe Ser Met Met Ile Leu Ser Asp Asp Gly Val Val Cys
            740                 745                 750

Tyr Asn Lys Asp Tyr Ala Glu Leu Gly Tyr Ile Ala Asp Ile Ser Ala
        755                 760                 765

Phe Lys Ala Thr Leu Tyr Tyr Gln Asn Asn Val Phe Met Ser Thr Ser
    770                 775                 780

Lys Cys Trp Val Glu Glu Asp Leu Thr Lys Gly Pro His Glu Phe Cys
785                 790                 795                 800

Ser Gln His Thr Met Gln Ile Val Asp Lys Asp Gly Thr Tyr Tyr Leu
                805                 810                 815

Pro Tyr Pro Asp Pro Ser Arg Ile Leu Ser Ala Gly Val Phe Val Asp
            820                 825                 830

Asp Val Val Lys Thr Asp Ala Val Val Leu Leu Xaa Arg Tyr Val Ser
        835                 840                 845

Leu Ala Ile Asp Ala Tyr Pro Leu Ser Lys His Pro Asn Ser Glu Tyr
    850                 855                 860

Arg Lys Val Phe Tyr Val Leu Leu Asp Trp Val Lys His Leu Asn Lys
865                 870                 875                 880

Asn Leu Asn Glu Gly Val Leu Glu Ser Phe Ser Val Thr Leu Leu Asp
                885                 890                 895

Asn Gln Glu Asp Lys Phe Trp Cys Glu Asp Phe Tyr Ala Ser Met Tyr
            900                 905                 910

Glu Asn Ser Thr Ile Leu Gln Ala Ala Gly Leu Cys Val Val Cys Gly
        915                 920                 925

Ser Gln Thr Val Leu Arg Cys Gly Asp Cys Leu Arg Lys Pro Met Leu
    930                 935                 940

Cys Thr Lys Cys Ala Tyr Asp His Val Phe Gly Thr Asp His Lys Phe
945                 950                 955                 960

Ile Leu Ala Ile Thr Pro Tyr Val Cys Asn Ala Ser Gly Cys Gly Val
                965                 970                 975

Ser Asp Val Lys Lys Leu Tyr Leu Gly Gly Leu Asn Tyr Tyr Cys Thr
            980                 985                 990

Asn His Lys Pro Gln Leu Ser Phe  Pro Leu Cys Ser Ala  Gly Asn Ile
        995                 1000                 1005

Phe Gly  Leu Tyr Lys Asn Ser  Ala Thr Gly Ser Leu  Asp Val Glu
    1010                 1015                 1020

Val Phe  Asn Arg Leu Ala Thr  Ser Asp Trp Thr Asp  Val Arg Asp
    1025                 1030                 1035

Tyr Lys  Leu Ala Asn Asp Val  Lys Asp Thr Leu Arg  Leu Phe Ala
    1040                 1045                 1050

Ala Glu  Thr Ile Lys Ala Lys  Glu Glu Ser Val Lys  Ser Ser Tyr
    1055                 1060                 1065

Ala Phe  Ala Thr Leu Lys Glu  Val Val Gly Pro Lys  Glu Leu Leu
    1070                 1075                 1080

Leu Ser  Trp Glu Ser Gly Lys  Val Lys Pro Pro Leu  Asn Arg Asn
    1085                 1090                 1095

Ser Val  Phe Thr Cys Phe Gln  Ile Ser Lys Asp Ser  Lys Phe Gln
    1100                 1105                 1110

Ile Gly Glu Phe Ile Phe Glu Lys Val Glu Tyr Gly Ser Asp Thr
    1115                1120                1125

Val Thr Tyr Lys Ser Thr Val Thr Thr Lys Leu Val Pro Gly Met
    1130                1135                1140

Ile Phe Val Leu Thr Ser His Asn Val Gln Pro Leu Arg Ala Pro
    1145                1150                1155

Thr Ile Ala Asn Gln Glu Lys Tyr Ser Ser Ile Tyr Lys Leu His
    1160                1165                1170

Pro Ala Phe Asn Val Ser Asp Ala Tyr Ala Asn Leu Val Pro Tyr
    1175                1180                1185

Tyr Gln Leu Ile Gly Lys Gln Lys Ile Thr Thr Ile Gln Gly Pro
    1190                1195                1200

Pro Gly Ser Gly Lys Ser His Cys Ser Ile Gly Leu Gly Leu Tyr
    1205                1210                1215

Tyr Pro Gly Ala Arg Ile Val Phe Val Ala Cys Ala His Ala Ala
    1220                1225                1230

Val Asp Ser Leu Cys Ala Lys Ala Met Thr Val Tyr Ser Ile Asp
    1235                1240                1245

Lys Cys Thr Arg Ile Ile Pro Ala Arg Ala Arg Val Glu Cys Tyr
    1250                1255                1260

Ser Gly Phe Lys Pro Asn Asn Thr Ser Ala Gln Tyr Ile Phe Ser
    1265                1270                1275

Thr Val Asn Ala Leu Pro Glu Cys Asn Ala Asp Ile Val Val Val
    1280                1285                1290

Asp Glu Val Ser Met Cys Thr Asn Tyr Asp Leu Ser Val Ile Asn
    1295                1300                1305

Gln Arg Leu Ser Tyr Lys His Ile Val Tyr Val Gly Asp Pro Gln
    1310                1315                1320

Gln Leu Pro Ala Pro Arg Val Met Ile Thr Lys Gly Val Met Glu
    1325                1330                1335

Pro Val Asp Tyr Asn Val Val Thr Gln Arg Met Cys Ala Ile Gly
    1340                1345                1350

Pro Asp Val Phe Leu His Lys Cys Tyr Arg Cys Pro Ala Glu Ile
    1355                1360                1365

Val Asn Thr Val Ser Glu Leu Val Tyr Glu Asn Lys Phe Val Pro
    1370                1375                1380

Val Lys Pro Ala Ser Lys Gln Cys Phe Lys Ile Phe Phe Lys Gly
    1385                1390                1395

Asn Val Gln Val Asp Asn Gly Ser Ser Ile Asn Arg Lys Gln Leu
    1400                1405                1410

Glu Ile Val Lys Leu Phe Leu Val Lys Asn Pro Ser Trp Ser Lys
    1415                1420                1425

Ala Val Phe Ile Ser Pro Tyr Asn Ser Gln Asn Tyr Val Ala Ser
    1430                1435                1440

Arg Phe Leu Gly Leu Gln Ile Gln Thr Val Asp Ser Ser Gln Gly
    1445                1450                1455

Ser Glu Tyr Asp Tyr Val Ile Tyr Ala Gln Thr Ser Asp Thr Ala
    1460                1465                1470

His Ala Cys Asn Val Asn Arg Phe Asn Val Ala Ile Thr Arg Ala
    1475                1480                1485

Lys Lys Gly Ile Phe Cys Val Met Cys Asp Lys Thr Leu Phe Asp
    1490                1495                1500

Ser Leu Lys Phe Phe Glu Ile Lys His Ala Asp Leu His Ser Ser
    1505                1510                1515

Gln Val Cys Gly Leu Phe Lys Asn Cys Thr Arg Thr Pro Leu Asn
    1520                1525                1530

Leu Pro Pro Thr His Ala His Thr Phe Leu Ser Leu Ser Asp Gln
    1535                1540                1545

Phe Lys Thr Thr Gly Asp Leu Ala Val Gln Ile Gly Ser Asn Asn
    1550                1555                1560

Val Cys Thr Tyr Glu His Val Ile Ser Phe Met Gly Phe Arg Phe
    1565                1570                1575

Asp Ile Ser Ile Pro Gly Ser His Ser Leu Phe Cys Thr Arg Asp
    1580                1585                1590

Phe Ala Ile Arg Asn Val Arg Gly Trp Leu Gly Met Asp Val Glu
    1595                1600                1605

Ser Ala His Val Cys Gly Asp Asn Ile Gly Thr Asn Val Pro Leu
    1610                1615                1620

Gln Val Gly Phe Ser Asn Gly Val Asn Phe Val Val Gln Thr Glu
    1625                1630                1635

Gly Cys Val Ser Thr Asn Phe Gly Asp Val Ile Lys Pro Val Cys
    1640                1645                1650

Ala Lys Ser Pro Pro Gly Glu Gln Phe Arg His Leu Val Pro Phe
    1655                1660                1665

Leu Arg Lys Gly Gln Pro Trp Leu Ile Val Arg Arg Arg Ile Val
    1670                1675                1680

Gln Met Ile Ser Asp Tyr Leu Ser Asn Leu Ser Asp Ile Leu Val
    1685                1690                1695

Phe Val Leu Trp Ala Gly Ser Leu Glu Leu Thr Thr Met Arg Tyr
    1700                1705                1710

Phe Val Lys Ile Gly Pro Ile Lys Tyr Cys Tyr Cys Gly Asn Ser
    1715                1720                1725

Ala Thr Cys Tyr Asn Ser Val Ser Asn Glu Tyr Cys Cys Phe Lys
    1730                1735                1740

His Ala Leu Gly Cys Asp Tyr Val Tyr Asn Pro Tyr Ala Phe Asp
    1745                1750                1755

Ile Gln Gln Trp Gly Tyr Val Gly Ser Leu Ser Gln Asn His His
    1760                1765                1770

Thr Phe Cys Asn Ile His Arg Asn Glu His Asp Ala Ser Gly Asp
    1775                1780                1785

Ala Val Met Thr Arg Cys Leu Ala Val His Asp Cys Phe Val Lys
    1790                1795                1800

Asn Val Asp Trp Thr Val Thr Tyr Pro Phe Ile Ala Asn Glu Lys
    1805                1810                1815

Phe Ile Asn Gly Cys Gly Arg Asn Val Gln Gly His Val Val Arg
    1820                1825                1830

Ala Ala Leu Lys Leu Tyr Lys Pro Ser Val Ile His Asp Ile Gly
    1835                1840                1845

Asn Pro Lys Gly Val Arg Cys Ala Val Thr Asp Ala Lys Trp Tyr
    1850                1855                1860

Cys Tyr Asp Lys Gln Pro Val Asn Ser Asn Val Lys Leu Leu Asp
    1865                1870                1875

Tyr Asp Tyr Ala Thr His Gly Gln Leu Asp Gly Leu Cys Leu Phe
    1880                    1885                    1890

Trp Asn Cys Asn Val Asp Met Tyr Pro Glu Phe Ser Ile Val Cys
    1895                    1900                    1905

Arg Phe Asp Thr Arg Thr Arg Ser Val Phe Asn Leu Glu Gly Val
    1910                    1915                    1920

Asn Gly Gly Ser Leu Tyr Val Asn Lys His Ala Phe His Thr Pro
    1925                    1930                    1935

Ala Tyr Asp Lys Arg Ala Phe Val Lys Leu Lys Pro Met Pro Phe
    1940                    1945                    1950

Phe Tyr Phe Asp Asp Ser Asp Cys Asp Val Val Gln Glu Gln Val
    1955                    1960                    1965

Asn Tyr Val Pro Leu Arg Ala Ser Ser Cys Val Thr Arg Cys Asn
    1970                    1975                    1980

Ile Gly Gly Ala Val Cys Ser Lys His Ala Asn Leu Tyr Gln Lys
    1985                    1990                    1995

Tyr Val Glu Ala Tyr Asn Thr Phe Thr Gln Ala Gly Phe Asn Ile
    2000                    2005                    2010

Trp Val Pro His Ser Phe Asp Val Tyr Asn Leu Trp Gln Ile Phe
    2015                    2020                    2025

Ile Glu Thr Asn Leu Gln Ser Leu Glu Asn Ile Ala Phe Asn Val
    2030                    2035                    2040

Val Lys Lys Gly Cys Phe Thr Gly Val Asp Gly Glu Leu Pro Val
    2045                    2050                    2055

Ala Val Val Asn Asp Lys Val Phe Val Arg Tyr Gly Asp Val Asp
    2060                    2065                    2070

Asn Leu Val Phe Thr Asn Lys Thr Thr Leu Pro Thr Asn Val Ala
    2075                    2080                    2085

Phe Glu Leu Phe Ala Lys Arg Lys Met Gly Leu Thr Pro Pro Leu
    2090                    2095                    2100

Ser Ile Leu Lys Asn Leu Gly Val Val Ala Thr Tyr Lys Phe Val
    2105                    2110                    2115

Leu Trp Asp Tyr Glu Ala Glu Arg Pro Phe Thr Ser Tyr Thr Lys
    2120                    2125                    2130

Ser Val Cys Lys Tyr Thr Asp Phe Asn Glu Asp Val Cys Val Cys
    2135                    2140                    2145

Phe Asp Asn Ser Ile Gln Gly Ser Tyr Glu Arg Phe Thr Leu Thr
    2150                    2155                    2160

Thr Asn Ala Val Leu Phe Ser Thr Val Val Ile Lys Asn Leu Thr
    2165                    2170                    2175

Pro Ile Lys Leu Asn Phe Gly Met Leu Asn Gly Met Pro Val Ser
    2180                    2185                    2190

Ser Ile Lys Ser Asp Lys Gly Val Glu Lys Leu Val Asn Trp Tyr
    2195                    2200                    2205

Thr Tyr Val Arg Lys Asn Gly Gln Phe Gln Asp His Tyr Asp Gly
    2210                    2215                    2220

Phe Tyr Thr Gln Gly Arg Asn Leu Ser Asp Phe Thr Pro Arg Ser
    2225                    2230                    2235

Asp Met Glu Tyr Asp Phe Leu Asn Met Asp Met Gly Val Phe Ile
    2240                    2245                    2250

Asn Lys Tyr Gly Leu Glu Asp Phe Asn Phe Glu His Val Val Tyr
    2255                    2260                    2265

Gly Asp Val Ser Lys Thr Thr Leu Gly Gly Leu His Leu Leu Ile
    2270                    2275                    2280

Ser Gln Phe Arg Leu Ser Lys Met Gly Val Leu Lys Ala Asp Asp
    2285                    2290                    2295

Phe Val Thr Ala Ser Asp Thr Thr Leu Arg Cys Cys Thr Val Thr
    2300                    2305                    2310

Tyr Leu Asn Glu Leu Ser Ser Lys Val Val Cys Thr Tyr Met Asp
    2315                    2320                    2325

Leu Leu Leu Asp Asp Phe Val Thr Ile Leu Lys Ser Leu Asp Leu
    2330                    2335                    2340

Gly Val Ile Ser Lys Val His Glu Val Ile Ile Asp Asn Lys Pro
    2345                    2350                    2355

Tyr Arg Trp Met Leu Trp Cys Lys Asp Asn His Leu Ser Thr Phe
    2360                    2365                    2370

Tyr Pro Gln Leu Gln Ser Ala Glu Trp Lys Cys Gly Tyr Ala Met
    2375                    2380                    2385

Pro Gln Ile Tyr Lys Leu Gln Arg Met Cys Leu Glu Pro Cys Asn
    2390                2395                2400

Leu Tyr Asn Tyr Gly Ala Gly Ile Lys Leu Pro Ser Gly Ile Met
    2405                2410                2415

Leu Asn Val Val Lys Tyr Thr Gln Leu Cys Gln Tyr Leu Asn Ser
    2420                2425                2430

Thr Thr Met Cys Val Pro His Asn Met Arg Val Leu His Tyr Gly
    2435                2440                2445

Ala Gly Ser Asp Lys Gly Val Ala Pro Gly Thr Thr Val Leu Lys
    2450                2455                2460

Arg Trp Leu Pro Pro Asp Ala Ile Ile Ile Asp Asn Asp Ile Asn
    2465                2470                2475

Asp Tyr Val Ser Asp Ala Asp Phe Ser Ile Thr Gly Asp Cys Ala
    2480                2485                2490

Thr Val Tyr Leu Glu Asp Lys Phe Asp Leu Leu Ile Ser Asp Met
    2495                2500                2505

Tyr Asp Gly Arg Ile Lys Phe Cys Asp Gly Glu Asn Val Ser Lys
    2510                2515                2520

Asp Gly Phe Phe Thr Tyr Leu Asn Gly Val Ile Arg Glu Lys Leu
    2525                2530                2535

Ala Ile Gly Gly Ser Val Ala Ile Lys Ile Thr Glu Tyr Ser Trp
    2540                2545                2550

Asn Lys Tyr Leu Tyr Glu Leu Ile Gln Arg Phe Ala Phe Trp Thr
    2555                2560                2565

Leu Phe Cys Thr Ser Val Asn Thr Ser Ser Ser Glu Ala Phe Leu
    2570                2575                2580

Ile Gly Ile Asn Tyr Leu Gly Asp Phe Ile Gln Gly Pro Phe Ile
    2585                2590                2595

Ala Gly Asn Thr Val His Ala Asn Tyr Ile Phe Trp Arg Asn Ser
    2600                2605                2610

Thr Ile Met Ser Leu Ser Tyr Asn Ser Val Leu Asp Leu Ser Lys
    2615                2620                2625

Phe Glu Cys Lys His Lys Ala Thr Val Val Val Thr Leu Lys Asp
    2630                2635                2640

Ser Asp Val Asn Asp Met Val Leu Ser Leu Ile Lys Ser Gly Arg
    2645                2650                2655

Leu Leu Leu Arg Asn Ser Gly Arg Phe Gly Gly Phe Ser Asn His
    2660                2665                2670

Leu Val Ser Thr Lys
    2675

<210> 9

<211> 226

<212> PRT

<213> EMCR Coronavirus

<400> 9

Met Pro Phe Gly Gly Leu Phe Gln Leu Thr Leu Glu Ser Thr Ile Asn
1               5                   10                  15

Lys Ser Val Ala Asn Leu Lys Leu Pro Pro His Asp Val Thr Val Leu
            20                  25                  30

Arg Asp Asn Leu Lys Pro Val Thr Thr Leu Ser Thr Ile Thr Ala Tyr
        35                  40                  45

Leu Leu Val Ser Leu Phe Val Thr Tyr Phe Ala Leu Phe Lys Pro Leu
    50                  55                  60

Thr Ala Arg Gly Arg Val Ala Cys Phe Val Leu Lys Leu Leu Thr Leu
65                  70                  75                  80

Ser Val Tyr Val Pro Leu Leu Val Leu Phe Gly Met Tyr Leu Asp Ser
                85                  90                  95

Phe Ile Ile Phe Phe Leu Arg Cys Cys Phe Asp Ser Tyr Met Leu Ala
            100                 105                 110

Ile Met Pro Ile Ser Asn Lys Asn Phe Ser Phe Val Leu Phe Asn Val
        115                 120                 125

Thr Lys Leu Cys Phe Val Ser Gly Lys Cys Trp Tyr Leu Glu Gln Ser
    130                 135                 140

Phe Tyr Glu Asn Arg Phe Ala Ala Ile Tyr Gly Gly Asp His Tyr Val
145                 150                 155                 160

Val Leu Gly Gly Glu Thr Ile Thr Phe Val Ser Phe Asp Asp Leu Tyr
                165                 170                 175

Val Ala Ile Arg Gly Ser Cys Glu Lys Asn Leu Gln Leu Met Arg Lys
            180                 185                 190

Val Asp Leu Tyr Asn Gly Ala Val Ile Tyr Ile Phe Ala Glu Glu Pro
        195                 200                 205

Val Val Gly Ile Val Tyr Ser Ser Gln Leu Tyr Glu Asp Val Pro Ser
    210                 215                 220

Ile Asn
225

## Description

The invention relates to the field of virology.

Recently, a respiratory illness (atypical pneumonia) was diagnosed in an 8 months old patient that could not be attributed to SARS (Severe Acute Respiratory Syndrome) virus or any other known viral infection. The patient tested negative for influenza, parainfluenza, mumps and RSV and yet the disease was identified to be caused by a virus which closely resembled SARS.

For being able to trace its origin, monitor its epidemiology and prevent possible spreading of the disease, it is of great importance to be able to recognise viral causes of pneumonia in an early stage. Especially, if severe diseases are found to be caused by viruses, it is necessary to detect the identity of the virus as soon as possible, in order to develop diagnostic tools and possibly therapies. The SARS epidemy has shown that it is paramount for prevention of spread of the disease to be able to get an early diagnosis in order to timely take effective isolation measures en initiate quarantaine precautions. Only then, world-wide contaminations can be prevented.

Furthermore, identification of the viral cause for the disease enables development of vaccines, which can be used prophylactically to protect people who are at risk of being infected. And, finally, knowledge of the viral cause enables to develop therapeutic measures.

Thus, there is great need in developing diagnostic tools and therapies for viral pneumonias in general, and particular to a novel disease-causing infectious agent, especially when this agent appears to be a virus.

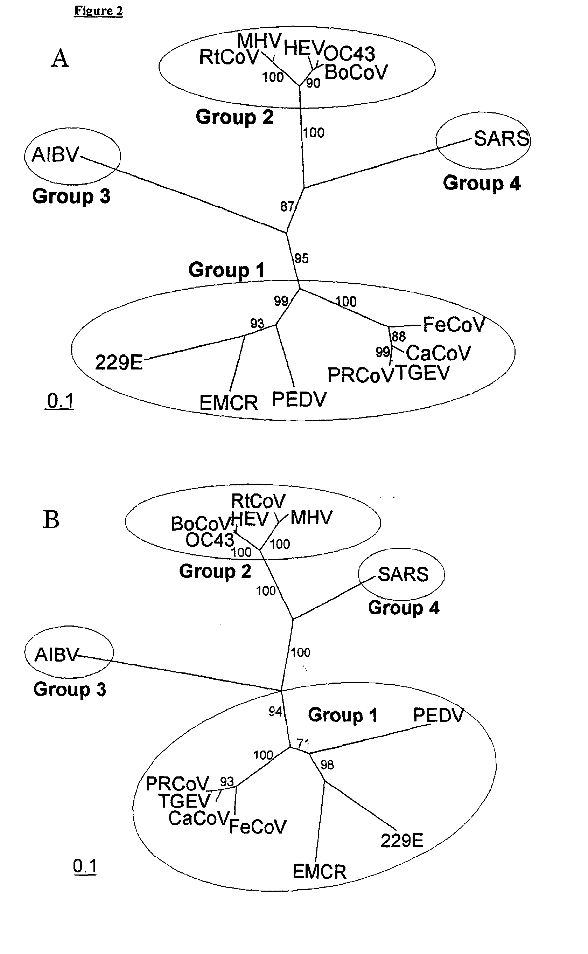

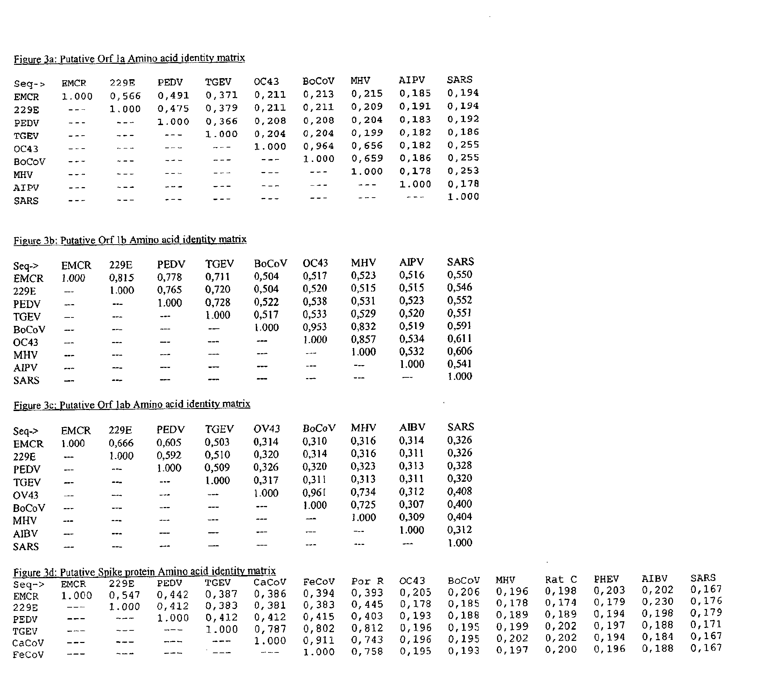

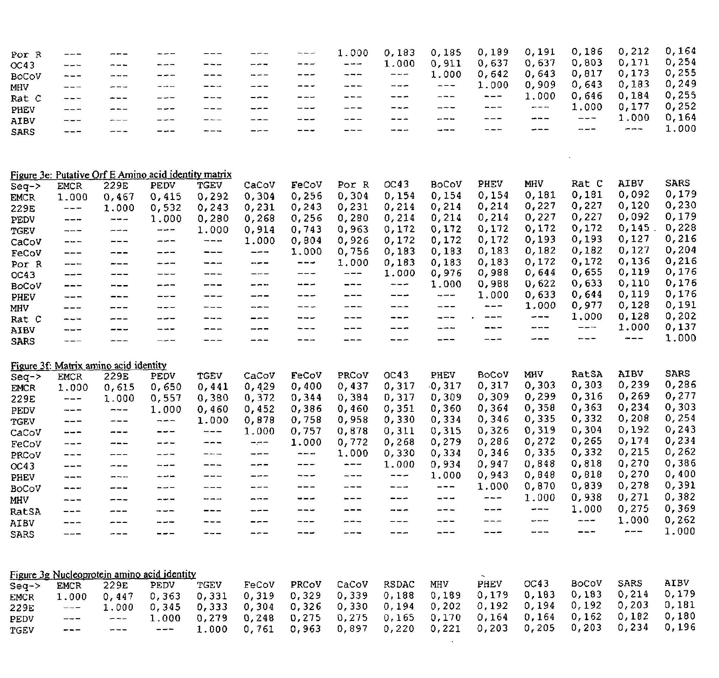

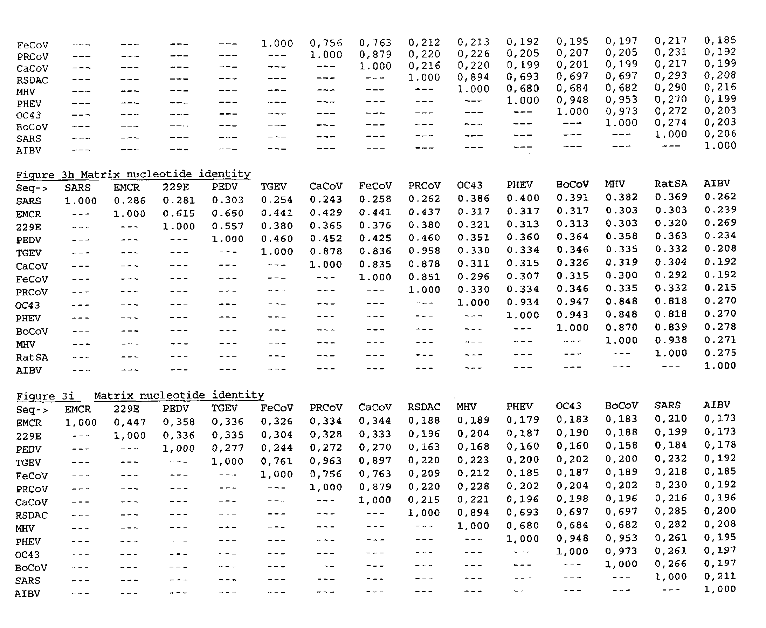

The invention provides the nucleotide sequence of an isolated essentially mammalian positive-sense single stranded RNA virus belonging to the Coronaviruses, which is the causative factor for the new disease, hereinafter referred to as EMCR-CoV and the disease being referred to as EMCR-CoV-caused pneumonia. From a phylogenetic analysis of the Matrix and Nucleocapsid gene sequences of the virus (Fig. 2a and 2b) it appears that the virus is a distinct member of the group formed by PEDV (porcine epidemic diarrhea virus), HCoV-229E (human coronavirus 229E), PRCoV (porcine respiratory coronavirus), TGEV (transmissible gastroenteritis virus), CaCoV (Canine coronavirus) and FeCoV (feline coronavirus). Based on amino acid identity matrices, human coronavirus 229E seems to be the closest relative (for all ORFs with the exception of Matrix which appears to be slightly more closely related to PEDV - see Figure 3).

Although phylogenetic analyses provide a convenient method of identifying a virus, several other possibly more straightforward albeit somewhat more coarse methods for identifying said virus or viral proteins or nucleic acids from said virus are herein also provided. As a rule of thumb an EMCR-Coronavirus can be identified by the percentages of homology of the virus, proteins or nucleic acids to be identified in comparison with viral proteins or nucleic acids identified herein by sequence. It is generally known that virus species, especially RNA virus species, often constitute a quasi species wherein a cluster of said viruses displays heterogeneity among its members. Thus it is expected that each isolate may have a somewhat different percentage relationship with the sequences of the isolate as provided herein.

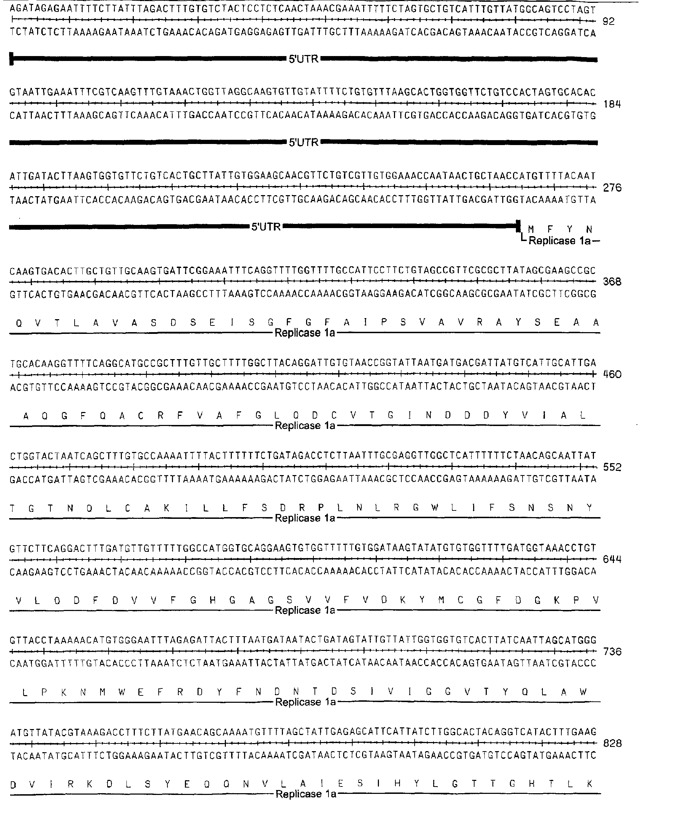

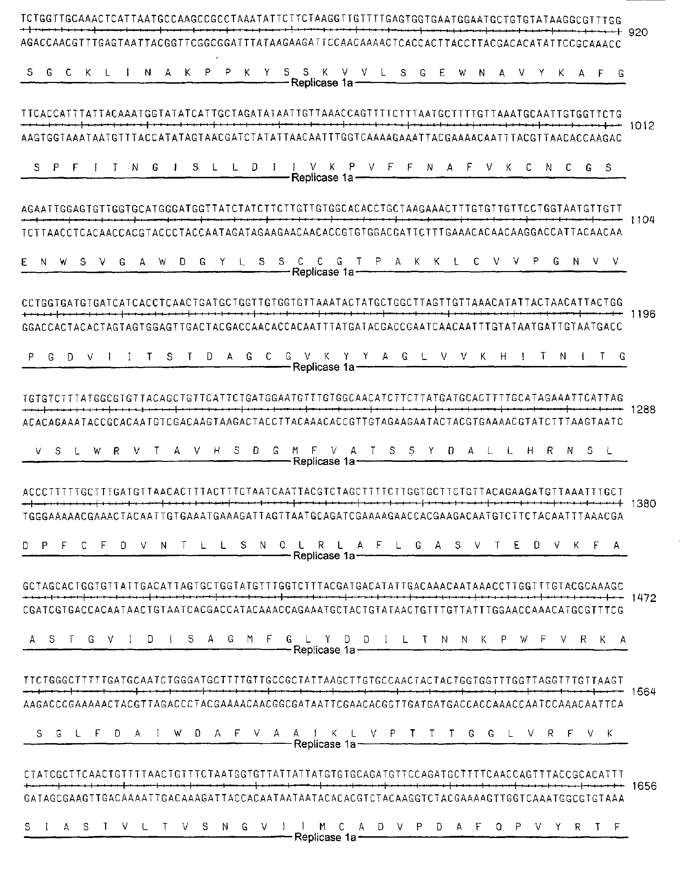

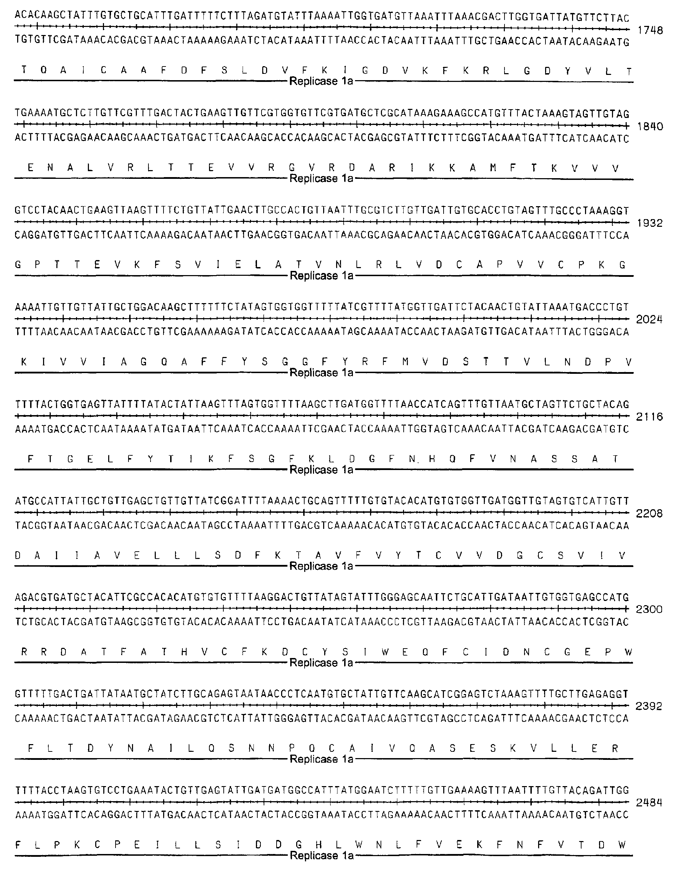

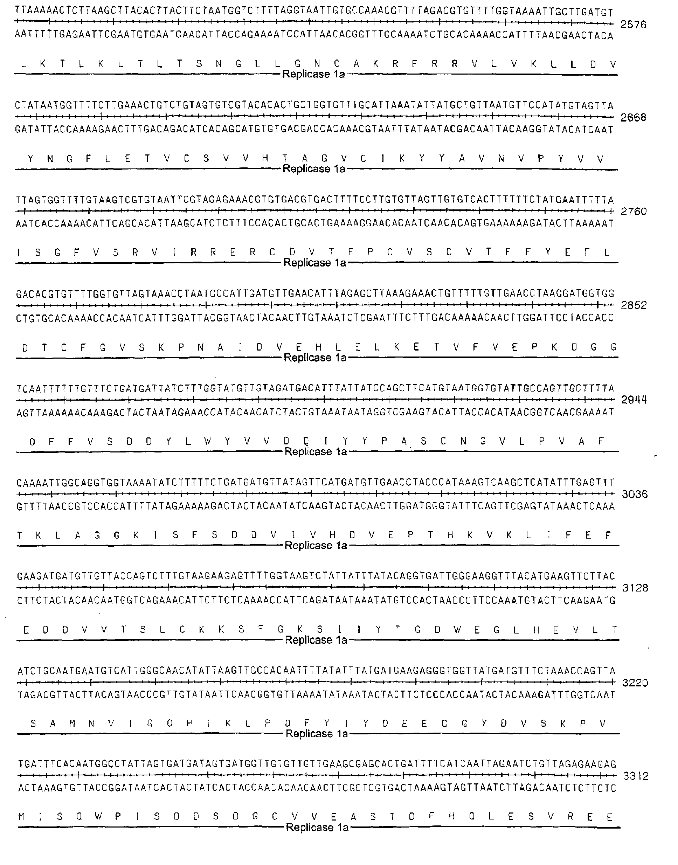

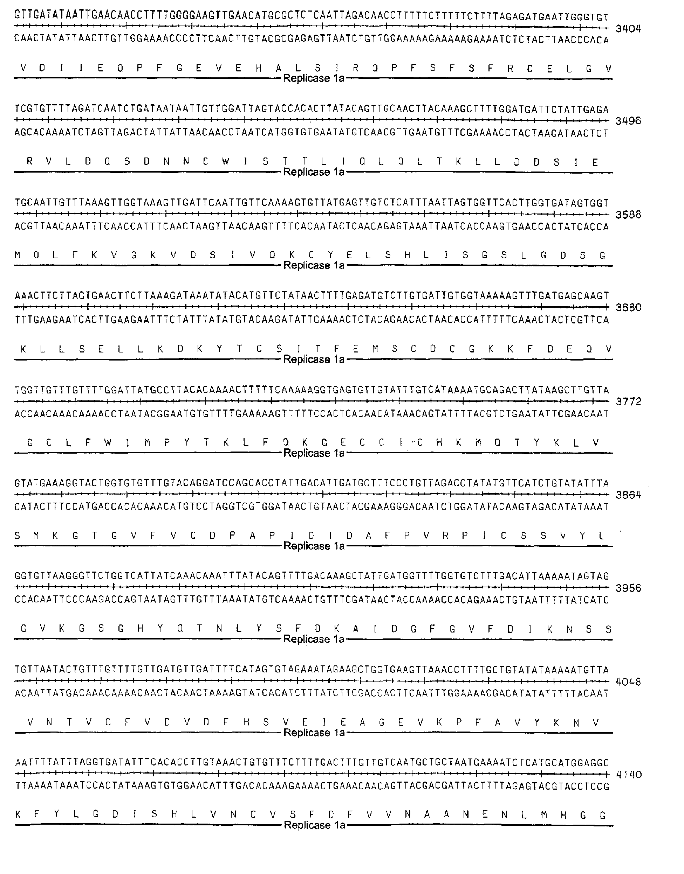

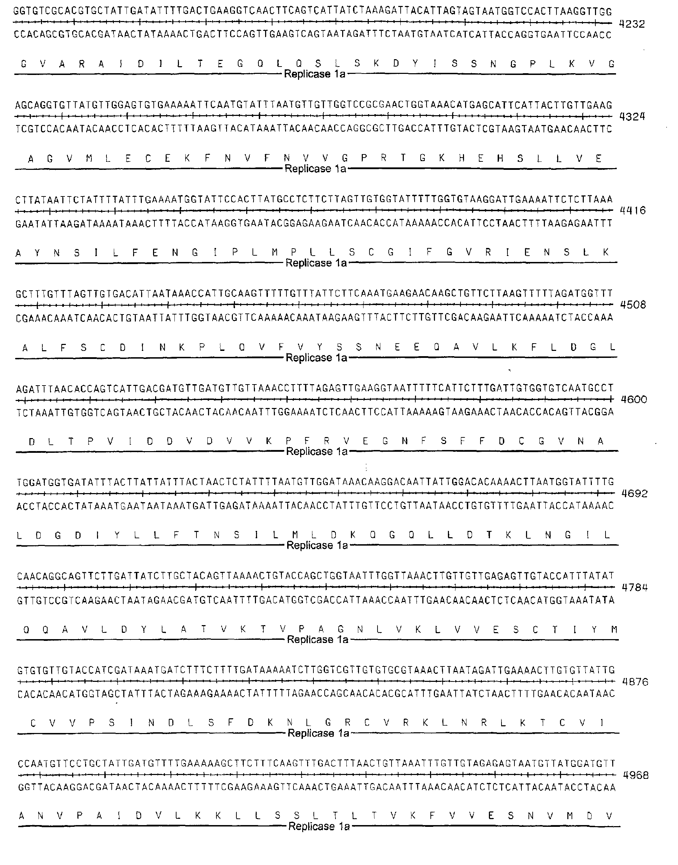

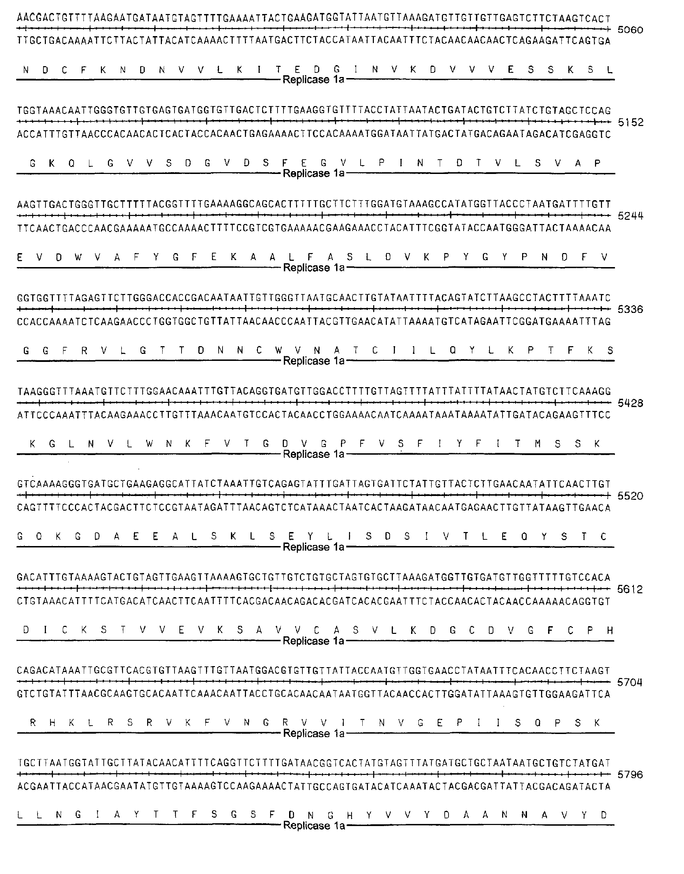

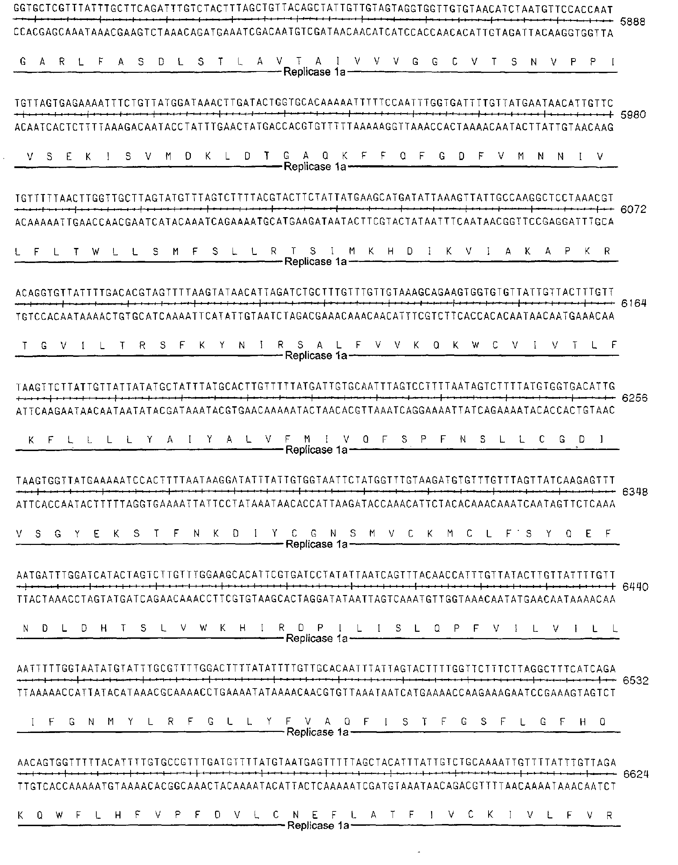

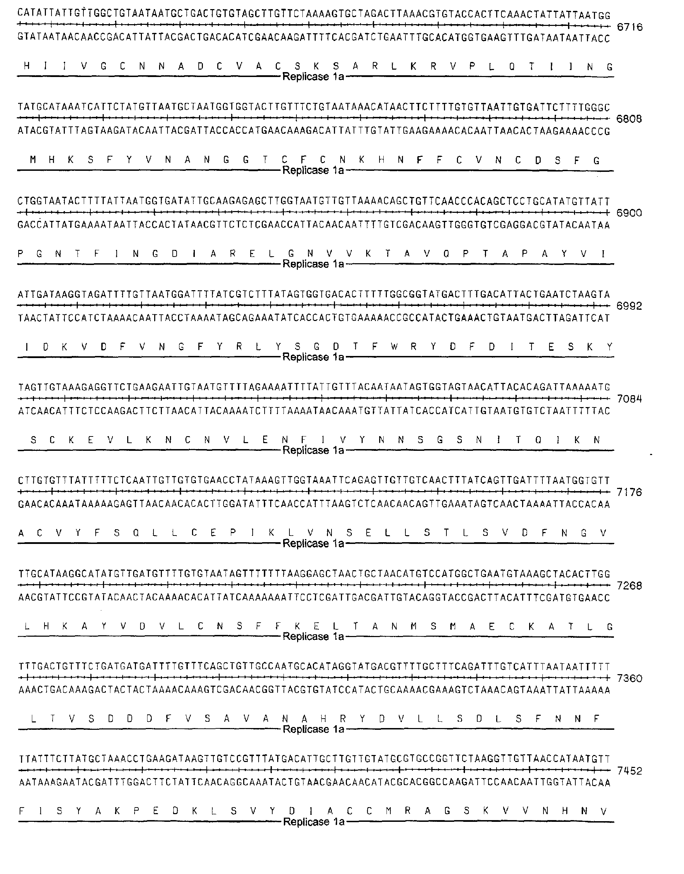

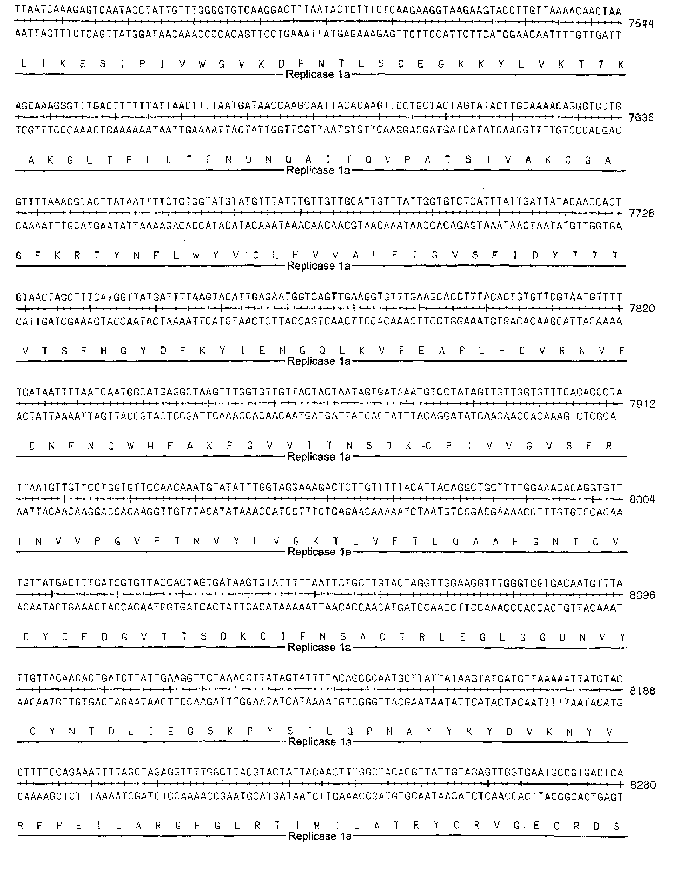

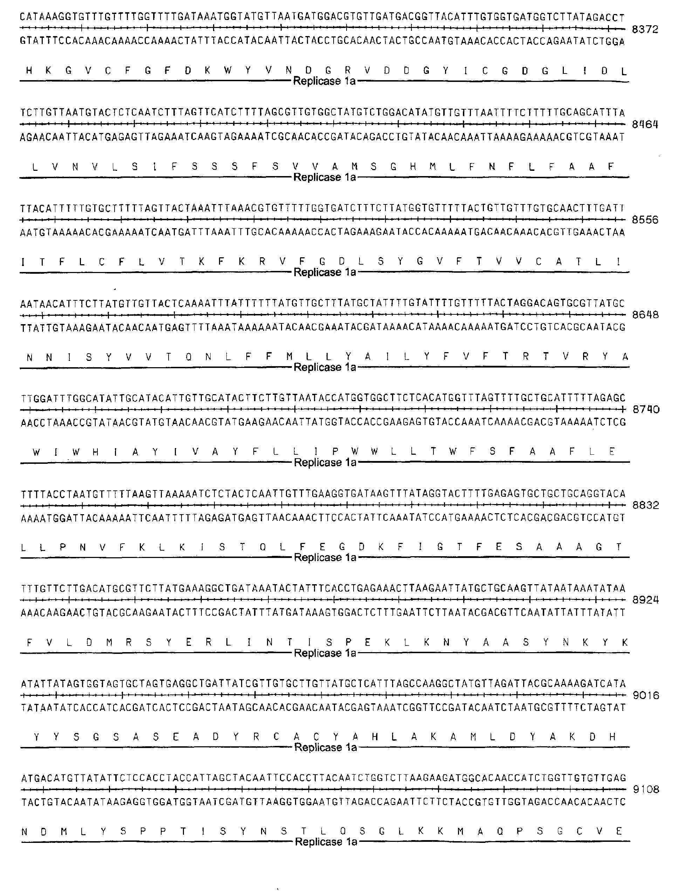

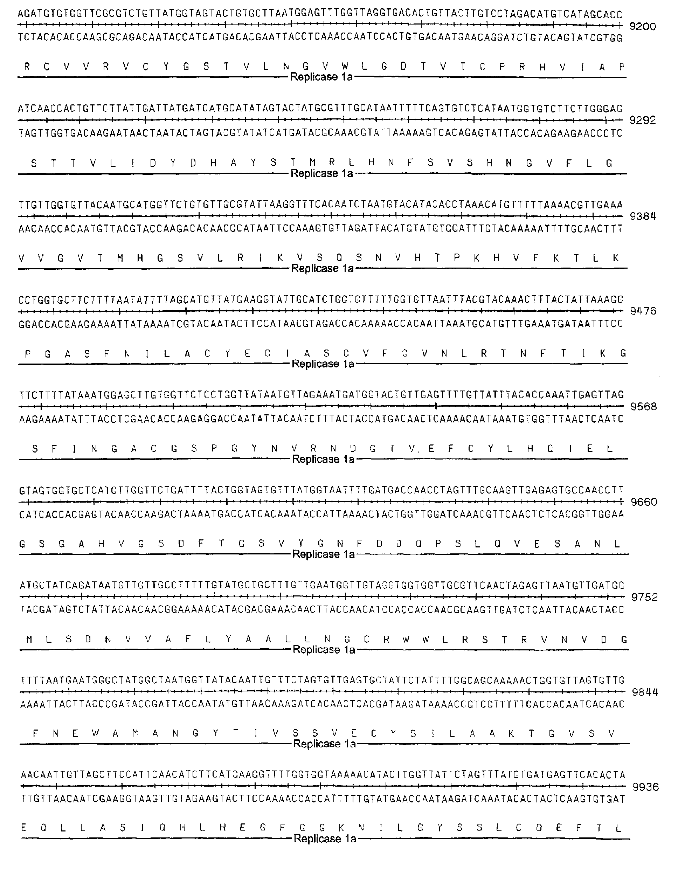

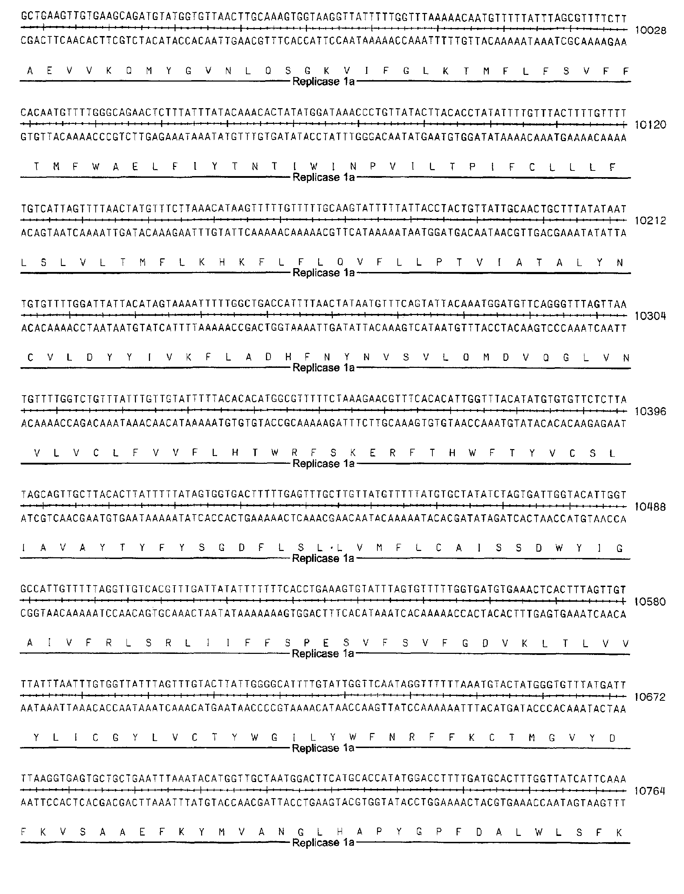

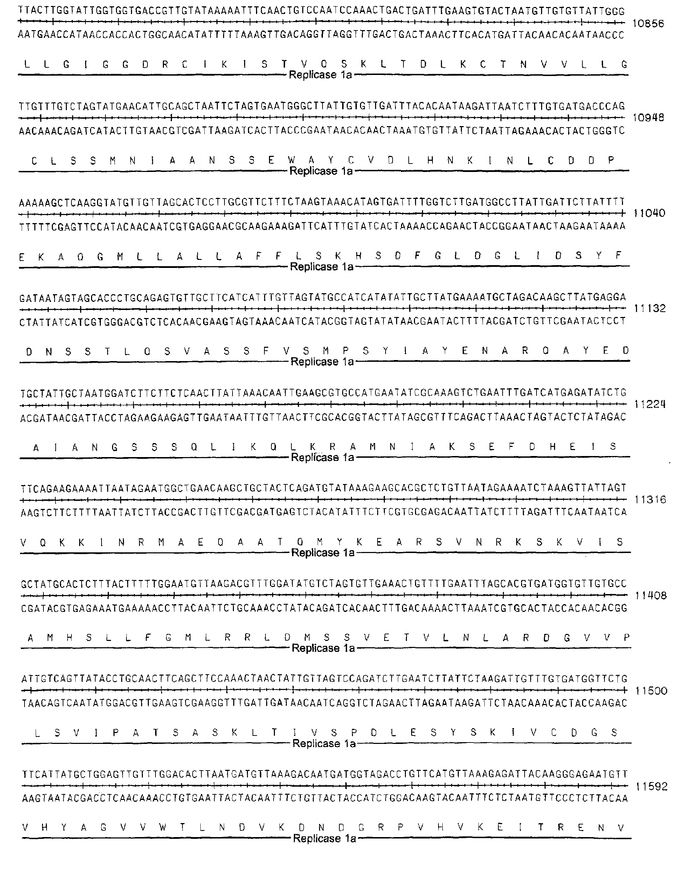

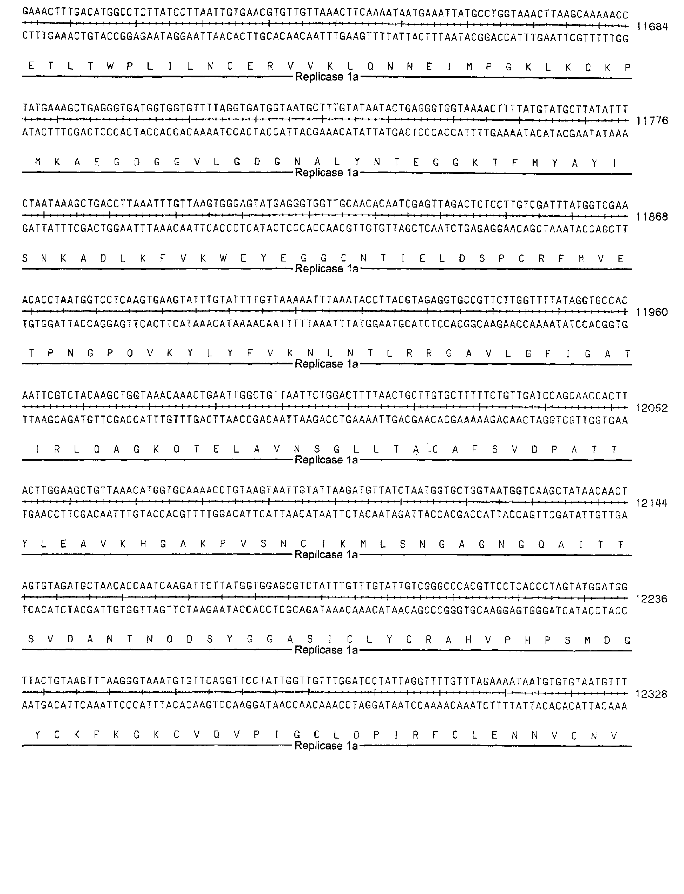

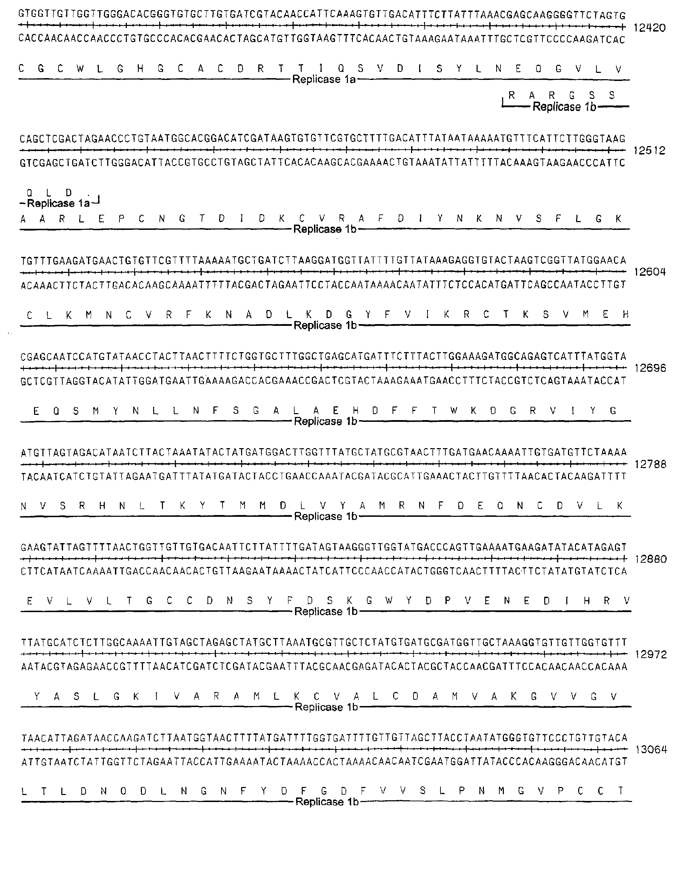

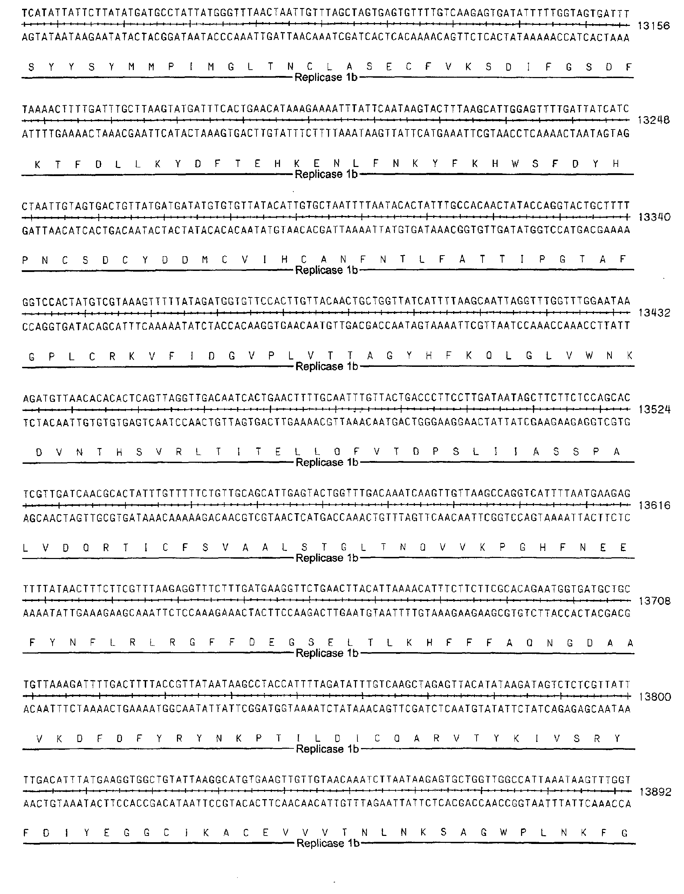

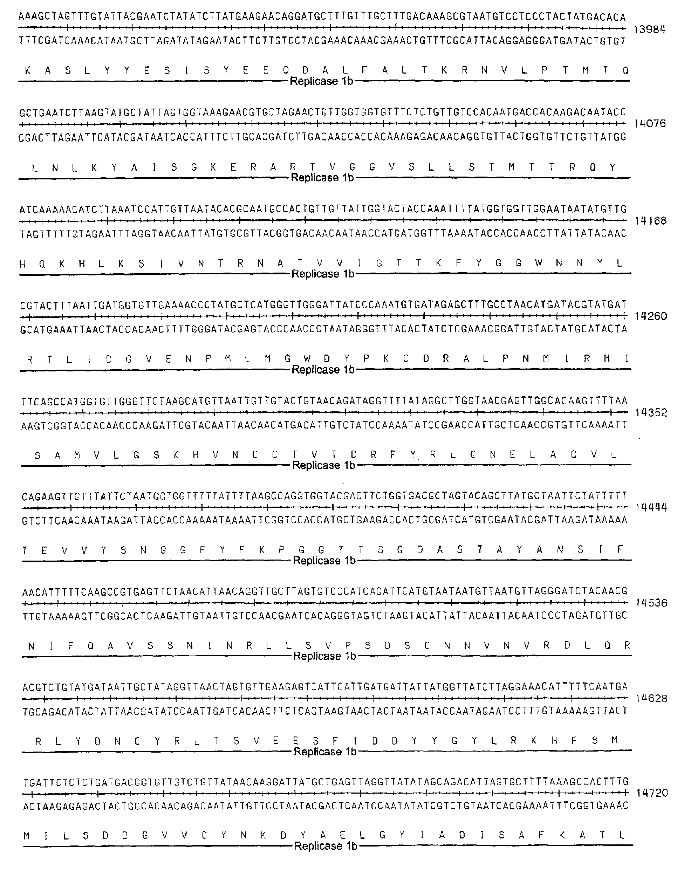

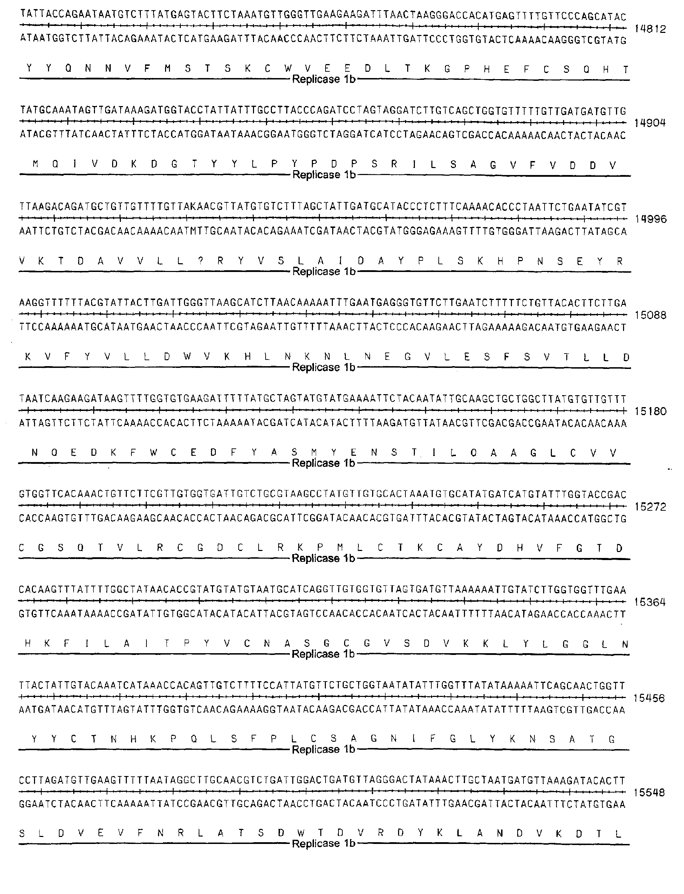

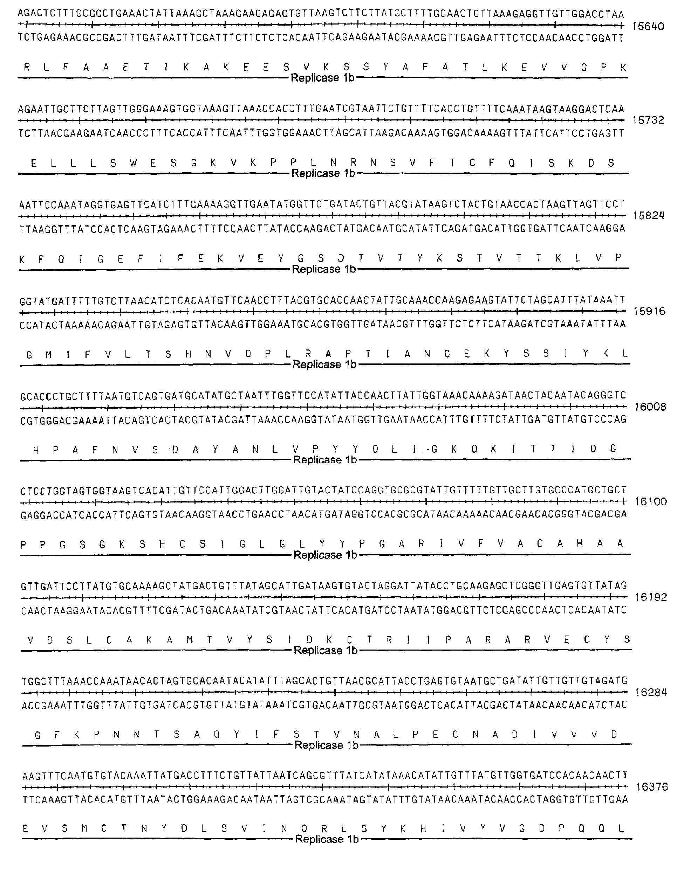

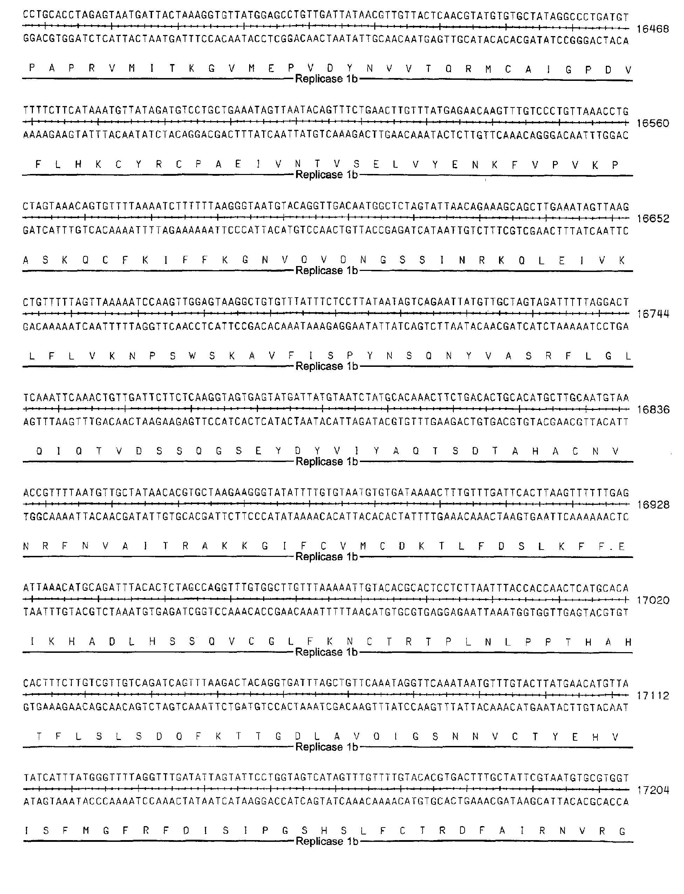

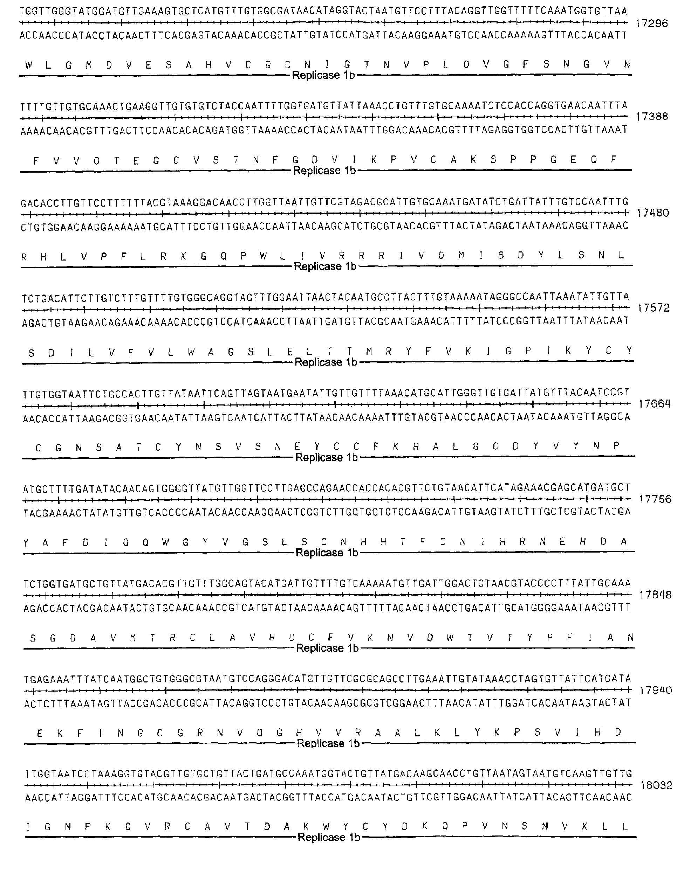

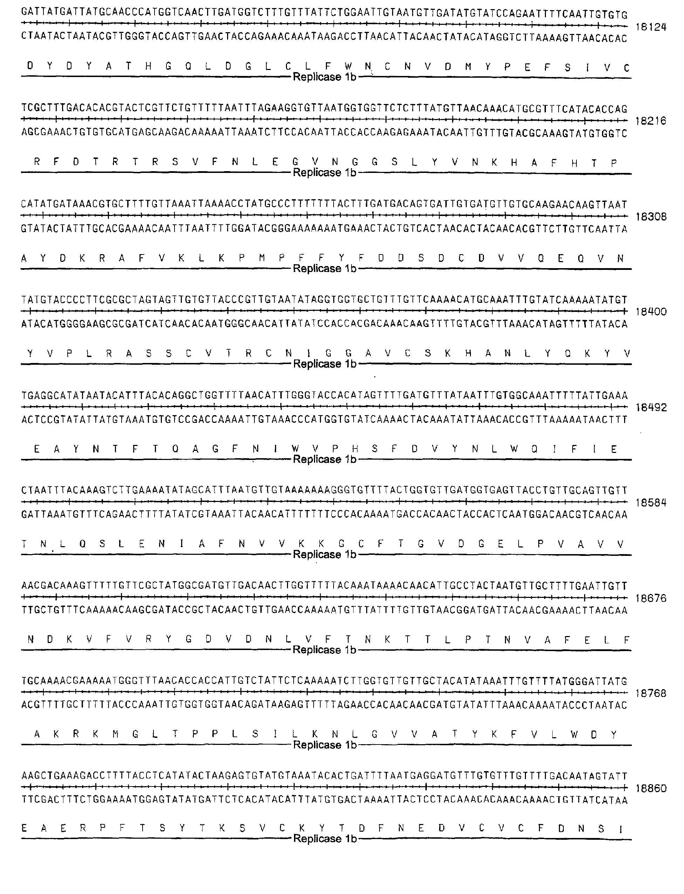

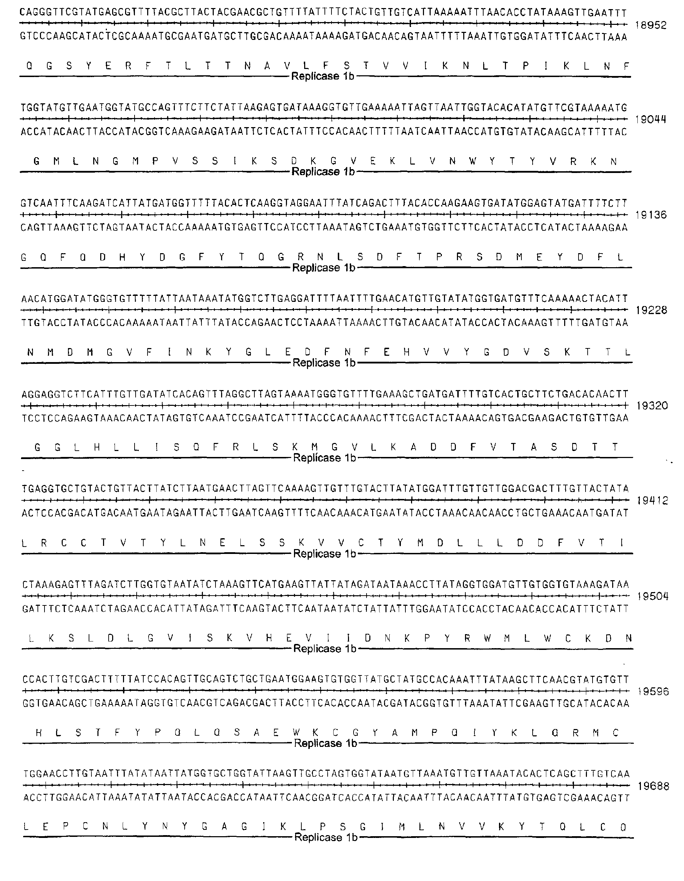

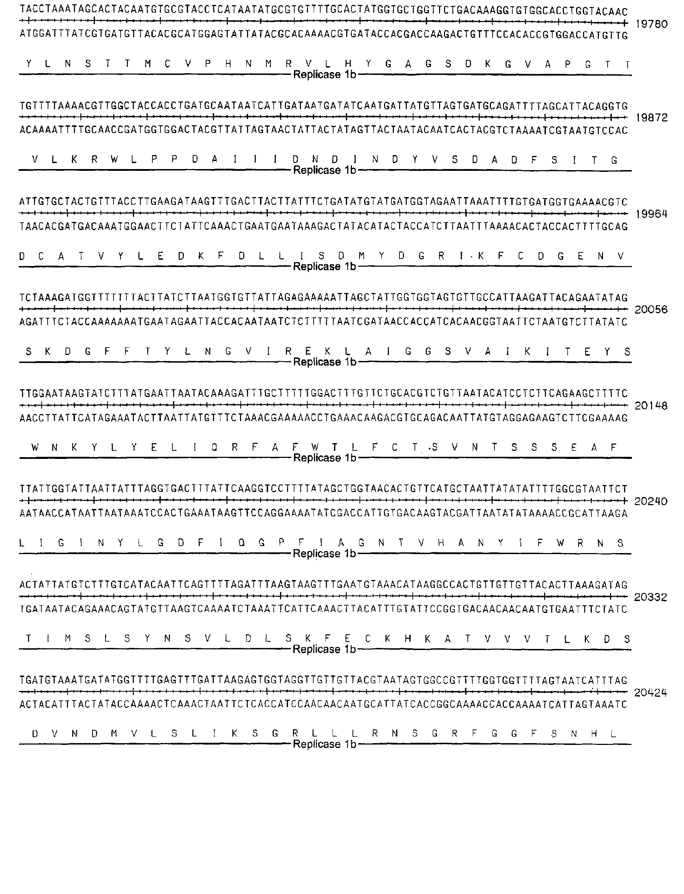

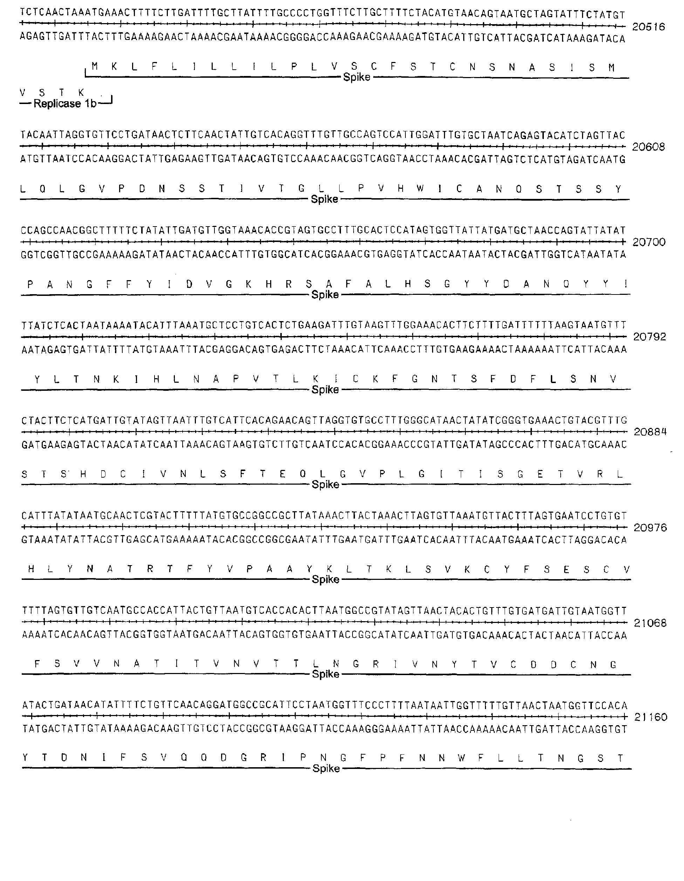

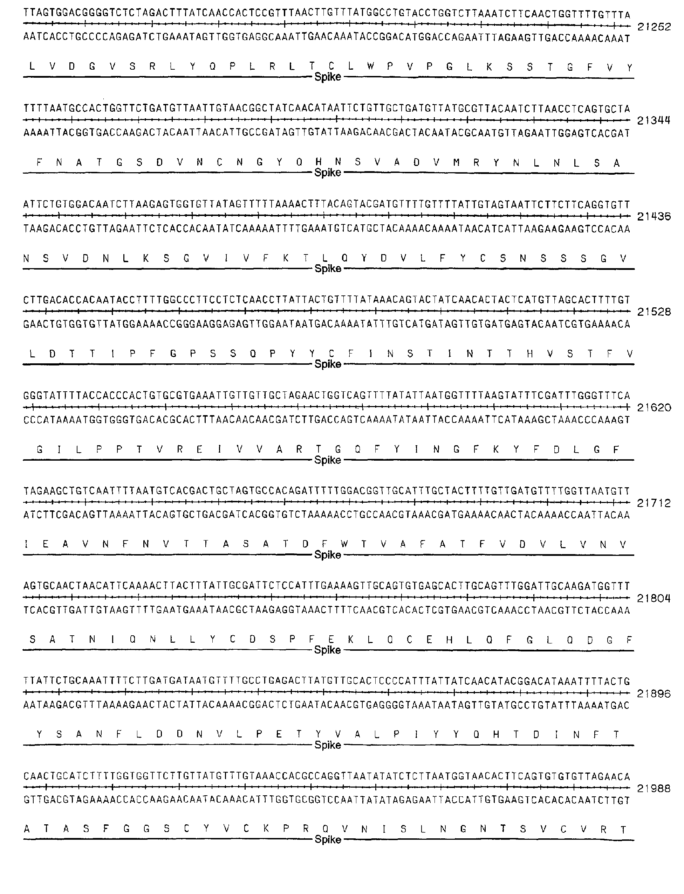

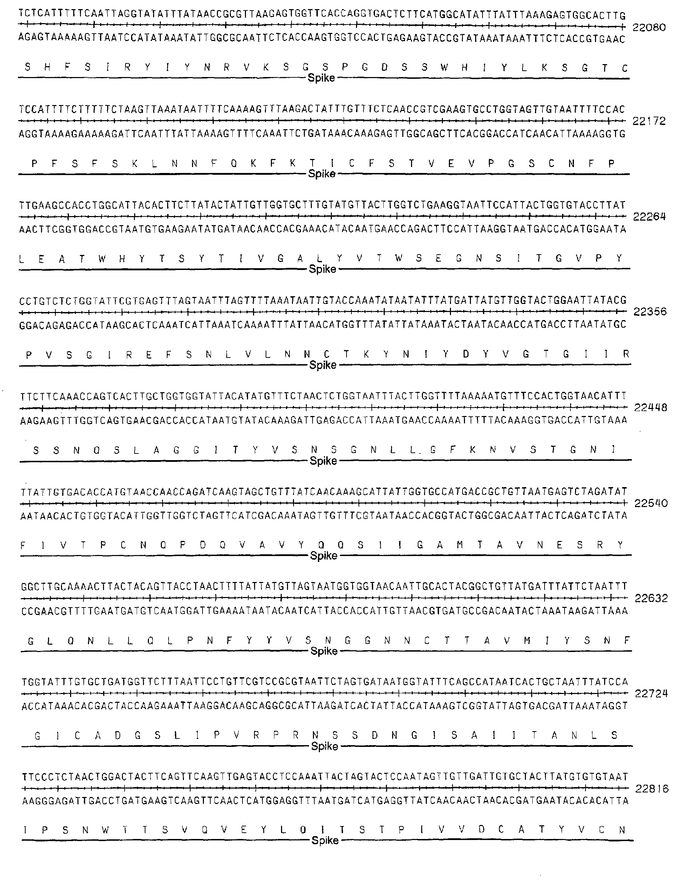

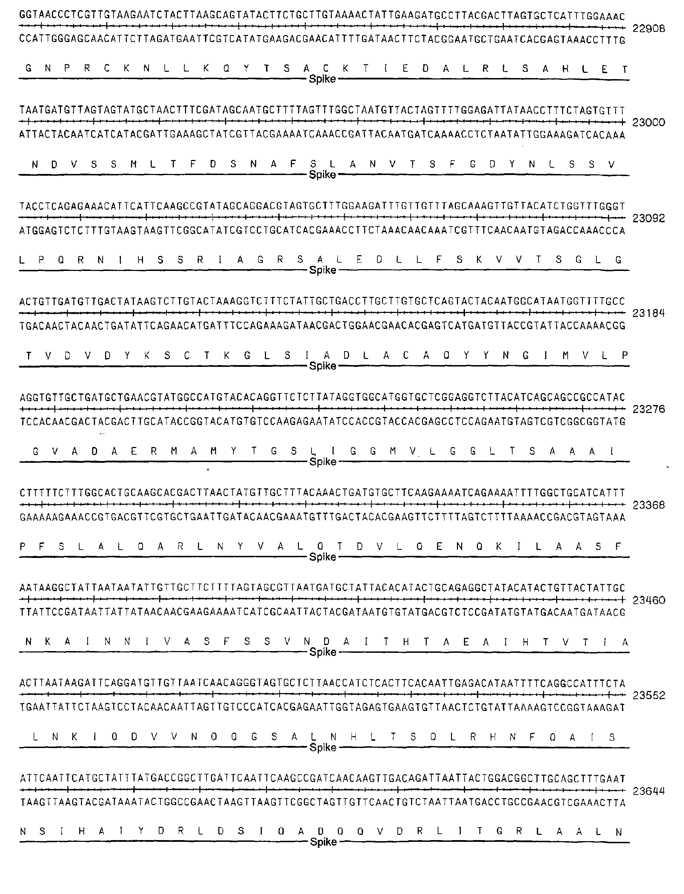

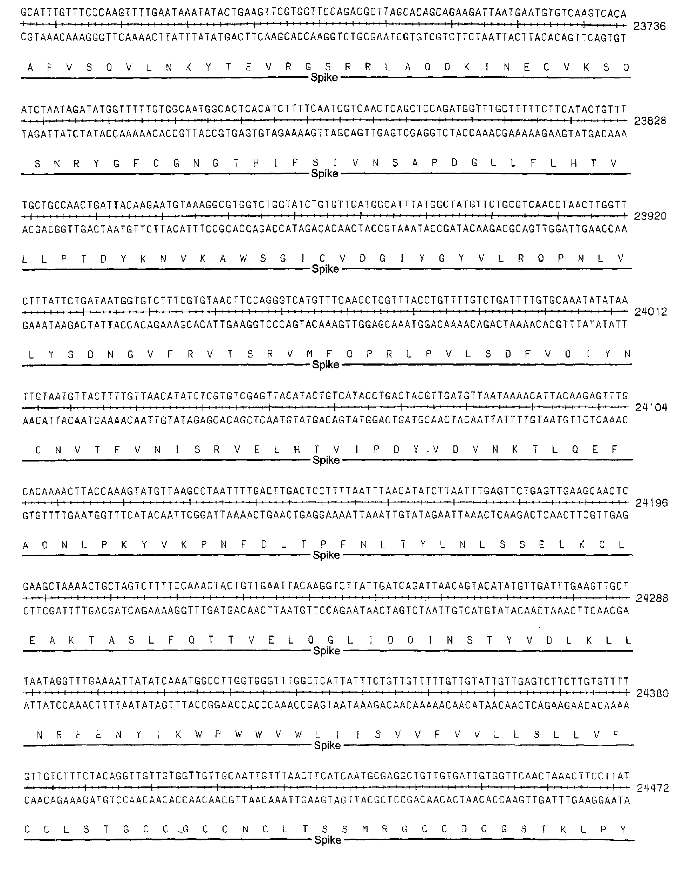

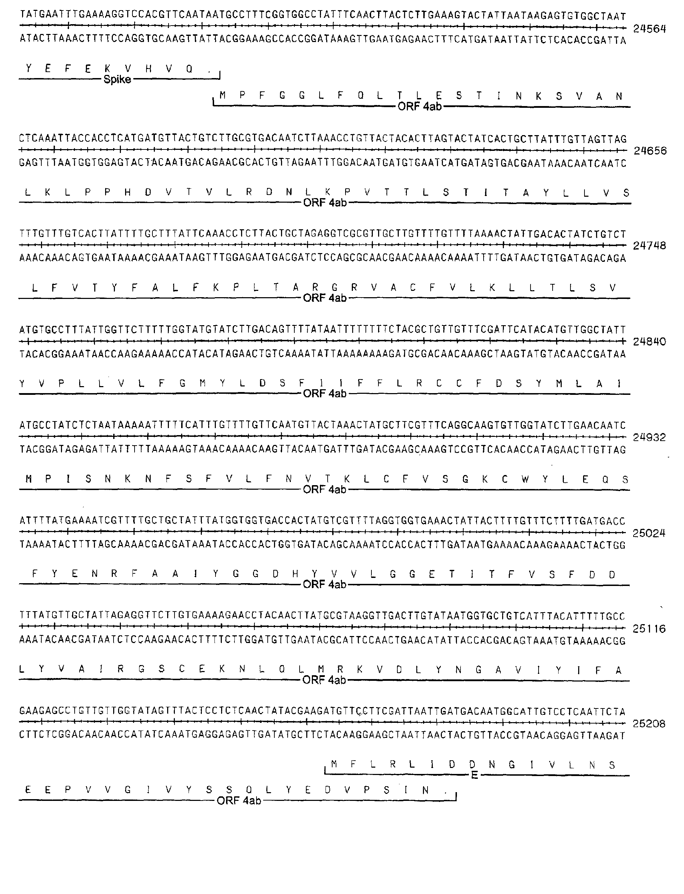

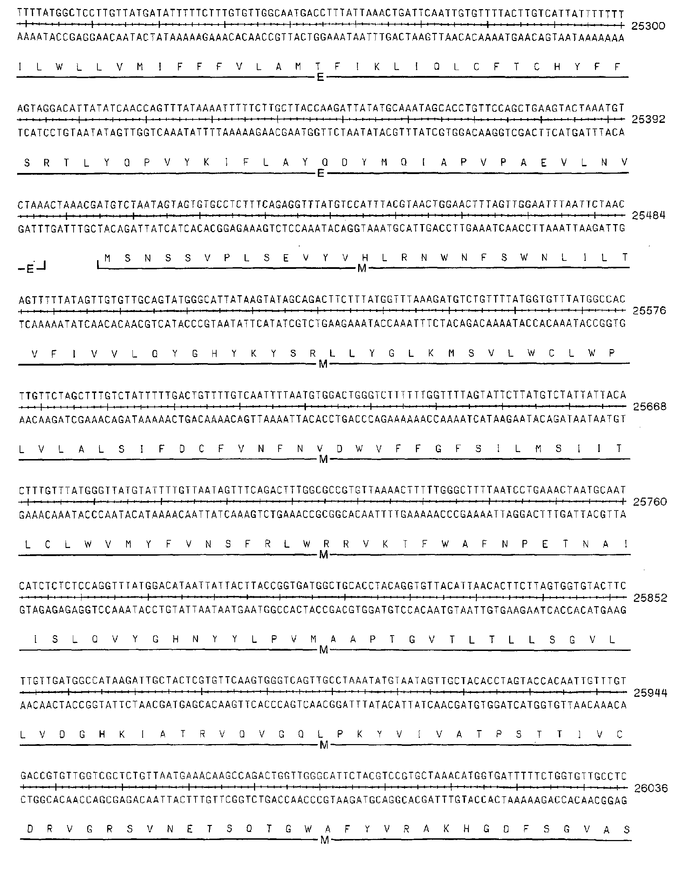

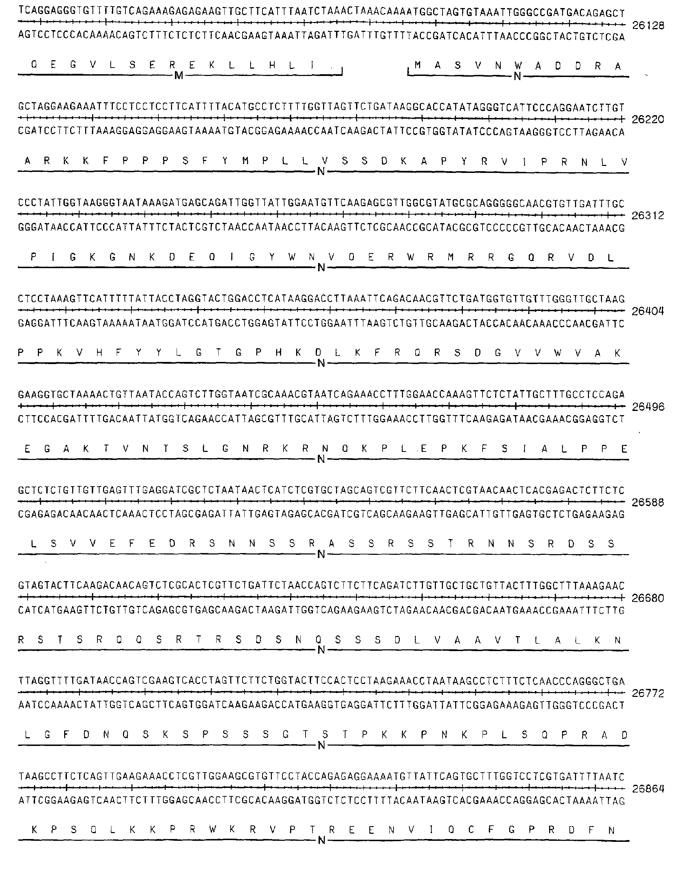

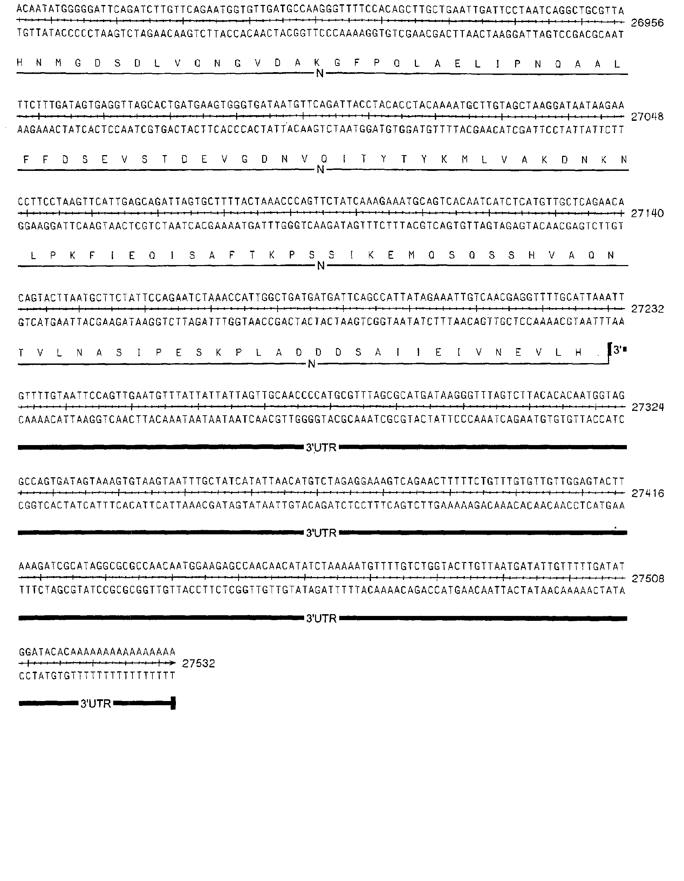

When one wishes to compare a virus isolate with the sequences as listed in figure 1molo, the invention provides an isolated essentially mammalian positive-sense single stranded RNA virus (EMCR-CoV) belonging to the Coronaviruses and identifiable as phylogenetically corresponding thereto by determining a nucleic acid sequence of said virus and determining that said nucleic acid sequence has a percentage nucleic acid identity to the sequences as listed higher than the percentages identified herein for the nucleic acids as identified herein below in comparison with PEDV, 229E, PRCoV, TGEV, CaCoV and FeCoV. Likewise, an isolated essentially mammalian positive-sense single stranded RNA virus (EMCR-CoV) belonging to the Coronaviruses and identifiable as phylogenetically corresponding thereto by determining an amino acid sequence of said virus and determining that said amino acid sequence has a percentage amino acid homology to the sequences as listed which is essentially higher than the percentages provided herein in comparison with PEDV, 229E, PRCoV, TGEV, CaCoV and FeCoV.

With the provision of the sequence information of this EMCR-Coronavirus (EMCR-CoV), the invention provides diagnostic means and methods, prophylactic means and methods and therapeutic means and methods to be employed in the diagnosis, prevention and/or treatment of disease, in particular of respiratory disease (atypical pneumonia), in particular of mammals, more in particular in humans associated with infection by this virus. In virology, it is most advisory that diagnosis, prophylaxis and/or treatment of a specific viral infection is performed with reagents that are most specific for said specific virus causing said infection. In this case this means that it is preferred that said diagnosis, prophylaxis and/or treatment of an EMCR-CoV virus infection is performed with reagents that are most specific for EMCR-CoV virus. This by no means however excludes the possibility that less specific, but sufficiently cross-reactive reagents are used instead, for example because they are more easily available and sufficiently address the task at hand.

The invention for example provides a method for virologically diagnosing an EMCR-CoV infection of an animal, in particular of a mammal, more in particular of a human being, comprising determining in a sample of said animal the presence of a viral isolate or component thereof by reacting said sample with an EMCR-CoV specific nucleic acid or antibody according to the invention, and a method for serologically diagnosing an EMCR-CoV infection of a mammal comprising determining in a sample of said mammal the presence of an antibody specifically directed against an EMCR-CoV virus or component thereof by reacting said sample with an EMCR-CoV virus-specific proteinaceous molecule or fragment thereof or an antigen according to the invention.

The invention also provides a diagnostic kit for diagnosing an EMCR-CoV infection comprising an EMCR-CoV virus, an EMCR-CoV virus-specific nucleic acid, proteinaceous molecule or fragment thereof, antigen and/or an antibody according to the invention, and preferably a means for detecting said EMCR-CoV virus, EMCR-CoV virus-specific nucleic acid, proteinaceous molecule or fragment thereof, antigen and/or an antibody, said means for example comprising an excitable group such as a fluorophore or enzymatic detection system used in the art (examples of suitable diagnostic kit format comprise IF, ELISA, neutralization assay, RT-PCR assay). To determine whether an as yet unidentified virus component or synthetic analogue thereof such as nucleic acid, proteinaceous molecule or fragment thereof can be identified as EMCR-CoV-virus-specific, it suffices to analyse the nucleic acid or amino acid sequence of said component, for example for a stretch of said nucleic acid or amino acid, preferably of at least 10, more preferably at least 25, more preferably at least 40 nucleotides or amino acids (respectively), by sequence homology comparison with the provided EMCR-CoV viral sequences and with known non-EMCR-CoV viral sequences (human coronavirus 299E is preferably used) using for example phylogenetic analyses as provided herein. Depending on the degree of relationship with said EMCR-CoV or non-EMCR-CoV viral sequences, the component or synthetic analogue can be identified.

The invention thus provides the nucleotide sequence of a novel etiological agent, an isolated essentially mammalian positive-sense single stranded RNA virus (herein also called EMCR-CoV virus) belonging to the Coronaviridae family, and EMCR-CoV virus-specific components or synthetic analogues thereof.

Coronaviruses were first isolated from chickens in 1937, while the first human coronavirus was propagated *in vitro* by Tyrell and Bonoe in 1965. There are now about 13 species in this family, which infect cattle, pigs, rodents, cats, dogs, birds and man. Coronavirus particles are irregularly shaped, about 60-220 nm in diameter, with an outer envelope bearing distinctive, 'club-shaped' peplomers ( about 20 nm long and 10 nm wide at the distal end). This 'crown-like' appearance give the family its name. The envelope carries two glycoproteins: S, the spike glycoprotein which is involved in cell fusion and is a major antigen, and M, the membrane glycoprotein, which is involved in budding and envelope formation. The genome is associated with a basic phosphoprotein, designated N. The genome of coronaviruses, a single stranded positive-sense RNA strand, is typically 27-31 Kb long and contains a 5' methylated cap and a 3' poly-A tail, by which it can directly function as an mRNA in the infected cell. Initially the 5' ORF 1 (about 20 Kb) is translated to produce a viral polymerase, which then produces a full length negative sense strand. This is used as a template to produce mRNA as a 'nested set' of transcripts, all with identical 5' non-translated leader sequence of 72 nucleotides and coincident 3' polyadenylated ends. Each mRNA thus produced is monocistronic, the genes at the 5' end being translated from the longest mRNA and so on. These unusual cytoplasmic structures are produced not by splicing, but by the polymerase during transcription. Between each of the genes there is a repeated intergenic sequence - AACUAAAC - which interacts with the transcriptase plus cellular factors to splice the leader sequence onto the start of each ORF. In some coronaviruses there are about 8 ORFs, coding for the proteins mentioned above, but also for a heamagglutenin esterase (HE), and several other non-structural proteins.

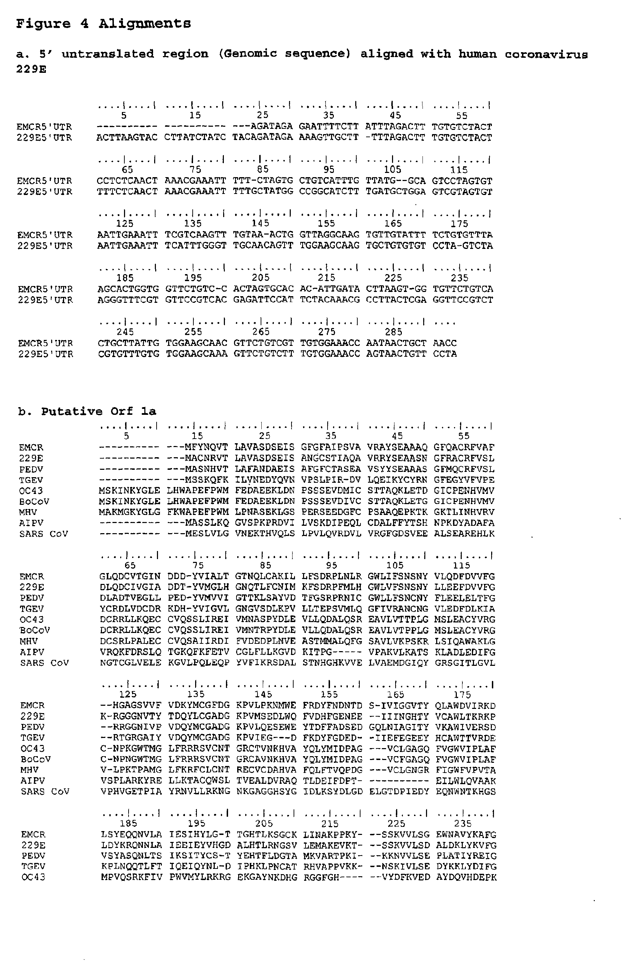

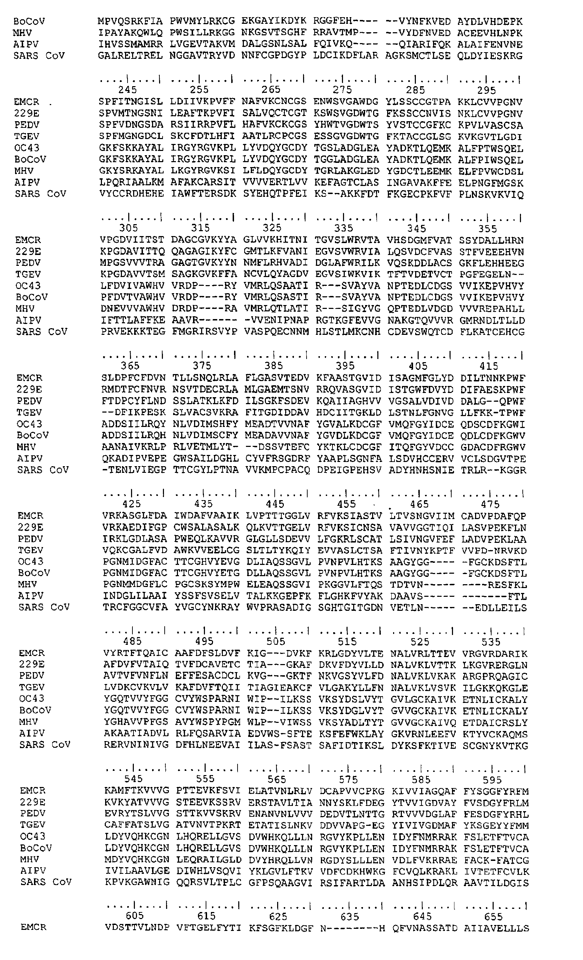

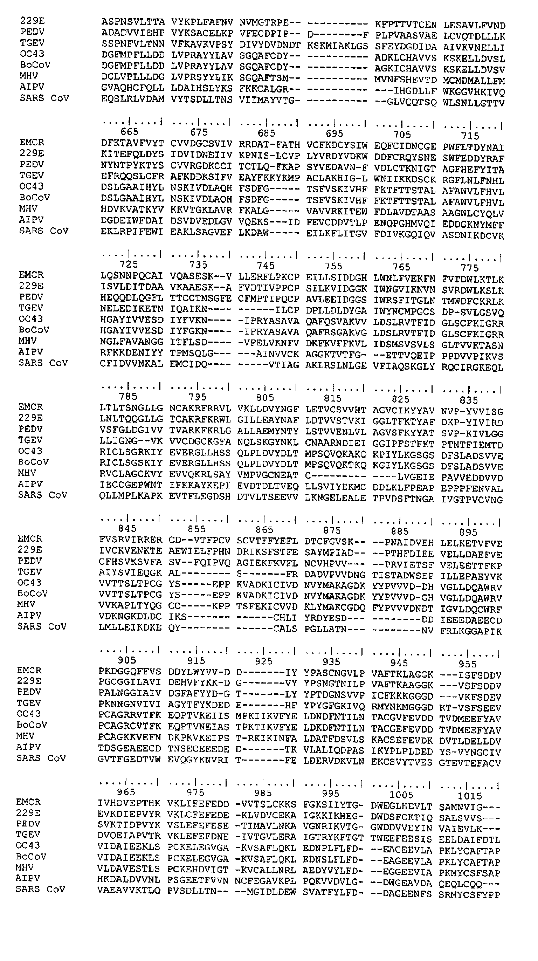

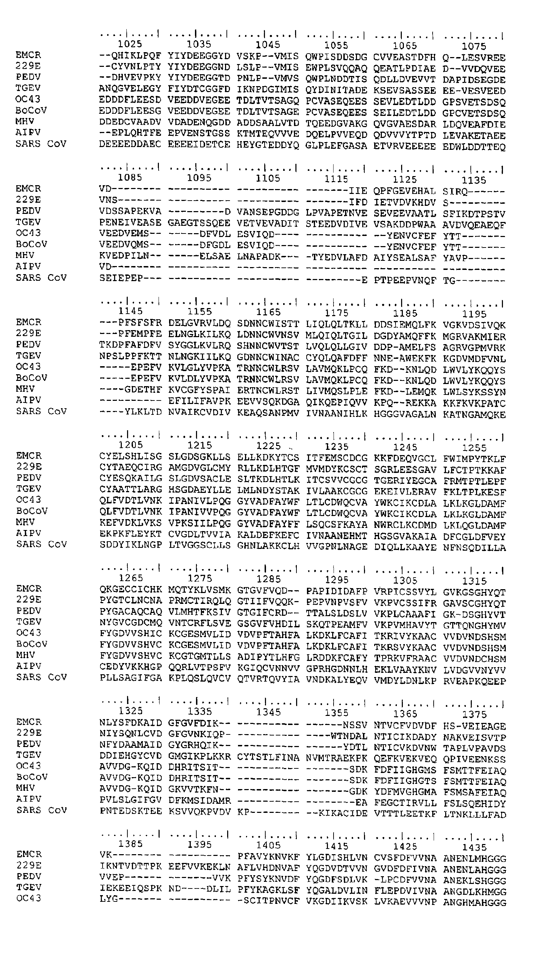

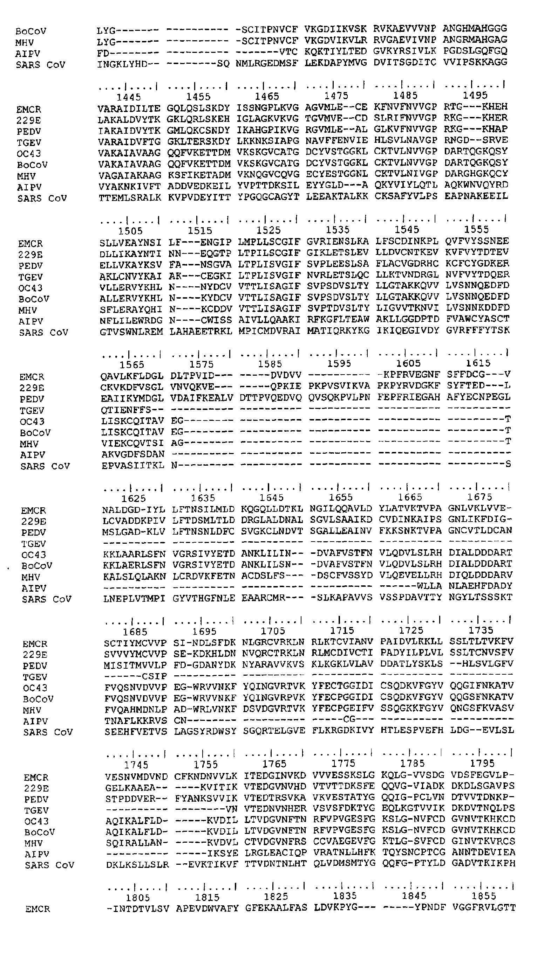

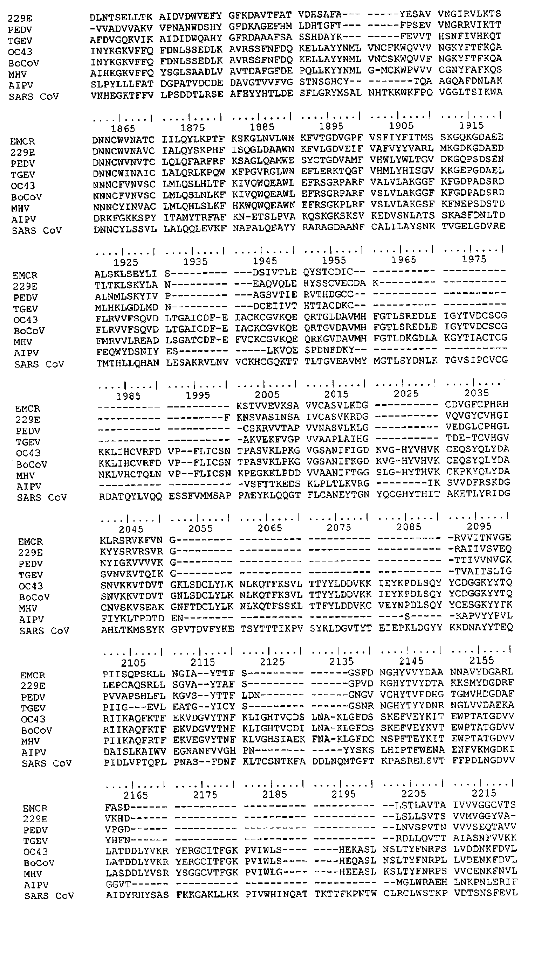

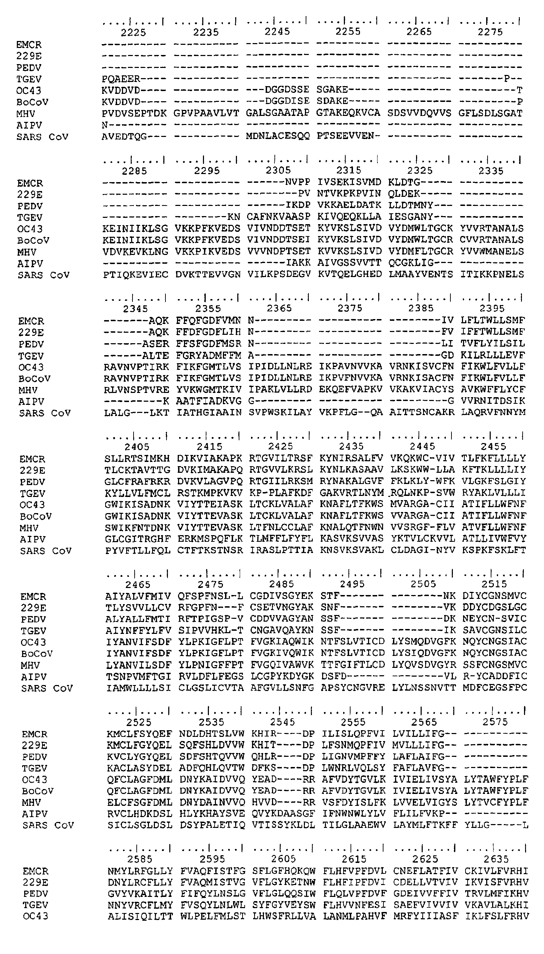

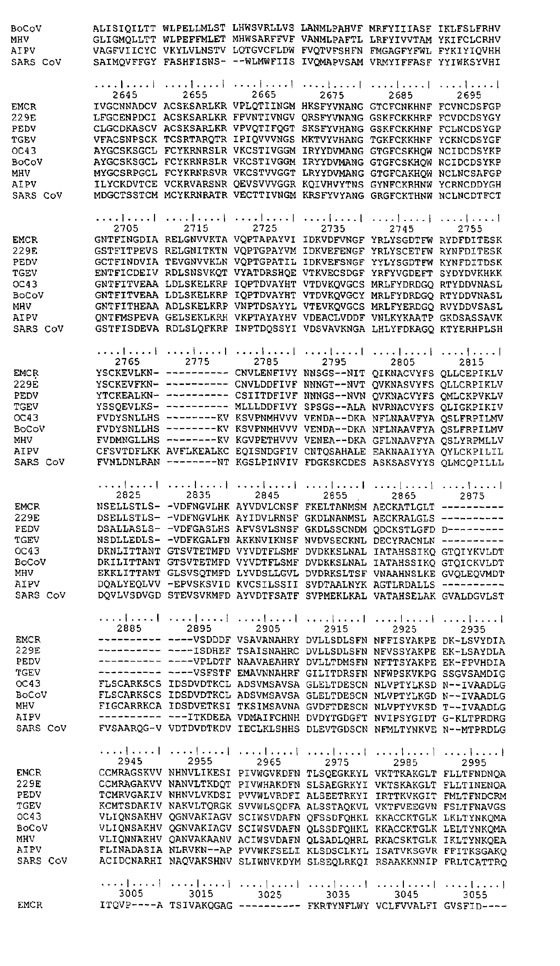

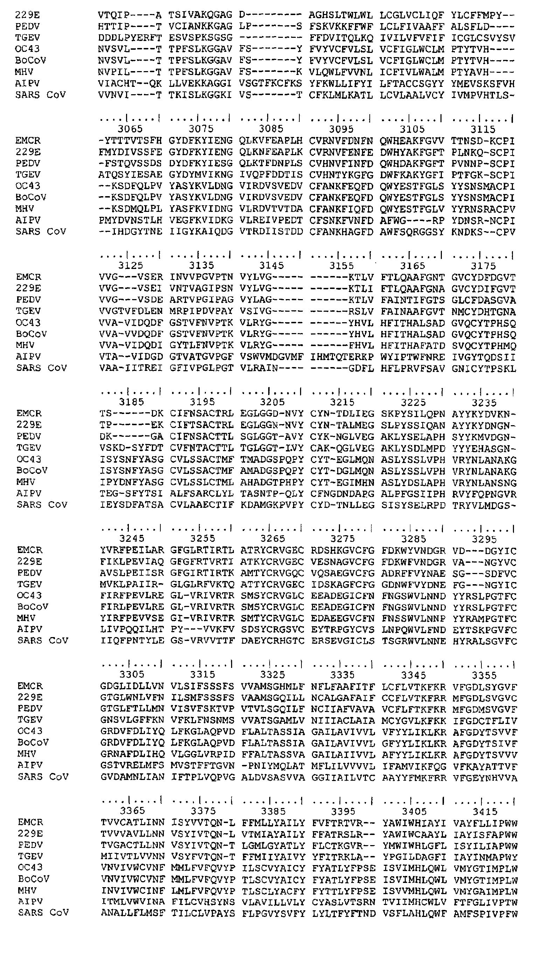

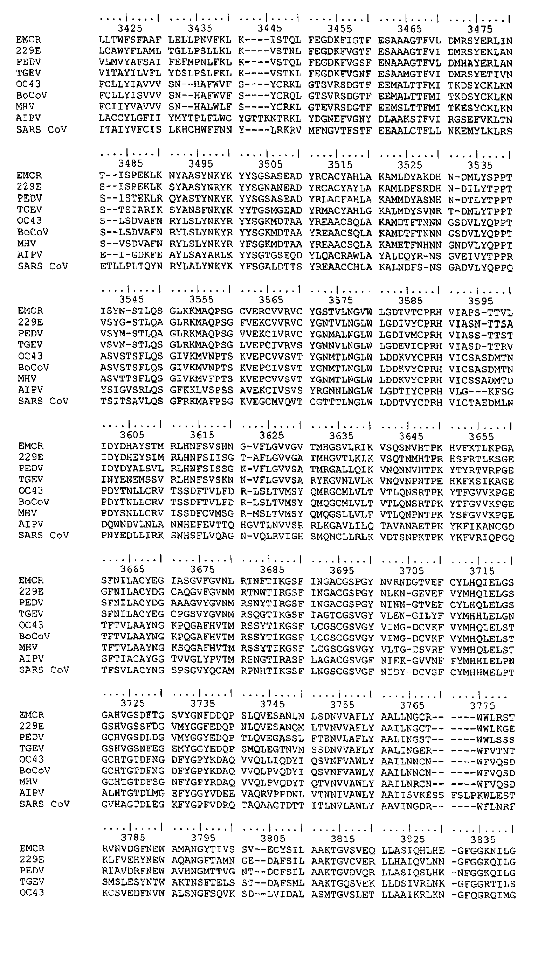

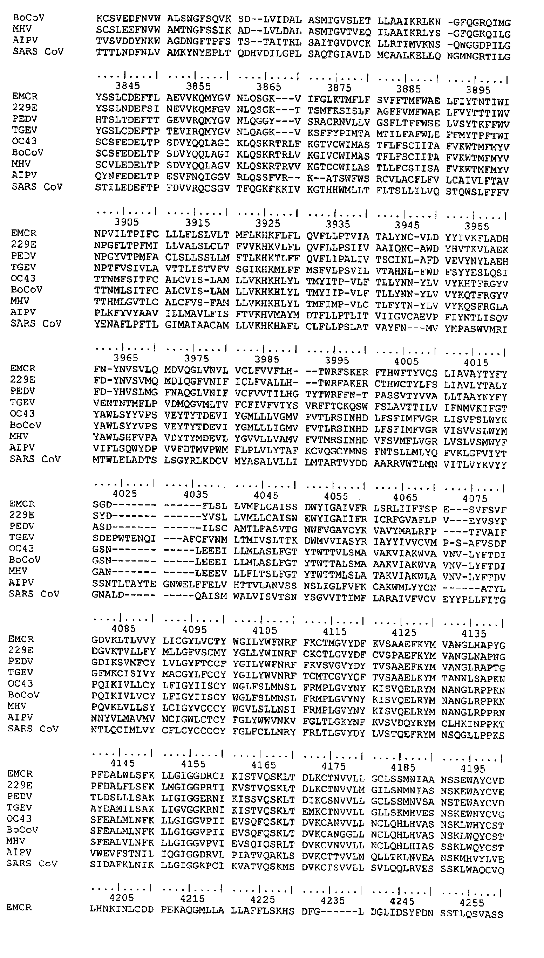

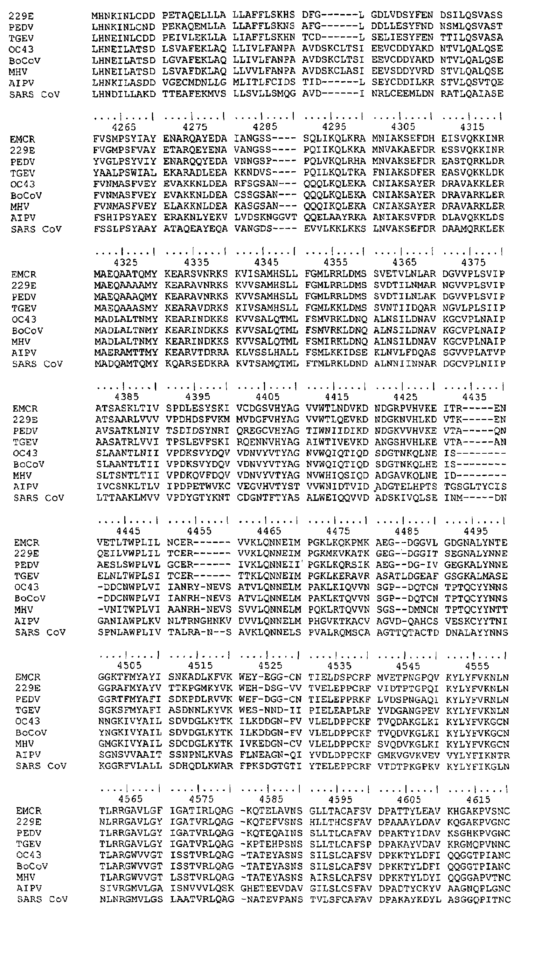

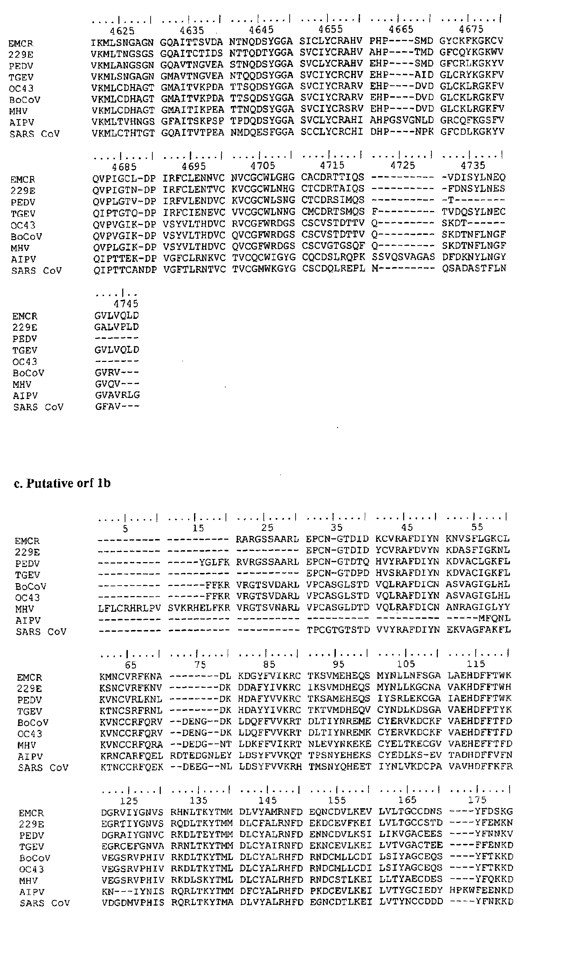

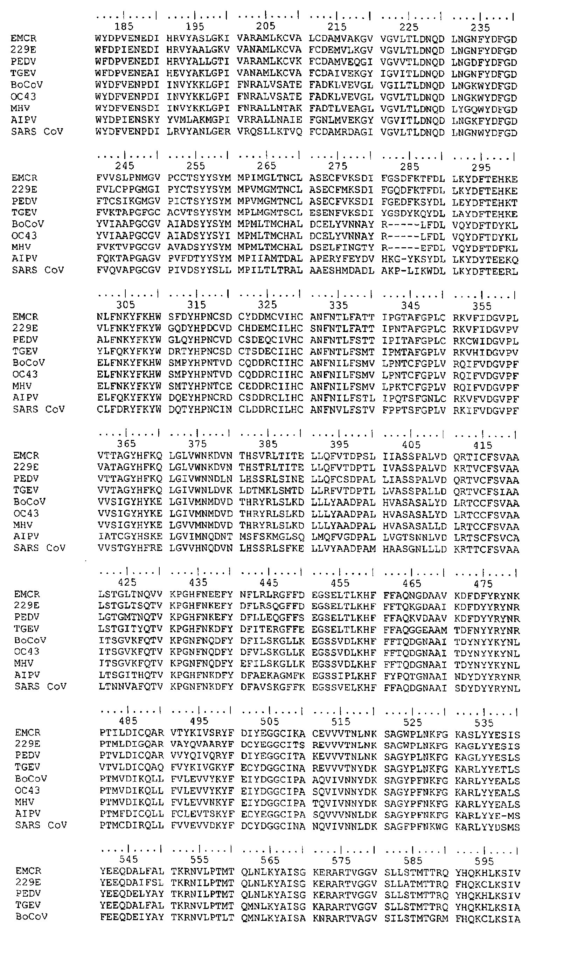

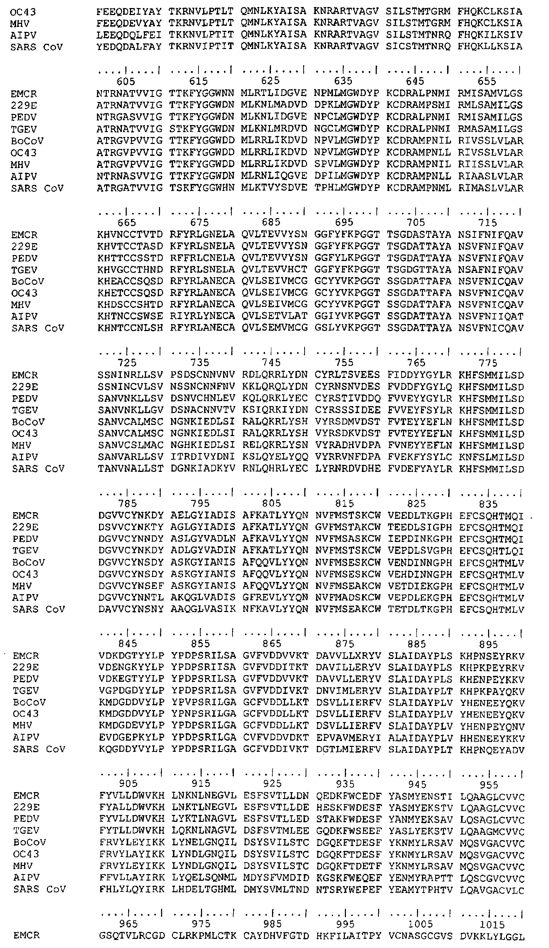

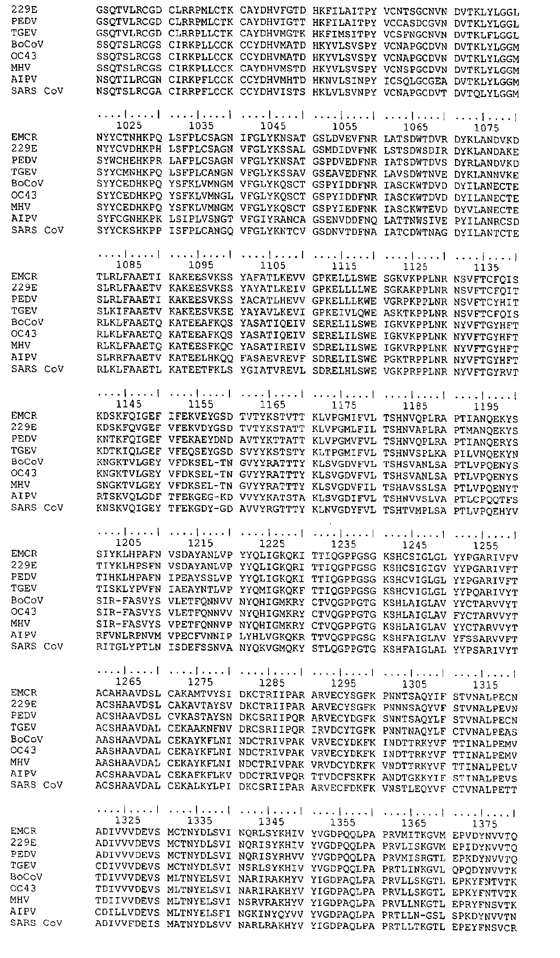

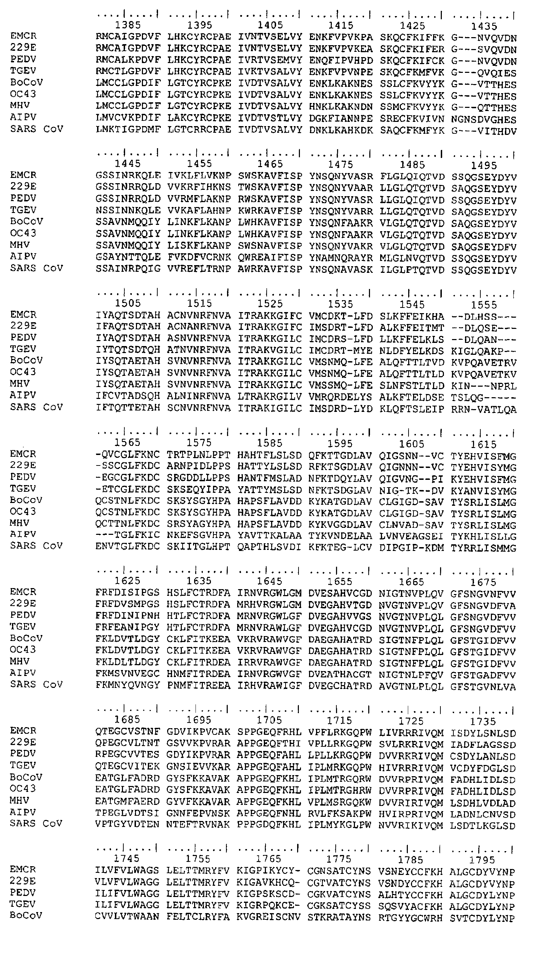

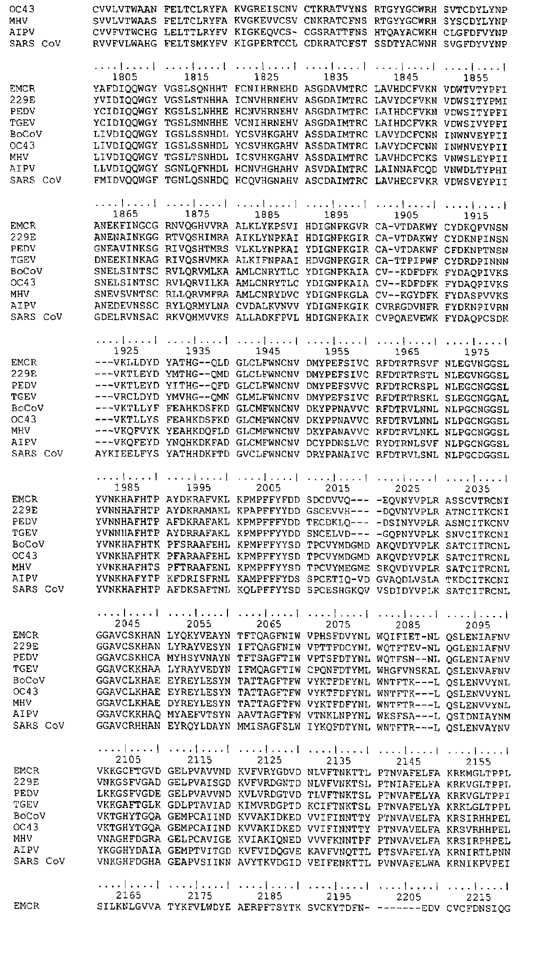

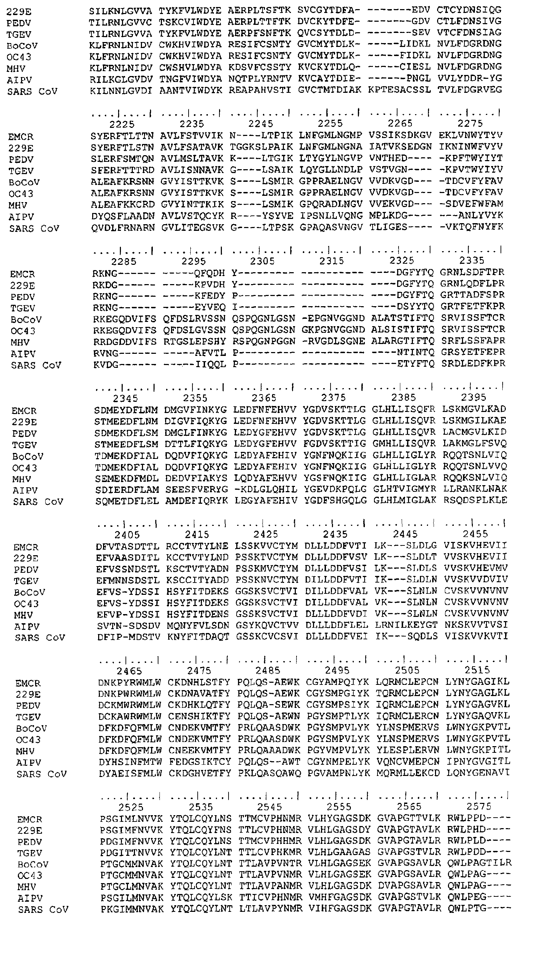

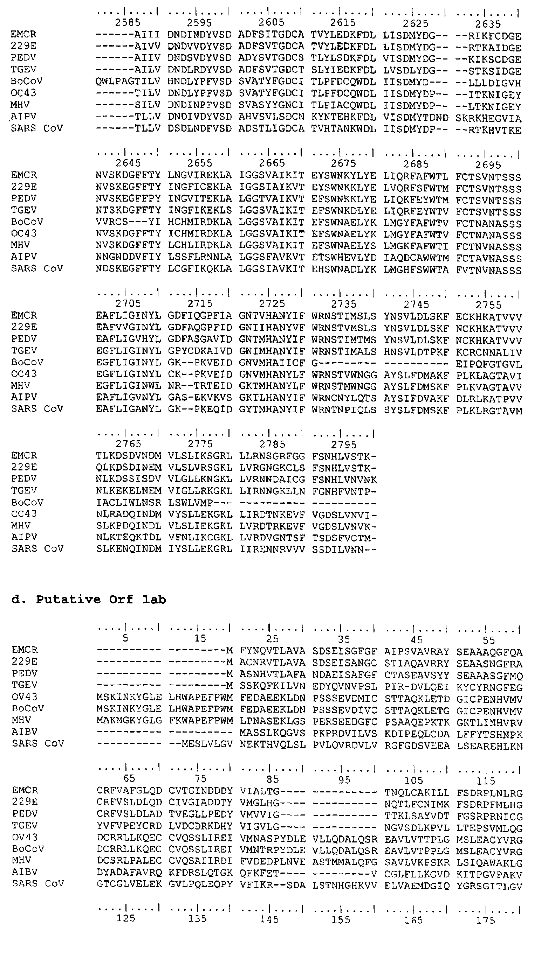

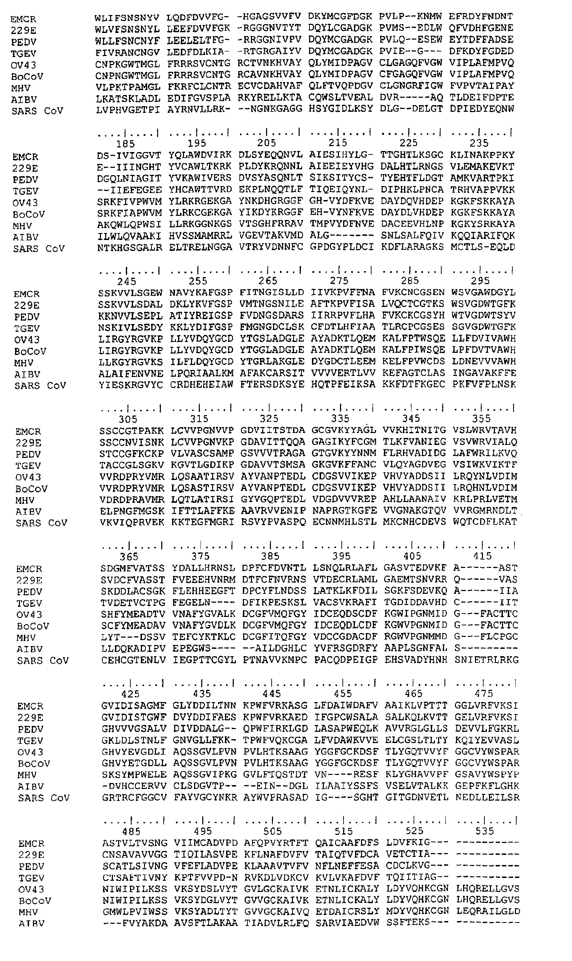

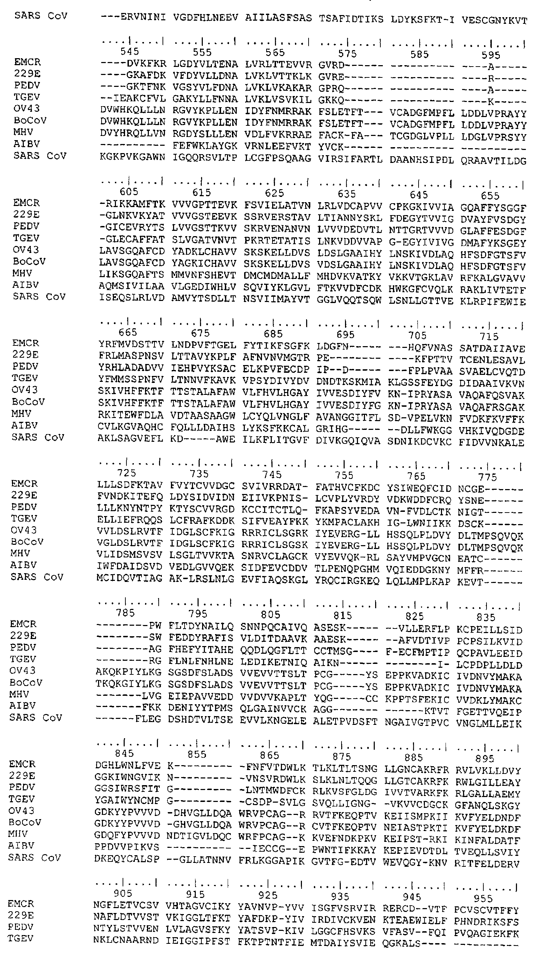

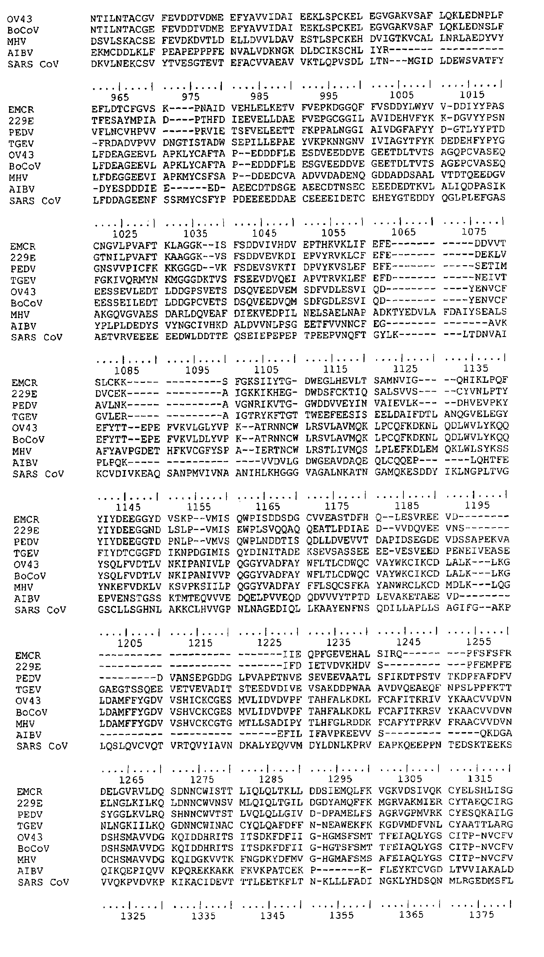

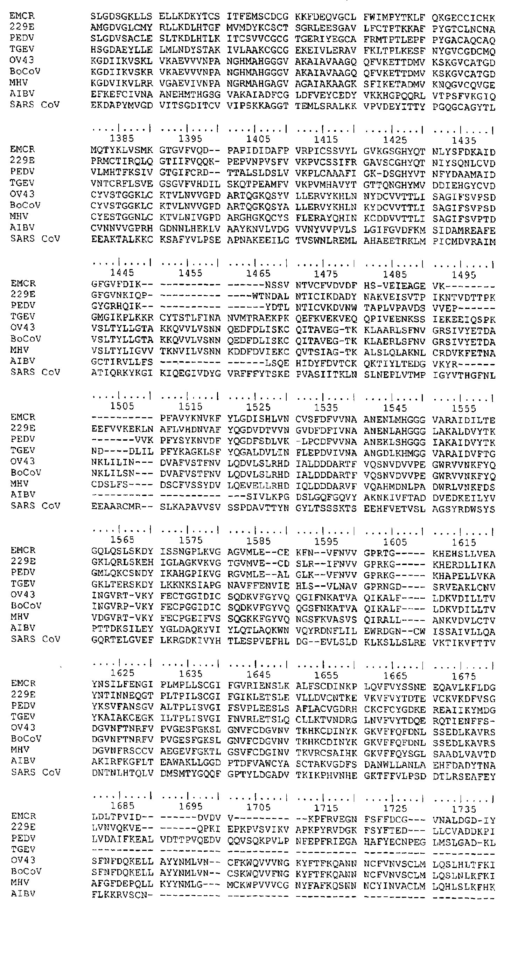

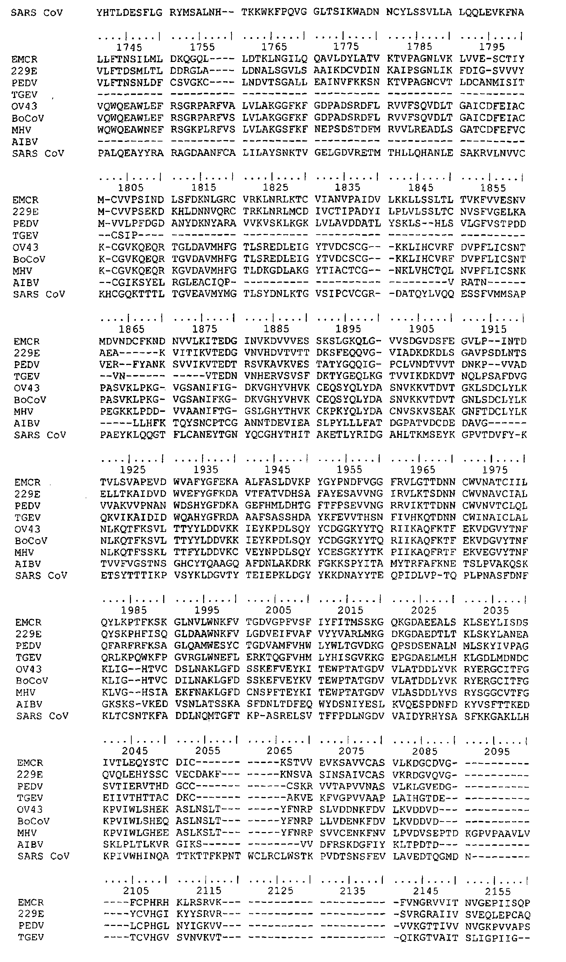

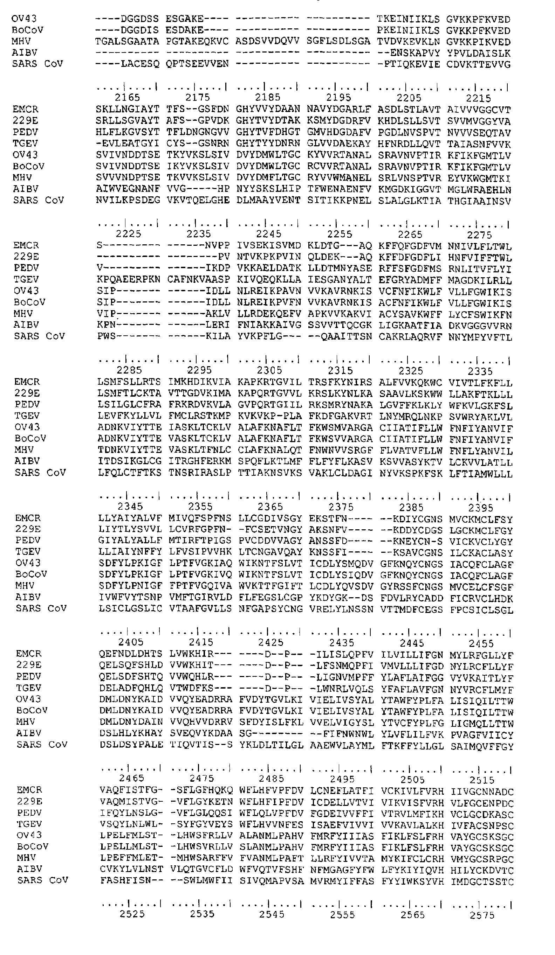

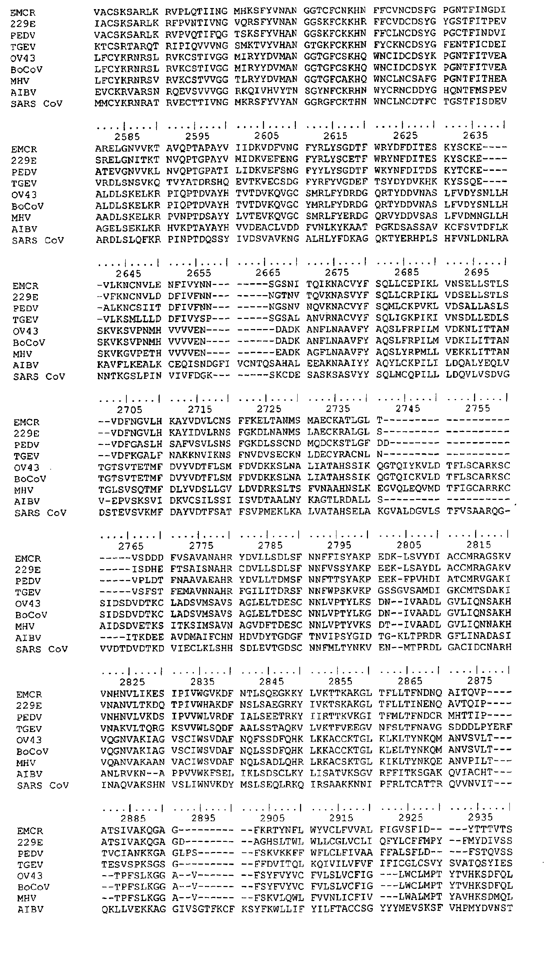

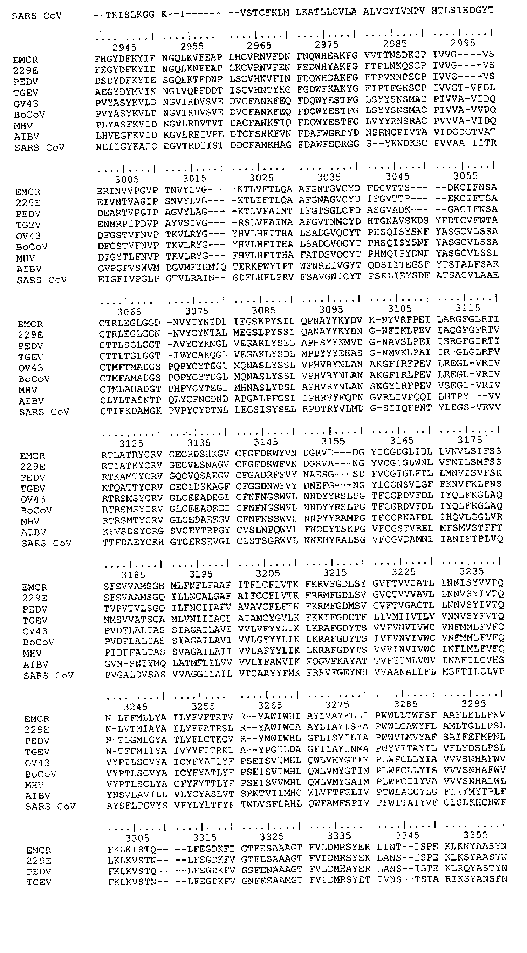

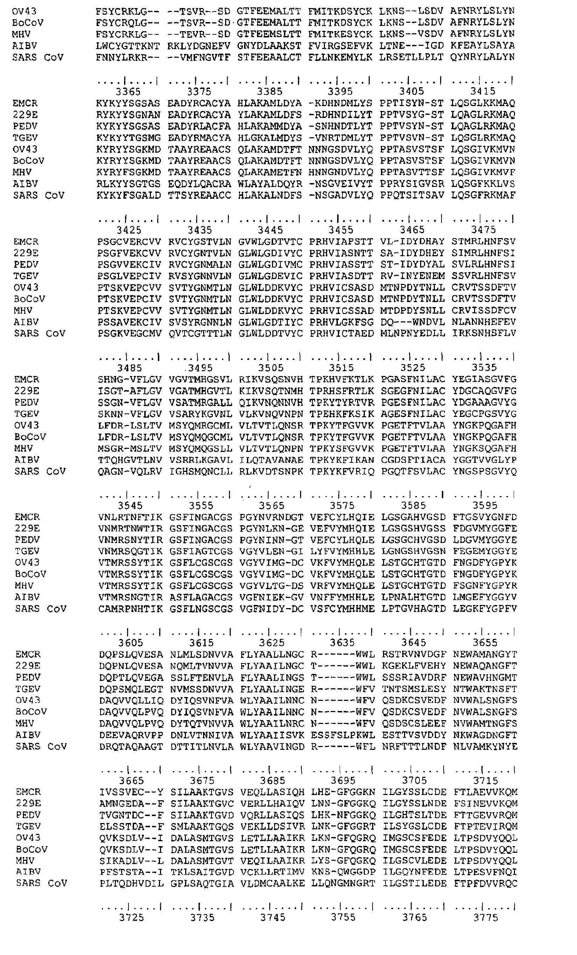

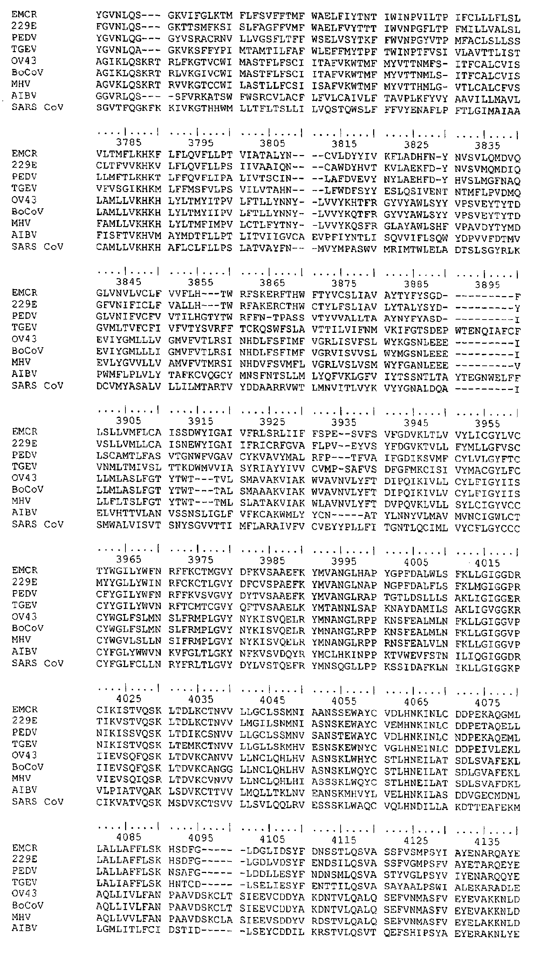

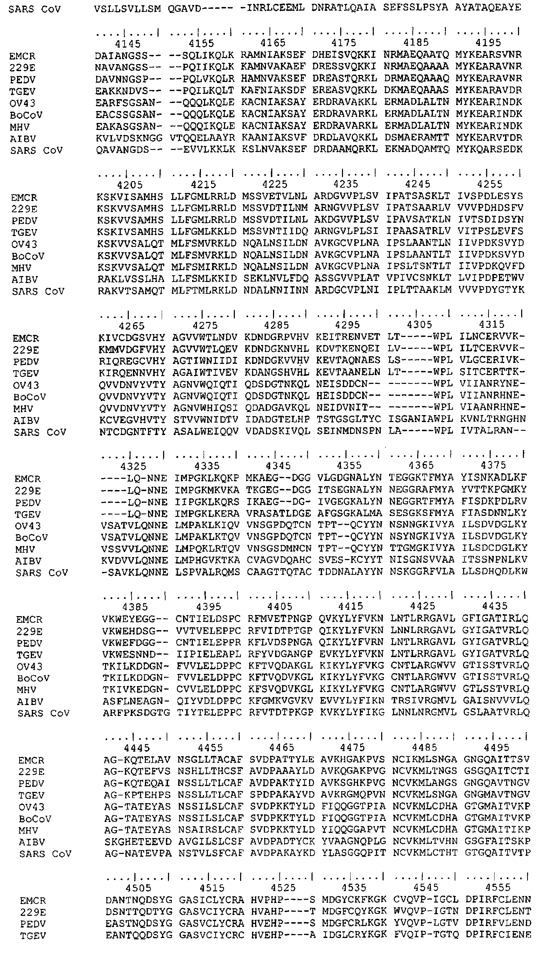

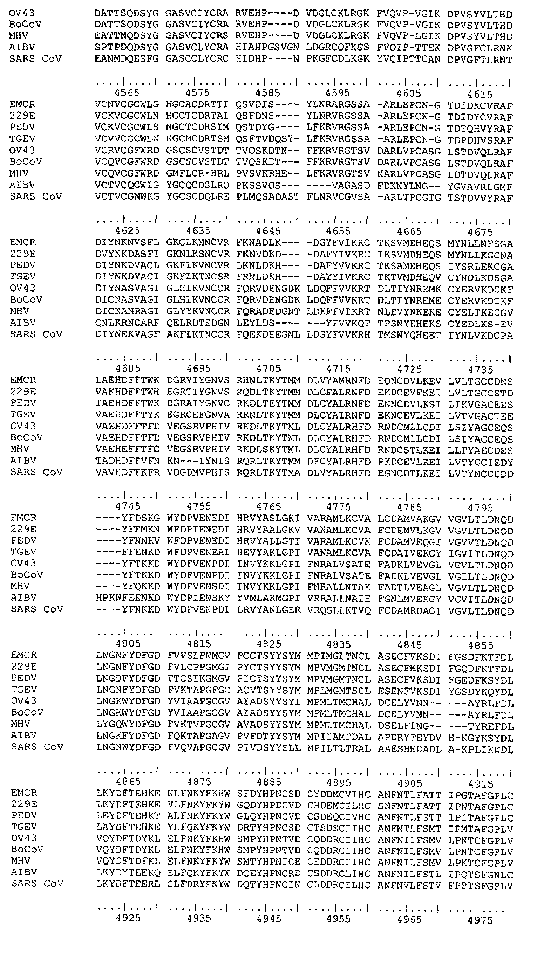

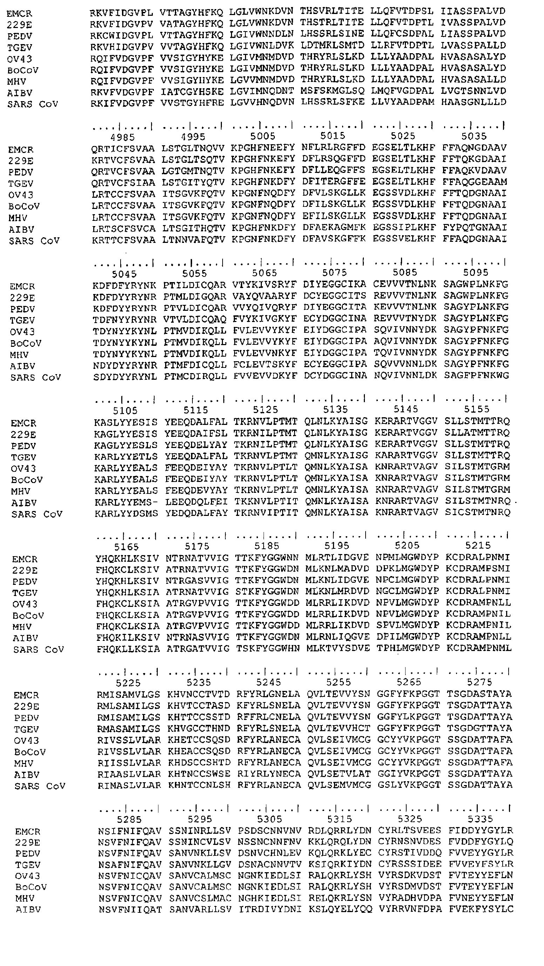

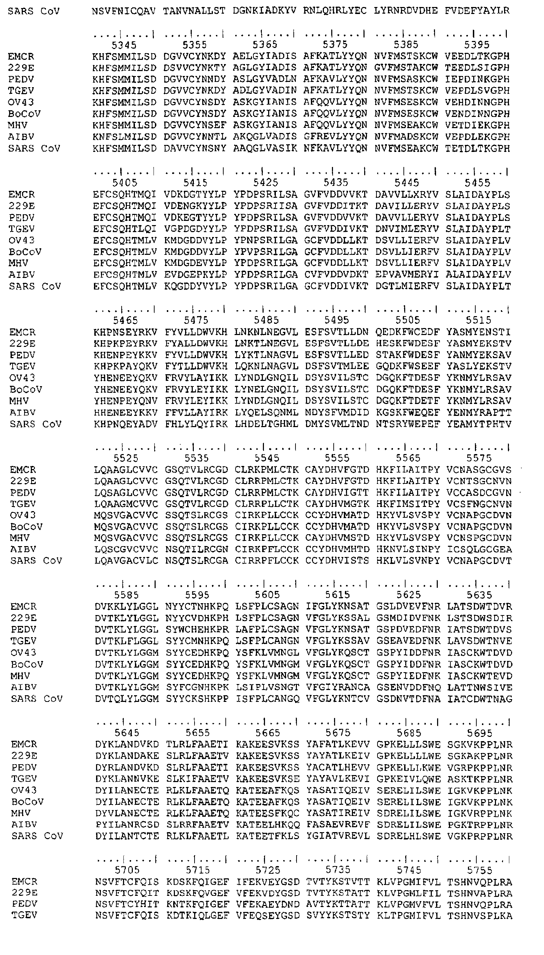

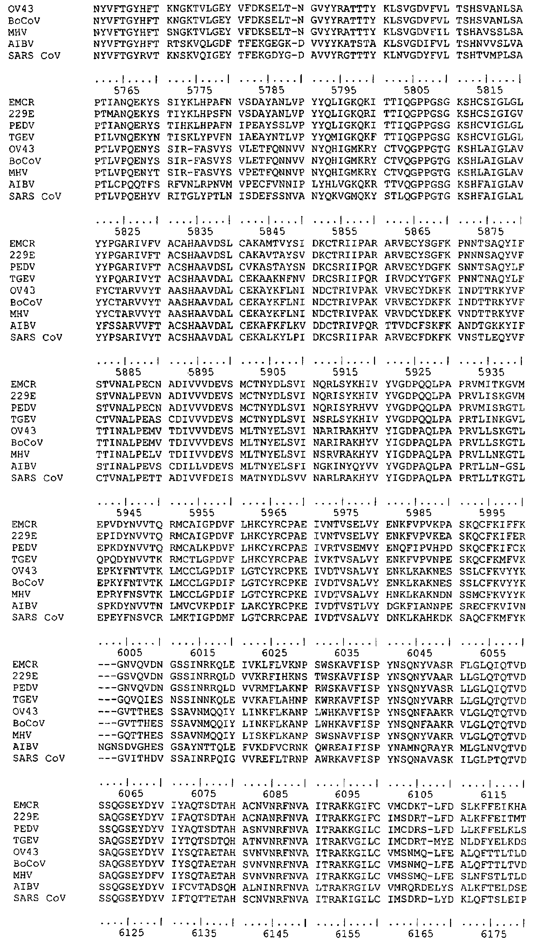

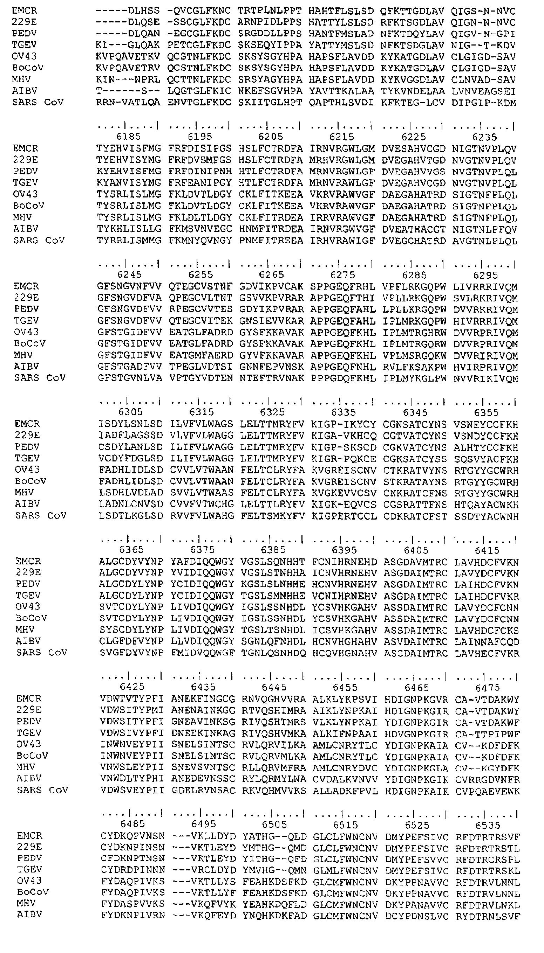

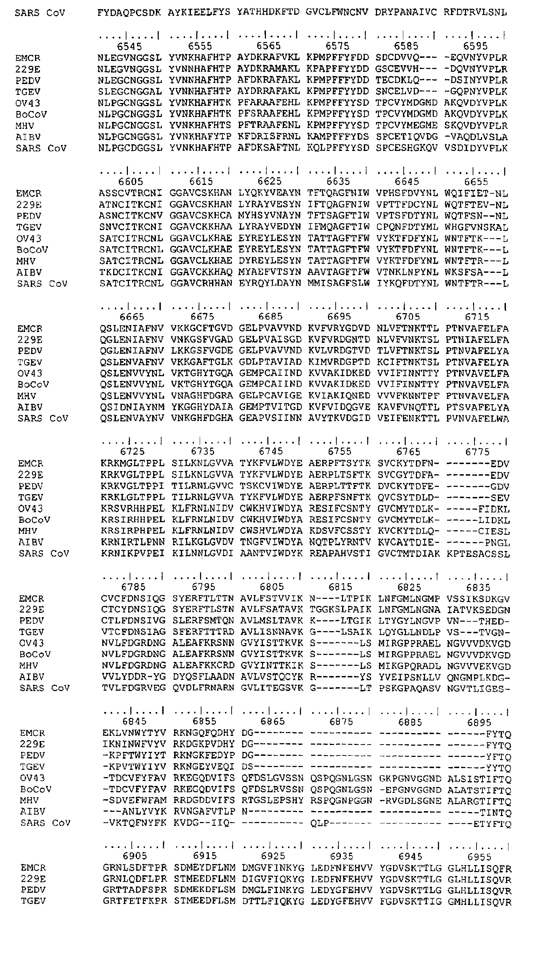

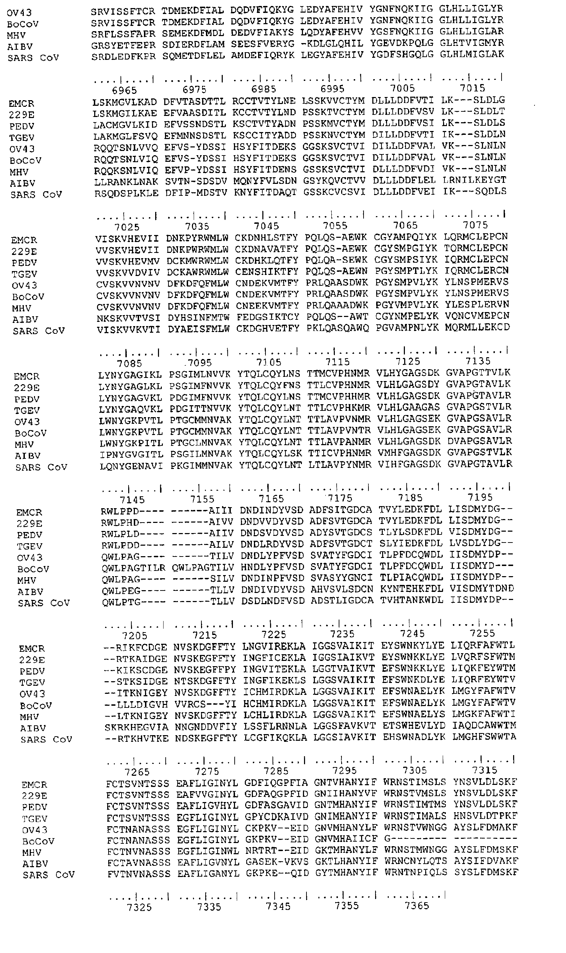

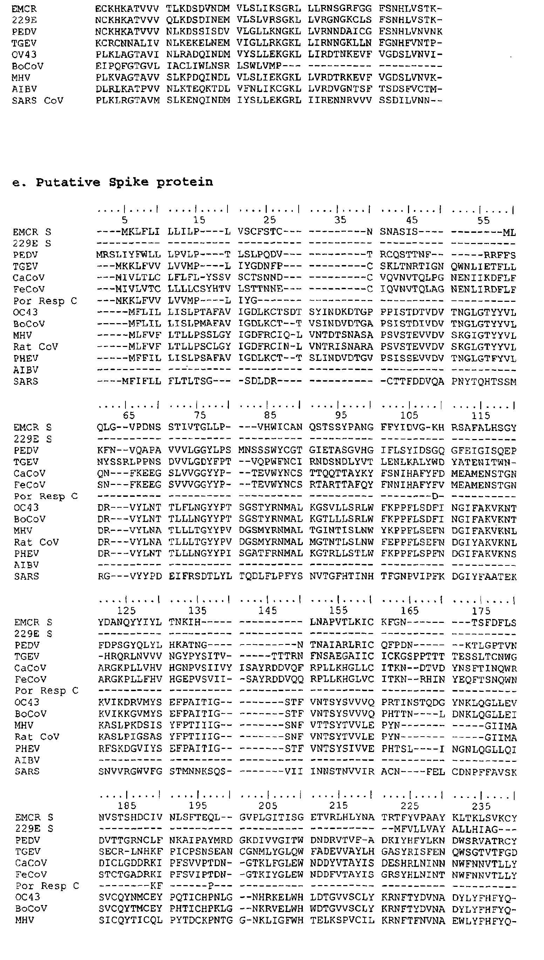

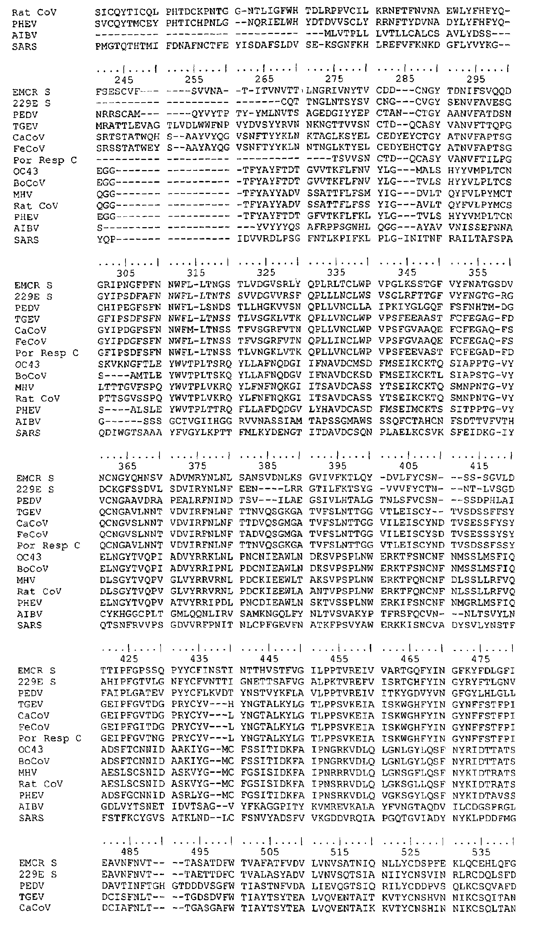

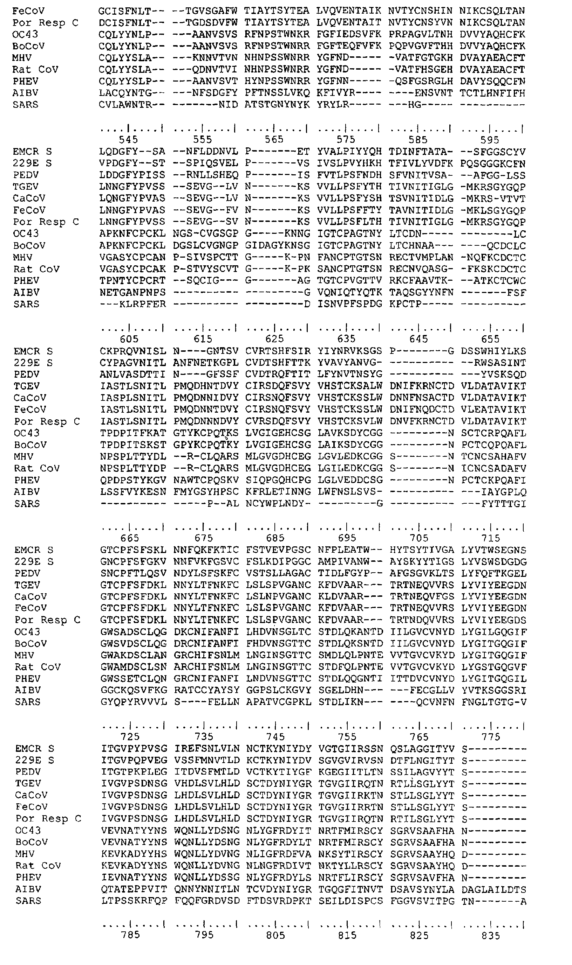

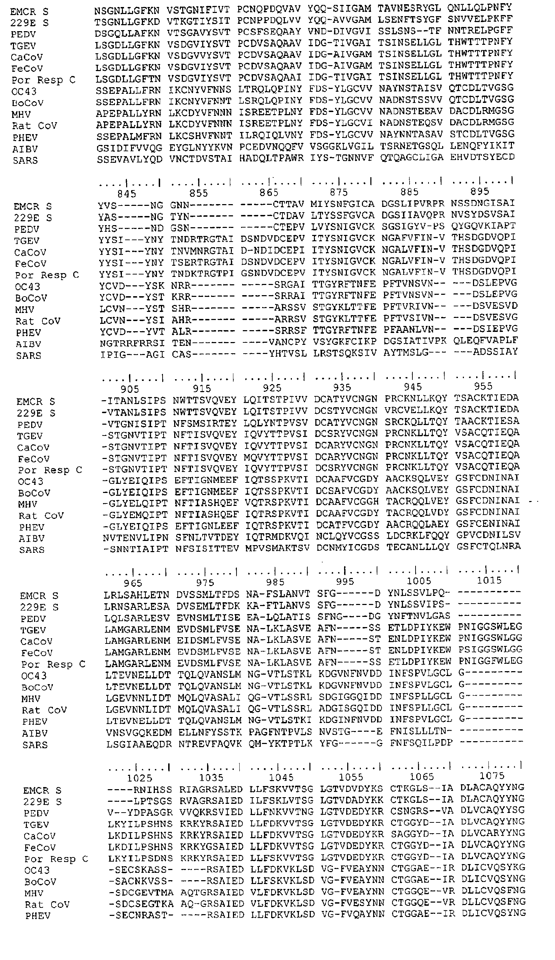

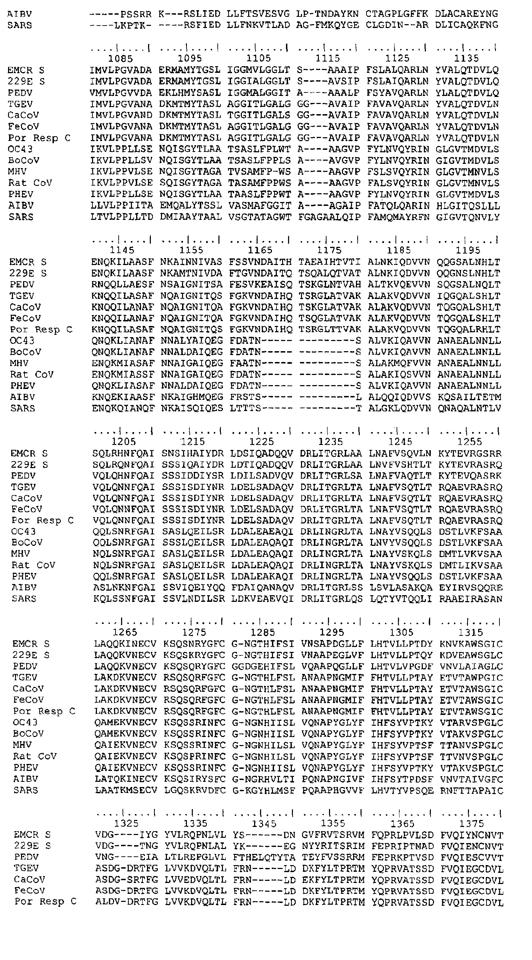

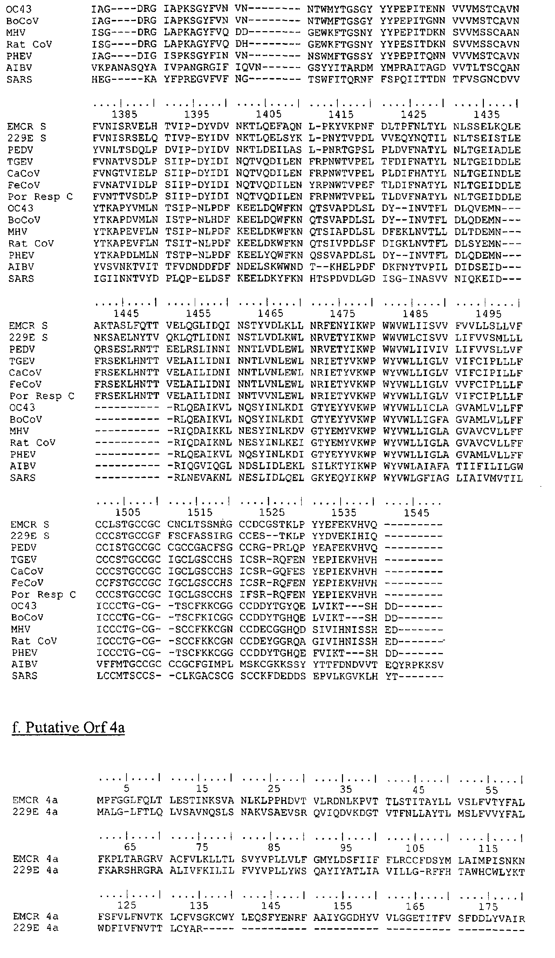

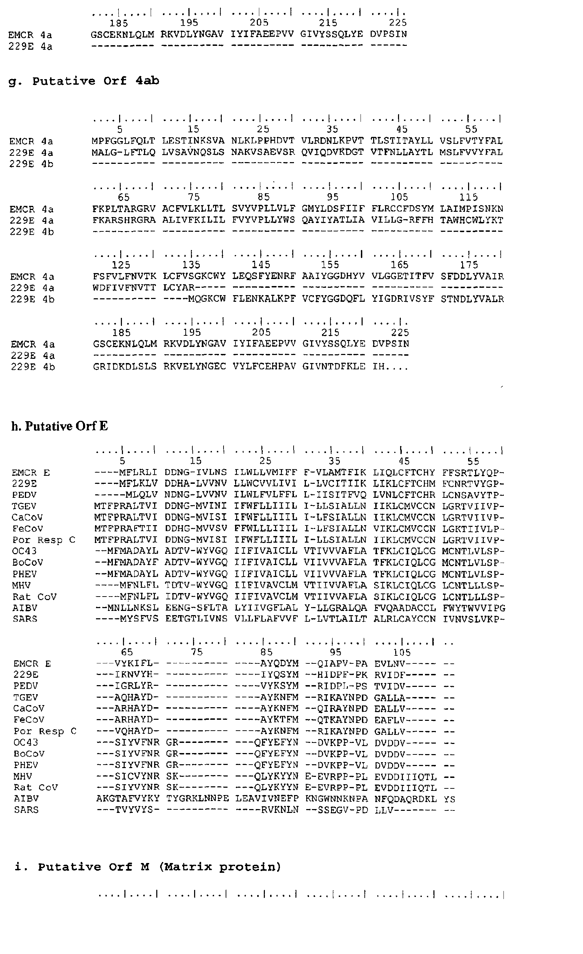

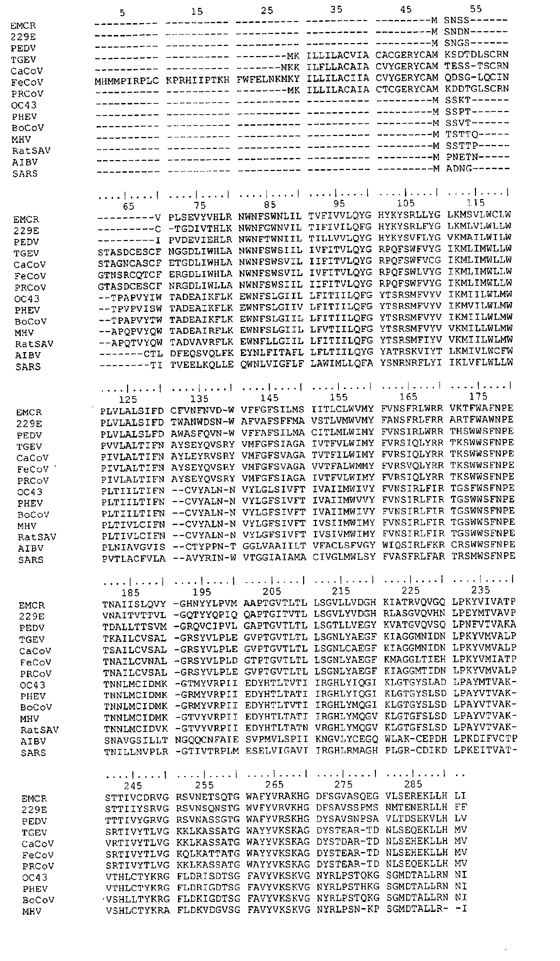

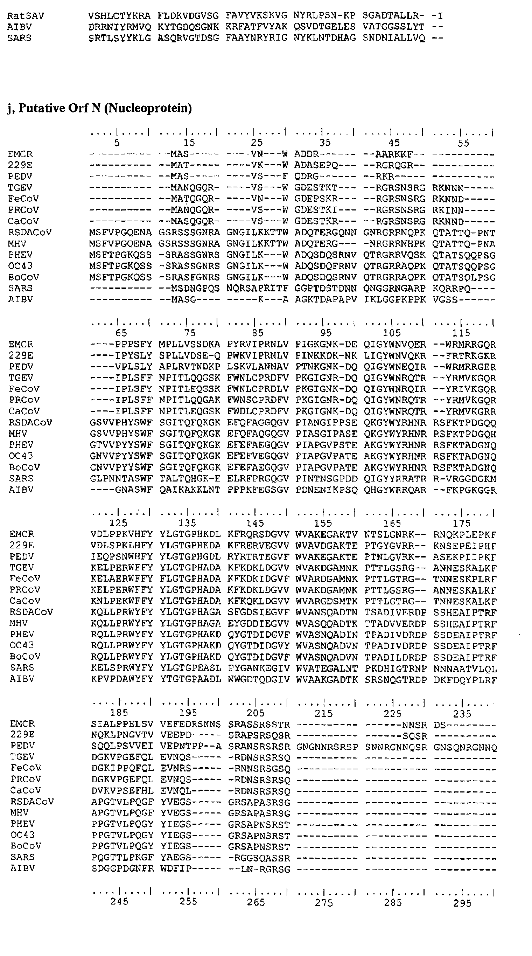

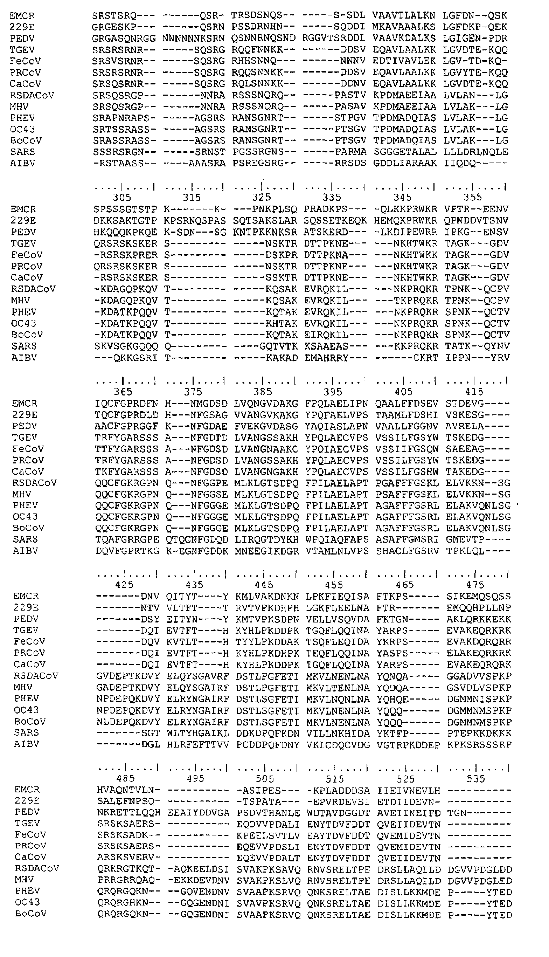

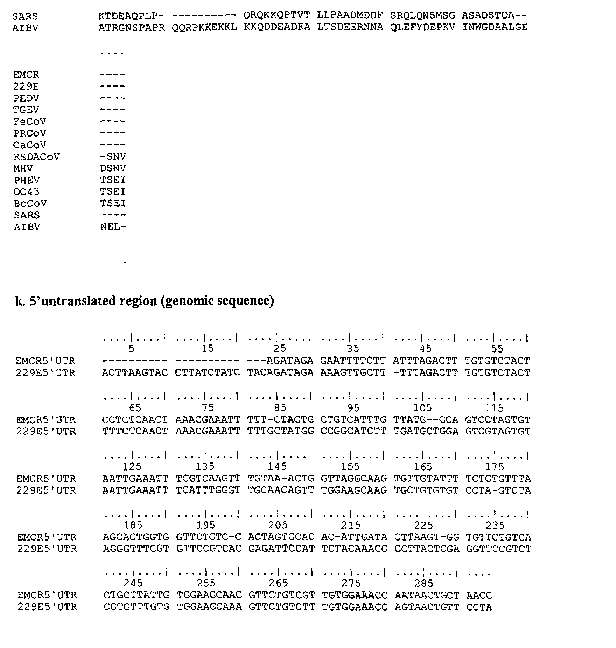

Newly isolated viruses are phylogenetically corresponding to and thus taxonomically corresponding to EMCR-CoV virus when comprising a gene order and/or amino acid sequence and/or nucleotide sequence sufficiently similar to our prototypic EMCR-CoV virus. The highest amino acid sequence identity, between ORFs of EMCR-CoV virus and any of the known other viruses of the same family to date are withhuman coronavirus 299E or Porcine Epidemic Diarrhea Virus (see Figures 3 and 4). The amino acid identities with human coronavirus 229E ranges from 45% (Nucleoprotein) to 81% (Replicase 1b); interestingly, Replicase 1a has an identity of just 56% contrasting with Replicase 1b's 81% identity. EMCR CoV has a closer identity with human coronavirus 229E than with any of the known other viruses of the same family to date for all putative ORFs, with the exception of Matrix, which is slightly more closely related to the Matrix ORF of PEDV. Individual proteins or whole virus isolates with, respectively, higher homology than these mentioned maximum values are considered phylogenetically corresponding and thus taxonomically corresponding to EMCR-CoV virus, and generally will be encoded by a nucleic acid sequence structurally corresponding with a sequence as shown in figure 1. Herewith the invention provides a virus phylogenetically corresponding to the isolated virus of which the sequences are depicted in figure 1.

It should be noted that, similar to other viruses, a certain degree of variation can be expected to be found between EMCR-CoV-viruses isolated from different sources.

Also, the viral sequence of the EMCR-CoV virus or an isolated EMCR-CoV virus gene as provided herein for example shows less than 95%, preferably less than 90%, more preferably less than 80%, more preferably less than 70% and most preferably less than 65% nucleotide sequence homology or less than 95%, preferably less than 90%, more preferably less than 80%, more preferably less than 70% and most preferably less than 65% amino acid sequence homology with the respective nucleotide or amino acid sequence of the human coronavirus 299E or Porcine Epidemic Diarrhea Virus as for example can be found in Genbank (for example in accession number af304460 (HCoV-299E) or af353511 (PEDV).

Sequence divergence of EMCR-CoV strains around the world may be somewhat higher, in analogy with other coronaviruses.

The term "nucleotide sequence homology" as used herein denotes the presence of homology between two (poly)nucleotides. Polynucleotides have "homologous" sequences if the sequence of nucleotides in the two sequences is the same when aligned for maximum correspondence. Sequence comparison between two or more polynucleotides is generally performed by comparing portions of the two sequences over a comparison window to identify and compare local regions of sequence similarity. The comparison window is generally from about 20 to 200 contiguous nucleotides. The "percentage of sequence homology" for polynucleotides, such as 50, 60, 70, 80, 90, 95, 98, 99 or 100 percent sequence homology may be determined by comparing two optimally aligned sequences over a comparison window, wherein the portion of the polynucleotide sequence in the comparison window may include additions or deletions (i.e. gaps) as compared to the reference sequence (which does not comprise additions or deletions) for optimal alignment of the two sequences. The percentage is calculated by: (a) determining the number of positions at which the identical nucleic acid base occurs in both sequences to yield the number of matched positions; (b) dividing the number of matched positions by the total number of positions in the window of comparison; and (c) multiplying the result by 100 to yield the percentage of sequence homology. Optimal alignment of sequences for comparison may be conducted by computerized implementations of known algorithms, or by inspection. Readily available sequence comparison and multiple sequence alignment algorithms are, respectively, the Basic Local Alignment Search Tool (BLAST) (Altschul, S.F. et al. 1990. J. Mol. Biol. 215:403; Altschul, S.F. et al. 1997. Nucleic Acid Res. 25:3389-3402) and ClustalW programs both available on the internet. Other suitable programs include GAP, BESTFIT and FASTA in the Wisconsin Genetics Software Package (Genetics Computer Group (GCG), Madison, WI, USA).
As used herein, "substantially complementary" means that two nucleic acid sequences have at least about 65%, preferably about 70%, more preferably about 80%, even more preferably 90%, and most preferably about 98%, sequence complementarity to each other. This means that the primers and probes must exhibit sufficient complementarity to their template and target nucleic acid, respectively, to hybridise under stringent conditions. Therefore, the primer sequences as disclosed in this specification need not reflect the exact sequence of the binding region on the template and degenerate primers can be used. A substantially complementary primer sequence is one that has sufficient sequence complementarity to the amplification template to result in primer binding and second-strand synthesis.

The term "hybrid" refers to a double-stranded nucleic acid molecule, or duplex, formed by hydrogen bonding between complementary nucleotides. The terms "hybridise" or "anneal" refer to the process by which single strands of nucleic acid sequences form double-helical segments through hydrogen bonding between complementary nucleotides.

The term "oligonucleotide" refers to a short sequence of nucleotide monomers (usually 6 to 100 nucleotides) joined by phosphorous linkages (e.g., phosphodiester, alkyl and aryl-phosphate, phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). Oligonucleotides may be naturally-occurring or synthetic molecules of double- and single-stranded DNA and double- and single-stranded RNA with circular, branched or linear shapes and optionally including domains capable of forming stable secondary structures (e.g., stem-and-loop and loop-stem-loop structures).

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in PCR amplification.

The term "probe" refers to a single-stranded oligonucleotide sequence that will recognize and form a hydrogen-bonded duplex with a complementary sequence in a target nucleic acid sequence analyte or its cDNA derivative.

The terms "stringency" or "stringent hybridization conditions" refer to hybridization conditions that affect the stability of hybrids, e.g., temperature, salt concentration, pH, formamide concentration and the like. These conditions are empirically optimised to maximize specific binding and minimize non-specific binding of primer or probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tm) for the specific sequence at a defined ionic strength and pH. The Tm is the temperature (under defined ionic strength and pH) at which 50% of a complementary target sequence hybridises to a perfectly matched probe or primer. Typically, stringent conditions will be those in which the salt concentration is less than about 1.0 M Na+ ion, typically about 0.01 to 1.0 M Na+ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel et al, Current Protocols in Molecular Biology, John Wiley & Sons Inc., 1994.

The term "antibody" includes reference to antigen binding forms of antibodies (e. g., Fab, F (ab) 2). The term "antibody" frequently refers to a polypeptide substantially encoded by an immunoglobulin gene or immunoglobulin genes, or fragments thereof which specifically bind and recognize an analyte (antigen). However, while various antibody fragments can be defined in terms of the digestion of an intact antibody, one of skill will appreciate that such fragments may be synthesized de novo either chemically or by utilizing recombinant DNA methodology. Thus, the term antibody, as used herein, also includes antibody fragments such as single chain Fv, chimeric antibodies (i. e., comprising constant and variable regions from different species), humanized antibodies (i. e., comprising a complementarity determining region (CDR) from a non-human source) and heteroconjugate antibodies (e. g., bispecific antibodies).

In short, the invention provides an isolated essentially mammalian positive-sense single stranded RNA virus (EMCR-CoV) belonging to the Coronaviruses and identifiable as phylogenetically corresponding thereto by determining a nucleic acid sequence of a suitable fragment of the genome of said virus and testing it in phylogenetic tree analyses wherein maximum likelihood trees are generated using 100 bootstraps and 3 jumbles and finding it to be more closely phylogenetically corresponding to a virus isolate having the sequences as depicted in figure 1 than it is corresponding to a virus isolate of PEDV (porcine epidemic diarrhea virus), HCoV-229E (human coronavirus 229E), PRCoV (porcine respiratory coronavirus), TGEV (transmissible gastroenteritis virus), CaCoV (Canine coronavirus) and FeCoV (feline coronavirus).

Suitable nucleic acid genome fragments each useful for such phylogenetic tree analyses are for example any of the fragments encoding the Matrix protein or the Nucleocapsid protein as disclosed in Figure 1, leading to the phylogenetic tree analysis as disclosed herein in figure 2a or 2b. Other suitable nucleic acid fragments useful for such phylogenetic tree analyses are for example any of the fragments encoding Replicase 1a and 1b, Spike, orf 4a and 4b, and E.

A suitable open reading frame (ORF) useful in phylogenetic analyses comprises the ORF encoding the viral replicase (ORF 1a). When an overall amino acid identity of at least 60%, preferably of at least 70%, more preferably of at least 80%, more preferably of at least 90%, most preferably of at least 95% of the analysed replicase with the replicase having a sequence comprising the amino acids of Figure 1 is found, the analysed virus isolate comprises an EMCR-CoV virus isolate according to the invention.

A suitable open reading frame (ORF) useful in phylogenetic analyses comprises the ORF encoding the viral replicase (ORF 1b). When an overall amino acid identity of at least 82%, more preferably of at least 90%, most preferably of at least 95% of the analysed replicase with the replicase having a sequence comprising the amino acids of Figure 1 is found, the analysed virus isolate comprises an EMCR-CoV virus isolate according to the invention.

Another suitable open reading frame (ORF) useful in phylogenetic analyses comprises the ORF encoding the Nucleocapsid protein. When an overall amino acid identity of at least 50%, more preferably of at least 60%, more preferably of at least 70%, more preferably of at least 80%, more preferably of at least 90%, most preferably of at least 95% of the analysed. Nucleocapsid protein with the Nucleocapsid protein encoded by a sequence comprising (part of) the sequence F of Figure 1 is found, the analysed virus isolate comprises an EMCR-CoV isolate according to the invention.

Another suitable open reading frame (ORF) useful in phylogenetic analyses comprises the ORF encoding the Matrix protein. When an overall amino acid identity of at least 60%, more preferably of at least 70%, more preferably of at least 80%, more preferably of at least 90%, most preferably of at least 95% of the analysed Matrix protein with the Matrix protein encoded by a sequence comprising (part of) the sequence F of Figure 1 is found, the analysed virus isolate comprises an EMCR-CoV isolate according to the invention.

Another suitable open reading frame (ORF) useful in phylogenetic analyses comprises the ORF encoding the spike protein S. When an overall amino acid identity of at least 55%, more preferably of at least 60%, more preferably of at least 70%, more preferably of at least 80%, more preferably of at least 90%, most preferably of at least 95% of the analysed S-protein encoded by a sequence comprising the sequence of translation 2 of E and translation 1 of the F sequence of the S-protein as depicted in Figure 1 is found, the analysed virus isolate comprises an EMCR-CoV virus isolate according to the invention. The S ORF of the EMCR-CoV virus seems to be located adjacent to the ORF 1ab (coding for the viral replicase), which would discriminate an EMCR-CoV viruses from the bovine coronavirus and the murine hepatitis virus, which have a so-called 2a gene and an HE-gene between the S protein and the viral polymerase.

The invention provides among others an isolated or recombinant nucleic acid or virus-specific functional fragment thereof obtainable from a virus according to the invention. The isolated or recombinant nucleic acids comprises the sequences as given in figure 1 or sequences of homologues which are able to hybridise with those under stringent conditions. In particular, the invention provides primers and/or probes suitable for identifying an EMCR-CoV virus nucleic acid.

Furthermore, the invention provides a vector comprising a nucleic acid according to the invention. To begin with, vectors such as plasmid vectors containing (parts of) the genome of the EMCR-CoV virus, virus vectors containing (parts of) the genome of the EMCR-CoV (for example, but not limited thereto, vaccinia virus, retroviruses, baculovirus), or EMCR-CoV virus containing (parts of) the genome of other viruse or other pathogens are provided.

Also, the invention provides a host cell comprising a nucleic acid or a vector according to the invention. Plasmid or viral vectors containing the replicase components of EMCR-CoV virus are generated in prokaryotic cells for the expression of the components in relevant cell types (bacteria, insect cells, eukaryotic cells). Plasmid or viral vectors containing full-length or partial copies of the EMCR-CoV virus genome will be generated in prokaryotic cells for the expression of viral nucleic acids *in-vitro* or *in-vivo*. The latter vectors may contain other viral sequences for the generation of chimeric viruses or chimeric virus proteins, may lack parts of the viral genome for the generation of replication defective virus, and may contain mutations, deletions or insertions for the generation of attenuated viruses.

Infectious copies of EMCR-CoV virus (being wild type, attenuated, replication-defective or chimeric) can be produced upon co-expression of the polymerase components according to the state-of-the-art technologies described above.

In addition, eukaryotic cells, transiently or stably expressing one or more full-length or partial EMCR-CoV virus proteins can be used. Such cells can be made by transfection (proteins or nucleic acid vectors), infection (viral vectors) or transduction (viral vectors) and may be useful for complementation of mentioned wild type, attenuated, replication-defective or chimeric viruses.

A chimeric virus may be of particular use for the generation of recombinant vaccines protecting against two or more viruses. For example, it can be envisaged that EMCR-CoV virus vector expressing one or more proteins of a human metapneumovirus or a human metapneumovirus vector expressing one or more proteins of EMCR-CoV virus will protect individuals vaccinated with such vector against both virus infections. Such a specific chimeric virus is particularly useful in the invention because it is suspected that co-infection of, for instance, human metapneumovirus frequently occurs in coronavirus infected patients. Attenuated and replication-defective viruses may be of use for vaccination purposes with live vaccines as has been suggested for other viruses.

In a preferred embodiment, the invention provides a proteinaceous molecule or coronavirus-specific viral protein or functional fragment thereof encoded by a nucleic acid according to the invention. Useful proteinaceous molecules are for example derived from any of the genes or genomic fragments derivable from a virus according to the invention. Such molecules, or antigenic fragments thereof, as provided herein, are for example useful in diagnostic methods or kits and in pharmaceutical compositions such as sub-unit vaccines and inhibitory peptides. Particularly useful are the viral replicase protein, the spike protein, the matrix protein, the nucleocapsid or antigenic fragments thereof for inclusion as antigen or subunit immunogen, but inactivated whole virus can also be used. Particulary useful are also those proteinaceous substances that are encoded by recombinant nucleic acid fragments that are identified for phylogenetic analyses, of course preferred are those that are within the preferred bounds and metes of ORFs useful in phylogenetic analyses, in particular for eliciting EMCR-CoV virus specific antibodies, whether in vivo (e.g. for protective puposes or for providing diagnostic antibodies) or in vitro (e.g. by phage display technology or another technique useful for generating synthetic antibodies).

Also provided herein are antibodies, be it natural polyclonal or monoclonal, or synthetic (e.g. (phage) library-derived binding molecules) antibodies that specifically react with an antigen comprising a proteinaceous molecule or EMCR-CoV virus-specific functional fragment thereof according to the invention. Such antibodies are useful in a method for identifying a viral isolate as an EMCR-CoV virus comprising reacting said viral isolate or a component thereof with an antibody as provided herein. This can for example be achieved by using purified or non-purified EMCR-CoV virus or parts thereof (proteins, peptides) using ELISA, RIA, FACS or similar formats of antigen detection assays (Current Protocols in Immunology). Alternatively, infected cells or cell cultures may be used to identify viral antigens using classical immunofluorescence or immunohistochemical techniques. Specifically useful in this respect are antibodies raised against EMCR-CoV virus proteins which are encoded by a nucleotide sequence comprising one or more of the sequences disclosed in figure 1.

Other methods for identifying a viral isolate as an EMCR-CoV virus comprise reacting said viral isolate or a component thereof with a virus specific nucleic acid according to the invention.

In this way the invention provides a viral isolate identifiable with a method according to the invention as a mammalian virus taxonomically corresponding to a positive-sense single stranded RNA virus identifiable as likely belonging to the EMCR-CoV virus genus within the family of Coronaviruses.

The method is useful in a method for virologically diagnosing an EMCR-CoV virus infection of a mammal, said method for example comprising determining in a sample of said mammal the presence of a viral isolate or component thereof by reacting said sample with a nucleic acid or an antibody according to the invention.

Methods of the invention can in principle be performed by using any nucleic acid amplification method, such as the Polymerase Chain Reaction (PCR; Mullis 1987, U.S. Pat. No. 4,683,195, 4,683,202, en 4,800,159) or by using amplification reactions such as Ligase Chain Reaction (LCR; Barany 1991, Proc. Natl. Acad. Sci. USA 88:189-193; EP Appl. No., 320,308), Self-Sustained Sequence Replication (3SR; Guatelli et al., 1990, Proc. Natl. Acad. Sci. USA 87:1874-1878), Strand Displacement Amplification (SDA; U.S. Pat. Nos. 5,270,184, en 5,455,166), Transcriptional Amplification System (TAS; Kwoh et al., Proc. Natl. Acad. Sci. USA 86:1173-1177), Q-Beta Replicase (Lizardi et al., 1988, Bio/Technology 6:1197), Rolling Circle Amplification (RCA; U.S. Pat. No. 5,871,921), Nucleic Acid Sequence Based Amplification (NASBA), Cleavase Fragment Length Polymorphism (U.S. Pat. No. 5,719,028), Isothermal and Chimeric Primer-initiated Amplification of Nucleic Acid (ICAN), Ramification-extension Amplification Method (RAM; U.S. Pat. Nos. 5,719,028 and 5,942,391) or other suitable methods for amplification of nucleic acids.
In order to amplify a nucleic acid with a small number of mismatches to one or more of the amplification primers, an amplification reaction may be performed under conditions of reduced stringency (e.g. a PCR amplification using an annealing temperature of 38°C, or the presence of 3.5 mM MgCl2). The person skilled in the art will be able to select conditions of suitable stringency.

The primers herein are selected to be "substantially" complementary (i.e. at least 65%, more preferably at least 80% perfectly complementary) to their target regions present on the different strands of each specific sequence to be amplified. It is possible to use primer sequences containing e.g. inositol residues or ambiguous bases or even primers that contain one or more mismatches when compared to the target sequence. In general, sequences that exhibit at least 65%, more preferably at least 80% homology with the target DNA or RNA oligonucleotide sequences, are considered suitable for use in a method of the present invention. Sequence mismatches are also not critical when using low stringency hybridization conditions.

The detection of the amplification products can in principle be accomplished by any suitable method known in the art. The detection fragments may be directly stained or labelled with radioactive labels, antibodies, luminescent dyes, fluorescent dyes, or enzyme reagents. Direct DNA stains include for example intercalating dyes such as acridine orange, ethidium bromide, ethidium monoazide or Hoechst dyes. Alternatively, the DNA or RNA fragments may be detected by incorporation of labelled dNTP bases into the synthesized fragments. Detection labels which may be associated with nucleotide bases include e.g. fluorescein, cyanine dye or BrdUrd.

When using a probe-based detection system, a suitable detection procedure for use in the present invention may for example comprise an enzyme immunoassay (EIA) format (Jacobs et al., 1997, J. Clin. Microbiol. 35, 791-795). For performing a detection by manner of the EIA procedure, either the forward or the reverse primer used in the amplification reaction may comprise a capturing group, such as a biotin group for immobilization of target DNA PCR amplicons on e.g. a streptavidin coated microtiter plate wells for subsequent EIA detection of target DNA -amplicons (see below). The skilled person will understand that other groups for immobilization of target DNA PCR amplicons in an EIA format may be employed.

Probes useful for the detection of the target DNA as disclosed herein preferably bind only to at least a part of the DNA sequence region as amplified by the DNA amplification procedure. Those of skill in the art can prepare suitable probes for detection based on the nucleotide sequence of the target DNA without undue experimentation as set out herein. Also the complementary nucleotide sequences, whether DNA or RNA or chemically synthesized analogs, of the target DNA may suitably be used as type-specific detection probes in a method of the invention, provided that such a complementary strand is amplified in the amplification reaction employed.

Suitable detection procedures for use herein may for example comprise immobilization of the amplicons and probing the DNA sequences thereof by e.g. southern blotting. Other formats may comprise an EIA format as described above. To facilitate the detection of binding, the specific amplicon detection probes may comprise a label moiety such as a fluorophore, a chromophore, an enzyme or a radio-label, so as to facilitate monitoring of binding of the probes to the reaction product of the amplification reaction. Such labels are well-known to those skilled in the art and include, for example, fluorescein isothiocyanate (FITC), β-galactosidase, horseradish peroxidase, streptavidin, biotin, digoxigenin, 35S or 125I. Other examples will be apparent to those skilled in the art.

Detection may also be performed by a so called reverse line blot (RLB) assay, such as for instance described by Van den Brule et al. (2002, J. Clin. Microbiol. 40, 779-787). For this purpose RLB probes are preferably synthesized with a 5' amino group for subsequent immobilization on e.g. carboxyl-coated nylon membranes. The advantage of an RLB format is the ease of the system and its speed, thus allowing for high throughput sample processing.

The use of nucleic acid probes for the detection of RNA or DNA fragments is well known in the art. Mostly these procedure comprise the hybridization of the target nucleic acid with the probe followed by post-hybridization washings. Specificity is typically the function of post-hybridization washes, the critical factors being the ionic strength and temperature of the final wash solution. For nucleic acid hybrids, the Tm can be approximated from the equation of Meinkoth and Wahl, Anal. Biochem., 138: 267-284 (1984): Tm = 81.5 °C + 16.6 (log M) + 0.41 (% GC)-0.61 (% form)-500/L; where M is the molarity of monovalent cations, % GC is the percentage of guanosine and cytosine nucleotides in the nucleic acid, % form is the percentage of formamide in the hybridization solution, and L is the length of the hybrid in base pairs. The Tm is the temperature (under defined ionic strength and pH) at which 50% of a complementary target sequence hybridizes to a perfectly matched probe. Tm is reduced by about 1 °C for each 1 % of mismatching; thus, the hybridization and/or wash conditions can be adjusted to hybridize to sequences of the desired identity. For example, if sequences with > 90% identity are sought, the Tm can be decreased 10°C. Generally, stringent conditions are selected to be about 5 °C lower than the thermal melting point (Tm) for the specific sequence and its complement at a defined ionic strength and pH. However, severely stringent conditions can utilize a hybridization and/or wash at 1,2,3, or 4 °C lower than the thermal melting point (Tm); moderately stringent conditions can utilize a hybridization and/or wash at 6, 7, 8, 9, or 10 °C lower than the thermal melting point (Tm); low stringency conditions can utilize a hybridization and/or wash at 11, 12, 13, 14, 15, or 20 °C lower than the thermal melting point (Tm). Using the equation, hybridization and wash compositions, and desired Tm, those of ordinary skill will understand that variations in the stringency of hybridization and/or wash solutions are inherently described. If the desired degree of mismatching results in a Tm of less than 45 °C (aqueous solution) or 32 °C (formamide solution) it is preferred to increase the SSC concentration so that a higher temperature can be used. An extensive guide to the hybridization of nucleic acids is found in Tijssen, Laboratory Techniques in Biochemistm and Molecular Biology―Hybridization with Nucleic Acid Probes, Part I, Chapter 2" Overview of principles of hybridization and the strategy of nucleic acid probe assays", Elsevier. New York (1993); and Current Protocols in Molecular Biology, Chapter 2, Ausubel, et al., Eds., Greene Publishing and Wiley-Interscience, New York (1995).

In another aspect, the invention provides oligonucleotide probes for the generic detection of target RNA or DNA. The detection probes herein are selected to be "substantially" complementary to one of the strands of the double stranded nucleic acids generated by an amplification reaction of the invention. Preferably the probes are substantially complementary to the immobilizable, e.g. biotin labelled, antisense strands of the amplicons generated from the target RNA or DNA.

It is allowable for detection probes of the present invention to contain one or more mismatches to their target sequence. In general, sequences that exhibit at least 65%, more preferably at least 80% homology with the target oligonucleotide sequences are considered suitable for use in a method of the present invention.

Antibodies, both monoclonal and polyclonal, can also be used for detection purpose in the present invention, for example, in immunoassays in which they can be utilized in liquid phase or bound to a solid phase carrier. In addition, the monoclonal antibodies in these immunoassays can be detectably labeled in various ways. A variety of immunoassay formats may be used to select antibodies specifically reactive with a particular protein (or other analyte). For example, solid-phase ELISA immunoassays are routinely used to select monoclonal antibodies specifically immunoreactive with a protein. See Harlow and Lane, Antibodies, A Laboratory Manual, Cold Spring Harbor Publications, New York (1988), for a description of immunoassay formats and conditions that can be used to determine selective binding. Examples of types of immunoassays that can utilize antibodies of the invention are competitive and non-competitive immunoassays in either a direct or indirect format. Examples of such immunoassays are the radioimmunoassay (RIA) and the sandwich (immunometric) assay. Detection of the antigens using the antibodies of the invention can be done utilizing immunoassays that are run in either the forward, reverse, or simultaneous modes, including immunohistochemical assays on physiological samples. Those of skill in the art will know, or can readily discern, other immunoassay formats without undue experimentation.

Antibodies can be bound to many different carriers and used to detect the presence of the target molecules. Examples of well-known carriers include glass, polystyrene, polypropylene, polyethylene, dextran, nylon, amylases, natural and modified celluloses, polyacrylamides, agaroses and magnetite. The nature of the carrier can be either soluble or insoluble for purposes of the invention. Those skilled in the art will know of other suitable carriers for binding monoclonal antibodies, or will be able to ascertain such using routine experimentation.

The invention also provides a method for serologically diagnosing an EMCR-CoV virus infection of a mammal comprising determining in a sample of said mammal the presence of an antibody specifically directed against an EMCR-CoV virus or component thereof by reacting said sample with a proteinaceous molecule or fragment thereof or an antigen according to the invention

Methods and means provided herein are particularly useful in a diagnostic kit for diagnosing an EMCR-CoV virus infection, be it by virological or serological diagnosis. Such kits or assays may for example comprise a virus, a nucleic acid, a proteinaceous molecule or fragment thereof, an antigen and/or an antibody according to the invention.

Use of a virus, a nucleic acid, a proteinaceous molecule or fragment thereof, an antigen and/or an antibody according to the invention is also provided for the production of a pharmaceutical composition, for example for the treatment or prevention of EMCR-CoV virus infections and/or for the treatment or prevention of atypical pneumonia, in particular in humans. Preferably a peptide comprising part of the amino acid sequence of the spike protein as depicted in the relevant translations of Figure 1, is used for the preparation of a therapeutic or prophylactic peptide. Also preferably, a protein comprising the amino acid sequence of the spike protein as depicted in the relevant translations of Figure 1, is used for the preparation of a sub-unit vaccine. Furthermore, the nucleocapsid of Coronaviruses, as depicted in the translation of Figure 1, is known to be particularly useful for eliciting cell-mediated immunity against Coronaviruses and can be used for the preparation of a sub-unit vaccine.

Attenuation of the virus can be achieved by established methods developed for this purpose, including but not limited to the use of related viruses of other species, serial passages through laboratory animals or/and tissue/cell cultures, serial passages through cell cultures at temparutes below 37°C (cold-adaption), site directed mutagenesis of molecular clones and exchange of genes or gene fragments between related viruses.

A pharmaceutical composition comprising a virus, a nucleic acid, a proteinaceous molecule or fragment thereof, an antigen and/or an antibody according to the invention can for example be used in a method for the treatment or prevention of an EMCR-CoV virus infection and/or a respiratory illness comprising providing an individual with a pharmaceutical composition according to the invention. This is most useful when said individual comprises a human. Antibodies against EMCR-CoV virus proteins, especially against the spike protein of EMCR-CoV virus, preferably against the amino acid sequence as depicted in the translation in figure 1, are also useful for prophylactic or therapeutic purposes, as passive vaccines. It is known from other coronaviruses that the spike protein is a very strong antigen and that antibodies against spike protein can be used in prophylactic and therapeutic vaccination.

The invention also provides method to obtain an antiviral agent useful in the treatment of atypical pneumonia comprising establishing a cell culture or experimental animal comprising a virus according to the invention, treating said culture or animal with an candidate antiviral agent, and determining the effect of said agent on said virus or its infection of said culture or animal. An example of such an antiviral agent comprises an EMCR-CoV virus-neutralising antibody, or functional component thereof, as provided herein, but antiviral agents of other nature are obtained as well.

The invention also provides use of an antiviral agent according to the invention for the preparation of a pharmaceutical composition, in particular for the preparation of a pharmaceutical composition for the treatment of atypical pneumonia, especifically when caused by an EMCR-CoV virus infection, and provides a pharmaceutical composition comprising an antiviral agent according to the invention, useful in a method for the treatment or prevention of an EMCR-CoV virus infection or atypical pneumonia, said method comprising providing an individual with such a pharmaceutical composition.

The invention also comprises an animal model usable for testing of prophylactic and/or therapeutic methods and/or preparations. It is hypothesized that apes can be infected with the EMCR-CoV virus, thereby showing clinical symptoms, and more importantly, similar tissue morphology as found in humans suffering from atypical pneumonia caused by the EMCR-CoV virus. Subjecting apes to a prophylactic or therapeutic treatment either before or during infection with the virus will have a good and useful predictionary value for application of such a prophylaxis or therapy in human subjects.

The invention is further explained in the Examples without limiting it thereto.

### Figure legends

Fig. 1: Nucleotide sequences from parts of the EMCR-CoV virus. Also included are the putative amino acid sequences of polypeptides.
Fig. 2: Phylogenetic relationship for the nucleotide sequences of isolate EMCR-CoV with its closest relatives genetically. Phylogenetic trees were generated by maximum likelihood analyses using 100 bootstraps and 3 jumbles. The scale representing the number of nucleotide changes is shown for each tree. Figure 1a. Maximum likelihood tree of matrix gene nucleotide sequences. Numbers in trees represent bootstrap values. The scale bar roughly reflects 10 % nucleotide differences between related sequences. Figure 1b. Maximum likelihood tree of nucleocapsid gene nucleotide sequences. Numbers in trees represent bootstrap values. The scale bar roughly reflects 10 % nucleotide differences between related sequences.
Fig. 3: Similarity matrices indicating amino acid identity for the putative Replicase 1a, Replicase 1b, Replicase 1ab, Spike, Orf E, Matrix and Nucleocapsid proteins 3a-g, respectively), and for the putative Matrix protein and Nucleoprotein (3h and 3i resp.) between the EMCR-CoV virus and closely related coronaviruses. See text for abbreviations.
Figure 4 Alignments with various coronaviruses: 5'untranslated region genomic sequence (a); Putative orf 1a amino acid sequence (b); Putative orf 1b amino acid sequence (c); Putative orf 1ab amino acid sequence (d); Putative Spike amino acid sequence (e); Putative orf 4a amino acid sequence (f); Putative orf 4ab amino acid sequence (g); Putative orf E amino acid sequence (h); Putative Matrix amino acid sequence (i); Putative Nucleoprotein amino acid sequence (j); Putative 3'untranslated genomic sequence (k); See text for abbreviations.

### Examples

### Specimen collection

Virus was collected from an 8 month old patient suffering from pneumonia using nasal swabs.

### Virus isolation and culture

Throat swabs were dipped into a culture of tMK cells and passaged four times. Virus was then in Vero-118 cells. One litre of virus containing cell culture supernatant was harvested, and the virus was pelleted in an ultracentrifuge and the virus pellet was resuspended inlml PBS.

### RNA isolation

RNA was isolated from the supernatant of infected cell cultures or sucrose gradient fractions using a High Pure RNA Isolation kit according to instructions from the manufacturer (Roche Diagnostics, Almere, The Netherlands).

### Sequencing

Purified RNA was sent to BaseClear holding BV (Leiden, The Netherlands) for sequencing.

### Phylogenetic analyses

Nucleotide sequences were aligned using Clustal W running under BioEdit version 5.0.9. Maximum likelihood trees were created using the Seqboot and DNA-ML packages of Phylip 5.6 using 100 bootstraps and 3 jumbles. The consensus trees were calculated using the Consense package of phylip 5.6. These consensus trees were used as usertree in DNA-ML to recalculate the branch lengths from the original sequences.

The sequences of EMCR-CoV were compared with those of reference viruses representing each species in the four groups of coronaviruses. These were: human coronavirus 229E (229E), af304460; porcine epidemic diarrhea virus (PEDV) af353511; transmissible gastroenteritis virus (TGEV), aj271965; bovine coronavirus (BoCoV), af220295; murine hepatitis virus (MHV), af201929; avian infectious bronchitis virus (AIBV), m95169, Canine coronavirus (CaCoV), d13096; feline coronavirus (FeCoV), ay204704; porcine respiratory coronavirus (PRCoV), z24675; human coronavirus OC43 (OC43), m76373, 114643, m933990; porcine haemagglutinating encephalomyelitis virus (HEV), ay078417; rat coronavirus (RtCoV) af 207551) References for the viruses are the numbers of the NCBI catalog (http://www.ncbi.nlm.nih.gov/entrezn.

In general, coronaviruses, such as EMCR-CoV can be isolated and identified according to the following protocol:

### Specimen collection

In order to find virus isolates nasopharyngeal aspirates, throat and nasal swabs, broncheo alveolar lavages, serum and plasma samples, and stools preferably from mammals such as humans, carnivores (dogs, cats, mustellits, seals etc.), horses, ruminants (cattle, sheep, goats etc.), pigs, rabbits, birds (poultry, ostriches, etc) should be examined. From birds cloaca swabs and droppings can be examined as well. Sera should be collected for immunological assays, such as ELISA, molecular-based assays, such as RT-PCR and virus neutralisation assays.
Collected virus specimens may be diluted with 5 ml Dulbecco MEM medium (BioWhittaker, Walkersville, MD) and thoroughly mixed on a vortex mixer for one minute. The suspension is thus centrifuged for ten minutes at 840 x g. The sediment is spread on a multispot slide (Nutacon, Leimuiden, The Netherlands) for immunofluorescence techniques, and the supernatant is used for virus isolation.

### Virus isolation

For virus isolation Vero-118 cells or tMK cells (RIVM, Bilthoven, The Netherlands) were cultured in 24 well plates containing glass slides (Costar, Cambridge, UK), with the medium described below supplemented with 10% fetal bovine serum (BioWhittaker, Vervier, Belgium). Before inoculation the plates were washed with PBS and supplied with Eagle's MEM with Hanks' salt (ICN, Costa mesa, CA) supplemented with 0.52/liter gram NaHCO₃, 0.025 M Hepes (Biowhittaker), 2 mM L-glutamine (Biowhittaker), 200 units/liter penicilline, 200 µg/liter streptomycine (Biowhittaker), 1gram/liter lactalbumine (Sigma-Aldrich, Zwijndrecht, The Netherlands), 2.0 gram/liter D-glucose (Merck, Amsterdam, The Netherlands), 10 gram/liter peptone (Oxoid, Haarlem, The Netherlands) and 0.02% trypsine (Life Technologies, Bethesda, MD). The plates were inoculated with supernatant of the patient samples, 0,2 ml per well in triplicate, followed by centrifuging at 840x g for one hour. After inoculation the plates were incubated at 37 °C for 1-7 days and cultures were checked daily for CPE. Extensive CPE was generally observed within 5-10 and included detachment of cells from the monolayer..

### Virus culture

Sub-confluent monolayers of tMK cells or Vero clone 118 cells in media as described above were inoculated with supernatants of samples that displayed CPE or with samples taken from a patient.

### RNA isolation

RNA was isolated from the supernatant of infected cell cultures or sucrose gradient fractions using a High Pure RNA Isolation kit according to instructions from the manufacturer (Roche Diagnostics, Almere, The Netherlands). RNA can also be isolated following other procedures known in the field *(Current Protocols in Molecular Biology).*

### Sequence analysis

Sequence analyses were performed by BaseClear holding BV (Leiden, The Netherlands).

## Claims

1. An isolated essentially mammalian positive-sense single stranded RNA virus (EMCR-CoV) comprising the sequence of figure 1 or homologues thereof.

2. An isolated positive-sense single stranded RNA virus (EMCR-CoV) belonging to the Coronaviruses and identifiable as phylogenetically corresponding thereto by determining a nucleic acid sequence of said virus and testing it in phylogenetic tree analyses wherein maximum likelihood trees are generated using 100 bootstraps and 3 jumbles and finding it to be more closely phylogenetically corresponding to a virus isolate having the sequences as depicted in figure 1 than it is corresponding to a virus isolate of PEDV (porcine epidemic diarrhea virus), HCoV-229E (human coronavirus 229E), PRCoV (porcine respiratory coronavirus), TGEV (transmissible gastroenteritis virus), CaCoV (Canine coronavirus) and FeCoV (feline coronavirus).

3. A virus according to claim 1 or 2 wherein said nucleic acid sequence comprises an open reading frame (ORF) encoding a viral protein of said virus.

4. A virus according to claim 3 wherein said open reading frame is selected from th group of ORFs encoding the viral replicase, nuclear capsid protein, matrix protein and the spike protein.

5. A virus according to claim 1-4 isolatable from a human with atypical pneumonia

6. An isolated or recombinant nucleic acid or EMCR-CoV virus-specific functional fragment thereof obtainable from a virus according to anyone of claims 1 to 5.

7. A vector comprising a nucleic acid according to claim 6.

8. A host cell comprising a nucleic acid according to claim 6 or a vector according to claim 7.

9. An isolated or recombinant proteinaceous molecule or EMCR-CoV virus-specific functional fragment thereof encoded by a nucleic acid according to claim 6.

10. An antigen comprising a proteinaceous molecule or EMCR-CoV virus-specific functional fragment thereof according to claim 9.

11. An antibody specifically directed against an antigen according to claim 10.

12. A method for identifying a viral isolate as an EMCR-CoV virus comprising reacting said viral isolate or a component thereof with an antibody according to claim 11.

13. A method for identifying a viral isolate as an EMCR-CoV virus comprising reacting said viral isolate or a component thereof with a nucleic acid according to claim 6.

14. A method for virologically diagnosing an EMCR-CoV infection of a mammal comprising determining in a sample of said mammal the presence of a viral isolate or component thereof by reacting said sample with a nucleic acid according to claim 6 or an antibody according to claim 11.

15. A method for serologically diagnosing an EMCR-CoV infection of a mammal comprising determining in a sample of said mammal the presence of an antibody specifically directed against an EMCR-CoV virus or component thereof by reacting said sample with a proteinaceous molecule or fragment thereof according to claim 9 or an antigen according to claim 10.

16. A diagnostic kit for diagnosing an EMCR-CoV infection comprising a virus according to anyone of claims 1 to 5, a nucleic acid according to claim 6, a proteinaceous molecule or fragment thereof according to claim 9, an antigen according to claim 10 and/or an antibody according to claim 11.

17. Use of a virus according to any one claims 1 to 5, a nucleic acid according to claim 6, a vector according to claim 7, a host cell according to claim 8, a proteinaceous molecule or fragment thereof according to claim 9, an antigen according to claim 10, or an antibody according to claim 11 for the production of a pharmaceutical composition.

18. Use according to claim 17 for the production of a pharmaceutical composition for the treatment or prevention of an EMCR-CoV virus infection.

19. Use according to claim 17 or 18 for the production of a pharmaceutical composition for the treatment or prevention of atypical pneumonia.

20. A pharmaceutical composition comprising a virus according to any one of claims 1 to 5, a nucleic acid according to claim 6, a vector according to claim 7, a host cell according to claim 8, a proteinaceous molecule or fragment thereof according to claim 9, an antigen according to claim 10, or an antibody according to claim 11.

21. A method for the treatment or prevention of an EMCR-CoV virus infection comprising providing an individual with a pharmaceutical composition according to claim 20.

22. A method for the treatment or prevention of atypical pneumonia comprising providing an individual with a pharmaceutical composition according to claim 20.

23. A viral replicase encoded by an RNA sequence comprising the indicated sequences, or homologues thereof as depicted in figure 1.

24. A viral spike protein comprising the indicated amino acid sequence as depicted in figure 1, or a homologue thereof.

25. A viral nuclear capsid protein encoded by an RNA sequence comprising the indicated sequence as depicted in figure 1 or a homologue thereof.

26. A viral nsp 3 or envelope protein encoded by an RNA sequence comprising the indicated sequence as depicted in figure 1, or a homologue thereof.

27. A nucleic acid sequence which comprises one or more of the sequences coding for sepearte viral proteins as depicted in figure 1 or a nucleic acid sequence which can hybridise with any of these sequences under stringent conditions.
